# EUROPEAN PATENT SPECIFICATION

(11) **EP 1 299 395 B1**
(45) Date of publication and mention of the grant of the patent: **15.09.2004**
(21) Application number: 01948231.4
(22) Date of filing: 29.06.2001
(51) Int. Cl.: C07D 498/04, C07D 498/20, A61K 31/535, A61P 31/22

(54) **OXAZINOQUINOLONES USEFUL FOR THE TREATMENT OF VIRAL INFECTIONS**
OXAZINOCHINOLONE FÜR DIE BEHANDLUNG VIRALER INFEKTIONEN
OXAZINOQUINOLONES UTILES DANS LE TRAITEMENT DES INFECTIONS VIRALES

(30) Priority: 12.07.2000 US 217555 P; 17.01.2001 US 262211 P; 13.02.2001 US 268255 P
(43) Date of publication of application: 09.04.2003
(73) Proprietor: PHARMACIA & UPJOHN COMPANY, Kalamazoo, Michigan 49001 (US)
(72) Inventor: THAISRIVONGS, Suvit, Kalamazoo, MI 49002 (US); TURNER, Steven, R., Kalamazoo, MI 49004 (US); THORARENSEN, Atli, Portage, MI 49024 (US)
(74) Representative: Perry, Robert Edward
(86) International application number: PCT/US2001/016507
(87) International publication number: WO 2002/004462

(56) References cited:
- WO-A-00/40561
- WO-A-01/25239
- WO-A-99/40093
- US-A- 4 959 363
- US-A- 5 792 774

## Description

The present invention provides oxazinoquinolone and thioxazinoquinolone derivatives having a ring connecting position 4 (N-4) and position 11 (C-11), and more specifically, provides compounds of formula (I) described herein below. These compounds are useful as antiviral agents, in particular, as agents against viruses of the herpes family.

The herpesviruses comprise a large family of double stranded DNA viruses. They are also a source of the most common viral illnesses in man. Eight of the herpes viruses, herpes simplex virus types 1 and 2 (HSV-1 and HSV-2), varicella zoster virus (VZV), human cytomegalovirus (HCMV), epstein-Barr virus (EBV), and human herpes viruses 6, 7, and 8 (HHV-6, HHV-7, and (HHV-8), have been shown to infect humans.

HSV-1 and HSV-2 cause herpetic lesions on the lips and genitals, respectively. They also occasionally cause infections of the eye and encephalitis. HCMV causes birth defects in infants and a variety of diseases in immunocompromised patients such as retinitis, pneumonia, and gastrointestinal disease. VZV is the causitive agent of chicken pox and shingles. EBV causes infectious mononucleosis. It can also cause lymphomas in immunocompromised patients and has been associated with Burkitt's lymphoma, nasopharyngeal carcinoma, and Hodgkins disease. HHV-6 is the causitive agent of roseola and may be associated with multiple sclerosis and chronic fatigue syndrome. HHV-7 disease association is unclear, but it may be involved in some cases of roseola. HHV-8 has been associated with Karposi's sarcoma, body cavity based lymphomas, and multiple myeloma.

Due to the unique position of the para-substitutent on the N-phenylmethyl of formula I described herein below, compounds of the present invention demonstrate unexpected activity against the above reference herpesviral infections, particularly, human cytomegaloviral infection.

US Patent 5,792,774 discloses oxazino 1,4-dihydro-4-oxoquinolines useful for the treatment of a large number of diseases modulated by tissue necrosis factor (TNF) or phosphodiesterase IV, includng cytomegalovirus (CMV) infections.

US Patent 4,847,375 discloses 1,8-bridged 4-quinoline-3-carboxylic acids useful as antibacterial agents.

US Patent 5,583,135 discloses heterotricyclic derivatives having a strong immunomodulating activity, anti-inflammatory activity and anti-cancer activity.

The abstract of Japanese Patent JP 10324631-A discloses IgE antibody production inhibitor comprise a pyrido(1,2,3-de1,4-benzoxazine or a pyrido (1,2,3,-de)-1,4-benzothiazine derivative.

PCT patent application, PCT/US00/21985 discloses oxazinoquinolones useful for the treatment of viral infections.

### SUMMARY OF THE INVENTION

The present, invention provides a compound of formula I, 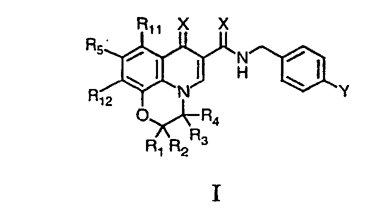 or a pharmaceutically acceptable salt, racemate, solvate, tautomer, optical isomer or prodrug derivative thereof wherein:
each X is independently O or S;
Y is Cl, F, Br, CN or NO₂;
R₁, R₂, R₃ and R₄ are independently
   a) hydrogen,
   b) N₃,
   c) CN,
   d) fluoro,
   e) trifluoromethyl,
   f) aryl,
   g) het,
   h) C₁₋₈ alkyl, optionally substituted with R₆ or OR₇, or
   i) R₁ and R₂ or R₃ and R₄ together with the carbon to which they are attached form C₃₋₈cycloalkyl or het;
R₅ is C₁₋₈alkyl, which may be partially unsaturated and optionally substituted with one to three N₃, halo, CN, R₆ or R₇;
R₆ is
   a) aryl,
   b) het,
   c) SOᵢR₈,
   d) OR₈,
   e) C(=O)OR₈,
   f) C(=O)R₈, or
   g) NR₈R₉;
R₇ is
   a) P(=O)(OR₁₀)₂,
   b) CO(CH₂)ⱼCON(CH₃)(CH₂)ₖSO₃⁻M⁺,
   c) an amino acid,
   d) C(=O)C₁₋₆alkyl, optionally substituted by NR¹⁰R¹⁰, or
   e) CO(CH₂)ₙCO₂H;
R₈ and R₉ are independently
   a) hydrogen,
   b) C₃₋₈cycloalkyl,
   c) aryl,
   d) het, or
   e) C₁₋₈alkyl which is further optionally substituted with one or more aryl, het, halo, CN, CO₂R₁₀, SOᵢR₁₀, OR₁₀, NR₁₀R₁₀, CF₃, or C₃₋₈cycloalkyl;
R₁₀ is
   a) H or
   b) C₁₋₇alkyl, optionally substituted with OH or OC₁₋₄alkyl;
R₁₁ and R₁₂ are independently
   a) hydrogen,
   b) halo,
   c) NO₂,
   d) CN,
   e) R₆,
   f) SOᵢNR₈R₉, or
   g) C₁₋₈alkyl, which may be partially unsaturated and optionally substituted with one to three N₃, halo, CN, R₆ or OR₇;
aryl is
   a phenyl radical, optionally fused with a saturated or unsaturated carbocyclic or heterocyclic ring; at each occurrence, aryl may be substituted with one or more halo, CN, CO₂R₁₀, SOᵢR₁₀, OR₁₀, NR₁₀R₁₀, CF₃, C₃₋₈cycloalkyl, or C₁₋₄alkyl wherein C₁₋₄alkyl is optionally substituted with OR₁₀;
het is
   a four- (4), five- (5), six- (6), or seven- (7) membered saturated or unsaturated heterocyclic ring having 1, 2, or 3 heteroatoms selected from the group consisting of O, S, and NW, wherein W is hydrogen, C₁₋₄alkyl, C(=O)OC₁₋₄alkyl or absent, wherein het is optionally fused with a benzene ring, a carbcyclic or a heterocyclic ring; at each occurrence, het may be substituted with one or more halo, CN, CO₂R₁₀, SOᵢR₁₀, OR₁₀, NR₁₀R₁₀, C₁₋₄alkyl, CF₃, C₃₋₈cycloalkyl, oxo or oxine;
at each occurrence, a cycloalkyl group may be substituted with C₁₋₄alkyl, OR¹⁰, oxo, oxine, or a spiro fused het;
i is 0, 1 or 2;
j is 1, 2, 3, 4, 5, or 6;
k is 1, 2, 3, 4, 5, or 6;
n is 1, 2, 3, 4, 5, or 6;
M is sodium, potassium, or lithium; and
with the following provisos:
a) at least one of R₁, R₂, R₃ and R₄ is other than hydrogen;
b) where R₁, R₂, R₃ and R₄ are independently C₁₋₈ alkyl, at least one of the alkyl groups is substituted with R₆ or OR₇.

The present invention further provides a pharmaceutical composition comprising a compound of formula I, or a pharmaceutically acceptable salt thereof, and a pharmaceutically acceptable carrier (the composition preferably comprises an effective antiviral amount of the compound or salt).

The present invention further provides a method of treating or preventing a herpesviral infection, comprising administering to a mammal in need of such treatment, a compound of formula (I) or a pharmaceutically acceptable salt thereof.

The present invention further provides a method of treating or preventing a herpesviral infection comprising administering orally, parenterally, topically, rectally, nasally, sublingually or transdermally an effective amount of a compound of claim 1.

The present invention further provides a compound of formula (1) or a pharmaceutically acceptable salt thereof for use in medical treatment.

The present invention further provides the use of a compound of formula (I) or a pharmaceutically acceptable salt thereof to prepare a medicament for treating or preventing a herpesviral infection in a mammal.

The present invention further provides a method for inhibiting a viral DNA polymerase, comprising contacting (*in vitro* or *in vivo*) the polymerase with an effective inhibitory amount of a compound of formula I, or a pharmaceutically acceptable salt thereof.

The invention also provides novel intermediates and processes disclosed herein that are useful for preparing compounds of formula I.

### DETAILED DESCRIPTION OF THE INVENTION

The following definitions are used, unless otherwise described. Halo denotes fluoro, chloro, bromo, or iodo. Alkyl, alkoxy, etc. denote both straight and branched groups; but reference to an individual radical such as "propyl" embraces only the straight chain radical, a branched chain isomer such as "isopropyl" being specifically referred to. When alkyl can be partially unsaturated, the alkyl chain may comprise one or more (e.g. 1, 2, 3, or 4) double or triple bonds in the chain.

The carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, (C₁₋₃)alkyl refers to alkyl of one to three carbon atoms, inclusive, or methyl, ethyl, propyl and isopropyl, straight and branched forms thereof.

Aryl is a phenyl radical, optionally fused with a saturated or unsaturated carbocyclic or heterocyclic ring. At each occurrence, aryl may be substituted with one or more halo, CN, CO₂R₁₀, SOᵢR₁₀, OR₁₀, NR₁₀R₁₀, CF₃, C₃₋₈cycloalkyl, or C₁₋₄alkyl wherein C₁₋₄alkyl is optionally substituted with OR₁₀.

Het is a four- (4), five- (5), six- (6), or seven- (7) membered saturated or unsaturated heterocyclic ring having 1, 2, or 3 heteroatoms selected from the group consisting of O, S, and NW, wherein W is hydrogen, C₁₋₄alkyl, C(=O)OC₁₋₄alkyl or absent, wherein het is optionally fused with a benzene ring, a carbcyclic or a heterocyclic ring. At each occurrence, het may be substituted with one or more halo, CN, CO₂R₁₀, SOᵢR₁₀, OR₁₀, NR₁₀R₁₀, C₁₋₄alkyl, CF₃, C₃₋₈cycloalkyl, oxo or oxine;

The term "het" also includes piperidinyl, morpholinyl, thiomorpholinyl, piperazinyl, N-C₁₋₄alky substituted piperazinyl such as 4-methyl piperazinyl, pyrrolidinyl, pyridyl, imidazolyl, N-C₁₋₄alky substituted imidazol such as 1-methyl-1H-imidazol, azetidyl, tetrahydrofuranyl, dioxolanyl, imidazolidinyl, oxathiolanyl, oxazolidinyl, pyran, thiopyran, tetrahydropyran or tetrahydrothiopyran, thiophene, furan, pyrazoline, pyrimidine, 2-pyridyl, 3-pyridyl, 4-pyridyl, 2-pyrimidinyl, 4-pyrimidinyl, 5-pyrimidinyl, 3-pyridazinyl, 4-pyridazinyl, 3-pyrazinyl, 2-quinolyl, 3-quinolyl, 1-isoquinolyl, 3-isoquinolyl, 4-isoquinolyl, 2-quinazolinyl, 4-quinazolinyl, 2-quinoxalinyl, I-phthalazinyl, 4-oxo-2-imidazolyl, 2-imidazolyl, 4-imidazolyl, 3-isoxazolyl, 4-isoxazolyl, 5-isoxazolyl, 3-pyrazolyl, 4-pyrazolyl, 5-pyrazolyl, 2-oxazolyl, 4-oxazolyl, 4-oxo-2-oxazolyl, 5-oxazolyl, 4,5,-dihydrooxazole, 1,2,3-oxathiole, 1,2,3-oxadiazole, 1,2,4-oxadiazole, 1,2,5-oxadiazole, 1,3,4-oxadiazole, 2-thiazolyl, 4-thiazolyl, 5-thiazolyl, 3-isothiazole, 4-isothiazole, 5-isothiazole, 2-indolyl, 3-indolyl, 3-indazolyl, 2-benzoxazolyl, 2-benzothiazolyl, 2-benzimidazolyl, 2-benzofuranyl, 3-benzofuranyl, benzoisothiazole, benzisoxazole, 2-furanyl, 3-furanyl, 2-thienyl, 3-thienyl, 2-pyrrolyl, 3-pyrrolyl, 3-isopyrrolyl, 4-isopyrrolyl, 5-isopyrrolyl, 1,2,3,-oxathiazole-1-oxide, 1,2,4-oxadiazol-3-yl, 1,2,4-oxadiazol-5-yl, 5-oxo-1,2,4-oxadiazol-3-yl, 1,2,4-thiadiazol-3-yl, 1,2,4-thiadiazol-5-yl, 3-oxo-1,2,4-thiadiazol-5-yl, 1,3,4-thiadiazol-5-yl, 2-oxo-1,3,4-thiadiazol-5-yl, 1,2,4-triazol-3-yl, 1,2,4-triazol-5-yl, 1,2,3,4-tetrazol-5-yl, 5-oxazolyl, 1-pyrrolyl, 1-pyrazolyl, 1,2,3-triazol-1-yl, 1,2,4-triazol-1-yl, 1-tetrazolyl, 1-indolyl, 1-indazolyl, 2-isoindolyl, 7-oxo-2-isoindolyl,1-purinyl, 3-isothiazolyl, 4-isothiazolyl and 5-isothiazolyl, 1,3,4,-oxadiazole, 4-oxo-2-thiazolinyl, or 5-methyl-1,3,4-thiadiazol-2-yl, thiazoledione, 1,2,3,4-thiatriazole, 1,2,4-dithiazolone. Each of these moieties may be substituted as appropriate.

"Amino acid," includes a residue of natural amino acid (e.g. Ala, Arg, Asn, Asp, Cys, Glu, Gln, Gly, His, Hyl, Hyp, Ile, Leu, Lys, Met, Phe, Pro, Ser, Thr, Trp, Tyr, and Val) in D or L form, as well as unnatural amino acids (e.g. phosphoserine, phosphothreonine, phosphotyrosine, hydroxyproline, gamma-carboxyglutamate; hippuric acid, octahydroindole-2-carboxylic acid, statine, 1,2,3,4,-tetrahydroisoquinoline-3-carboxylic acid, penicillamine, omithine, citruline, -methyl-alanine, para-benzoylphenylalanine, phenylglycine, propargylglycine, sarcosine, and tert-butylglycine). An amino acid can conveniently be linked to the remainder of a compound of formula I through the carboxy terminus, the amino terminus, or through any other convenient point of attachment, such as, for example, through the sulfur of cysteine. In particular, an amino acid can conveniently be linked to the remainder of a compound of formula I through the carboxy terminus.

Mammal denotes human and animals, specifically including food animals and companion animals.

It will be appreciated by those skilled in the art that compounds of the invention have one or more achiral center and be isolated in optically active and racemic forms.
Some compounds may exhibit polymorphism. It is to be understood that the present invention encompasses any racemic, optically-active, polymorphic, tautomeric, or stereoisomeric form, or mixture thereof, of a compound of the invention, which possesses the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine antiviral activity using the standard tests described herein, or using other similar tests which are well known in the art.

The compounds of the present invention are generally named according to the IUPAC or CAS nomenclature system. Other nomenclature systems may also be used. Abbreviations which are well known to one of ordinary skill in the art may be used (e.g. "Ph" for phenyl, 'Me" for methyl, "Et" for ethyl, "h" for hour or hours and "rt" for room temperature).

Specific and preferred values listed below for radicals, substituents, and ranges, are for illustration only; they do not exclude other defined values or other values within defined ranges for the radicals and substituents.

Specifically, the term "C₁₋₈alkyl, " or "C₁₋₄alkyl" refers to an alkyl group having one to eight or one to four carbon atoms such as, for example, methyl, ethyl, propyl, butyl, pentyl, hexyl, heptyl, octyl, and their isomeric forms thereof.

Specifically, a 5- or 6-membered heterocyclic ring includes piperidinyl, morpholinyl, thiomorpholinyl, piperazinyl, N-C₁₋₄alky substituted piperazinyl such as 4-methyl piperazinyl, or pyrrolidinyl.

Specifically, a 5- or 6-membered heterocyclic ring includes pyridyl, imidazolyl, N-C₁₋₄alky substituted imidazol such as 1-methyl-1H-imidazol.

Specifically, R₅ is C₁₋₈alkyl substituted with OR₇ or het.

Specifically, R₅ is C₁₋₄alkyl substituted with OH.

Specifically, R₅ is C₁₋₄alkyl substituted with het.

Specifically, het is morpholinyl or thiomorpholinyl.

Specifically, R₅ is 4-morpholinylmethyl.

Specifically, R₅ is C₁₋₈alkyl, optionally substituted with OR₉.

Specifically, R₅ is C₁₋₈alkyl which is partially unsaturated and optionally substituted with OR₉.

Specifically, R₅ is propynyl substituted with OH.

Specifically, R₅ is 3-hydroxypropyl.

Specifically, R₃ and R₄ are independently hydrogen.

Specifically, R₁ and R₂ are independently hydrogen, fluoro, or C₁₋₈ alkyl substituted with R₆ or OR₇.

Specifically, R₁ and R₂ are independently hydrogen, fluoro, C₁₋₈ alkyl substituted with R₆ or OR₇; aryl, het, or R₁ and R₂ together with the carbon to which they are attached form a six-(6) membered cycloalkyl or a het; wherein R₆ is het, SOᵢR₈, OR₈ or NR₈R₉; wherein R₇ is P(=O)(OR₁₀)₂, CO(CH₂)ₙCON(CH₃)(CH₂)ₙSO₃⁻M⁺, or C(=O)C₁₋₆alkyl, wherein R₈ and R₉ are independently hydrogen, aryl, het, or C₁₋₈alkyl which is further optionally substituted with one or more aryl, het, halo, CO₂R₁₀, SOᵢR₁₀, or OR₁₀; wherein R₁₀ is H or C₁₋₄alkyl, optionally substituted with OH.

Specifically, R₁ and R₂ are indepandetly H, C₁₋₄alkylsubstituted with OR₈ wherein R₈ is H, or C₁₋₄alkylsubstituted with OR₁₀.

Specifically, R₁ is H; R₂ is aryl wherein aryl is optinoally substituted with one or two halo, CN, OR₁₀, or C₁₋₄alkylsubstituted with OR₁₀.

Specifically, R₁ is H; R₂ is aryl wherein aryl is fused with a heterocyclic ring.

Specifically, R₂ is 1,3-benzodioxolyl or 1,4-benxodioxinyl.

Specifically, R₁ is H; R₂ is het.

Specifically, het is a five- (5) or six- (6) membered saturated or unsaturated heterocyclic ring having 1, 2, or 3 heteroatoms selected from the group consisting of O, S, and NW, wherein W is hydrogen, C₁₋₄alkyl, C(=O)OC₁₋₄alkyl or absent, wherein het may be substituted with one or more halo, C₁₋₄alkyl, CF₃, oxo or oxine.

Specifically, het is pyridinyl.

Specifically, het is a five- (5) membered heterocyclic ring.

Specifically, R₁ and R₂ together with the carbon to which they are attached form a het, wherein het is a five- (5) or six- (6) membered heterocyclic ring having 1, 2, or 3 heteroatoms selected from the group consisting of O, S, and NW, wherein W is hydrogen, C₁₋₄alkyl, or C(=O)OC₁₋₄alkyl, wherein het may be substituted with one or more halo, OR₁₀, C₁₋₄alkyl, CF₃, oxo or oxine; more specifically, het is a (6) membered heterocyclic ring; even more specifically, het is pyran, piperdine, or thiopyran.

Specifically, R₁ and R₂ together with the carbon to which they are attached form a six- (6) membered cycloalkyl; more specifically, cycloalkyl is optionally substituted with oxo, or OR₁₀.

Specifically, R₆ is het, SOᵢR₈, OR₈ or NR₈R₉.

Specifically, R₇ is P=O)(OH)₂, (P=O)(C₁₋₄alkoxy)₂, C(=O)C₁₋₆alkyl, or CO(CH₂)ₙCON(CH₃)(CH₂)ₙSO₃⁻M⁺.

Specifically, R₈ and R₉ are independently hydrogen, aryl, het, or C₁₋₈alkyl which is further optionally substituted with one or more aryl, het, halo, CO₂R₁₀, SOᵢR₁₀, or OR₁₀;

Specifically, R₁₀ is H or C₁₋₄alkyl, optionally substituted with OH.

Specifically, R₂ is hydrogen, and R₁ is C₁₋₈ alkyl substituted with R₆ or OR₇; where R₆ is het, SR₈, OR₈ or NR₈R₉; wherein R₇ is (P=O)(OCH₃)₂, CO(CH₂)ₙCON(CH₃)(CH₂)ₙSO₃⁻M⁺, or C(=O)CH₃, R₈ and R₉ are independently hydrogen, het, or C₁₋₈alkyl, which is optionally substituted with one or two het, CO₂R₁₀, SOR₁₀, or OR₁₀; Rio is H or C₁₋₄alkyl, optionally substituted with OH.

Specifically, R₁₁ is H, halo, or C₁₋₄alkyl optionally substituted with one to three halo.

Specifically, R₁₂ is H, SOᵢR₈, OR₈, C(=O)OR₈, C(=O)R₈, NR₈R₉; or C₁₋₈alkyl, which may be partially unsaturated and optionally substituted with one to three N₃, halo, CN, or R₆.

Specifically, R₁₁ and R₁₂ are hydrogen.

Specifically, het is piperidinyl, morpholinyl, thiomorpholinyl, piperazinyl, N-C₁₋₄alky substituted piperazinyl, pyrrolidinyl, pyridyl, imidazolyl, or N-C₁₋₄alky substituted imidazol.

Specifically, R₁, R₂, R₃ and R₄ are independently hydrogen, hydroxymethyl, morpholinylmethyl, (pyridinylmethyl)aminomethyl, (dimethylamino)methyl, (hydroxyethyl)sulfanylmethyl, (1-methyl-1H-imidazol-2-yl)sulfanylmethyl,-CH₂OCO(CH₂)₆CON(CH₃)(CH₂)₂SO₃⁻M⁺, -CH₂OC(=O)CH₃, (4-methyl-1-piperazinyl)methyl, 1-pyrrolidinylmethyl, (2,3-dihydroxypropyl)aminomethyl, (2-hydroxyethyl)aminomethyl, 1-piperidinylmethyl, bis(2-hydroxyethyl)aminomethyl, 1H-imidazol-1-ylmethyl, (methylsulfanyl)methyl, (tert-butylsulfanyl)methyl, methylsulfanyl acetate, (2,3-dihydroxypropyl)sulfanylmethyl, phenyl, or fluoro. or CH₂OP(=O)(OCH₃)₂.

Specifically, R₁, R₂, R₃ and R₄ are independently hydrogen, hydroxymethyl, morpholinylmethyl, (2-pyridinylmethyl)aminomethyl, (3-pyridinylmethyl)aminomethyl, (dimethylamino)methyl, (2-hydroxyethyl)sulfanylmethyl, (1-methyl-1H-imidazol-2-yl)sulfanylmethyl, -CH₂OCO(CH₂)₆CON(CH₃)(CH₂)₂SO₃⁻M⁺, -CH₂OC(=O)CH₃, or CH₂OP(=O)(OCH₃)₂.

Specifically, R₁ and R₂ are independently hydrogen, R₃ and R₄ are independently fluoro, phenyl, or C₁₋₈ alkyl substituted with het or OH.

Specifically, R₁ is hydrogen and R₂ is het.

Specifically, R₁ is hydrogen and R₂ is 2-pyridinyl, 3-pyridinyl, or 4-pyridinyl.

Specifically, R₃ and R₄ are independently fluoro or hydroxymethyl.

Specifically, R₃ is hydrogen and R₄ is phenyl, morpholinylmethyl, or hydroxymethyl.

Specifically, R₃ is morpholinylmethyl.

Specifically, a compound of formula I is the following structure I-A;

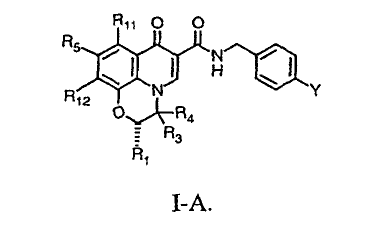

Specifically, a compound of formula I is the following structure I-B;

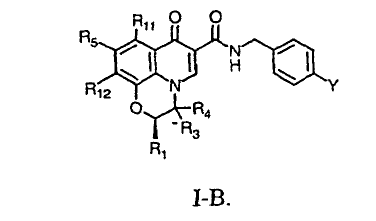

Specifically, a compound of formula I is the following structure I-C;

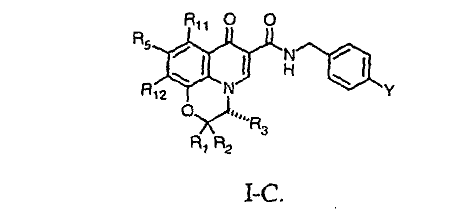

Specifically, a compound of formula I is the following structure I-D;

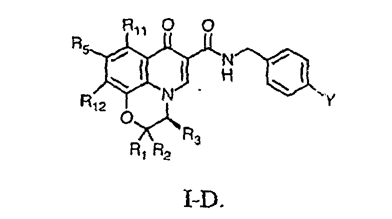

Specifically, a compound of formula I is the following structure I-E;

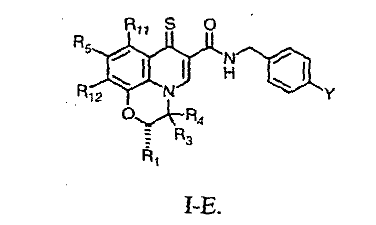

Specifically, a compound of formula I is the following structure I-F;

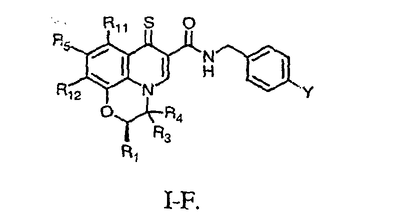

Specifically, a compound of formula I is the following structure I-G;

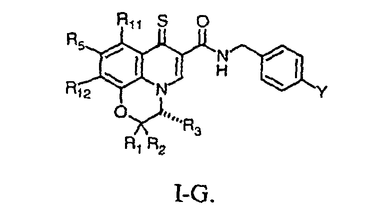

Specifically, a compound of formula I is the following structure I-H;

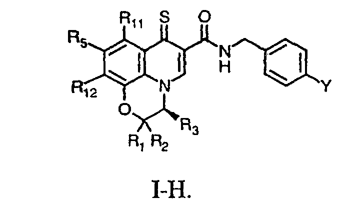

Specifically, a compound of formula I is the following structure I-I;

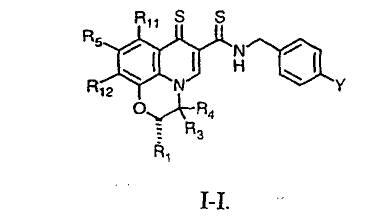

Specifically, a compound of formula I is the following structure I-J;

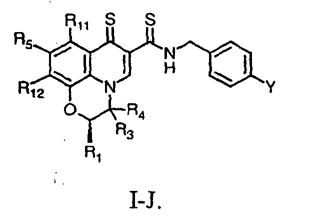

Specifically, a compound of formula I is the following structure I-K;

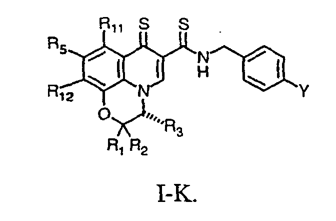

Specifically, a compound of formula I is the following structure I-L;

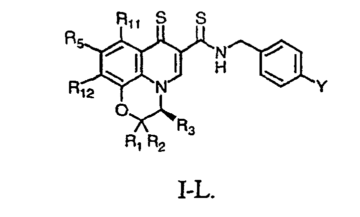

Specifically, a compound of formula I is the following structure I-M;

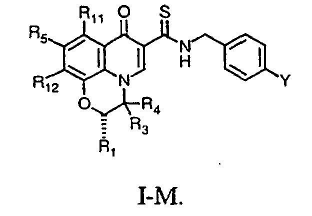

Specifically, a compound of formula I is the following structure I-N;

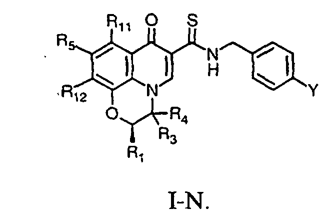

Specifically, a compound of formula I is the following structure I-O;

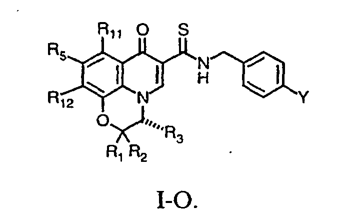

Specifically, a compound of formula I is the following structure I-P;

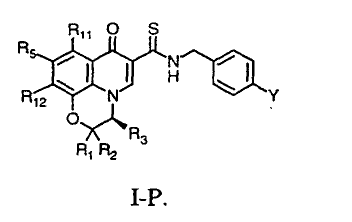

More specifically, Y is Cl in the formulas I-A to I-P.
Examples of the compounds of the present invention are:
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxamide,
c) N-(4-Chlorobenzyl)-9-(morpholin-4-ylmethyl)-7-oxo-2-pyridin-3-yl-2,3-dihydro-7H-[ 1,4]oxazino[2,3,4-ij]quinoline-6-carboxamide,
d) N-(4-Chlorobenzyl)-9-(morpholin-4-ylmethyl)-7-oxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-oxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-oxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-oxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]quinoline-6-carboxamide,
k) N-(4-Chlorobenzyl)-9-(morpholin-4-ylmethyl)-7-oxo-2-{[(3-pyridinylmethyl)amino]methyl}-2,3-dihydro-7H-[1,4]oxazino[2,3,4-ij]quinoline-6-carboxamide,
l) [6- {[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-oxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-oxo-2,3-dihydro-7H-[1,4]oxazino[2,3,4-ij]quinoline-6-carboxamide,
o) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-oxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-oxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-oxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-oxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-oxo-2,3-dihydro-7H-[1,4]oxazino[2,3,4-ij]quinoline-6-carboxamide,
v) N-(4-Chlorobenzyl)-9-(morpholin-4-ylmethyl)-7-oxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-oxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-oxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-oxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
bb) N-(4-Chlorobenzyl)-9-(morpholin-4-ylmethyl)-7-oxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-oxo-2,3-dihydro-7H-[1,4]oxazino[2,3,4-ij]quinoline-6-carboxamide,
dd) N-[(4-Chlorophenyl)methyl]-9'-(4-morphoIinylmethyl)-4,7'-dioxospiro[cyclohexane-1,2'(3'*H*)-[7*H*]pyrido[1,2,3-*de*][1,4]benzoxazine]-6'-carboxaxnide,
ee) N-[(4-Chlorophenyl)methyl]-4-hydroxy-9'-(4-morpholinylmethyl)-7'-oxospiro[cyclohexane-1,2'(3'*H*)-[7*H*]pyrido[1,2,3-*de*] [1,4]benzoxazine]-6'-carboxamide,
ff) N-(4-Chlorobenzyl)-3,9-bis(morpholin-4-ylmethyl)-7-oxo-2,3-dihydro-7H-[1,4]oxazino[2,3,4-ij]quinoline-6-carboxamide,
gg) N-(4-Chlorobenzyl)-9-(morpholin-4-ylmethyl)-7-oxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-oxo-2,3-dihydro-7H-[1,4]oxazino[2,3,4-ij]quinoline-6-carboxamide,
ii) N-(4-Chlorobenzyl)-2-[(methylsulfanyl)methyl]-9-(morpholin-4-ylmethyl)-7-oxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-oxo-2,3-dihydro-7H-[1,4]oxazino[2,3,4-ij]quinoline-6-carboxamide,
kk) N-(4-Chlorobenzyl)-2-[(4-methyl-1-piperazinyl)methyl]-9-(morpholin-4-ylmethyl)-7-oxo-2,3-dihydro-7H-[1,4]oxazino[2,3,4-ij]quinoline-6-carboxamide,
11) Methyl ({[6-{[(4-chlorobenzyl)amino]carbonyl}-9-(morpholin-4-ylmethyl)-7-oxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-oxo-2-(1-pyrrolidinylmethyl)-2,3-dihydro-7H-[1,4]oxazino[2,3,4-ij]quinoline-6-carboxamide,
nn) N-(4-Chlorobenzyl)-2-{[(2,3-dihydroxypropyl)sulfanyl]methyl}-9-(morpholin-4-ylmethyl)-7-oxo-2,3-dihydro-7H-[1,4]oxazino[2,3,4-ij]quinoline-6-carboxamide,
oo) N-(4-Chlorobenzyl)-2- {[(2,3-dihydroxypropyl)amino]methyl}-9-(morpholin-4-ylmethyl)-7-oxo-2,3-dihydro-7H-[1,4]oxazino[2,3,4-ij]quinoline-6-carboxamide,
pp) N-(4-Chlorobenzyl)-2-{[(2-hydroxyethyl)amino]methyl}-9-(morpholin-4-ylmethyl)-7-oxo-2,3-dihydro-7H-[1,4]oxazino[2,3,4-ij]quinoline-6-carboxamide,
qq) N-(4-Chlorobenzyl)-9-(morpholin-4-ylmethyl)-7-oxo-2-(1-piperidinylmethyl)-2,3-dihydro-7H-[1,4]oxazino[2,3,4-ij]quinoline-6-carboxamide,
rr) 2- {[bis(2-Hydroxyethyl)amino]methyl}-N-(4-chlorobenzyl)-9-(morpholin-4-ylmethyl)-7-oxo-2,3-dihydro-7H-[1,4]oxazino[2,3,4-ij]quinoline-6-carboxamide,
ss) N-(4-Chlorobenzyl)-9-(morpholin-4-ylmethyl)-7-oxo-2-{[(2-pyridinylmethyl)amino]methyl}-2,3-dihydro-7H-[1,4]oxazino[2,3,4-ij]quinoline-6-carboxamide,
tt) 2-[(8-{[6-{[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6- {[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-oxo-2-{[(4-pyridinylmethyl)amino]methyl}-2,3-dihydro-7H-[1,4]oxazino[2,3,4-ij]quinoline-6-carboxamide,
ww) N-(4-Chlorobenzyl)-2-(1H-imidazol-1-ylmethyl)-9-(morpholin-4-ylmethyl)-7-oxo-2,3-dihydro-7H-[1,4]oxazino[2,3,4-ij]quinoline-6-carboxamide,
xx) N-(4-Chlorobenzyl)-2-{[(4-chlorobenzyl)amino]methyl)}-9-(morpholin-4-ylmethyl)-7-oxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-oxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-oxo-2,3-dihydro-7H-[1,4]oxazino[2,3,4-ij]quinoline-b-carboxamide,
aaa) N-(4-Chlorobenzyl)-2-{3-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-oxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-oxospiro[4*H*-pyran-4,2'(3'*H*)-[7*H*]pyrido[1,2,3-*de*][1,4]benzoxazine]-6'-carboxamide,
eee) 1,1-Dimethylethyl 6-[[[(4-chlorophenyl)methyl]amino]carbonyl]-9'-(4-morpholinylmethyl)-7'-oxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-oxospiro[piperidine-4,2'(3'*H*)-[7*H*]pyrido[1,2,3-*de*][1,4]benzoxazine]-6'-carboxamide,
ggg) N-(4-Chlorobenzyl)-2,2-bis(hydroxymethyl)-9-(morpholin-4-ylmethyl)-7-oxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-oxospiro[7*H*-pyrido[1,2,3-*de*]-1,4-benzoxazine-2(3*H*),4'-[4H]thiopyran]-6-carboxamide,
iii) N-(4-Chlorobenzyl)-9-(morpholin-4-ylmethyl)-7-oxo-3-phenyl-2,3-dihydro-7H-[1,4]oxazino[2,3,4-ij]quinoline-6-carboxamide,
jjj) N-(4-Chlorobenzyl)-3,3-bis(hydroxymethyl)-9-(3-hydroxy-1-propynyl)-7-oxo-2,3-dihydro-7H-[1,4]oxazino[2,3,4-ij]quinoline-6-carboxamide,
kkk) N-(4-Chlorobenzyl)-3,3-bis(hydroxymethyl)-9-(3-hydroxypropyl)-7-oxo-2,3-dihydro-7H-[1,4]oxazino[2,3,4-ij]quinoline-6-carboxamide,
lll) N-(4-Chlorobenzyl)-2-[2-(methoxymethoxy)phenyl]-9-(morpholin-4-ylmethyl)-7-oxo-2,3-dihydro-7H-[1,4]oxazino[2,3,4-ij]quinoline-6-carboxamide,
mmm) N-(4-Chlorobenzyl)-2-{4-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
nnn) 2-[2,3-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
ooo) N-[(4-Chlorophenyl)methyl]-1-methyl-9'-(4-morpholinylmethyl)-7'-oxospiro[piperidine-4,2'(3'*H*)-[7*H*]pyrido[1,2,3-*de*][1,4]benzoxazine]-6'-carboxamide, or
ppp) N-[(4-Chlorophenyl)methyl]-9"-(4-morpholinylmethyl)dispiro[1,3-dioxolane-2,1'-cyclohexane-4',2"(3"*H*)-[7*H*]pyrido[1,2,3-*de*][1,4]benzoxazine]-6"-carboxamide.

Additional examples of the compounds of the present invention are:
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxamide,
b) N-(4-Chlorobenzyl)-2-(*R* or *S*)- (hydroxymethyl)-9-(morpholin-4-ylmethyl)-7-thioxo-2,3-dihydro-7H-[1,4]oxazino[2,3,4-ij]quinoline-6-carboxamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-thioxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-thioxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-thioxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-thioxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]quinoline-6-carboxamide,
k) N-(4-Chlorobenzyl)-9-(morpholin-4-ylmethyl)-7-thioxo-2-{[(3-pyridinylmethyl)amino]methyl}-2,3-dihydro-7H-[1,4]oxazino[2,3,4-ij]quinoline-6-carboxamide,
l) [6- {[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-thioxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-thioxo-2,3-dihydro-7H-[1,4]oxazino[2,3,4-ij]quinoline-6-carboxamide,
o) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-thioxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-thioxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-thioxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-thioxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-thioxo-2,3-dihydro-7H-[1,4]oxazino[2,3,4-ij]quinoline-6-carboxamide,
v) N-(4-Chlorobenzyl)-9-(morpholin-4-ylmethyl)-7-thioxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-thioxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
bb) N-(4-Chlorobenzyl)-9-(morpholin-4-ylmethyl)-7-thioxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-thioxo-2,3-dihydro-7H-[1,4]oxazino[2,3,4-ij]quinoline-6-carboxamide,
dd) N-[(4-Chlorophenyl)methyl]-9'-(4-morpholinylmethyl)-4-oxo-7'-thioxospiro[cyclohexane-1,2'(3'*H*)-[7*H*]pyrido[1,2,3-*de*] [1,4]benzoxazine]-6'-carboxamide,
ee) N-[(4-Chlorophenyl)methyl]-4-hydroxy-9'-(4-morpholinylmethyl)-7'-thioxospiro[cyclohexane-1,2'(3'*H*)-[7*H*]pyrido[1,2,3-*de*] [1,4]benzoxazine]-6'-carboxamide,
ff) N-(4-Chlorobenzyl)-3,9-bis(morpholin-4-ylmethyl)-7-thioxo-2,3-dihydro-7H-[1,4]oxazino[2,3,4-ij]quinoline-6-carboxamide,
gg) N-(4-Chlorobenzyl)-9-(morpholin-4-ylmethyl)-7-thioxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-thioxo-2,3-dihydro-7H-[ 1,4]oxazino[2,3,4-ij]quinoline-6-carboxamide,
ii) N-(4-Chlorobenzyl)-2-[(methylsulfanyl)methyl]-9-(morpholin-4-ylmethyl)-7-thioxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-thioxo-2,3-dihydro-7H-[1,4]oxazino[2,3,4-ij]quinoline-6-carboxamide,
kk) N-(4-Chlorobenzyl)-2-[(4-methyl-1-piperazinyl)methyl]-9-(morpholin-4-ylmethyl)-7-thioxo-2,3-dihydro-7H-[1,4]oxazino[2,3,4-ij]quinoline-6-carboxamide,
ll) Methyl ({[6-{[(4-chlorobenzyl)amino]carbonyl}-9-(morpholin-4-ylmethyl)-7-thioxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-thioxo-2-(1-pyrrolidinylmethyl)-2,3-dihydro-7H-[1,4]oxazino[2,3,4-ij]quinoline-6-carboxamide,
nn) N-(4-Chlorobenzyl)-2- {[(2,3-dihydroxypropyl)sulfanyl]methyl}-9-(morpholin-4-ylmethyl)-7-thioxo-2,3-dihydro-7H-[1,4]oxazino[2,3,4-ij]quinoline-6-carboxamide,
oo) N-(4-Chlorobenzyl)-2-{[(2,3-dihydroxypropyl)amino]methyl}-9-(morpholin-4-ylmethyl)-7-thioxo-2,3-dihydro-7H-[1,4]oxazino[2,3,4-ij]quinoline-6-carboxamide,
pp) N-(4-Chlorobenzyl)-2-{[(2-hydroxyethyl)amino]methyl]-9-(morpholin-4-ylmethyl)-7-thioxo-2,3-dihydro-7H-[1,4]oxazino[2,3,4-ij]quinoline-6-carboxamide,
qq) N-(4-Chlorobenzyl)-9-(morpholin-4-ylmethyl)-7-thioxo-2-(1-piperidinylmethyl)-2,3-dihydro-7H-[1,4]oxazino[2,3,4-ij]quinoline-6-carboxamide,
rr) 2- {[bis(2-Hydroxyethyl)amino]methyl}-N-(4-chlorobenzyl)-9-(morpholin-4-ylmethyl)-7-thioxo-2,3-dihydro-7H-[1,4]oxazino[2,3,4-ij]quinoline-6-carboxamide,
ss) N-(4-Chlorobenzyl)-9-(morpholin-4-ylmethyl)-7-thioxo-2-{[(2-pyridinylmethyl)amino]methyl}-2,3-dihydro-7H-[1,4]oxazino[2,3,4-ij]quinoline-6-carboxamide,
tt) 2-[(8-{[6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6- {[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-thioxo-2-{[(4-pyridinylmethyl)amino]methyl}-2,3-dihydro-7H-[1,4]oxazino[2,3,4-ij]quinoline-6-carboxamide,
) ww) N-(4-Chlorobenzyl)-2-(1H-imidazol-1-ylmethyl)-9-(morpholin-4-ylmethyl)-7-thioxo-2,3-dihydro-7H-[1,4]oxazino[2,3,4-ij]quinoline-6-carboxamide,
xx) N-(4-Chlorobenzyl)-2-{[(4-chlorobenzyl)amino]methyl}-9-(morpholin-4-ylmethyl)-7-thioxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-thioxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-thioxo-2,3-dihydro-7H-[1,4]oxazino[2,3,4-ij]quinoline-6-carboxamide,
aaa) N-(4-Chlorobenzyl)-2-{3-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-thioxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-thioxospiro[4*H*-pyran-4,2'(3'*H*)-[7*H*]pyrido[1,2,3-*de*][1,4]benzoxazine]-6'-carboxamide,
eee) 1,1-Dimethylethyl 6-[[[(4-chlorophenyl)methyl]amino]carbonyl]-9'-(4-morpholinylmethyl)-7'-thioxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-thioxospiro[piperidine-4,2'(3'*H*)-[7*H*]pyrido[1,2,3-*de*][1,4]benzoxazine]-6'-carboxamide,
ggg) N-(4-Chlorobenzyl)-2,2-bis(hydroxymethyl)-9-(morpholin-4-ylmethyl)-7-thioxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-thioxospiro[7*H*-pyrido[1,2,3-*de*]-1,4-benzoxazine-2(3*H*),4'-[4H]thiopyran]-6-carboxamide,
iii) N-(4-Chlorobenzyl)-9-(morpholin-4-ylmethyl)-7-thioxo-3-phenyl-2,3-dihydro-7H-[1,4]oxazino[2,3,4-ij]quinoline-6-carboxamide,
jjj) N-(4-Chlorobenzyl)-3,3-bis(hydroxymethyl)-9-(3-hydroxy-1-propynyl)-7-thioxo-2,3-dihydro-7H-[1,4]oxazino[2,3,4-ij]quinoline-6-carboxamide,
kkk) N-(4-Chlorobenzyl)-3,3-bis(hydroxymethyl)-9-(3-hydroxypropyl)-7-thioxo-2,3-dihydro-7H-[1,4]oxazino[2,3,4-ij]quinoline-6-carboxamide,
lll) N-(4-Chlorobenzyl)-2-[2-(methoxymethoxy)phenyl]-9-(morpholin-4-ylmethyl)-7-thioxo-2,3-dihydro-7H-[1,4]oxazino[2,3,4-ij]quinoline-6-carboxamide,
mmm) N-(4-Chlorobenzyl)-2-{4-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
nnn) 2-[2,3-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
ooo) N-[(4-Chlorophenyl)methyl]-1-methyl-9'-(4-morpholinylmethyl)-7'-thioxospiro[piperidine-4,2'(3'*H*)-[7*H*]pyrido[1,2,3-*de*][1,4]benzoxazine]-6'-carboxamide, or a pharmaceutically acceptable salt.

Additional examples of the compounds of the present invention are:
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxthioamide,
b) N-(4-Chlorobenzyl)-2-(*R* or *S*)-(hydroxymethyl)-9-(morpholin-4-ylmethyl)-7-thioxo-2,3-dihydro-7H-[1,4]oxazino[2,3,4-ij]quinoline-6-carboxthioamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxthioamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxthioamide,
e) N-(4-Chlorobenzyl)-9-(morpholin-4-ylmethyl)-7-thioxo-2-pyridin-2-yl-2,3-dihydro-7H-[ 1,4]oxazino[2,3,4-ij]quinoline-6-carboxthioamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxthioamide,
g) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxthioamide, or a pharmaceutically acceptable salt.

Additional examples of the compounds of the present invention are:
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxthioamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxthioamide,
c) N-(4-Chlorobenzyl)-9-(morpholin-4-ylmethyl)-7-oxo-2-pyridin-3-yl-2,3-dihydro-7H-[ 1,4]oxazino[2,3,4-ij]quinoline-6-carboxthioamide,
d) N-(4-Chlorobenzyl)-9-(morpholin-4-ylmethyl)-7-oxo-2-pyridin-4-yl-2,3-dihydro-7H-[1,4]oxazino[2,3,4-ij]quinoline-6-carboxthioamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxthioamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxthioamide,
g) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxthioamide, or a pharmaceutically acceptable salt.

The following Charts A-N describe the preparation of the compounds of formula I of the present invention. All of the starting materials are prepared by procedures described in these charts, by procedures well known to one of ordinary skill in organic chemistry or can be obtained commercially. All of the final compounds of the present invention are prepared by procedures described in these charts or by procedures analogous thereto, which would be well known to one of ordinary skill in organic chemistry. All of the variables used in the charts are as defined below or as in the formula I above.

As shown in CHART **A**, acid **A-1**, which is 3-hydroxy-4-nitrobenzoic acid, is reacted with thionyl chloride to give the corresponding acid chloride, which is treated with morpholine to provide amide **A-2**. Reduction of the nitro group using hydrogen gas and palladium catalyst gives aminophenol **A-3**, which is alkylated using sodium hydride and diethyl chloromalonate in dimethylformamide to give lactam **A-4**. Simultaneous reduction of the three carbonyl groups using borane-methyl sulfide in tetrahydrofuran affords amine **A-5**. Treatment of the amine with diethyl ethoxymethylenemalonate provides **A-6,** which is derivatized to acetate **A-7** using acetic anhydride and pyridine. Cyclization to **A-8** is effected by phosphorus pentoxide in methanesulfonic acid. Treatment of **A-8** with p-chlorobenzylamine at 150°C cleaves the acetate and aminolyzes the ester, giving amide **A-9**. The alcohol is reacted with methanesulfonyl chloride and 2,4,6-collidine to give the intermediate mesylate **A-10**, which is subsequently reacted with a nucleophile of formula -R₈R₉NH, R₈SH or R₈OH (wherein R₈ and R₉ are defined previously), or anions thereof, to provide compounds of formula **A-11** wherein R₆ is SOᵢR₈, OR₈, NR₈R₉, aryl, or het.

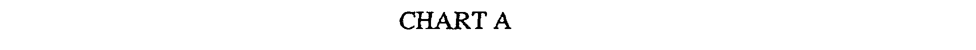

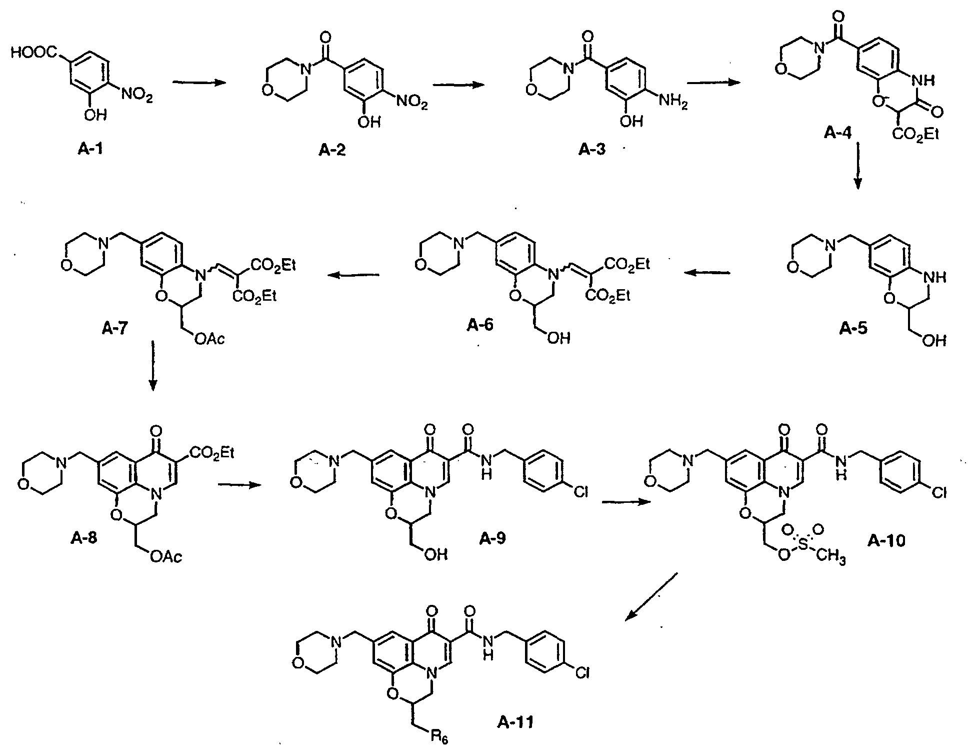

As shown in CHART B, compound **A-8,** which is a racemic mixture, is resolved into its component enantiomers **B-1a** and **B-1b** by preparative chiral HPLC. The individual isomers are carried forth separately through Formulae **B-2** and **B-3,** according to the protocol described for Chart A.

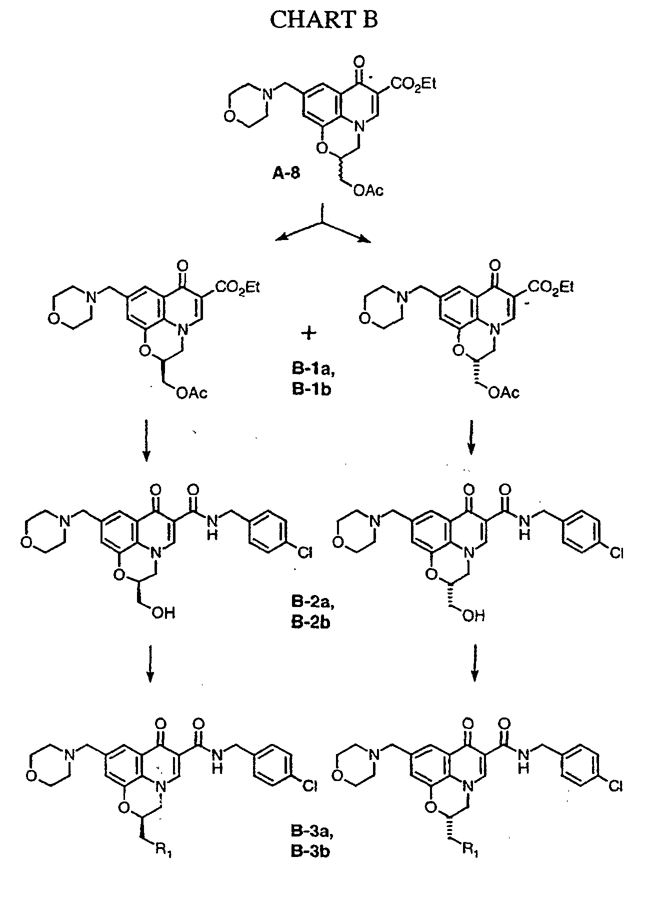

As shown in CHART **C**, alcohol of Formula **A-9** is reacted with acetic anhydride to provide acetate **C-1**. Alternately, the alcohol can be coupled with a salt of suleptanic acid, which is 8-[Methyl(2-sulfoethyl)amino]-8-oxooctanoic acid, using diisopropylcarbodiimide to afford **C-2.** Alternately, **C-9** can be reacted sequentially with phosphorus oxychoride and then methanol to provide phosphate **C-3**.

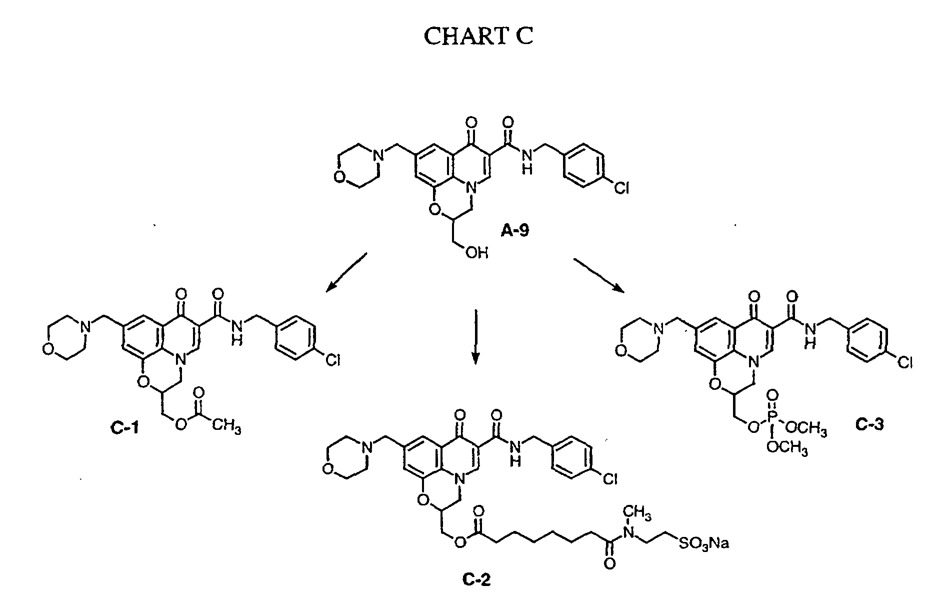

As shown in CHART **D,** aminophenol of Formula **A-3** is treated with methyl α-bromophenylacetate and potassium carbonate in refluxing acetone to afford **D-1**. Reduction of the carbonyl groups using lithium aluminum hydride provides amine **D-2,** which is reacted with diethyl ethoxymethylenemalonate to furnish **D-3**. Cyclization to **D-4** is effected with polyphosphoric acid, and aminolysis of the ester using *p*-chlorobenzylamine at 150 °C affords compound **D-5**.

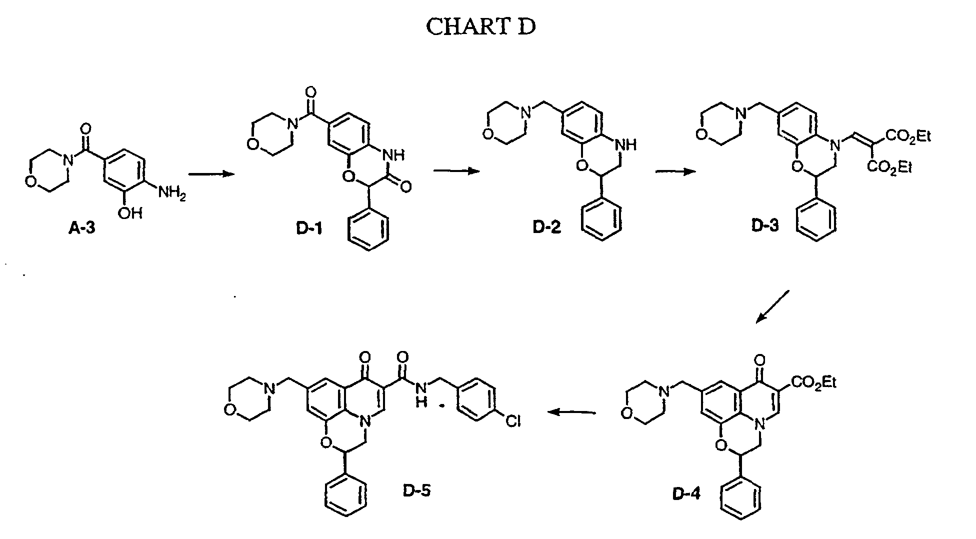

As shown in CHART **E**, aminophenol of Formula **A-3** is treated with ethyl bromodifluoroacetate and sodium hydride in dimethylformamide to afford **E-1**. Reduction of the carbonyl groups using lithium aluminum hydride provides amine **E-2**, which is reacted with diethyl ethoxymethylenemalonate to furnish **E-3**. Cyclization to **E-4** is effected with polyphosphoric acid, and aminolysis of the ester using *p*-chlorobenzylamine at 150 °C affords compound **E-5**.

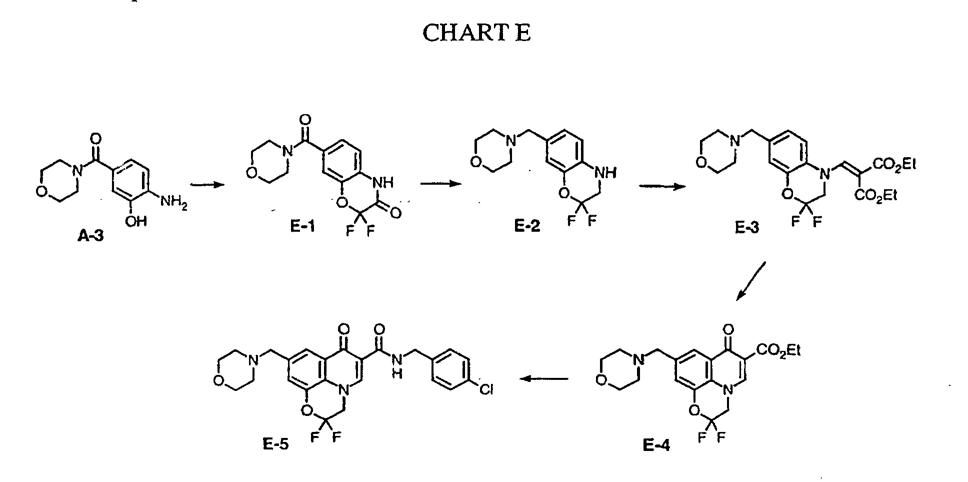

As shown in CHART F, nitrophenol of Formula **A-2** is alkylated using phenacyl bromide and tetrabutylammonium bisulfate as a phase transfer catalyst, giving compound **F-1**. Reduction of the nitro group with tin(II) chloride is followed by spontaneous cyclization to enamine **F-2.** Reduction of the enamine with sodium borohydride furnishes **F-3**, which is further reduced using lithium aluminum hydride to afford amine **F-4**. Reaction with diethyl ethoxymethylenemalonate provides **F-5**, which is cyclized using polyphosphoric acid to give **F-6.** Aminolysis of the ester using *p*-chlorobenzylamine at 150 °C affords compound **F-7**.

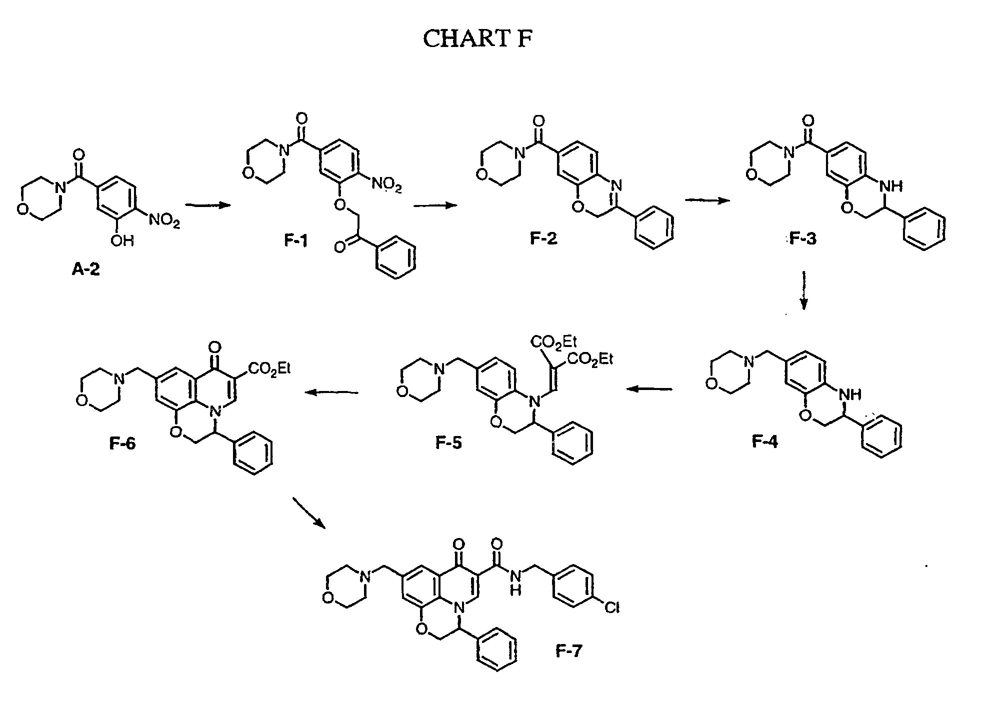

As shown in CHART **G,** nitro ketone of Formula **A-2** is reacted with epichlorohydrin in the presence of sodium hydroxide to give epoxide **G-1**. Reaction of the epoxide with morpholine in refluxing methanol provides amino alcohol **G-2.** The alcohol is oxidized with dimethyl sulfoxide and trifluoroacetic anhydride, and the resulting ketone is immediately hydrogenated using hydrogen gas and Raney nickel catalyst to give **G-3.** Reduction of the remaining keto group is accomplished with lithium aluminum hydride, affording amine **G-4**. Treatment of the amine with diethyl ethoxymethylenemalonate, followed by polyphosphoric acid mediated cyclization of the intermediate enamine, provides compound **G-5**. Aminolysis of the ester using *p*-chlorobenzylamine at 150 °C provides compound **G-6**.

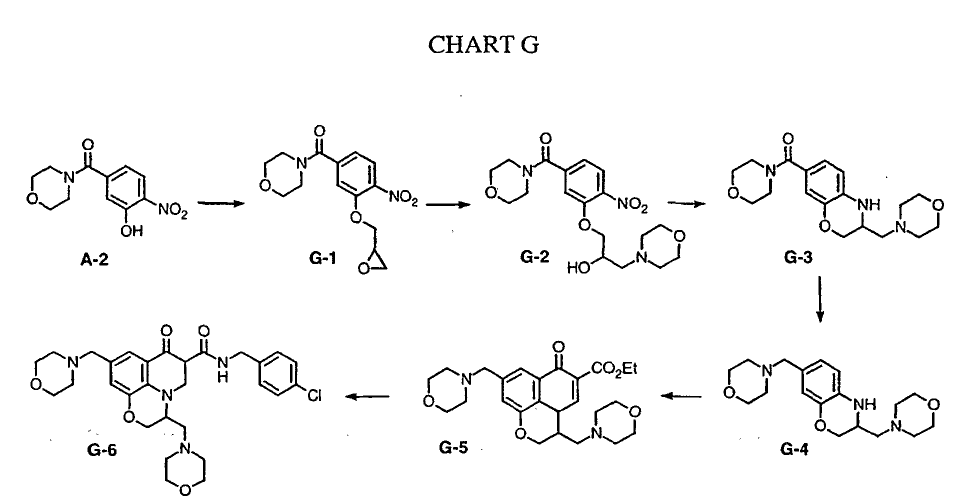

As shown in CHART H, iodination of commercially available 2,3-difluorobenzoic acid **H-1** using *N*-iodosuccinimide in trifluoromethanesulfonic acid provides trihalo-acid **H-2.** The acid is reacted with carbonyldiimidazole to form the intermediate acyl imidazolide, which is treated with ethyl trimethylsilyl malonate and 1,8-diazabicyclo[5.4.0]undec-7-ene to provide β-ketoester **H-3.** Compound **H-3** is reacted with triethyl orthoformate and acetic acid, followed by tris(hydroxymethyl) methylamine to afford triol **H-4.** Cyclization is effected using potassium carbonate in DMF, giving tricycle **H-5,** and aminolysis using *p*-chlorobenzylamine at 150 °C affords **H-6.** The hydroxyl groups are masked as *tert*-butyldimethylsilyl ethers by reaction of the diol with *tert*-butyldimethylsilyl chloride and imidazole in dimethylformamide, giving **H-7.** Coupling of the iodide with propargyl alcohol using catalytic bis(triphenylphosphine)palladium (II) dichloride and copper (I) iodide provides **H-8.** Removal of the silyl ethers using hydrochloric acid in ethanol affords compound **H-9.** Alternately, the triple bond in **H-8** is reduced with hydrogen gas and catalytic platinum on carbon to afford **H-10**, which is desilylated using hydrochloric acid in ethanol to provide compound **H-11**.

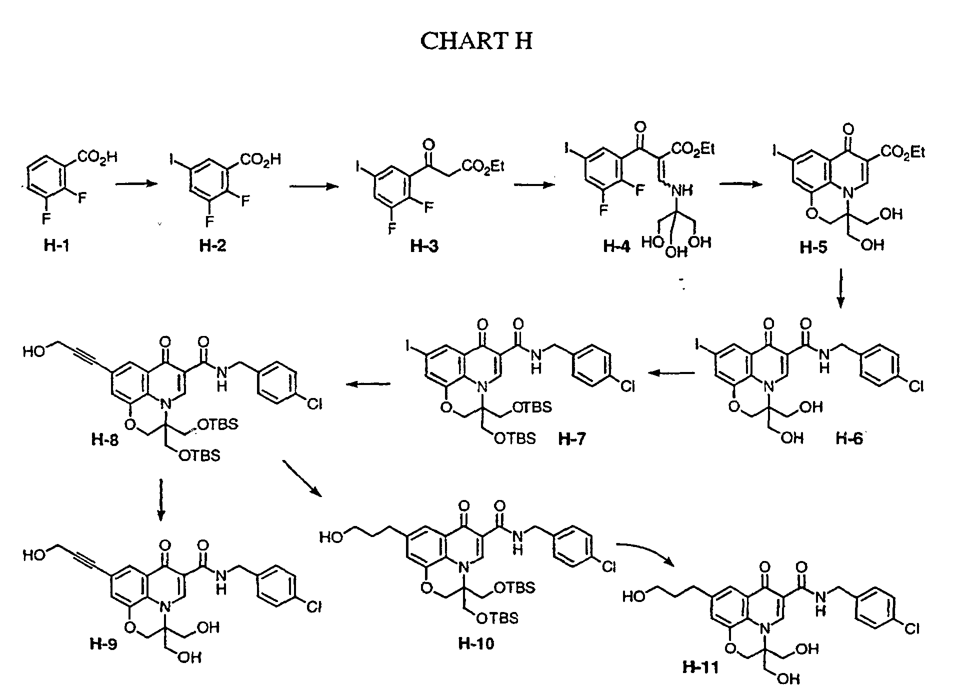

As shown in CHART I, treatment of β-ketoester **H-3** (Chart H) with triethyl orthoformate in refluxing acetic anhydride forms intermediate enol **I-1,** which then reacts with amino alcohols **I-7** to provide enamines **I-2.** In Chart I, substitutent T refers to either a het or an aryl group. In some cases, groups T in Chart I may contain functional groups which are protected by suitable protective groups, such as methoxymethyl or *tert*-butyldimethylsilyl during the course of the synthesis. The protective groups are installed and then removed at the end of the synthesis using methods known to those skilled the art. Cyclization of the enamines to tricycles **I-3** is accomplished using cesium carbonate in DMF. Aminolysis of the ethyl ester using neat *p*-chlorobenzylamine affords amides **I-4**. The iodide is converted to the aldehyde **I-5** using carbon monoxide and tri-*n*-butylstannane under palladium catalysis. Finally, reductive amination of the aldehyde provides **I-6**.

Amino alcohols **I-7** required for the sequence are prepared in two steps from the corresponding aldehydes **I-8**, by treatment of the aldehyde with trimethylsilyl cyanide and subsequent reduction of the TMS cyanohydrin with lithium aluminum hydride.

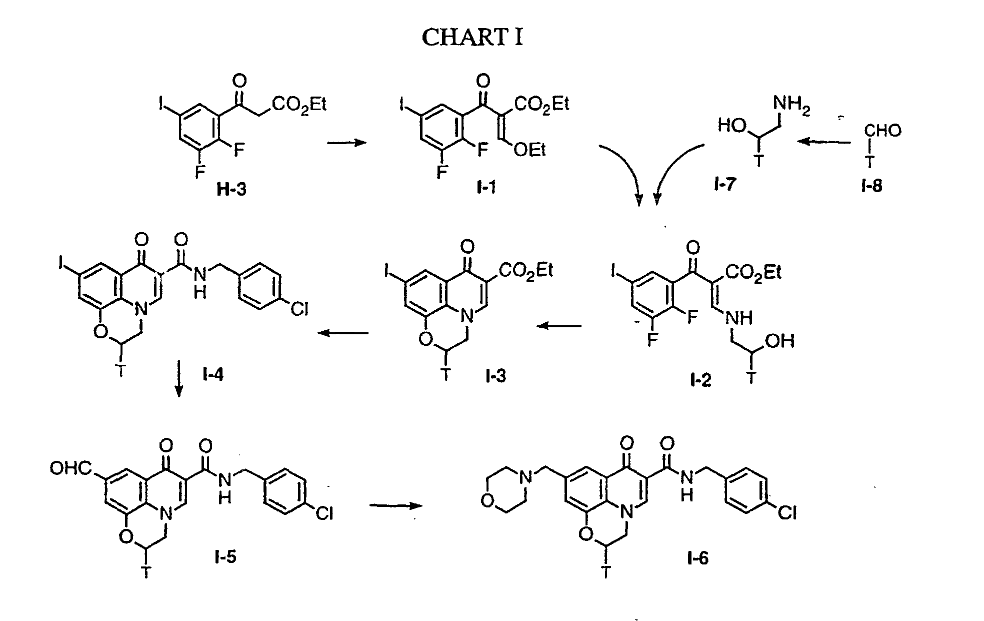

CHART J illustrates a method for converting oxazinoquinolones to thioxazinoquinolones.

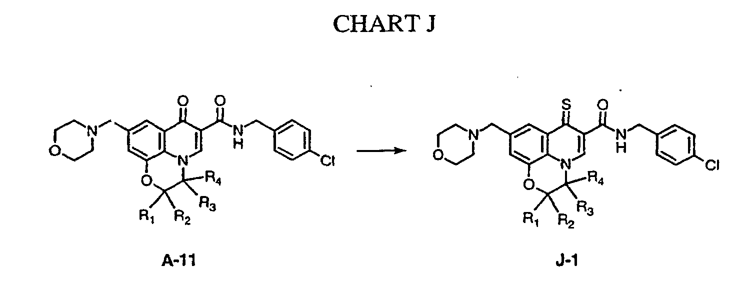

A morpholino substituted 7-thioxo-2,3-dihydro-7H-[1,4]oxazino[2,3,4-ij]quinoline-6-carboxamide, **A-11**, in a suitable solvent such as toluene and dichloroethane can be reacted with Lawesson's reagent in the presence of KHMDS. The mixture is then reacted at a suitable temperature range providing the desired thioketone **J-1**.

CHART K provides an alternative method to convert oxazinoquinolones to thioxazinoquinolones when they are substituted with one or more alkyl hydroxy groups. For example, reacting compound **H-11** in DMF with TIPSCl in the presence of imidiazole provides **K-1** (See Wuts, P. G. *Protecting Groups in Organic Chemistry* **1999**, 123). Next, ketone **K-1** is reacted with Lawesson's reagent in the presence of KHMDS in refluxing toluene and dichloroethane provides the thioketone **K-2**. The protected alcohol **K-2** is then treated with Bu₄N⁺F⁻ in THF affording the hydroxyl compound **K-3** (In Wuts, P. G. *Protecting Groups in Organic Chemistry* **1999**, 124).

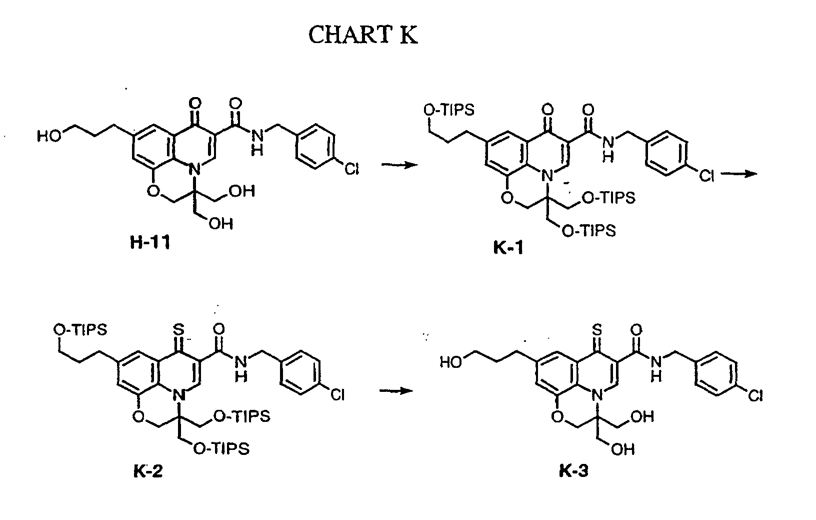

CHART L illustrates a method for converting oxazinoquinolones to thioxazinoquinolone thioamides. A morpholino substituted 7-thioxo-2,3-dihydro-7H-[1,4]oxazino[2,3,4-ij]quinoline-6-carboxamide, **A-11,** in a suitable solvent such as toluene and dichloroethane can be reacted with excess Lawesson's reagent. The mixture is then reacted at a suitable temperature range providing the desired thioketone **L-1**.

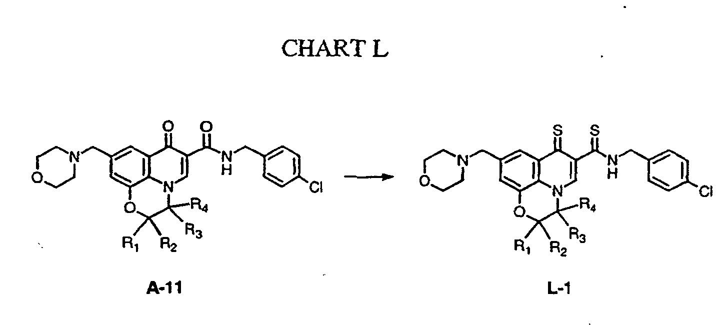

As shown in CHART M, commercially available 2-fluoro-4-iodoaniline of Formula **M-1** is reacted with diethyl ethoxymethylenemalnoate under thermal conditions to give the substituted 4-hydroxyquinoline of Formula **M-2**. Aminolysis of the ethyl ester using *p*-chlorobenzylamine under thermal conditions provides amide **M-3**. Palladium catalyzed formylation of the iodide affords aldehyde **M-4**, which is treated with morpholine and sodium triacetoxyborohydride to furnish amine **M-5**. Compound **M-5** is reacted with various epoxides **M-7**, using cesium carbonate or calcium ethoxide as base, to provide compounds **M-8** or **M-9.** Where not commercially available, epoxides **M-7** may be prepared by treatment of the corresponding carbonyl compounds **M-6** with trimethylsulfonium methylsulfate and sodium hydroxide. Epoxides **M-7** may also be prepared by other routes familiar to those skilled in the art; for example, by epoxidation of the corresponding olefins. If epoxides **M-7** are prepared in optically enriched form, either by synthesis or resolution, the resulting products **M-8** and **M-9** will also be obtained in optically enriched form. This method for the preparation of the compounds of the present invention is further illustrated in the Preparation **46** and Examples **36-54.**

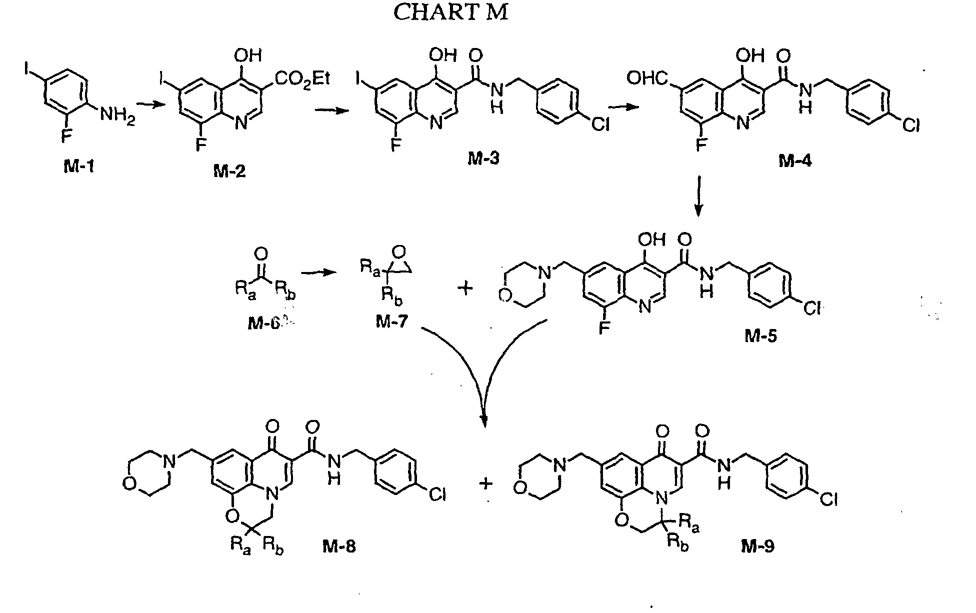

In CHART N, hydroxyalkylbenzyl alcohols **N-1** are protected with an appropriate blocking group, such as methoxymethyl or tert-butyldimethylsilyl, giving monoprotected alcohols **N-2**. Oxidation of the remaining hydroxyl group provides aldehydes **N-3.** Where hydroxyalkylbenzaldehydes **N-4** are commercially available, they may be converted to compounds **N-3** using similar protection chemistry. Addition of trimethylsilyl cyanide provides amino alcohols **N-5,** which are carried forth through the sequence **N-6, N-7, N-8, N-9,** and **N-10** as described for Chart I. Finally, the protecting group is removed from compounds **N-10** using standard conditions familiar to those proficient in the art, for example, by treatment with acid in ethanol, to give compounds **N-11.**

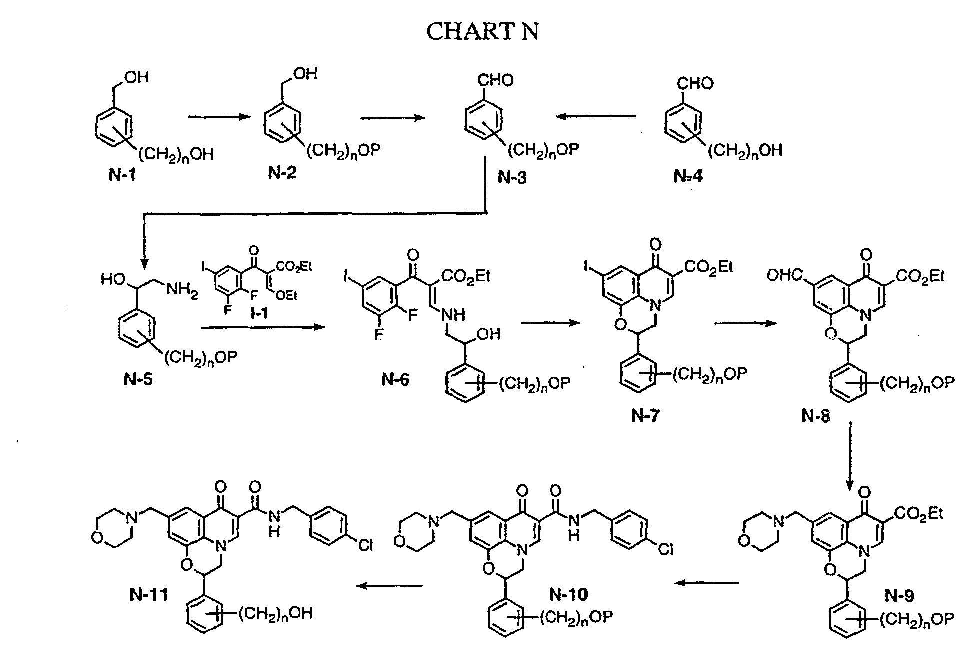

It will be apparent to those skilled in the art that the described synthetic procedures are merely representative in nature and alternative synthetic processes are known to one of ordinary skill in organic chemistry.

The inventive compounds may be used in their native form or as salts. In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, etoglutarate, and glycerophosphate. Suitable inorganic salts may also be formed, including hydrochloride, hydrobromide, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

"Pharmaceutically acceptable salts" refers to those salts which possess the biological effectiveness and properties of the parent compound and which are not biologically or otherwise undesirable.

Compounds of the present invention can conveniently be administered in a pharmaceutical composition containing the compound in combination with a suitable excipient, the composition being useful in combating viral infections. Pharmaceutical compositions containing a compound appropriate for antiviral use are prepared by methods and contain excipients which are well known in the art. A generally recognized compendium of such methods and ingredients is Remington's Pharmaceutical Sciences by E.W. Martin (Mark Publ. Co., 15th Ed., 1975).

The compounds and compositions of the present invention can be administered parenterally (for example, by intravenous, intraperitoneal or intramuscular injection), topically, orally, or rectally, depending on whether the preparation is used to treat internal or external viral infections.

For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, com starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The compounds or compositions can also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

Pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of formula I to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949.

The compound is conveniently administered in unit dosage form; for example, containing 5 to 1000 mg, conveniently 10 to 750 mg, most conveniently, 50 to 500 mg of active ingredient per unit dosage form. The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

For internal infections, the compositions can be administered orally or parenterally at dose levels, calculated as the free base, of about 0.1 to 300 mg/kg, preferably 1.0 to 30 mg/kg of mammal body weight, and can be used in man in a unit dosage form, administered one to four times daily in the amount of 1 to 1000 mg per unit dose.

For parenteral administration or for administration as drops, as for eye infections, the compounds are presented in aqueous solution in a concentration of from about 0.1 to about 10%, more preferably about 0.1 to about 7%. The solution may contain other ingredients, such as emulsifiers, antioxidants or buffers.

Generally, the concentration of the compound(s) of formula I in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The exact regimen for administration of the compounds and compositions disclosed herein will necessarily be dependent upon the needs of the individual subject being treated, the type of treatment and, of course, the judgment of the attending practitioner.

The exact regimen for administration of the compounds and compositions disclosed herein will necessarily be dependent upon the needs of the individual subject being treated, the type of treatment and, of course, the judgment of the attending practitioner. The compounds of the present invention can be administered to an animal in need of treatment. In most instances, this will be a human being, but the treatment of livestock and companion animals is also specifically contemplated as falling within the scope of the instant invention.

The antiviral activity of a compound of the invention can be determined using pharmacological models which are well known to the art, or using Test A described below.

The compounds of formula (I) and pharmaceutically acceptable salts thereof are useful as antiviral agents. Thus, they are useful to combat viral infections in animals, including man. The compounds are generally active against herpes viruses, and are particularly useful against the varicella zoster virus, the Epstein-Barr virus, the herpes simplex virus, the human herpes virus type 8 (HHV-8) and the cytomegalovirus (CMV).

While many of the compounds of the present invention have shown activity against the CMV polymerase, these compounds may be active against the cytomegalovirus by this or other mechanisms of action. Thus, the description below of these compounds' activity against the CMV polymerase is not meant to limit the present invention to a specific mechanism of action.

The HCMV polymerase assay is performed using a scintillation proximity assay (SPA) as described in several references, such as N.D. Cook, et al., Pharmaceutical Manufacturing International, pages 49-53 (1992); K. Takeuchi, Laboratory Practice, September issue (1992); US Patent No. 4,568,649 (1986); which are incorporated by reference herein. Reactions are performed in 96-well plates. The assay is conducted in 100 µl volume with 5.4 mM HEPES (pH 7.5), 11.7 mM KCl, 4.5 mM MgCl₂, 0.36 mg/ml BSA, and 90 nM ³H-dTTP. Assays are run with and without CHAPS, (3-[(3-Cholamidopropyl)-dimethylammonio]-1-propane-sulfonate) at a final concentration of 2 mM. HCMV polymerase is diluted in enzyme dilution buffer containing 50% glycerol, 250 mM NaCl, 10 mM HEPES (pH 7.5), 100 µg/ml BSA, and 0.01% sodium azide. The HCMV polymerase, which is expressed in recombinant baculovirus-infected SF-9 cells and purified according to literature procedures, is added at 10% (or 10 µl) of the final reaction volume, i.e., 100 µl.

Compounds are diluted in 50% DMSO and 10 µl are added to each well. Control wells contain an equivalent concentration of DMSO. Unless noted otherwise, reactions are initiated via the addition of 6 nM biotinylated poly(dA)-oligo(dT) template/primer to reaction mixtures containing the enzyme, substrate, and compounds of interest. Plates are incubated in a 25 C or 37 C H₂O bath and terminated via the addition of 40 µl/reaction of 0.5 M EDTA (pH 8) per well. Reactions are terminated within the time-frame during which substrate incorporation is linear and varied depending upon the enzyme and conditions used, i.e., 30 min. for HCMV polymerase. Ten µl of streptavidin-SPA beads (20 mg/ml in PBS/10% glycerol) are added following termination of the reaction. Plates are incubated 10 min. at 37 C, then equilibrated to room temperature, and counted on a Packard Topcount. Linear regressions are performed and IC₅₀'s are calculated using computer software.

A modified version of the above HCMV polymerase assay is performed as described above, but with the following changes: Compounds are diluted in 100% DMSO until final dilution into assay buffer. In the previous assay, compounds are diluted in 50% DMSO. 4.5 mM dithiotherotol (DTT) is added to the polymerase buffer. Also, a different lot of CMV polymerase is used, which appears to be more active resulting in a more rapid polymerase reaction. Results of the testing of representative compounds of formula I in this assay are shown in Table 1 below.

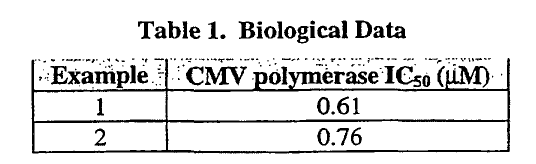

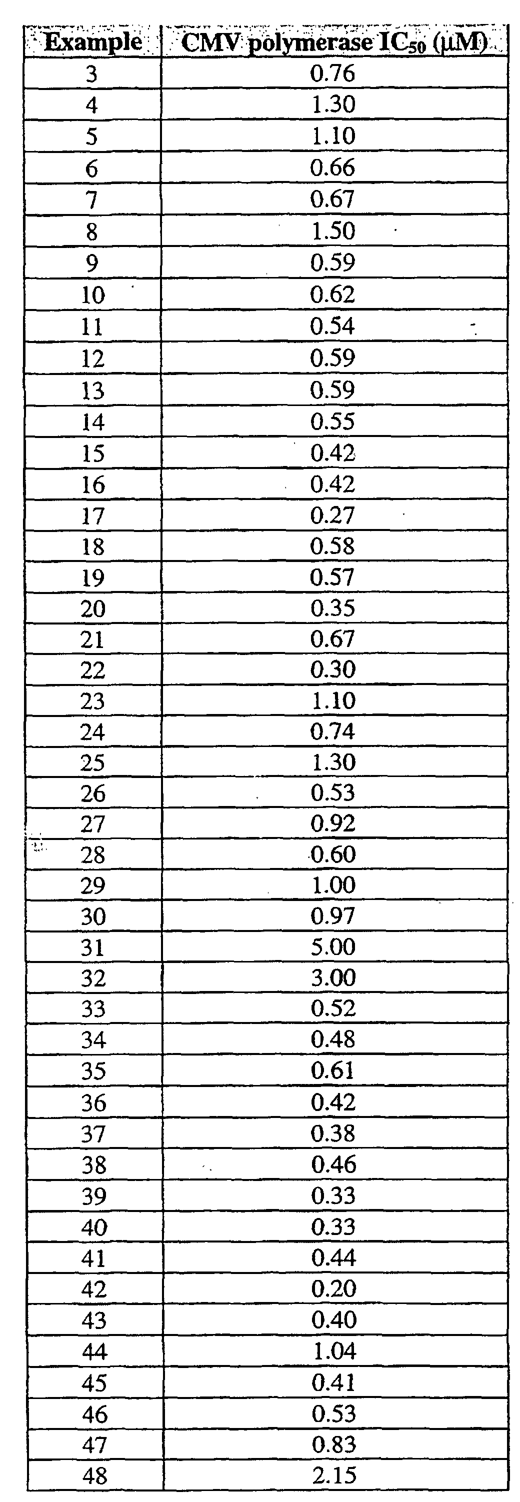

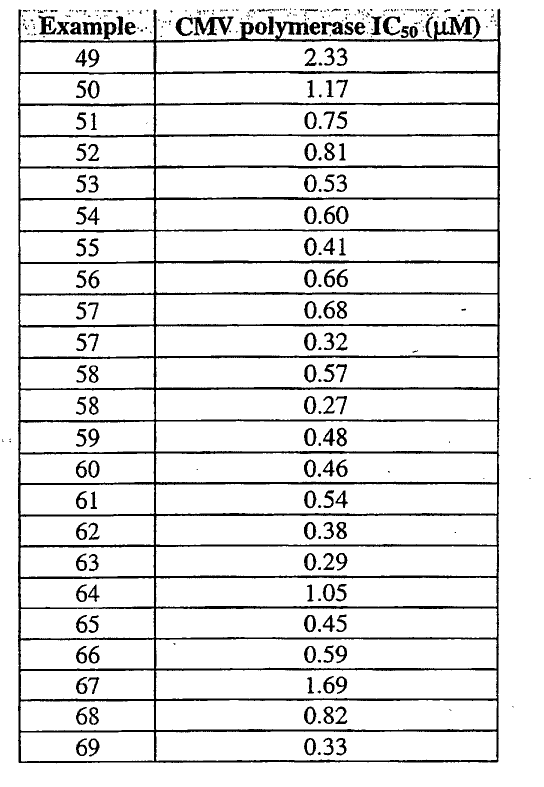

The symbol "--" refers to the data are not determined.

The compounds and their preparation of the present invention will be better understood in connection with the following Examples, which are intended as an illustration of and not a limitation upon the scope of the invention.

### EXAMPLES

### Preparation 1: (3-Hydroxy-4-nitrophenyl)(4-morpholinyl)methanone (Formula A-2 of Chart A)

To a stirred mixture of 55.0 g of 3-hydroxy-4-nitrobenzoic acid in 600 mL of dichloromethane is added 35 mL of thionyl chloride and 5.0 mL of DMF. The mixture is stirred and refluxed with exclusion of moisture for 1-2 h, when it suddenly becomes a clear solution. Refluxing is continued for another hour, then volatiles are removed under reduced pressure. The residual amber oil is dissolved in dichloromethane (200 mL), toluene (200 mL) is added, and the solution again concentrated under reduced pressure. The resulting amber oil is dissolved in 300 mL of dichloromethane, and to this solution, stirred and cooled to 0 °C, is added dropwise 65 mL of morpholine in 200 mL of dichloromethane. The resulting mixture is stirred overnight, then washed with water containing sufficient 6N HCl to render the aqueous phase acidic. The aqueous phase is extracted with one additional portion (100 mL) of dichloromethane, and the combined extracts dried (Na₂SO₄) and concentrated under reduced pressure, affording an orange solid. Recrystallization from 1:3 ethyl acetate in heptane provides 70.34 g of the amide as a solid, mp 105.5-106.5 °C.
¹H NMR (CDCl₃) δ 3.40, 3.64, 3.79, 7.01, 7.18, 8.18, 10.63 ppm
HRMS (FAB) calcd for C₁₁H₁₂N₂O₅ +H₁ 253.0824, found 253.0832.
Anal. Calcd for C₁₁H₁₂N₂O₅: C, 52.38, H, 4.80, N, 11.11, Found: C, 52.46; H, 4.85; N, 11.11.

### Preparation 2: 2-Amino-5-(4-morpholinylcarbonyl)phenol (Formula A-3 of Chart A)

A mixture of 15.36 g of the product of Preparation **1** and 1.25 g of 5% palladium on carbon in 450 mL of methanol is shaken under 30 psi of hydrogen gas for 3 h, then filtered through Celite. The solid is washed thoroughly with chloroform and methanol, and the combined filtrates concentrated under reduced pressure. The solid residue is suspended in ether, and the resulting solid filtered, washed with ether, and dried *in vacuo* to provide 13.34 g of the aminophenol as a solid.
¹H NMR (DMSO-d₆) δ 3.48, 3.57, 6.57, 6.68, 6.76, 9.28 ppm.

### Preparation 3: Ethyl 7-(4-morpholinylcarbonyl)-3-oxo-3,4-dihydro-2H-1,4-benzoxazine-2-carboxylate (Formula A-4 of Chart A)

To a stirred, cooled (0 °C) mixture of 13.24 g the product of Preparation **2** in 60 mL of dry DMF, under argon, is added in portions 2.4 g of sodium hydride (60% dispersion in mineral oil). The mixture is stirred at 0°C for 45 m, then 10.7 mL of diethyl chloromalonate is added, and the mixture allowed to warm to ambient temperature. After 18 h, the mixture is partitioned between dichloromethane and water containing sufficient dil. aq. HCl to bring the pH of the aqueous phase to *ca.* 4. The aqueous phase is extracted with two additional portions of dichloromethane, and the combined extracts dried (MgSO₄) and concentrated under reduced pressure to a DMF solution of an intermediate amino ester. This solution is heated at 80 °C under argon for 5 h, and the DMF is then distilled off under reduced pressure. Flash chromatography of the residue on silica using 3% methanol in dichloromethane affords 15.66 g of the title compound as a solid.
¹H NMR (CDCl₃) δ 1.27, 3.7, 4.26, 5.22, 6.89, 7.08, 7.15, 9.15.

### Preparation 4: [7-(4-Morpholinylmethyl)-3,4-dihydro-2H-1,4-benzoxazin-2-yl]methanol (Formula A-5 of Chart A)

To a stirred, cold (0 °C) solution of 15.59 g of the product of Preparation **3** in 90 mL of dry THF, under argon, is added 34 mL of borane-dimethyl sulfide complex. Following the addition, the ice bath is removed, whereupon the reaction warms spontaneously nearly to reflux. After the initial exothermic reaction, heat is supplied from an oil bath, and the temperature of the reaction maintained at 50 °C for 18 h. The solution is then cooled to 0 °C and quenched cautiously with methanol. Additional methanol (200 mL) is added, and the solution distilled through a Vigreux column at atmospheric pressure. Methanol is added at intervals until a total of 650 mL has distilled. Flash chromatography of the residual oil on silica using 5-6% methanol in dichloromethane provides 8.07 g of the title comopound as a solid.
¹H NMR (CDCl₃) δ 2.4, 3.3, 3.70, 3.8, 4.34, 6.56, 6.72, 6.80.

### Preparation 5: Diethyl 2-{[2-(hydroxymethyl)-7-(4-morpholinylmethyl)-2,3-dihydro-4H-1,4-benzoxazin-4-yl]methylene}malonate (Formula A-6 of Chart A)

A mixture of 8.05 g of the product of Preparation **4** and 7.3 g of diethyl ethoxymethylenemalonate is heated at 130 °C under a slow stream of argon for 2 h, then held under vacuum at 130 °C to remove volatile components. Flash chromatography of the residual amber oil on silica using 3% methanol in dichloromethane affords 11.51 g of the title compound as a solid.
¹H NMR (CDCl₃) δ 1.30, 1.33, 2.43, 3.42, 3.58, 3.71, 3.82, 4.19, 4.24, 4.28, 6.9, 8.01.
MS (ES+) m/z 435.3.

### Preparation 6: Diethyl 2-{[2-[(acetyloxy)methyl]-7-(4-morpholinylmethyl)-2,3-dihydro-4H-1,4-benzoxazin-4-yl]methylene}malonate (Formula A-7 of Chart A)

A solution of 11.48 g of the product of Preparation **5**, 11 mL of pyridine, and 5.0 mL of acetic anhydride in 25 mL of dichloromethane is stirred at room temperature for 18 h, then concentrated under reduced pressure with toluene azeotrope to remove pyridine and acetic acid. Flash chromatography of the residual oil on silica using 2% methanol in dichloromethane provides 13.08 g of the title compound as a solid.
¹H NMR (CDCl₃) δ 1.31, 1.33, 2.11, 2.42, 3.42, 3.48, 3.58, 3.71, 4.28, 6.95, 7.99.
TLC R_{*f*} 0.35 (3% methanol in dichloromethane).
HRMS (FAB) calcd for C₂₄H₃₂N₂O₈ +H₁ 477.2237, found 477.2236.

### Preparation 7: Ethyl 2-[(acetyloxy)methyl]-9-(4-morpholinylmethyl)-7-oxo-2,3-dihydro-7H-[1,4]oxazino[2,3,4-ij]quinoline-6-carboxylate (Formula A-8 of Chart A)

To 7.1 g of phosphorus pentoxide is added 50 mL of methanesulfonic acid, and the mixture is stirred under argon at 80-100 °C until a clear solution is obtained. The solution is cooled to 10 °C, and into this is cannulated a solution of 13.08 g of the product of Preparation **6** in 10 mL of dichloromethane. The resulting solution is stirred at 50 °C for 18 h, then cooled, diluted with dichloromethane (100 mL), and cautiously added to a stirred slurry of 72 g of sodium bicarbonate in 200 mL of water. Phases are separated and the aqueous extracted with four additional portions of dichloromethane, and the combined organic phases dried (Na₂SO₄) and concentrated under reduced pressure. Flash chromatography of the residue on silica gel using 3.5-4.5% methanol in dichloromethane affords 5.58 g of the title compound as a solid.
¹H NMR (CDCl₃) δ 1.39, 2.16, 2.44, 3.54, 3.70, 4.17, 4.29, 4.34, 4.45, 4.50, 4.59, 7.31, 7.91,8.28.
TLC R_{*f*} 0.30 (5% methanol in dichloromethane).
MS (ES+) m/z 431.2.

### Example 1: N-(4-Chlorobenzyl)-2-(hydroxymethyl)-9-(4-morpholinylmethyl)-7-oxo-2,3-dihydro-7H-[1,4]oxazino[2,3,4-ij]quinoline-6-carboxamide (Formula A-9 of Chart A)

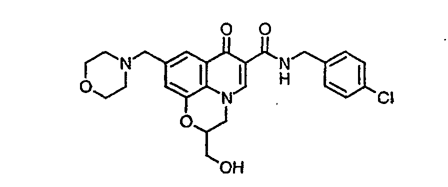

A mixture of 5.17 g of the product of Preparation **7** and 10.2 g of *p*-chlorobenzylamine is stirred and heated at 145 °C under argon for 18 h, after which the bulk of excess amine is distilled off under reduced pressure. The residual solid is triturated well with ether, filtered and washed with ether, and dried under vacuum to provide 5.91 g of cream colored solid. This is triturated with boiling acetonitrile, filtered and washed with acetonitrile, and dried under vacuum to afford 5.76 g of the title compound as a solid.
¹H NMR (DMSO-d₆) δ 2.38, 3.34, 3.58, 3.75, 4.24, 4.42, 4.56, 4.62, 5.27, 7.27, 7.39, 7.77, 8.75, 10.43.
TLC R_{*f*} 0.38 (10% methanol in dichloromethane)
IR (mull) 1656, 1611, 1572, 1556, 1539, 1504, 1492, 1417, 1347, 1286, 1123, 1112, 1105, 1090, 806 cm⁻¹.
UV λₘₐₓ 225 (32600, 95% ethanol).

### Preparation 8: [6-{[(4-Chlorobenzyl)amino]carbonyl}-9-(4-morpholinylmethyl)-7-oxo-2,3-dehydro-7H-[1,4]oxazino[2,3,4-ij]quinolin-2-yl]methyl methanesulfonate (Formula A-10 of Chart A)

A solution of 726 mg of the product of Example **1** and 0.80 mL of 2,4,6-collidine is prepared with the aid of heat in 12 mL of DMF, and the solution is cooled to 10 °C for the addition of 0.35 mL of methanesulfonyl chloride. The mixture is allowed to warm to ambient temperature and stirred overnight, then partitioned between dichloromethane and water containing 25 mL of saturated aqueous NaHCO₃. The aqueous phase is extracted with two additional portions of dichloromethane, and the combined organic phases dried (MgSO₄) and concentrated under high vacuum to remove DMF. Flash chromatography of the residue on silica gel using 3% methanol in dichloromethane provides 692 mg of the title compound as a solid.
¹H NMR (CDCl₃ + CD₃OD) δ 2.45, 3.14, 3.57, 3.70, 4.26, 4.41, 4.57, 7.28, 7.38, 7.94, 8.67, 10.36.
TLC R_{*f*} 0.29 (5% methanol in dichloromethane).
MS (ES+) m/z 561.9.

### Example 2: General procedure for mesylate displacement using nitrogen nucleophiles: N-(4-Chlorobenzyl)-2,9-bis(4-morpholinylmethyl)-7-oxo-2,3-dihydro-7H-[1,4]oxazino[2,3,4-ij]quinoline-6-carboxamide (Formula A-11 of Chart A)

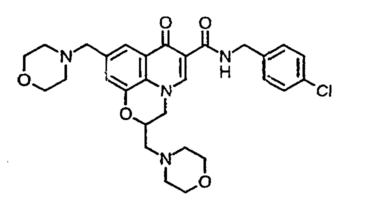

A solution of 91 mg of the product of Preparation **8** and 0.17 mL of morpholine in 1.0 mL of N-methylpyrrolidinone is heated at 80 °C for 6 h, then cooled and partitioned between water and dichloromethane or ethyl acetate. The aqueous phase is extracted with sufficient dichloromethane or ethyl acetate to remove product, and the organic phase is dried (MgSO₄). Volatiles are removed under high vacuum, and the residue purified by flash chromatography on silica using 2-5% methanol in dichloromethane to afford 45.2 mg of the title compound as a white solid. Further purification may be effected by recrystallization from acetonitrile or ethyl acetate-hexane.
Mp 178-180 °C. ¹H NMR (CDCl₃) δ 2.46, 2.60, 2.72, 2.86, 3.59, 3.77, 4.18, 4.37, 4.47, 4.64, 7.30, 7.93, 8.66, 10.44.
TLC R_{*f*} 0.22 (5% methanol in dichloromethane).
HRMS (FAB) calcd for C₂₉H₃₃CLN₄O₅ +H₁ 553.2217, found 553.2228.
Anal. Calcd for C₂₉H₃₃ClN₄O₅: C, 62.98; H, 6.01; N, 10.13; Cl, 6.41; found: C, 62.85; H, 6.10; N, 9.97.

### Example 3: N-(4-Chlorobenzyl)-2-[(dimethylamino)methyl]-9-(4-morpholinylmethyl)-7-oxo-2,3-dihydro-7H-[1,4]oxazino[2,3,4-ij]quinoline-6-carboxamide (Formula A-11 of Chart A)

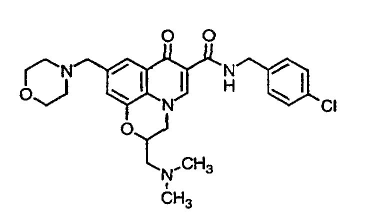

Following the procedure in Example **2**, the titled compound is obtained as a solid, mp 168-170 °C.
¹H NMR (CDCl₃) δ 2.35, 2.45, 2.64, 2.77, 3.57, 3.70, 4.13, 4.35, 4.44, 4.63, 7.30, 7.37, 7.93, 8.65, 10.46.
TLC R_{*f*} 0.47 (5% methanolic ammonia in dichloromethane).
HRMS (FAB) calcd for C₂₇H₃₁CLN₄O₄+H₁ 511.2112, found 511.2111.
Anal. Calcd for C₂₇H₃₁ClN₄O₄: C, 63.46; H, 6.11; N, 10.96; Cl, 6.94; found: C, 63.22; H, 6.24; N, 10.63.

### Example 4: N-(4-Chlorobenzyl)-2-[(4-methyl-1-piperazinyl)methyl]-9-(4-morpholinylmethyl)-7-oxo-2,3-dihydro-7H-[1,4]oxazino[2,3,4-ij]quinoline-6-carboxamide (Formula A-11 of Chart A)

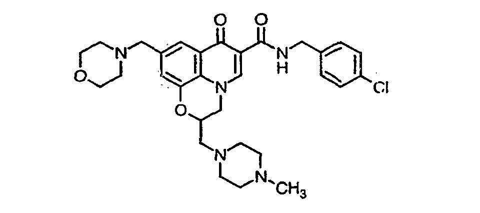

Following the procedure in Example **2**, the titled compound is obtained as a solid, mp 201-208 (d).
¹H NMR (CDCl₃) δ 2.33, 2.4-2.7, 2.72, 2.86, 3.57, 3.70, 4.15, 4.36, 4.48, 4.64, 7.29, 7.35, 7.92, 8.65, 10.46.
TLC R_{*f*}0.37 (7% methanolic ammonia in dichloromethane).
HRMS (FAB) calcd for C₃₀H₃₆CLN₅O₄ +H₁ 566.2534, found 566.2547.
Anal. Calcd for C₃₀H₃₆ClN₅O₄: C, 63.65; H, 6.41; N, 12.37; Cl, 6.26; found (av): C, 63.00; H, 6.37; N, 11.95.

### Example 5: N-(4-Chlorobenzyl)-9-(4-morpholinylmethyl)-7-oxo-2-(1-pyrrolidinylmethyl)-2,3-dihydro-7H-[1,4]oxazino[2,3,4-ij]quinoline-6-carboxamide (Formula A-11 of Chart A)

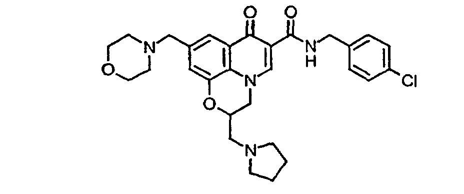

Following the procedure in Example 2, the titled compound is obtained as a solid, mp 209.5-212.0 °C.
¹H NMR (CDCl₃) δ 1.82, 2.45, 2.63, 2.89, 3.58, 3.70, 4.16, 4.38, 4.45, 4.64, 7.30, 7.37, 7.93, 8.65, 10.47.
TLC R_{*f*} 0.31 (3% methanolic ammonia in dichloromethane).
IR (drift) 2960, 2799, 1655, 1627, 1608, 1553, 1501, 1412, 1348, 1330, 1319, 1281, 1221, 1117, 808 cm⁻¹
HRMS (FAB) calcd for C₂₉H₃₃CLN₄O₄ +H₁ 537.2268, found 537.2259
Anal. Calcd for C₂₉H₃₃ClN₄O₄: C, 64.86; H, 6.19; N, 10.43; Cl, 6.60; found: C, 64.89; H, 6.25; N, 10.38.

### Example 6: N-(4-Chlorobenzyl)-2-{[(2,3-dihydroxypropyl)amino]methyl}-9-(4-marpholinylmethyl)-7-oxo-2,3-dihydro-7H-[1,4]oxazino[2,3,4-ij]quinoline-6-carboxamide (Formula A-11 of Chart A)

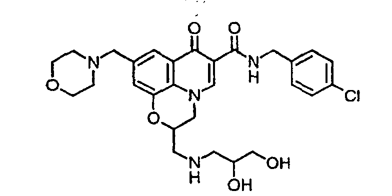

Following the procedure in Example 2, the titled compound is obtained.
¹H NMR (CDCl₃+ CD₃OD) δ 2.47, 2.79, 3.53, 3.65, 3.74, 4.15, 4.39, 4.63, 7.30, 7.37, 7.92, 8.63, 10.58.
TLC R_{*f*} 0.22 (12% methanolic ammonia in dichloromethane).
IR (drift) 1654, 1626, 1608, 1555, 1503, 1414, 1349, 1330, 1282, 1223, 1114, 1094, 1014, 869, 807 cm⁻¹
HRMS (FAB) calcd for C₂₈H₃₃CLN₄O₆ +H₁ 557.2167, found 557.2153
Anal. Calcd for C₂₈H₃₃ClN₄O₆: C, 60.37; H, 5.97; N, 10.06; Cl, 6.36; found (av): C, 59.87; H, 5.99; N, 9.94.

### Example 7: N-(4-Chlorobenzyl)-2-{[(2-hydroxyethyl)amino]methyl}-9-(4-morpholinylmethyl)-7-oxo-2,3-dihydro-7H-[1,4]oxazino[2,3,4-ij]quinoline-6-carboxamide (Formula A-11 of Chart A)

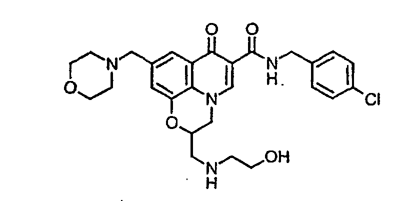

Following the procedure in Example **2**, the titled compound is obtained.
¹H NMR (CDCl₃ + CD₃OD) δ 2.47, 2.85, 3.07, 3.60, 3.71, 4.24, 4.45, 4.63, 7.31, 7.38, 7.92, 8.63, 10.59.
TLC R_{*f*} 0.15 (6% methanolic ammonia in dichloromethane).
IR (drift) 1653, 1626, 1607, 1580, 1557, 1502, 1414, 1348, 1293, 1281, 1273, 1224, 1116, 869, 807 cm⁻¹
HRMS (FAB) calcd for C₂₇H₃₁CLN₄O₅ +H₁ 527.2061, found 527.2067
Anal. Calcd for C₂₇H₃₁ClN₄O₅: C, 61.53; H, 5.93; N, 10.63; Cl, 6.73; found (av): C, 61.15; H, 5.98; N, 10.64.

### Example 8: N-(4-Chlorobenzyl)-9-(4-morpholinylmethyl)-7-oxo-2-(1-piperidinylmethyl)-2,3-dihydro-7H-[1,4]oxazino[2,3,4-ij]quinoline-6-carboxamide (Formula A-11 of Chart A).

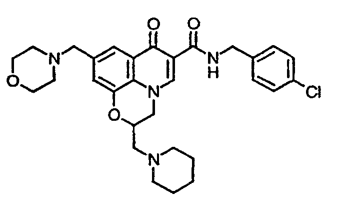

Following the procedure in Example **2**, the titled compound is obtained as a solid, mp 213-215 °C. ¹H NMR (CDCl₃) δ 1.46, 1.60, 2.5, 2.63, 2.81, 3.57, 3.70, 4.13, 4.39, 4.46, 4.64, 7.30, 7.34, 7.92, 8.66, 10.46.
TLC R_{*f*} 0.25 (5% methanol in dichloromethane).
IR (drift) 2931, 1653, 1607, 1572, 1552, 1500, 1411, 1349, 1330, 1284, 1225, 1113, 1087, 810, 800 cm⁻¹
HRMS (FAB) calcd for C₃₀H₃₅CLN₄O₄ +H₁ 551.2425, found 551.2435
Anal. Calcd for C₃₀H₃₅ClN₄O₄: C, 65.39; H, 6.40; N, 10.17; Cl, 6.43; found: C, 65.34; H, 6.46; N, 10.12.

### Example 9: 2-{[bis(2-Hydroxyethyl)amino]methyl}-N-(4-chlorobenzyl)-9-(4-morpholinylmethyl)-7-oxo-2,3-dihydro-7H-[1,4]oxazino[2,3,4-ij]quinoline-6-carboxamide (Formula A-11 of Chart A)

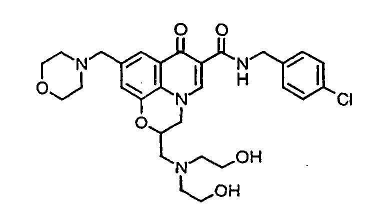

Following the procedure in Example **2**, the titled compound is obtained. ¹H NMR (CDCl₃) δ 2.44, 2.7, 2.97, 3.54, 3.58, 3.69, 4.24, 4.3, 4.5, 4.55, 7.27, 7.28, 7.86, 8.59, 10.51.
TLC R_{*f*} 0.28 (10% methanol in dichloromethane).
IR (drift) 2953, 2843, 2810, 1660, 1608, 1552, 1500, 1411, 1355, 1329, 1284, 1113, 1085, 808, 799 cm⁻¹
HRMS (FAB) calcd for C₂₉H₃₅CLN₄O₆ +H₁ 571.2323, found 571.2328
Anal. Calcd for C₂₉H₃₅ClN₄O₆: C, 60.99; H, 6.18; N, 9.81; Cl, 6.21; found: C, 60.67; H, 6.24; N, 9.68.

### Example 10: N-(4-Chlorobenzyl)-9-(4-morpholinylmethyl)-7-oxo-2-{[(2-pyridinylmethyl)amino]methyl}-2,3-dihydro-7H-[1,4]oxazino[2,3,4-ij]quinoline-6-carboxamide (Formula A-11 of Chart A)

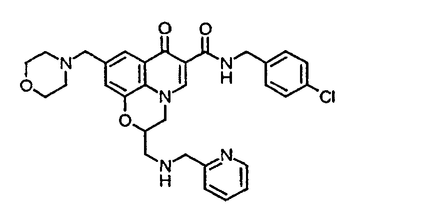

Following the procedure in Example **2**, the titled compound is obtained.
¹H NMR (CDCl₃) δ 2.48, 3.13, 3.60, 3.72, 4.02, 4.30, 4.48, 4.63, 7.21, 7.29, 7.33, 7.40, 7.68, 7.93, 8.58, 8.64, 10.44.
TLC R_{*f*} 0.51 (7% methanolic ammonia in dichloromethane).
IR (drift) 1654, 1626, 1607, 1554, 1500, 1453, 1412, 1348, 1330, 1282, 1223, 1113, 810, 801, 760 cm⁻¹
HRMS (FAB) calcd for C₃₁H₃₂CLN₅O₄ +H₁ 574.2221, found 574.2233
Anal. Calcd for C₃₁H₃₂ClN₅O₄: C, 64.86; H, 5.62; N, 12.20; Cl, 6.18; found: C, 64.66; H, 5.62; N, 12.05.

### Example 11: N-(4-Chlorobenzyl)-9-(4-morpholinylmethyl)-7-oxo-2-{[(3-pyridinylmethyl)amino]methyl}-2,3-dihydro-7H-[1,4]oxazino[2,3,4-ij]quinoline-6-carboxamide (Formula A-11 of Chart A)

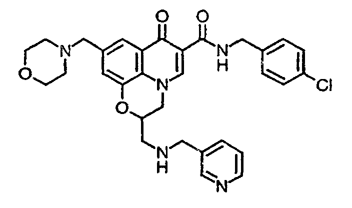

Following the procedure in Example **2**, the titled compound is obtained.
¹H NMR (CDCl₃) δ 2.46, 3.06, 3.08, 3.59, 3.71, 3.91, 4.27, 4.43, 4.63, 7.30, 7.36, 7.71, 7.93, 8.54, 8.60, 8.64, 10.44.
TLC R_{*f*} 0.36 (6% methanolic ammonia in dichloromethane).
HRMS (FAB) calcd for C₃₁H₃₂CLN₅O₄ +H₁ 574.2221, found 574.2221
Anal. Calcd for C₃₁H₃₂ClN₅O₄: C, 64.86; H, 5.62; N, 12.20; Cl, 6.18; found: C, 64.65; H, 5.68; N, 12.07.

### Example 12: N-(4-Chlorobenzyl)-9-(4-morpholinylmethyl)-7-oxo-2-{[(4-pyridinylmethyl)amino]methyl}-2,3-dihydro-7H-[1,4]oxazino[2,3,4-ij]quinoline-6-carboxamide (Formula A-11 of Chart A)

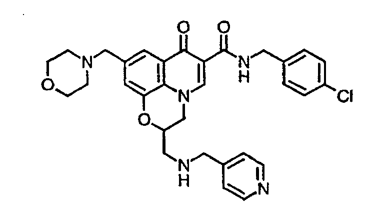

Following the procedure in Example **2**, the titled compound is obtained as a solid,
mp 164-167 °C.
¹H NMR (CDCl₃) δ 2.47, 3.04, 3.10, 3.59, 3.71, 3.92, 4.3, 4.44, 4.63, 7.30, 7.37, 7.94, 8.57, 8.65, 10.44.
TLC R_{*f*} 0.40 (6% methanolic ammonia in dichloromethane).
HRMS (FAB) calcd for C₃₁H₃₂CLN₅O₄ +H₁ 574.2221, found 574.2216
Anal. Calcd for C₃₁H₃₂ClN₅O₄: C, 64.86; H, 5.62; N, 12.20; Cl, 6.18; found: C, 64.17; H, 5.70; N, 12.07.

### Example 13: N-(4-Chlorobenzyl)-2-(1H-imidazol-1-ylmethyl)-9-(4-morpholinylmethyl)-7-oxo-2,3-dihydro-7H-[1,4]oxazino[2,3,4-ij]quinoline-6-carboxamide (Formula A-11 of Chart A)

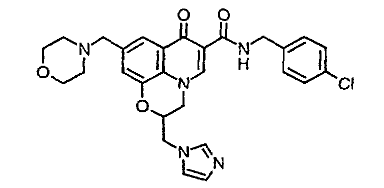

Following the procedure in Example **2**, the titled compound is obtained as a solid,
mp 217-219 °C (d).
¹H NMR (CDCl₃ + CD₃OD) δ 2.49, 3.62, 3.73, 3.97, 4.29, 4.40, 4.44, 4.58, 4.61, 7.10, 7.15, 7.30, 7.42, 7.72, 7.94, 8.59, 10.44.
TLC R_{*f*} 0.30 (5% methanol in dichloromethane).
IR (drift) 2916, 2804, 1656, 1608, 1570, 1550, 1500, 1411, 1347, 1295, 1280, 1231, 1223, 1110, 800 cm⁻¹
HRMS (FAB) calcd for C₂₈H₂₈CLN₅O₄ +H₁ 534.1908, found 534.1907
Anal. Calcd for C₂₈H₂₈ClN₅O₄: C, 62.98; H, 5.28; N, 13.11; Cl, 6.64; found: C, 62.65; H, 5.30; N, 13.21.

### Example 14: N-(4-Chlorobenzyl)-2-[(methylsulfanyl)methyl]-9-(4-morpholinylmethyl)-7-oxo-2,3-dihydro-7H-[1,4]oxazino[2,3,4-ij]quinoline-6-carboxamide (Formula A-11 of Chart A)

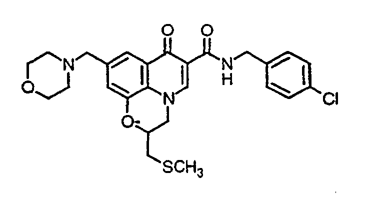

A mixture of 112 mg of the product of Preparation **8** and 112 mg of sodium methanethiolate in 0.5 mL of DMF is stirred at ambient temperature for 18 h, then partitioned between water and chloroform. The aqueous layer is extracted with two additional portions of chloroform, and the combined organic phases dried (MgSO₄) and concentrated under reduced pressure. Flash chromatography of the residue on silica using 1-2% methanol in dichloromethane provides 72.5 mg of the title compound as a yellow solid. Recrystallization from acetonitrile gives a white solid, mp 190-191 °C.
¹H NMR (CDCl₃) δ 2.26, 2.46, 2.83, 3.01, 3.58, 3.70, 4.21, 4.48, 4.64, 7.29, 7.36, 7.94, 8.66, 10.43.
TLC R_{*f*} 0.45 (5% methanol in dichloromethane).
HRMS (FAB) calcd for C₂₆H₂₈CLN₃O₄S +H₁ 514.1567, found 514.1568.
Anal. Calcd for C₂₆H₂₈ClN₃O₄S: C, 60.75; H, 5.49; N, 8.17; Cl, 6.90; S, 6.24; found: C, 60.60; H, 5.52; N, 8.12; Cl, 6.85; S, 6.15.

### Example 15: 2-[(tert-Butylsulfanyl)methyl]-N-(4-chlorobenzyl)-9-(4-morpholinylmethyl)-7-oxo-2,3-dihydro-7H-[1,4]oxazino[2,3,4-ij]quinoline-6-carboxamide (Formula A-11 of Chart A)

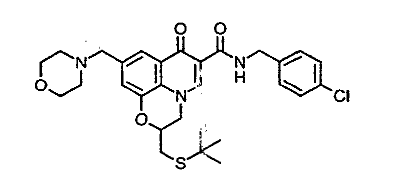

Following the procedure in Example **14,** the titled compound is obtained as a solid,
mp 198-201 °C. ¹H NMR (CDCl₃) δ 1.37, 2.45, 2.81, 3.06, 3.57, 3.69, 4.13, 4.43, 4.63, 7.29, 7.35, 7.92, 8.64, 10.42.
TLC R_{*f*} 0.33 (3% methanol in dichloromethane).
HRMS (FAB) calcd for C₂₉H₃₄CLN₃O₄S +H₁ 556.2037, found 556.2052
Anal. Calcd for C₂₉H₃₄ClN₃O₄S: C, 62.63; H, 6.16; N, 7.56; Cl, 6.38; S, 5.76; found: C, 62.46; H, 6.24; N, 7.50; Cl, 6.34; S, 5.74.

### Example 16: N-(4-Chlorobenzyl)-2-{[(2-hydroxyethyl)sulfanyl]methyl}-9-(4-morpholinylmethyl)-7-oxo-2,3-dihydro-7H-[1,4]oxazino[2,3,4-ij]quinoline-6-carboxamide (Formula A-11 of Chart A)

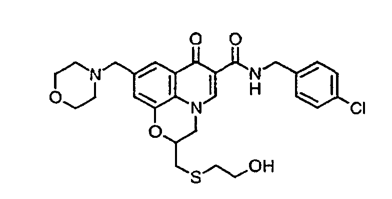

Following the procedure in Example **14,** the titled compound is obtained.
¹H NMR (CDCl₃+ CD₃OD) δ 2.48, 2.85, 2.93, 3.04, 3.60, 3.72, 3.80, 4.24, 4.55, 4.63, 7.31, 7.38, 7.92, 8.63, 10.51.
TLC R_{*f*} 0.40 (10% methanol in dichloromethane).
IR (drift) 1651, 1607, 1578, 1555, 1500, 1413, 1347, 1329, 1281, 1272, 1232, 1113, 1014, 869, 807 cm⁻¹
HRMS (FAB) calcd for C₂₇H₃₀CLN₃O₅S +H₁ 544.1673, found 544.1672
Anal. Calcd for C₂₇H₃₀ClN₃O₅S: C, 59.60; H, 5.56; N, 7.72; Cl, 6.52; S, 5.89; found (av): C, 59.15; H, 5.55; N, 7.55.

### Example 17: N-(4-Chlorobenzyl)-2-{[(1-methyl-1H-imidazol-2-yl)sulfanyl]methyl}-9-(4-morpholinylmethyl)-7-oxo-2,3-dihydro-7H-[1,4]oxazino[2,3,4-ij]quinoline-6-carboxamide (Formula A-11 of Chart A)

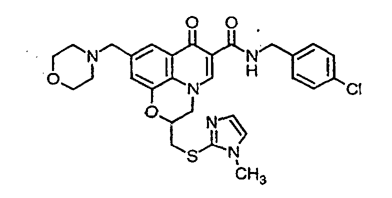

Following the procedure in Example **14**, the titled compound is obtained as a solid, ¹H NMR (CDCl₃) δ 2.45, 3.39, 3.55, 3.56, 3.63, 3.69, 4.27, 4.50, 4.63, 4.72, 6.92, 6.95, 7.29, 7.35, 7.92, 8.64, 10.42.
TLC R_{*f*} 0.22 (5% methanol in dichloromethane).
IR (drift) 1655, 1626, 1607, 1578, 1560, 1499, 1457, 1414, 1357, 1347, 1280, 1273, 1117, 869, 807 cm⁻¹
HRMS (FAB) calcd for C₂₉H₃₀CLN₅O₄S +H₁ 580.1785, found 580.1776
Anal. Calcd for C₂₉H₃₀ClN₅O₄S: C, 60.04; H, 5.21; N, 12.07; Cl, 6.11; S, 5.53; found: C, 59.93; H, 5.15; N, 12.03.

### Example 18: Methyl({[6-{[(4-chlorobenzyl)amino]carbonyl}-9-(4-morpholinylmethyl)-7-oxo-2,3-dihydro-7H-[1,4]oxazino[2,3,4-ij]quinolin-2-yl]methyl}sulfanyl)acetate (Formula A-11 of Chart A)

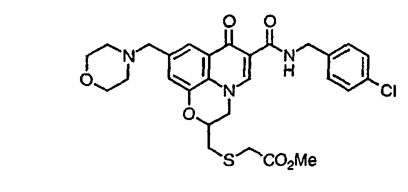

Following the procedure described in Example **14**, the titled compound is obtained as a solid, mp 182.5-183.0 °C.
¹H NMR (CDCl₃) δ 2.45, 3.03, 3.16, 3.40, 3.58, 3.70, 3.75, 4.25, 4.41, 4.56, 4.63, 7.30, 7.36, 7.94, 8.66, 10.42.
TLC R_{*f*} 0.42 (5% methanol in dichloromethane).
IR (drift) 1735, 1653, 1607, 1553, 1501, 1411, 1352, 1329, 1281, 1226, 1141, 1113, 1008, 810, 802 cm⁻¹
HRMS (FAB) calcd for C₂₈H₃₀CLN₃O₆S +H₁ 572.1622, found 572.1624
Anal. Calcd for C₂₈H₃₀ClN₃O₆S: C, 58.79; H, 5.28; N, 7.34; Cl, 6.20; S, 5.60; found: C, 58.67; H, 5.26; N, 7.33.

### Example 19: N-(4-Chlorobenzyl)-2-{[(2,3-dihydroxypropyl)sulfanyl]methyl}-9-(4-morpholinylmethyl)-7-oxo-2,3-dihydro-7H-[1,4]oxazino[2,3,4-ij]quinoline-6-carboxamide (Formula A-11 of Chart A)

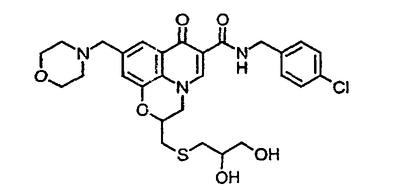

Following the procedure described in Example **14**, the titled compound is obtained.
¹H NMR (CDCl₃) δ 2.45, 2.8, 3.06, 3.54, 3.58, 3.68, 3.83, 4.16, 4.46, 4.58, 7.27, 7.32, 7.90, 8.62, 10.52.
TLC R_{*f*} 0.32 (10% methanol in dichloromethane).
MS (ES+) m/z 574.4

### Preparation 9: Ethyl 2-(R or S)-[(acetyloxy)methyl]-9-(4-morpholinylmethyl)-7-oxo-2,3-dihydro-7H-[1,4]oxazino[2,3,4-ij]quinoline-6-carboxylate (Formula B-1 of Chart B)

Racemic acetate (3.2 g of the product of Preparation **7**) is resolved using a 5x50 cm Chiralcel OD column at 30 °C with absolute ethanol as the mobile phase at a flow rate of 75 mL/min with UV detection at 326 nm. 1 g injections are made in 35 mL of 1:4 CHCl₃/EtOH. Following HPLC resolution, the compounds are purified by silica gel chromatography using 4% methanol in dichloromethane. Of the first eluting isomer, there is obtained 1.228 g; of the second peak, there is obtained 1.47 g. Analytical samples are prepared by recrystallization from ethyl acetate-hexane.
For **Isomer 1**: mp 142.5-144.0 °C.
¹H NMR (CDCl₃) δ 1.39, 2.16, 2.44, 3.54, 3.70, 4.17, 4.27, 4.35, 4.45, 4.49, 4.59, 7.32, 7.91,8.29.
TLC R_{*f*} 0.38 (8% methanol in dichloromethane).
IR (drift) 1745, 1682, 1637, 1609, 1556, 1505, 1324, 1292, 1273, 1237, 1222, 1181, 1113, 879, 799 cm⁻¹
HRMS (FAB) calcd for C₂₂H₂₆N₂O₇ +H₁ 431.1818, found 431.1821
Anal. Calcd for C₂₂H₂₆N₂O₇: C, 61.39; H, 6.09; N, 6.51; found: C, 61.24; H, 6.13; N, 6.49.
For **isomer 2:**
Mp 143.0-144.5 °C.
¹H NMR (CDCl₃) δ 1,39, 2.16, 2.44, 3.54, 3.70, 4.17, 4.27, 4.35, 4.45, 4.49, 4.59, 7.31, 7.91, 8.28.
TLC R_{*f*} 0.38 (8% methanol in dichloromethane).
IR (drift) 1745, 1726, 1682, 1637, 1609, 1556, 1505, 1324, 1291, 1273, 1237, 1223, 1181, 1113, 799 cm⁻¹
HRMS (FAB) calcd for C₂₂H₂₆N₂O₇ +H₁ 431.1818, found 431.1831
Anal. Calcd for C₂₂H₂₆N₂O₇: C, 61.39; H, 6.09; N, 6.51; found: C, 61.29; H, 6.12; N, 6.50.

### Example 20: N-(4-Chlorobenzyl)-2-(R or S)-(hydroxymethyl)-9-(4-morpholinylmethyl)-7-oxo-2,3-dihydro-7H-[1,4]oxazino[2,3,4-ij]quinoline-6-carboxamide (Formula B-2 of Chart B, isomer 1)

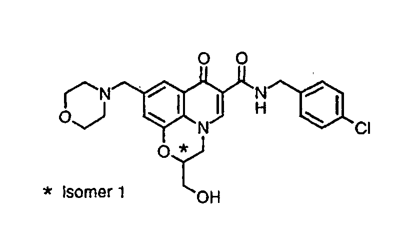

A mixture of 1.20 g of the product of Preparation **9** (isomer 1) and 2.4 g of *p*-chlorobenzylamine is heated under argon at 150 °C for 18 h, then excess amine is distilled off *in vacuo*. Flash chromatography of the residue on silica using 5-8% methanol in dichloromethane provides 1.27 g of the title amide as a tan solid. The solid is triturated well with ether, filtered, washed well with ether, and dried under vacuum to afford 1.22 g of the title compound as a solid.
¹H NMR (CDCl₃ + CD₃OD) δ 2.49, 3.62, 3.73, 3.95, 4.4, 4.64, 7.31, 7.40, 7.91, 8.65, 10.63. TLC R_{*f*} 0.30 (10% methanol in dichloromethane).

### Example 21: N-(4-Chlorobenzyl)-2-(R or S)-(hydroxymethyl)-9-(4-morpholinylmethyl)-7-oxo-2,3-dihydro-7H-[1,4]oxazino[2,3,4-ij]quinoline-6-carboxamide (Formula B-2 of Chart B, isomer 2)

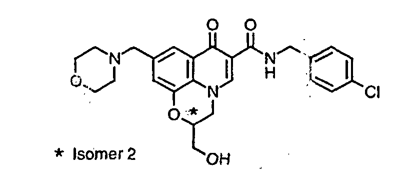

According to the procedure described for isomer 1 in Example **20**, 1.41 g of the product of Preparation **9** (isomer 2) is converted to 1.43 g of the corresponding amide.
¹H NMR (CDCl₃+ CD₃OD) δ 2.48, 3.61, 3.72, 3.95, 4.4, 4.64, 7.31, 7.38, 7., 8.64 (s, 1H), 10.62.
TLC R_{*f*} 0.30 (10% methanol in dichloromethane).

### Example 22: N-(4-chlorobenzyl)-9-(4-morpholinyhnethyl)-7-oxo-2-(R or S)-{[(3-pyridinylmethyl)amino]methyl}-2,3-dihydro-7H-[1,4]oxazino[2,3,4-ij]quinoline-6-carboxamide Isomer 1 (Formula B-3 of Chart B, where R₁=3-pyridinylmethyl, R₂=H, and X=N)

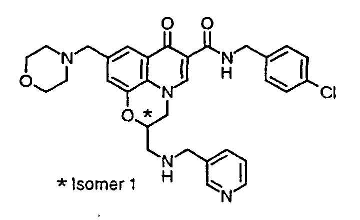

The compound is prepared using the procedures described in Preparation **8** and Example **2**, using resolved isomer 1 of Example **20** and proceeding through the mesylate.
¹H NMR (CDCl₃) δ 2.45, 3.05, 3.09, 3.57, 3.70, 3.91, 4.27, 4.43, 4.63, 7.29, 7.35, 7.70, 7.93, 8.54, 8.60, 8.64, 10.44.
TLC R_{*f*} 0.40 (6% methanolic ammonia in dichloromethane).
MS (ES+) m/z 574.2.

### Example 23: N-(4-Chlorobenzyl)-9-(4-morpholinylmethyl)-7-oxo-2-(R or S)-{[(3-pyridinylmethyl)amino]methyl}-2,3-dihydro-7H-[1,4]oxazino[2,3,4-ij]quinoline-6-carboxamide Isomer 2 (Formula B-3 of Chart B, where R₁=3-pyridinylmethyl, R₂=H, and X=N)

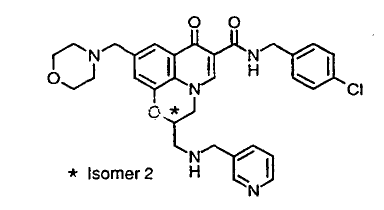

The compound is prepared using the procedures described in Preparation **8** and Example **2**, using resolved isomer 2 of Example **21** and proceeding through the mesylate.
¹H NMR (CDCl₃) δ 2.45, 3.05, 3.09, 3.57, 3.70, 3.91, 4.27, 4.43, 4.63, 7.29, 7.35, 7.70, 7.93, 8.54, 8.60, 8.64, 10.44.
TLC R_{*f*} 0.40 (6% methanolic ammonia in dichloromethane).
MS (ES+) m/z 574.3.

### Example 24: [6-{[(4-Chlorobenzyl)amino]carbonyl}-9-(4-morpholinylmethyl)-7-oxo-2,3-dihydro-7H-[1,4]oxazino[2,3,4-ij]quinolin-2-yl]methyl acetate (Formula C-1 of Chart C)

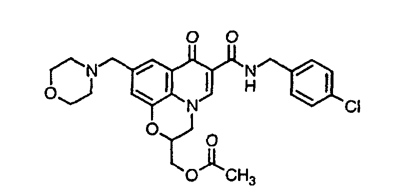

A mixture of 97 mg of the product of Example **1**, 0.19 mL of acetic anhydride, and 1 mL of chloroform is stirred for 18 h, then concentrated under reduced pressure with toluene azeotrope to remove acetic acid. Flash chromatography of the residue on silica using 2-3% methanol in dichloromethane provides 106.5 mg of the title compound as a white solid. An analytical sample is recrystallized from acetonitrile, giving white solid, mp 215-218 °C.
¹H NMR (CDCl3) δ 2.16, 2.46, 3.59, 3.71, 4.22, 4.33, 4.4, 4.56, 4.63, 7.30, 7.40, 7.95, 8.65, 10.41.
TLC R_{*f*} 0.39 (5% methanol in dichloromethane).
IR (drift) 1745, 1652, 1626, 1607, 1558, 1502, 1413, 1347, 1280, 1274, 1223, 1119, 1094, 1043, 808 cm⁻¹
HRMS (FAB) calcd for C₂₇H₂₈CLN₃O₆ +H₁ 526.1744, found 526.1741
Anal. Calcd for C₂₇H₂₈ClN₃O₆: C, 61.65; H, 5.36; N, 7.99; Cl, 6.74; found: C, 61.56; H, 5.44; N, 7.96.

### Example 25: 2-[(8-{[6-{[(4-Chlorobenzyl)amino]carbonyl}-9-(4-morpholinylmethyl)-7-oxo-2,3-dihydro-7H-[1,4]oxazino[2,3,4-ij]quinolin-2-yl]methoxy}-8-oxooctanoyl)(methyl)amino]ethanesulfonic acid sodium salt (Formula C2 of Chart C)

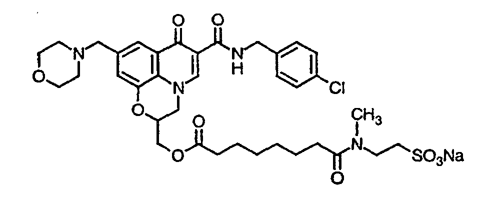

To a mixture of 97 mg of the product of Example **1** and 27 mg of DMAP in 0.5 mL of dry DMF is added 0.46 mL of a 0.65 M solution of suleptanic acid triethylammonium salt, which is 8-[methyl(2-sulfoethyl)amino]-8-oxooctanoic acid triethylammonium salt, in acetonitrile, followed by 38 µL of diisopropylcarbodiimide. After 5 days, volatile components are removed under vacuum, and the residue chromatographed on silica using 3-6% methanolic ammonia in dichloromethane to afford the triethylammonium salt of the product. This is dissolved in *n*-butanol and chloroform, and the solution stirred with an equal volume of saturated aqueous sodium sulfate solution. The aqueous phase is extracted with two additional portions of chloroform, and the combined organic phases filtered through anhydrous sodium sulfate and concentrated under reduced pressure to provide 92.5 mg of the title compound as a solid.
¹H NMR (CDCl3) δ 1.3, 1.6, 2.4, 3.1, 3.6, 3.7, 4.2-4.7, 7.3, 7.9, 8.68, 10.45 ppm.
TLC R_{*f*} 0.21 (10% methanol and 5% methanolic ammonia in dichloromethane).
MS (ES+) m/z 761.3.
MS (FAB) *m*/*z* 761 (MH⁺), 97, 95, 85, 83, 81, 69, 67, 55, 43, 23
HRMS (FAB) calcd for C₃₆H₄₅CLN₄O₁₀S +H₁ 761.2623, found 761.2643.

### Example 26: [6-{[(4-Chlorobenzyl)amino]carbonyl}-9-(4-morpholinylmethyl)-7-oxo-2,3-dihydro-7H-[1,4]oxazino[2,3,4-ij]quinolin-2-yl]methyl dimethyl phosphate (Formula C-3 of Chart C)

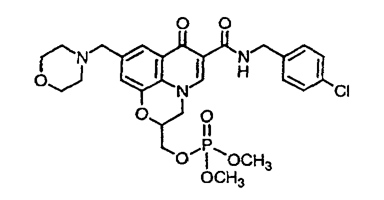

To a stirred mixture of 97 mg of the product of Example **1** in 1 mL of chloroform is added 0.19 mL of phosphorus oxychloride. After the mixture is stirred for 18 h, 15 mL of methanol is added, and the solution is allowed to stand for 3 days. The solution is then concentrated under reduced pressure, and the residue partitioned between chloroform and aqueous NaHCO₃. The aqueous phase is extracted with additional chloroform, and the combined organic phases are dried (Na₂SO₄) and concentrated under reduced pressure. Flash chromatography of the residue on silica using 4% methanol in dichloromethane provides 56.0 mg of the title compound as a solid.
¹H NMR (CDCl3) δ 2.46, 3.58, 3.71, 3.82, 3.86, 4.4, 4.58, 4.63, 7.29, 7.38, 7.95, 8.67, 10.38.
TLC R_{*f*} 0.25 (5% methanol in dichloromethane).
MS (ES+) m/z 592.2.

### Preparation 10: 7-(4-Morpholinylcarbonyl)-2-phenyl-2H-1,4-benzoxazin-3(4H)-one (Formula D-1 of Chart D)

A mixture of 1.11 g of the product of Preparation **2**, 760 mg of potassium carbonate, and 0.86 mL of methyl α-bromophenylacetate in 10 mL of acetone is refluxed for 18 h, then cooled and partitioned between water and dichloromethane. The aqueous phase is extracted with additional dichloromethane, and the combined organic phases dried (Na₂SO₄) and concentrated under reduced pressure. Flash chromatography of the residue on silica using 1.5-3% methanol in dichloromethane provides 1.27 g of the title compound as a solid.
¹H NMR (CDCl₃) δ 3.68, 5.70, 6.85, 7.01, 7.09, 7.33, 7.43, 9.82.
TLC R_{*f*} 0.20 (3% methanol in dichloromethane).
MS (ES+) m/z 339.2

### Preparation 11: 7-(4-Morpholinylmethyl)-2-phenyl-3,4-dihydro-2H-1,4-benzoxazine (Formula D-2 of Chart D)

To a stirred, cooled (0 °C) solution of 1.27 g of the product of Preparation **10** in 15 mL of dry THF under argon is added in portions 290 mg of LAH. The mixture is allowed to warm to room temperature, stirred for 18 h, and then quenched with 0.29 mL of water, 0.29 mL of 3N NaOH, and 0.85 mL of water. The mixture is filtered and the solid washed well with dichloromethane, and the filtrate is concentrated under reduced pressure. Flash chromatography of the residue on silica using 3% methanol in dichloromethane provides 1.00 g of the title compound as a slolid
¹H NMR (CDCl₃) δ 2.43, 3.32, 3.37, 3.46, 3.69, 3.92, 5.05, 6.60, 6.75, 6.89, 7.4.
TLC R_{*f*} 0.33 (5% methanol in dichloromethane).

### Preparation 12: Diethyl 2-{[7-(4-morpholinylmethyl)-2-phenyl-2,3-dihydro-4H-1,4-benzoxazin-4-yl]methylene}malonate (Formula D-3 of Chart D)

A mixture of 1.00 g of the product of Preparation **11** and 0.80 g of diethyl ethoxymethylenemalonate is heated under argon at 140 °C for 1 h, then under vacuum for 20 m. The mixture is cooled and chromatographed on silica gel using 2-3% methanol in dichloromethane to provide 1.32 g of the title compound as a solid.
¹H NMR (CDCl₃) δ 1.19, 1.31, 2.46, 3.46, 3.56, 3.64, 3.72, 4.16, 4.25, 5.05, 6.97, 7.04, 7.07, 7.4, 8.04.
TLC R_{*f*} 0.38 (5% methanol in dichloromethane).
MS (ES+) m/z 481.3.

### Preparation 13: Ethyl 9-(4-morpholinylmethyl)-7-oxo-2-phenyl-2,3-dihydro-7H-[1,4]oxazino[2,3,4-ij]quinoline-6-carboxylate (Formula D-4 of Chart D)

An intimate mixture of 1.32 g of the product of Preparation **12**, 7.0 g of polyphosphoric acid, and 4 mL of toluene is heated at 90 °C for 18 h, then transferred using a spatula to a stirred slurry of 8 g of NaHCO₃ in 100 mL of water. The mixture is extracted with three portions of dichloromethane, and the combined organic phases dried (Na₂SO₄) and concentrated under reduced pressure. Flash chromatography of the residue on silica using 3% methanol in dichloromethane affords 960 mg of the title compound as an orange solid. An analytical sample is recrystallized from acetonitrile, giving white solid, mp 213-215 °C.
¹H NMR (CDCl₃) δ 1.40, 2.47, 3.59, 3.70, 4.24, 4.30, 4.37, 5.31, 7.4-7.5, 7.98, 8.31.
TLC R_{*f*} 0.27 (5% methanol in dichloromethane).
HRMS (FAB) calcd for C₂₅H₂₆N₂O₅ +H₁ 435.1920, found 435.1923
Anal. Calcd for C₂₅H₂₆N₂O₅: C, 69.11; H, 6.03; N, 6.45; found: C, 69.06; H, 6.13; N, 6.54.

### Example 27: N-(4-Chlorobenzyl)-9-(4-morpholinylmethyl)-7-oxo-2-phenyl-2,3-dihydro-7H-[1,4]oxazino[2,3,4-ij]quinoline-6-carboxamide (Formula D-5 of Chart D)

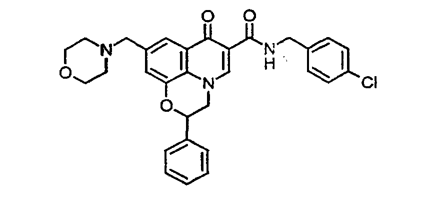

A mixture of 219 mg of the product of Preparation **13** and 0.5 g of *p*-chlorobenzylamine is heated at 150 °C under nitrogen for 18 h, after which time excess amine is removed *in vacuo*. Flash chromatography of the residue on silica using 1.5-3% methanol in dichloromethane provides 251 mg of the title compound.
¹H NMR (CDCl₃) δ 2.48, 3.62, 3.71, 4.30, 4.40, 4.61, 5.30, 7.29, 7.49, 7.98, 8.68, 10.45.
TLC R_{*f*} 0.28 (3% methanol in dichloromethane).
MS (ES+) m/z 530.3.

### Preparation 14: 2,2-Difluoro-7-(4-morpholinylcarbonyl)-2H-1,4-benzoxazin-3(4H)-one (Formula E-1 of Chart E)

To a cold (0 °C), stirred slurry of 1.11 g of the product of Preparation **2** in 5 mL of dry DMF under argon is added in portions 200 mg of sodium hydride (60% oil dispersion). The mixture is stirred for 20 m at 0 °C, then 0.63 mL of ethyl bromodifluoroacetate is added dropwise. After 15 m, the mixture is allowed to warm to room temperature, and after 1 h is heated to 90 °C. After 3 h, the mixture is cooled and partitioned between ethyl acetate and water, and the aqueous phase is extracted with two additional portions of ethyl acetate. The combined organic phases are washed with water and brine, dried (MgSO₄), and concentrated under reduced pressure. Flash chromatography of the residue on silica using 2.5-3.5% methanol in dichloromethane affords 1.05 g of yellow solid. This is triturated well with 10 mL of 1:1 ethyl acetate-hexane and the solid filtered, washed with 1:1 ethyl acetate-hexane, and dried under vacuum to provide 824 mg of the title compound as an off-white solid. The solid is further purified by recrystallization from ethyl acetate-hexane, mp 214-217 °C.
¹H NMR (CDCl₃ + CD₃OD) δ 3.74, 7.09, 7.22, 7.25.
TLC R_{*f*} 0.27 (5% methanol in dichloromethane).
IR (drift) 1739, 1617, 1586, 1464, 1433, 1288, 1265, 1256, 1228, 1217, 1117, 1111, 1070, 824, 758 cm⁻¹
HRMS (FAB) calcd for C₁₃H₁₂F₂N₂O₄+H₁ 299.0843, found 299.0836
Anal. Calcd for C₁₃H₁₂F₂N₂O₄: C, 52.35; H, 4.05; N, 9.39; found: C, 52.09; H, 4.07; N, 9.47.

### Preparation 15: 2,2-Difluoro-7-(4-morpholinylmethyl)-3,4-dithydro-2H-1,4-benzoxazine (Formula E-2 of Chart E)

To a cold (0 °C), stirred solution of 805 mg of the product of Preparation **14** in 15 mL of dry THF, under argon, is added in portions 205 mg of LAH. The mixture is allowed to warm to room temperature and stirred overnight, then quenched cautiously with 0.2 mL of water, 0.2 mL of 3N NaOH, and 0.6 mL of water. Dichloromethane and Na₂SO₄ are added, and the mixture is filtered and the solid washed with dichloromethane. The filtrate is concentrated under reduced pressure, and the residue chromatographed on silica using 2% methanol in dichloromethane to afford 423 mg of the title compound as a solid.
¹H NMR (CDCl₃) δ 2.41, 3.38, 3.50, 3.69, 4.02, 6.69, 6.88, 6.94.
TLC R_{*f*} 0.34 (5% methanol in dichloromethane).
IR (drift) 3309, 1526, 1354, 1320, 1312, 1266, 1250, 1216, 1188, 1108, 999, 964, 885, 856, 793 cm⁻¹
HRMS (FAB) calcd for C₁₃H₁₆F₂N₂O₂ +H₁ 271.1258, found 271.1264.

### Preparation 16: Diethyl 2-{[2,2-difluoro-7-(4-morpholinylmethyl)-2,3-dihydro-4H-1,4-benzoxazin-4-yl]methylene}malonate (Formula E-3 of Chart E)

A mixture of 414 mg of the product of Preparation **15** and 870 mg of diethyl ethoxymethylenemalonate is heated under argon at 150 °C for 90 m, then cooled and chromatographed on silica using 2% methanol in dichloromethane to provide 739 mg of the title compound as a thick yellow oil.
¹H NMR (CDCl₃) δ 1.31, 1.34, 2.44, 3.47, 3.71, 3.78, 4.26, 4.30, 7.1, 7.91.
TLC R_{*f*} 0.31 (3% methanol in dichloromethane).
HRMS (FAB) calcd for C₂₁H₂₆F₂N₂O₆ +H₁ 441.1837, found 441.1844.

### Preparation 17: Ethyl 2,2-difluoro-9-(4-morpholinylmethyl)-7-oxo-2,3-dihydro-7H-[1,4]oxazino[2,3,4-ij]quinoline-6-carboxylate (Formula E-4 of Chart E)

An intimate mixture of 675 mg of the product of Preparation **15** and 4.0 g of polyphosphoric acid is heated at 90 °C under argon, with occasional mechanical stirring, for 90 m, then cooled and added to a stirred slurry of 5 g of NaHCO₃ in 50 mL of water. The mixture is extracted three times with dichloromethane, and the combined organic phases dried (MgSO₄) and concentrated under reduced pressure. Flash chromatography of the residue on silica using 2-3% methanol in dichloromethane provides 477 mg of the title compound. An analytical sample is recrystallized from ethyl acetate-hexane, giving the titled compound as crystals,
mp176-177°C.
¹H NMR (CDCl₃) δ 1.37, 2.45, 3.58, 3.71, 4.32, 4.56, 7.46, 8.02, 8.35.
TLC R_{*f*} 0.34 (5% methanol in dichloromethane).
IR (drift) 1678, 1614, 1560, 1510, 1325, 1285, 1261, 1242, 1228, 1201, 1191, 1112, 943, 847, 809 cm⁻¹
HRMS (FAB) calcd for C₁₉H₂₀F₂N₂O₅ +H₁ 395.1418, found 395.1421.
Anal. Calcd for C₁₉H₂₀F₂N₂O₅: C, 57.87; H, 5.11; N, 7.10; found: C, 57.67; H, 5.16; N, 7.10.

### Example 28: N-(4-Chlorobenzyl)-2,2-difluoro-9-(4-morpholinylmethyl)-7-oxo-2,3-dihydro-7H-[1,4]oxazino[2,3,4-ij]quinoline-6-carboxamide (Formula E-5 of Chart 5)

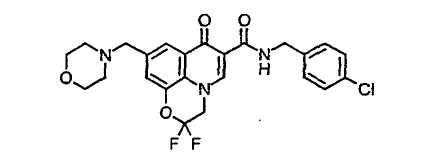

A mixture of 118 mg of the product of Preparation **17** and 0.21 g of *p*-chlorobenzylamine is heated at 160 °C for 18 h, then placed under vacuum to remove excess amine. Chromatography of the residue on silica using 2-3% methanol in dichloromethane affords 135.8 mg of product as a yellow solid. Recrystallization from acetonitrile provides 111 mg of the title compound as white needles, mp 213.5-216.5 °C.
¹H NMR (CDCl₃) δ 2.46, 3.61, 3.72, 4.57, 4.63, 7.29, 7.54, 8.08, 8.76, 10.26.
TLC R_{*f*} 0.25 (3% methanol in dichloromethane).
IR (drift) 1658, 1611, 1582, 1562, 1508, 1493, 1327, 1285, 1258, 1243, 1229, 1192, 1126, 1116, 808 cm⁻¹
HRMS (FAB) calcd for C₂₄H₂₂CLF₂N₃O₄ +H₁ 490.1345, found 490.1343
Anal. Calcd for C₂₄H₂₂ClF₂N₃O₄: C, 58.84; H, 4.53; N, 8.58; Cl, 7.24; found: C, 58.78; H, 4.56; N, 8.52.

### Preparation 18: 2-[5-(4-Morpholinylcarbonyl)-2-nitrophenoxy]-1-phenylethanone (Formula F-1 of Chart F)

A mixture of 1.01 g of the product of Preparation **1**, 800 mg of phenacyl bromide, 1.11 g of potassium carbonate, and 20 mg of tetrabutylammonium bisulfate in 4.0 mL of dichloromethane and 3 mL of water is stirred vigorously at room temperature for 18 h. The phases are then separated, and the aqueous phase extracted with one additional portion of dichloromethane. The combined organic phases are dried (Na₂SO₄) and concentrated under reduced pressure. Flash chromatography of the residue on silica using 50% ethyl acetate in dichloromethane affords 1.465 g of the title compound as a white solid. An analytical sample is prepared by recrystallization from ethyl acetate-hexane, giving white crystals, mp 171.5-173 °C.
¹H NMR (CDCl₃) δ 3.35, 3.6, 5.52, 6.97, 7.08, 7.53, 7.66, 7.90, 7.96.
TLC R_{*f*} 0.30 (50% ethyl acetate in dichloromethane).
HRMS (FAB) calcd for C₁₉H₁₈N₂O₆ +H₁ 371.1243, found 371.1241
Anal. Calcd for C₁₉H₁₈N₂O₆: C, 61.62; H, 4.90; N, 7.56; found: C, 61.52; H, 4.90; N, 7.51.

### Preparation 19: 7-(4-morpholinylcarbonyl)-3-phenyl-2H-1,4-benzoxazine (Formula F-2 of Chart F)

To a solution of 3.13 g of stannous chloride dihydrate.in 12 mL of ethanol is added 1.026 g of the product of Preparation **18**, and the mixture is stirred and heated at 60 °C for 30 m. Dilute aqueous NaOH is added in sufficient quantity to dissolve the precipitate which initially forms, and the mixture is extracted thrice with dichloromethane. The combined organic phases are dried (Na₂SO₄) and concentrated under reduced pressure, and the residue chromatographed on silica using 50-75% ethyl acetate in dichloromethane to provide 622 mg of the title compound as a pale yellow crystalline solid. Recrystallization from ethyl acetate-hexane gives white needles, mp 175-177 °C.
¹H NMR (CDCl₃) δ 3.7, 5.09, 6.98, 7.05, 7.5, 7.94.
TLC R_{*f*} 0.32 (50% ethyl acetate in dichloromethane).
IR 2856, 1633, 1568, 1458, 1432, 1280, 1244, 1114, 1025, 732, 691 cm⁻¹.
HRMS (FAB) calcd for C₁₉H₁₈N₂O₃ +NA₁ 345.1215, found 345.1212
Anal. Calcd for C₁₉H₁₈N₂O₃: C, 70.79; H, 5.63; N, 8.69; found: C, 70.63; H, 5.77; N, 8.57.

### Preparation 20: 7-(4-Morpholinylcarbonyl)-3-phenyl-3,4-dihydro-2H-1,4-benzoxazine (Formula F-3 of Chart F)

A mixture of 586 mg of the product of Preparation **19** and 138 mg of sodium borohydride in 10 mL of ethanol is stirred at room temperature for 18 h, then added to 100 mL of stirred water. The resulting solid is filtered, washed with water, and dried under vacuum to give 401 mg of white solid. The aqueous phases are extracted thrice with dichloromethane, and the combined organic phases dried (Na₂SO₄) and concentrated under reduced pressure to provide an additional 203 mg of solid. The combined solids are chromatographed on silica using 50-75% ethyl acetate in dichloromethane to afford 510 mg of the title compound as a solid.
¹H NMR (CDCl₃) δ 3.68, 3.98, 4.30, 4.52, 6.66, 6.93, 6.94, 7.4.
TLC R_{*f*} 0.32 (50% ethyl acetate in dichloromethane).
IR 3306, 2856, 1612, 1456, 1430, 1303, 1284, 1243, 1114, 1022, 730, 701 cm⁻¹.
MS (ES+) m/z 325.2.

### Preparation 21: 7-(4-Morpholinylmethyl)-3-phenyl-3,4-dihydro-2H-1,4-benzoxazine (Formula F-4 of Chart F)

To a cold (0 °C), stirred solution of 576 mg of the product of Preparation **20** in 5.0 mL of dry THF is added in portions 135 mg of LAH. The mixture is allowed to warm to room temperature and stirred for 3 h, then quenched cautiously with 0.16 mL of water, 0.16 mL of 3N NaOH, and 0.48 mL of water. Dichloromethane and Na₂SO₄ are added, the mixture is filtered, and the solid washed with additional dichloromethane. The filtrates are concentrated under reduced pressure, and the residue flash chromatographed on silica using ethyl acetate and 3% methanol in ethyl acetate to afford 389 mg of white crystalline solid. An analytical sample is recrystallized from ethyl acetate-hexane to give the title compound as white crystals, mp 135.5-136.5 °C.
¹H NMR (CDCl₃) δ 2.44, 3.38, 3.70, 3.98, 4.0, 4.27, 4.49, 6.61, 6.75, 6.82, 7.4.
TLC R_{*f*} 0.28 (ethyl acetate).
IR 3348, 2806, 1516, 1454, 1292, 1116, 1006, 701 cm⁻¹.
HRMS (FAB) calcd for C₁₉H₂₂N₂O₂ +H₁ 311.1759, found 311.1761
Anal. Calcd for C₁₉H₂₂N₂O₂: C, 73.52; H, 7.14; N, 9.02; found: C, 73.38; H, 7.19; N, 8.93.

### Preparation 22: Diethyl 2-{[7-(4-morpholinylmethyl)-3-phenyl-2,3-dihydro-4H-1,4-benzoxazin-4-yl]methylene}malonate (Formula F-5 of Chart F)

A mixture of 378 mg of the product of Preparation **21** and 350 mg of diethyl ethoxymethylenemalonate is heated at 140 °C under argon for 2 h. Additional malonate *(ca.* 150 mg) is added, and the mixture heated at 120 °C for 18 h. Flash chromatography of the residue on silica using ethyl acetate provides 509 mg of the title compound as a solid.
¹H NMR (CDCl₃) δ 0.90, 1.26, 2.42, 3.43, 3.70, 3.9-4.2, 4.32, 4.50, 5.27, 6.83, 6.99, 7.1-7.3,8.11.
IR 2980, 2808, 1708, 1598, 1509, 1249, 1219, 1196, 1117, 1071 cm⁻¹.
HRMS (FAB) calcd for C₂₇H₃₂N₂O₆ +H₁ 481.2338, found 481.2344.

### Preparation 23: Ethyl 9-(4-morpholinylmethyl)-7-oxo-3-phenyl-2,3-dihydro-7H-[1,4]oxazino[2,3,4-ij]quinoline-6-carboxylate (Formula F-6 of Chart 6)

An intimate mixture of 509 mg of the product of Preparation **22** and 2.4 g of polyphosphoric acid is heated at 90 °C, with occasional mechanical stirring, for 18 h. The resulting gum is added to a stirred slurry of 2.5 g of Na₂HCO₃ in 50 mL of water, and the mixture is extracted with three portions of dichloromethane. The combined organic phases are dried (MgSO₄) and concentrated under reduced pressure. Flash chromatography of the residue on silica using 3-5% methanol in dichloromethane affords 375 mg of solid. Recrystallization from acetonitrile-water provides 184 mg of white crystals, mp >190 °C.
¹H NMR (CDCl₃) δ 1.35, 2.47, 3.59, 3.71, 4.33, 4.56, 5.30, 7.15, 7.33, 7.4, 8.03, 8.23..
TLC R_{*f*} 0.25 (5% methanol in dichloromethane).
IR 2809, 1723, 1691, 1608, 1556, 1500, 1315, 1226, 1116, 1073, 808, 730 cm⁻¹.
HRMS (FAB) calcd for C₂₅H₂₆N₂O₅ +H₁ 435.1920, found 435.1920
Anal. Calcd for C₂₅H₂₆N₂O₅: C, 69.11; H, 6.03; N, 6.45; found: C, 68.87; H, 6.19; N, 6.32.

### Example 29: N-(4-Chlorobenzyl)-9-(4-morpholinylmethyl)-7-oxo-3-phenyl-2,3-dihydro-7H-[1,4]oxazino[2,3,4-ij]quinoline-6-carboxamide (Formula F-7 of Chart F)

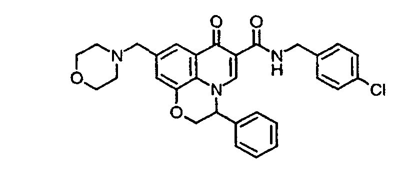

A mixture of 103 mg of the product of Preparation **23** and 200 mg of *p*-chlorobenzylamine is heated at 150 °C for 18 h, then placed under vacuum to remove excess amine. Flash chromatography of the residue on silica using 2% methanol in dichloromethane affords 125.3 mg of the title compound as a white solid. An analytical sample is recrystallized from toluene to give the title compound as white crystals.
¹H NMR (CDCl₃) δ 2.48, 3.61, 3.72, 4.58, 4.59, 5.37, 7.13, 7.28, 7.40, 8.02, 8.59, 10.44. TLC R_{*f*} 0.28 (3% methanol in dichloromethane).
HRMS (FAB) calcd for C₃₀H₂₈CLN₃O₄ +H₁ 530.1846, found 530.1841
Anal. Calcd for C₃₀H₂₈ClN₃O₄: C, 67.98; H, 5.32; N, 7.93; Cl, 6.69; found: C, 68.12; H, 5.38; N, 7.71.

### Preparation 24: 5-(4-Morpholinylcarbonyl)-2-nitrophenyl 2-oxiranylmethyl ether (Formula G-1 of Chart G)

A mixture of 504 mg of the product of Preparation **1** and 120 mg of powdered NaOH in 2 mL of epichlorohydrin is heated at 120 °C for 18 h, then cooled and partitioned between dil aqueous HCl and ethyl acetate. The organic phase is washed with brine, dried (MgSO₄) and concentrated under reduced pressure. Flash chromatography of the residue on silica using 2% methanol in dichloromethane provides 496 mg of the title compound. Recrystallization from ethyl acetate-hexane gives the title compound as solid, mp 156.5-158.0 °C.
¹H NMR (CDCl₃) δ 2.85, 2.93, 3.39, 3.7, 4.13, 4.49, 7.06, 7.21, 7.87
TLC R_{*f*} 0.47 (5% methanol in dichloromethane).
IR 2859, 1637, 1607, 1522, 1437, 1288, 1240, 1114, 1016, 842 cm⁻¹.
HRMS (FAB) calcd for C₁₄H₁₆N₂O₆ +NA₁ 331.0906, found 331.0893
Anal. Calcd for C₁₄H₁₆N₂O₆: C, 54.54; H, 5.23; N, 9.09; found: C, 54.16; H, 5.25; N, 8.92.

### Preparation 25: 1-(4-Morpholinyl)-3-[5-(4-morpholinylcarbonyl)-2-nitrophenoxy]-2-propanol (Formula G-2 of Chart G)

A solution of 442 mg of the product of Preparation **24** and 0.25 mL of morpholine in 2 mL of methanol is refluxed for 18 h, then concentrated under reduced pressure. Flash chromatography of the residue on silica using 2-5% methanol in dichloromethane provides 546 mg of the title compound as a solid.
¹H NMR (CDCl₃) δ 2.6, 3.41, 3.7, 4.15, 4.23, 7.05, 7.21, 7.89.
TLC R_{*f*} 0.19 (5% methanol in dichloromethane).
IR 3425, 2856, 1637, 1607, 1523, 1437, 1288, 1242, 1115, 1031, 842 cm⁻¹.
MS (ES+) m/z 396.3.

### Preparation 26: 7-(4-Morpholinylcarbonyl)-3-(4-morpholinylmethyl)-3,4-dihydro-2H-1,4-benzoxazine (Formula G-3 of Chart G)

To a cold (-78 °C), stirred solution of 0.17 mL of DMSO in 2 mL of dichloromethane, under argon, is added dropwise 0.29 mL of trifluoroacetic anhydride. The solution is stirred 15 m, then a solution of 546 mg of the product of Preparation **25** in 1 mL of dichloromethane is added *via* cannula. The solution is warmed to -30 °C and stirred at that temperature for 30 m, then 0.96 mL of diisopropylethylamine is added. The solution is warmed to room temperature and partitioned between dichloromethane and water containing 2.0 mL of 1N HCl. The aqueous phase is extracted with two additional portions of dichloromethane, and the combined organic phases dried (Na₂SO₄), diluted with 40 mL of ethanol, and concentrated under reduced pressure to *ca.* 5 mL volume. To this solution is added Raney 2800 nickel catalyst, which is isolated from 3 mL of commercially available aqueous slurry by filtration and washing with ethanol with minimal exposure to air. The mixture is shaken under 50 psi hydrogen gas for 18 h, then filtered through Celite with dichloromethane and methanol rinses. The filtrate is concentrated under reduced pressure, and the residue chromatographed on silica using 2-4% methanol in dichloromethane to provide 264 mg of the title compound as a white crystalline solid. Recrystallization from ethyl acetate in hexane gives the title compound as white crystals, mp 125-126 °C.
¹H NMR (CDCl₃) δ 2.37, 2.60, 3.5-3.9, 3.84, 4.21, 4.72, 6.59, 6.88.
TLC R_{*f*} 0.36 (5% methanol in dichloromethane).
HRMS (FAB) calcd for C₁₈H₂₅N₃O₄ +H₁ 348.1923, found 348.1921
Anal. Calcd for C₁₈H₂₅N₃O₄: C, 62.23; H, 7.25; N, 12.10; found: C, 62.21; H, 7.22; N, 11.97.

### Preparation 27: 3,7-bis(4-Morpholinylmethyl)-3,4-dihydro-2H-1,4-benzoxazine (Formula G-4 of Chart G)

To a cold (0 °C), stirred solution of 237 mg of the product of Preparation **26** in 2.0 mL of dry THF, under argon, is added 52 mg of LAH. The mixture is allowed to warm to room temperature and stirred for 30 m, then quenched cautiously with 50 µL of water, 50 µL of 3N NaOH, and 0.15 mL of water. Dichloromethane and Na₂SO₄ are added, and the mixture filtered through Celite. The filtrate is concentrated under reduced pressure, and the residue flash chromatographed on silica using 2-6% methanol in dichloromethane to afford 211 mg of the title compound as a solid.
¹H NMR (CDCl₃) δ 2.40, 2.59, 3.36, 3.55, 3.72, 3.86, 4.21, 4.4, 6.58, 6.76.
TLC R_{*f*} 0.31 (5% methanol in dichloromethane).
IR 3355, 2808, 1517, 1290, 1116, 1005, 865 cm⁻¹.
MS (ES+) m/z 334.3.

### Preparation 28: Ethyl 3,9-bis(4-morpholinylmethyl)-7-oxo-2,3-dihydro-7H-[1,4]oxazino[2,3,4-ij]quinoline-6-carboxylate (Formula G-5 of Chart G)

A mixture of 211 mg of the product of Preparation **27** and 274 mg of diethyl ethoxymethylenemalonate is heated under argon at 140 °C for 2 h, then cooled and chromatographed on silica using 2-4% methanol in dichloromethane to provide 230 mg of the intermediate enamide, which is diethyl 2-{[3,7-bis(4-morpholinylmethyl)-2,3-dihydro-4H-1,4-benzoxazin-4-yl]methylene}malonate, as an orange foam. This is mixed thoroughly with 1.5 g of polyphosphoric acid, and the mixture is heated at 90 °C for 18 h, then cooled and added to excess aqueous NaHCO₃. The mixture is extracted thrice with dichloromethane, and the organic phases dried (Na₂SO₄) and concentrated under reduced pressure. Flash chromatography of the residue on silica using 2-5% methanol in dichloromethane affords 117 mg of the title compound.
¹H NMR (CDCl₃) δ 1.41, 2.44, 2.67, 3.55, 3.69, 4.22, 4.40, 4.57, 7.27, 7.95, 8.37.
TLC R_{*f*} 0.25 (5% methanol in dichloromethane).
IR 2811, 1722, 1600, 1555, 1502, 1319, 1292, 1227, 1116, 912, 867, 731 cm⁻¹.
MS (ES+) m/z 458.3.

### Example 30: N-(4-Chlorobenzyl)-3,9-bis(4-morpholinylmethyl)-7-oxo-2,3-dihydro-7H-[1,4]oxazino[2,3,4-ij]quinoline-6-carboxamide (Formula G-6 of Chart G)

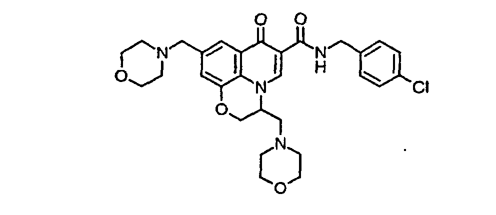

A mixture of 117 mg of the product of Preparation **28** and 0.20 g of *p*-chlorobenzylamine is heated at 150 °C for 18 h, then excess amine is removed under vacuum. Flash chromatography of the residue on silica using 2-4% methanol in dichloromethane affords 11.6 mg of the title compound.
¹H NMR (CDCl₃) δ 2.41, 2.63, 2.77, 3.57, 3.69, 4.31, 4.66, 7.30, 7.33, 7.96, 8.68 (s, 1H), 10.46.
TLC R_{*f*} 0.31 (5% methanol in dichloromethane).
IR 2812, 1658, 1608, 1551, 1500, 1286, 1116, 867, 809, 730 cm⁻¹.
HRMS (FAB) calcd for C₂₉H₃₃CLN₄O₅ +H₁ 553.2217, found 553.2213.

### Preparation 29: 2,3-Difluoro-5-iodobenzoic acid (Formula H-2 of Chart H)

To a cold (0 °C), stirred solution of 4.74 g of 2,3-difluorobenzoic acid in 15.0 mL of trifluoromethanesulfonic acid is added in portions 8.1 g of powdered *N*-iodosuccinimide. Following the addition, the mixture is allowed to warm to room temperature, stirred for 5 h, then poured onto 200 mL of cracked ice containing 5 g of sodium bisulfite. The mixture is stirred well for 15 m, then filtered, and the solid washed well with water and dried under vacuum to provide 6.78 g of the title compound as a solid.
¹H NMR (CDCl₃) δ 7.81, 8.09.
IR 3082, 1713, 1473, 1280 cm⁻¹.
MS (ES-) *m*/*z* 282.8.

### Preparation 30: Ethyl 3-(2,3-difluoro-5-iodophenyl)-3-oxopropanoate (Formula H-3 of Chart H)

To a stirred solution of 2.02 g of the product of Preparation **29** in 10 mL of dry THF, under argon, is added 1.42 g of carbonyldiimidazole and 20 mg of DMAP. In a separate flask, 1.49 g of ethyl potassium malonate is suspended in 10 mL of CH₃CN at 0 °C under argon, and to the slurry is added 1.0 mL of chlorotrimethylsilane. The mixture is stirred at ambient temperature for 18 h, then re-cooled to 0 °C. To the mixture is added 2.3 mL of DBU, and after 30 min the acyl imidazolide solution prepared above is added via cannula. The mixture is stirred for 4 h at room temperature, then partitioned between ether and excess dilute HCl. The aqueous phase is back extracted with one additional portion of ether, and the combined organic phases washed with 0.1 N HCl and brine, dried (MgSO₄), and concentrated under reduced pressure. Flash chromatography of the residue on silica using 5% EtOAc in heptane provides 2.13 g of the title compound as a white solid.
¹NMR (CDCl₃) δ 1.34, 4.26, 5.81, 7.57, 7.95.
TLC R_{*f*} 0.44 (10% EtOAc in hexane).
IR 1622, 1490, 1420, 1213, 1036, 958, 800 cm⁻¹.
MS (ES-) *m*/*z* 352.9.

### Preparation 31: Ethyl (2-E and Z)-2-(2,3-difluoro-5-iodobenzoyl)-3-{[2-hydroxy-1,1-bis(hydroxymethyl)ethyl]amino}-2-propenoate (Formula H-4 of Chart H)

A solution of 2.12 g of the product of Preparation **30** and 1.5 mL of triethyl orthoformate in 6 mL of acetic anhydride is refluxed under nitrogen for 2 h, then concentrated under reduced pressure to a viscous oil. This is dissolved in 6 mL of ethanol, and 1.45 g of tris(hydroxymethyl) aminomethane is added. The solution is stirred at room temperature for 18 h, then concentrated under reduced pressure. Flash chromatography of the residue on silica using 5-7% methanol in dichloromethane affords 2.77 g of the title compound as a tan foam.
¹NMR (CDCl₃) (*E*/*Z* mixture of isomers) δ 0.87, 0.93, 3.78, 3.98, 4.23, 7.35, 7.49, 8.46, 10.09, 11.33.
TLC R_{*f*} 0.32 (10% methanol in dichloromethane).
IR 3400, 1670, 1622, 1481, 1430, 1323, 1296, 1274, 1256, 1222, 1056, 912, 733 cm⁻¹.
HRMS (FAB) calcd for C₁₆H₁₈F₂INO₆ +H₁ 486.0227, found 486.0240.

### Preparation 32: Ethyl 3,3-bis(hydroxymethyl)-9-iodo-7-oxo-2,3-dihydro-7H-[1,4]oxazino[2,3,4-ij]quinoline-6-carboxylate (Formula H-5 of Chart H)

A mixture of 2.77 g of the product of Preparation **31** and 1.74 g of potassium carbonate in 20 mL of DMF is heated at 130 °C under argon for 2 days, after which DMF is distilled off under reduced pressure. To the brown residue is added water, and the mixture is stirred for 20 m then filtered. The solid is washed with water, ethyl acetate, and ether, and dried under vacuum to provide 1.76 g of the title compound as a solid.
¹NMR (CDCl₃ + TFA) δ1.48, 4.24, 4.42, 4.60, 4.63, 8.03, 8.62, 9.49.
HRMS (FAB) calcd for C₁₆H₁₆INO₆ +H₁ 446.0102, found 446.0100.

### Preparation 33: N-(4-Chlorobenzyl)-3,3-bis(hydroxymethyl)-9-iodo-7-oxo-2,3-dihydro-7H-[1,4]oxazino[2,3,4-ij]quinoline-6-carboxamide (Formula H-6 of Chart H)

A mixture of 1.69 g of the product of Preparation **32** and 3.2 g of *p*-chlorobenzylamine is heated under argon at 160 °C for 18 h, then cooled and triturated with dichloromethane. The resulting solid is filtered, washed with dichloromethane, and dried under vacuum to provide 1.50 g of the title compound as a solid.
¹NMR (CDCl₃ + TFA) δ 4.12, 4.35, 4.56, 4.64, 7.3, 7.91, 8.54, 9.25, 9.4.
MS (ES+) m/z 541.0.

### Preparation 34: 3,3-bis({[tert-Butyl(dimethyl)silyl]oxy}methyl)-N-(4-chlorobenzyl)-9-iodo-7-oxo-2,3-dihydro-7H-[1,4]oxazino[2,3,4-ij]quinaline-6-carboxamide (Formula H-7 of Chart H)

A mixture of 1.49 g of the product of Preparation **33**, 661 mg of imidazole, and 1.25 g of *tert*-butyldimethylsilyl chloride in 6 mL of DMF is stirred at room temperature for 18 h, then partitioned between ethyl acetate and aqueous NaHCO₃. The organic phase is washed with water and brine, dried (Na₂SO₄), and concentrated under reduced pressure. Flash chromatography of the residue on silica using 20% ethyl acetate in heptane affords 1.72 g of the title compound as a white solid. An analytical sample is recrystallized from methanol to give the title compound as a solid, mp 157.5-159.0 °C.
¹NMR (CDCl₃) δ 0.00 (used as reference peak), 0.03, 0.82, 3.94, 4.02, 4.37, 4.92, 7.26, 7.53, 8.39, 8.81, 10.27.
TLC R_{*f*} 0.38 (20% ethyl acetate in hexane).
IR (drift) 2953, 2930, 2856, 1670, 1572, 1543, 1483, 1467, 1252, 1122, 1088, 841, 837, 800, 776 cm⁻¹
HRMS (FAB) calcd for C₃₃H₄₆CLIN₂O₅Si₂ +H₁ 769.1758, found 769.1748
Anal. Calcd for C₃₃H₄₆ClIN₂O₅Si₂: C, 51.52; H, 6.03; N, 3.64; Cl, 4.61; found (av): C, 51.14; H, 6.06; N, 3.59.

### Preparation 35: 3,3-bis({[tert-Butyl(dimethyl)silyl]oxy}methyl)-N-(4-chlorobenzyl)-9-(3-hydroxy-1-propynyl)-7-oxo-2,3-dihydro-7H-[1,4]oxazino[2,3,4-ij]quinoline-6-carboxamide (Formula H-8 of Chart H)

To a mixture of 385 mg of the product of Preparation **34**, 70 mg of copper (I) iodide, and 18 mg of bis(triphenylphosphine)palladium (II) dichloride is added 40 µL of propargyl alcohol in 4 mL of diethylamine. The mixture is stirred overnight at room temperature, becoming a clear red solution. Volatile components are removed under reduced pressure and the residue flash chromatographed on silica using 10-15% ethyl acetate in dichloromethane to provide 333 mg of the title compound as a solid.
¹NMR (CDCl₃) δ 0.00 (used as reference peak), 0.03, 0.82, 4.02, 4.38, 4.43, 4.63, 7.02, 7.3, 7.78, 8.73, 10.34.
TLC R_{*f*} 0.35 (10% ethyl acetate in dichloromethane).
MS (ES+) m/z 697.3.

### Example 31: N-(4-Chlorobenzyl)-3,3-bis(hydroxymethyl)-9-(3-hydroxy-1-propynyl)-7-oxo-2,3-dihydro-7H-[1,4]oxazino[2,3,4-ij]quinoline-6-carboxamide (Formula H-9 of Chart H)

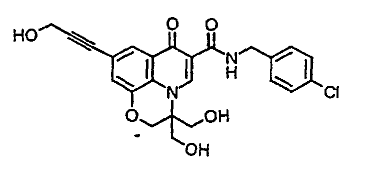

A mixture of 52 mg of the product of Preparation **35**, 0.5 mL of concentrated hydrochloric acid, and 1 mL of ethanol is stirred at room temperature for 3 days, then added to 10 mL of rapidly stirred water. The resulting solid is filtered, washed with water, and dried under vacuum to provide 34 mg of buff solid. Chromatography of this material on silica using 5-10% methanol in dichloromethane affords 29.4 mg of the title compound as a solid.
¹NMR (CDCl₃+CD₃OD) δ 3.88, 4.09, 4.45, 4.63, 7.28, 7.31, 8.03, 8.92.
TLC R_{*f*} 0.38 (10% methanol in dichloromethane).
MS (ES+) m/z 469.0.

### Preparation 36: 3,3-bis({[tert-Butyl(dimethyl)silyl]oxy}methyl)-N-(4-chlorobenzyl)-9-(3-hydroxypropyl)-7-oxo-2,3-dihydro-7H-[1,4]oxazino[2,3,4-ij]quinoline-6-carboxamide (Formula H-10 of Chart H)

A mixture of 175 mg of the product of Preparation **35** and 70 mg of 5% platinum on carbon in 6 mL of ethyl acetate is shaken under 40 psi H₂ for 5 h, then filtered through Celite. The filtrate is concentrated under reduced pressure and the residue chromatographed on silica using 30% ethyl acetate in dichloromethane to provide 142 mg of the title compound as a pale yellow solid. An analytical sample is recrystallized from ethanol to give the title compound as white needles.

Physical properties are as follows:
¹NMR (CDCl₃) δ 0.00 (used as reference peak), 0.03, 0.83, 1.94, 2.82, 3.68, 3.97, 4.02, 4.37, 4.63, 7.11, 7.3, 7.90, 8.80, 10.43.
TLC R_{*f*} 0.32 (30% ethyl acetate in dichloromethane).
IR (drift) 2951, 2930, 2858, 1662, 1604, 1572, 1554, 1491, 1253, 1115, 1092, 1064, 840, 802, 780 cm⁻¹
HRMS (FAB) calcd for C₃₆H₅₃CLN₂O₆Si₂ +H₁ 701.3209, found 701.3202
Anal. Calcd for C₃₆H₅₃ClN₂O₆Si₂: C, 61.64; H, 7.61; N, 3.99; Cl, 5.05; found: C, 61.35; H, 7.74; N, 3.97.

### Example 32: N-(4-Chlorobenzyl)-3,3-bis(hydroxymethyl)-9-(3-hydroxypropyl)-7-oxo-2,3-dihydro-7H-[1,4]oxazino[2,3,4-ij]quinoline-6-carboxamide (Formula H-11 of Chart H)

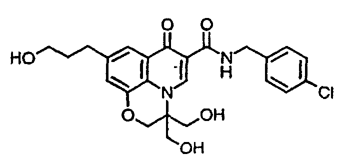

A solution of 132 mg of the product of Preparation **36** in 2.0 mL of ethanol and 1.0 mL of concentrated hydrochloric acid is stirred at room temperature for 18 h, then concentrated under reduced pressure. Flash chromatography of the residue on silica using 5-8% methanol in dichloromethane provides 85.3 mg of the title compound as a white solid. An analytical sample is recrystallized from methanol to give the title compound as white crystals, mp 173.0-174.5 °C.
¹NMR (CDCl₃ + CD₃OD) δ 1.90, 2.81, 3.61, 3.88, 4.10, 4.43, 4.64, 7.16, 7.31, 7.84, 8.90.
TLC R_{*f*} 0.35 (10% methanol in dichloromethane).
IR (drift) 3363, 1654, 1628, 1605, 1552, 1492, 1367, 1348, 1338, 1301, 1291, 1266, 1090, 1061, 801 cm⁻¹
HRMS (FAB) calcd for C₂₄H₂₅CLN₂O₆ +H₁ 473.1479, found 473.1471
Anal. Calcd for C₂₄H₂₅ClN₂O₆: C, 60.95; H, 5.33; N, 5.92; Cl, 7.50; found: C, 59.00; H, 5.63; N, 5.70.

### Preparation 37: 2-Amino-1-(2-pyridinyl)ethanol (I-7 of Chart I, T=2-pyridyl)

To 2.68 g of 2-pyridylcarboxaldehyde, stirred under argon at 0 °C, is added 3.7 mL of trimethylsilyl cyanide. After 10 min the ice bath is removed and the yellow liquid stirred for 30 min at ambient temperature. The resulting TMS cyanohydrin is dissolved in 10 mL of dry ether. In a three-necked flask fitted with argon inlet and overhead mechanical stirrer is placed 1.05 g of LAH and 50 mL of dry ether. The slurry is cooled to 0 °C under argon and stirred vigorously while the cyanohydrin solution is added dropwise via cannula. Following the addition, the reaction is stirred at room temperature for 2 h, then recooled to 0 °C and cautiously quenched with 1.0 mL of water (dissolved in 10 mL of THF), 1.0 mL of 3*N* NaOH, and 3.0 mL of water, added sequentially. CH₂Cl₂ (30 mL) and Na₂SO₄ are added, and the mixture is well stirred for 30 min and then filtered. The filter cake is washed well with 1:1 CH₂Cl₂:ether, and the combined filtrates concentrated under reduced pressure to provide 5.02 g of crude title compound as a deep red oil. The crude material is used directly in the subsequent reaction.
¹H NMR (CDCl₃) δ 2.87, 3.12, 4.71, 7.20, 7.35, 7.69, 8.55.

### Preparation 38: Ethyl 2-(2,3-difluoro-5-iodobenzoyl)-3-{[2-hydroxy-2-(2-pyridinyl)ethyl]amino}-2-propenoate (I-2 of Chart I, T=2-pyridyl)

A solution of 1.79 g of β-ketoester **H-3** of Preparation **30** and 1.3 mL of triethyl orthoformate in 5 mL of acetic anhydride is refluxed for 2 h, then concentrated under reduced pressure. The residual oil is stirred at 70 °C under 0.1 mmHg for 20 min to remove volatile components, providing the crude enol ether. This is dissolved in 2 mL of ethanol, and a solution of approximately 25 mmol of amino alcohol **1-7** of Preparation **37** in 10 mL of ethanol is added. The resulting deep red solution is stirred at room temperature for 15 h, then concentrated under reduced pressure. The residual oil is partitioned between EtOAc and water, and the organic phase is washed with two additional portions of water, once with brine, dried (MgSO₄), and concentrated under reduced pressure. Flash chromatography of the crude material on silica using EtOAc provides 2.14 g of the title compound as an orange gum. The NMR spectrum is complex due to E/Z isomerism.
TLC R_{*f*} 0.36 (EtOAc).

### Preparation of 39: Ethyl 9-iodo-7-oxo-2-(2-pyridinyl)-2,3-dihydro-7H-[1,4]oxazino[2,3,4-ij]quinoline-6-carboxylate (I-3 of Chart I, T=2-pyridyl)

A stirred mixture of 2.13 g of **I-2** of Preparation **38** and 3.04 g of cesium carbonate in 8 mL of DMF is heated at 100 °C under argon for 18 h, then cooled and diluted with 100 mL of water. The resulting solid is filtered, washed well with water, and dried under vacuum. Flash chromatography of the solid on silica using 3% MeOH in CH₂Cl₂ affords 2.06 g of the title compound as a peach-colored solid. A sample recrystallized from acetonitrile provides white crystals, mp 224-225 °C.
¹NMR (CDCl₃) δ 1.38, 4.34, 4.53, 4.64, 5.54, 7.33, 7.62, 7.64, 7.81, 8.27, 8.30, 8.61.
TLC R_{*f*} 0.45 (5% MeOH in CH₂Cl₂.)
IR 1682, 1637, 1611, 1589, 1550, 1491, 1399, 1367, 1316, 1267, 1248, 1235, 1183, 800, 770 cm⁻¹
OAMS supporting ions at: ESI+ 463.0
HRMS (FAB) 463.0150
Anal. found: C, 49.36; H, 3.30; N, 6.09.

### Preparation 40: N-(4-Chlorobenzyl)-9-iodo-7-oxo-2-(2-pyridinyl)-2,3-dihydro-7H-[1,4]oxazino[2,3,4-ij]quinoline-6-carboxamide (I-4 of Chart I, T=2-pyridyl)

A mixture of 1.86 g of **I-3** of Preparation **39** and 3.5 g of *p*-chlorobenzylamine is stirred and heated at 150 °C. After 18 h, excess amine is distilled off under vacuum, and the residue is adsorbed onto silica gel and flash chromatographed on silica using 1-3% MeOH in CH₂Cl₂ to provide 1.69 g of the amide as a pale yellow solid.
¹NMR (CDCl₃+CD₃OD) δ 4.61, 4.62, 4.76, 5.55, 7.30, 7.38, 7.62, 7.73, 7.85, 8.38, 8.66, 8.71.
TLC R_{*f*} 0.37 (2% MeOH in CH₂Cl₂)
OAMS supporting ions at: ESI+ 558.0.

### Preparation 41: N-(4-Chlorobenzyl)-9-formyl-7-oxo-2-(2-pyridinyl)-2,3-dihydro-7H-[1,4]oxazino[2,3,4-ij]quinoline-6-carboxamide (1-5 Chart I, T=2-pyridyl)

In a dry 50 mL recovery flask fitted with a Liebig condenser topped with a rubber septum is placed 520 mg of iodide **1-4** of Preparation **40**, 80 mg of tetrakis(triphenylphosphine) palladium (0), 5 mL of dry DMF, and 5 mL of dry THF. The mixture is stirred and heated at 55 °C while a slow stream of carbon monoxide is introduced below the surface of the liquid using a long 18 gauge needle. To the mixture is added, via syringe pump over about 5 h, a solution of 0.32 mL of tri-n-butylstannane in 10 mL of dry THF. Following the addition, solvents are removed *in vacuo*, and the remaining solid is washed with several portions of EtOAc and ether, and then dried under vacuum to provide 393 mg of the aldehyde as a poorly soluble tan solid.
¹NMR (DMSO-*d*₆) δ 4.58, 4.75, 4.98, 5.76, 7.3, 7.70, 7.78, 7.95, 8.45, 8.64, 8.93, 10.10, 10.24.
TLC R_{*f*} 0.44 (3% MeOH in dichoromethane).

### Example 33: N-(4-Chlorobenzyl)-9-(4-morpholinylmethyl)-7-oxo-2-(2-pyridinyl)-2,3-dihydro-7H-[1,4]oxazino[2,3,4-ij]quinoline-6-carboxamide (I-6 of Chart I, T=2-pyridyl)

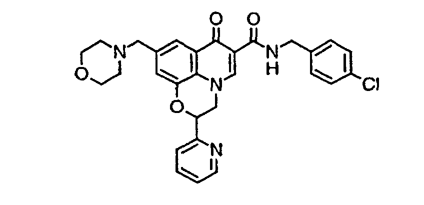

A mixture of 138 mg of aldehyde **1-5** of Preparation **41**, 79 µL of morpholine, and 34 µL of acetic acid in 3.0 mL of dry THF is stirred at ambient temperature for 30 minutes, then 50 mg of sodium triacetoxyborohydride is added. After 30 min, a second 50 mg portion of triacetoxyborohydride is added, and the resulting mixture stirred overnight. The following day, additional portions of triacetoxyborohydride sufficient to result in complete reduction are added over the course of 8 h. The reaction is partitioned between CH₂Cl₂ and aqueous sodium bicarbonate, and the aqueous phase is extracted with additional CH₂Cl₂. The combined organic phases are dried (Na₂SO₄) and concentrated under reduced pressure, and the residue flash chromatographed on silica using 3% MeOH in CH₂Cl₂ to afford 152 mg of the product as a white solid.. Recrystallization from 10 mL of acetonitrile provides 129 mg of the title compound as fine needles, mp 208-216 °C.
¹NMR (CDCl₃) δ 2.47, 3.61, 3.72, 4.58, 4.64, 4.72, 5.51, 7.3, 7.51, 7.63, 7.79, 7.97, 8.63, 8.72, 10.44.
TLC R_{*f*} 0.38 (5% MeOH in CH₂Cl₂).
IR 3035, 1655, 1608, 1569, 1551, 1499, 1452, 1410, 1357, 1346, 1329, 1283, 1212, 1113, 810 cm⁻¹
OAMS supporting ions at: ESI+ 531.2
HRMS (FAB) 531.1813
Anal. found: C, 65.39; H, 5.13; N, 10.47.

### Example 34: N-(4-Chlorobenzyl)-9-(4-morpholinylmethyl)-7-oxo-2-(3-pyridinyl)-2,3-dihydro-7H-[1,4]oxazino[2,3,4-ij]quinoline-6-carboxamide (I-6 of Chart I, T=3-pyridyl)

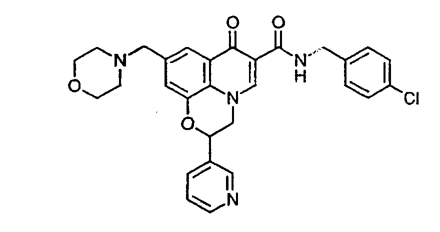

Following the procedures in Preparations **37-41** and Example **33** without making major changes except using 3-pyridylcarboxaldehyde as a starting material, the title compound is obtained as solid.
¹H NMR (CDCl₃) δ 2.47, 3.61, 3.71, 4.33, 4.43, 4.63, 5.38, 7.30, 7.45, 7.85, 8.00, 8.69, 8.74, 8.79, 10.40.
TLC R_{*f*} 0.28 (5% MeOH in CH₂Cl₂).
IR 3038, 1656, 1607, 1580, 1553, 1498, 1457, 1409, 1345, 1331, 1320, 1282, 1115, 810, 711 cm⁻¹
OAMS supporting ions at: ESI+ 531.2
HRMS (FAB) 531.1798
Anal. found: C, 65.62; H, 5.18; N, 10.53.

### Example 35: N-(4-Chlorobenzyl)-9-(4-morpholinylmethyl)-7-oxo-2-(4-pyridinyl)-2,3-dihydro-7H-[1,4]oxazino[2,3,4-ij]quinoline-6-carboxamide (I-6 of Chart I, T=4-pyridyl)

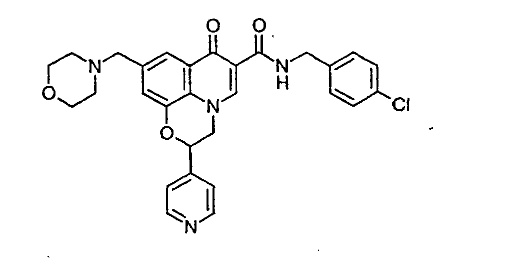

Following the procedure in Preparations **37-41** and Example **33** without making major changes except using 4-pyridylcarboxaldehyde as a starting material, the title compound is obtained as solid.
¹H NMR (CDCl₃) δ 2.47, 3.61, 3.71, 4.25, 4.49, 4.60, 5.34, 7.27, 7.45, 7.49, 7.99, 8.70, 8.74, 10.41.
TLC R_{*f*} 0.26 (5% MeOH in CH₂Cl₂).
IR 2859, 1666, 1608, 1551, 1500, 1411, 1352,1331, 1285, 1271, 1220, 1114, 1090, 821, 806 cm⁻¹
OAMS supporting ions at: ESI+ 531.2
HRMS (FAB) 531.1791
Anal. found: C, 65.26; H, 5.18; N, 10.50.

### Preparation 42: Ethyl 8-fluoro-4-hydroxy-6-iodoquinoline-3-carboxylate (Formula M-2 of Chart M)

A mixture of 2-fluoro-4-iodoaniline (11.85 g) and diethylethoxymethylene malonate (10.81 g) is heated to 130 °C in a flask equipped with a Dean-Stark trap to collect EtOH formed. The mixture is then cooled to 75 °C and diluted with hexanes. The resulting solid is collected and dried. The solid is then dissolved in 60 mL Ph₂O and heated to 250 °C for 3 h in a flask equipped with a Dean-Stark trap to collect the EtOH formed. The solution is allowed to cool to room temperature and the resulting solid is collected and dried to yield 11.73 g of ethyl-8-fluoro-4-hydroxy-6-iodoquinoline-3-carboxylate.
M.p. 287-289 °C
¹H NMR (DMSO-*d*_{*6*}) δ 12.60, 8.38, 8.23, 8.04, 4.22, 1.28
IR (drift) 3165, 3080, 3070, 3059, 2969, 1709, 1616, 1603, 1564, 1527, 1296, 1249, 1173, 1141, 865 cm⁻¹
HRMS (FAB) calcd for C₁₂H₉FINO₃+H₁ 361.9691, found 361.9696
Anal. Calcd for C₁₂H₉FINO₃: C, 39.91; H, 2.51; N, 3.88. found: C, 39.71; H, 2.42; N, 3.88.

### Preparation 43: N-(4-Chlorobenzyl)-8-fluoro-4-hydroxy-6-iodoquinoline-3-carboxamide (Formula M-3 of Chart M)

Compound **M-2** of Preparation **42** (0.55 g) and 4-chlorobenzylamine (3 mL) are heated at 180 °C for 1 h. The reaction is cooled and poured into 75 mL diethyl ether. The resulting solid is filtered and recrystallized from EtOAc/hexanes to give the product as an off-white solid (0.45 g), m.p. 268-270 °C
¹H NMR (DMSO-*d*_{*6*}) δ 10.17, 8.59, 8.29, 8.05, 7.37, 7.33, 4.51
IR (mull) 3180, 3078, 3059, 3004, 1647, 1607, 1551, 1524, 1489, 1344, 1297, 1285, 1240, 1183, 805 cm⁻¹; MS (ESI) for *m*/*z* 456.9 (M+H)⁺, 454.9 (M-H)⁻
HRMS (FAB) calcd for C₁₇H₁₁ClFIN₂O₂+H₁ 456.9618, found 456.9628
Anal. Calcd for C₁₇H₁₁ClFIN₂O₂: C, 44.72; H, 2.43; N, 6.14. found: C, 45.45; H, 2.82; N, 6.05.

### Preparation 44: N-(4-Chlorobenzyl)-8-fluoro-6-formyl-4-hydroxyquinoline-3-carboxamide (Formula M-4 of Chart M)

A mixture of 4.57 g of compound **M-3** of Preparation **43** and 810 mg of tetrakis(triphenylphosphine)palladium (0) in 35 mL of dry DMF and 10 mL of dry THF is stirred and purged with CO for 10 minutes, and then heated to 60 °C. Passage of CO through the mixture is continued while 3.3 mL of tributyltin hydride in 6.7 mL of dry THF is added very slowly, *via* syringe pump over about 6 h. The reaction mixture is then cooled and concentrated under reduced pressure to a semisolid. Ether (70 mL) is added and the resulting solid filtered, washed well with ether, and dried *in vacuo* to provide 3.54 g of the aldehyde.
¹H NMR (CDCl₃ + CD₃OD) δ 4.65, 7.32, 7.93, 8.68, 8.75, 10.07, 10.43 ppm.
TLC R_{*f*} 0.29 (3% methanol in dichloromethane).
OAMS supporting ions at: ESI- 358.1.

### Preparation 45: N-(4-Chlorobenzyl)-8-fluoro-4-hydroxy-6-(morpholin-4-ylmethyl)quinoline-3-carboxamide (Formula M-5 of Chart M)

To a stirred solution of 2.42 g of aldehyde **M-4** of Preparation **44** in 50 mL of THF is added 1.8 mL of morpholine and 0.77 mL of acetic acid. The resulting slurry is stirred well for 15 min, then 1.42 g of sodium triacetoxyborohydride is added. Additional portions of triacetoxyborohydride are periodically added until TLC analysis indicates completion of reaction. The reaction is then partitioned between dichloromethane and aqueous NaHCO₃, and the aqueous phase extracted with additional dichloromethane. The combined organic extract is dried (Na₂SO₄) and concentrated under reduced pressure. Flash chromatography of the residue on silica gel using 4-5% methanol in dichloromethane affords 2.29 g of the title compound as a tan solid. Recrystallization from acetonitrile provides tan plates, mp 227.5-230.0°C.
¹H NMR (CDCl₃ + CD₃OD) δ 2.47, 3.62, 3.72, 4.64, 7.31, 7.56, 8.06, 8.70, 10.63 ppm.
TLC R_{*f*} 0.33 (5% methanol in dichoromethane).
IR (diffuse reflectance) 3080, 3025, 2971, 2929, 2861, 1660, 1613, 1575, 1542, 1508, 1349, 1268, 1118, 806, 799 cm⁻¹
OAMS supporting ions at: ESI+ 430.3
HRMS (FAB) calcd for C₂₂H₂₁CLFN₃O₃ +H₁ 430.1334, found 430.1339
Anal. Calcd for C₂₂H₂₁ClFN₃O₃: C, 61.47; H, 4.92; N, 9.77; Cl, 8.25; F, 4.42; found: C, 61.09; H, 4.92; N, 9.66.

### Preparation 46: General procedure for preparation of epoxides (M-7)

**Step 1: Trimethylsulfonium methylsulfate:** Dimethyl sulfide (25.0 mL) is cooled to 0 °C, and dimethyl sulfate (25.0 mL) is added. The liquid is stirred at 0 °C and allowed to warm slowly as the ice in the cooling bath melts. The following day, ether is added and the solid mass crushed with a stirring rod. The solid is filtered, washed well with ether, and dried *in vacuo* to provide 48.0 g of the salt as a white crystalline solid.
**Step 2: Epoxidation: 3-Oxiran-2-ylbenzonitrile** (Formula M-7 of Chart M, where Rₐ=3-cyanophenyl and R_{b}=H)

To a solution of 1.31 g of 3-cyanobenzaldehyde (Formula **M-6** of Chart M where Rₐ is 3-cyanophenyl and R_{b} is H) and 25 mg of tetra-*n*-butylammonium bromide in 30 mL of dichloromethane is added a solution of 3.92 g of trimethylsulfonium methylsulfate in 10 mL of water, followed by 20 mL of 50% aqueous NaOH. The biphasic mixture is stirred and refluxed for 5 h, then cooled, diluted with brine (~20 mL) and ether (~50 mL), and filtered to remove solids. The phases are separated and the aqueous phase extracted with additional ether as necessary to remove product. The combined organic phase is dried (Na₂SO₄) and concentrated under reduced pressure, or at atmospheric pressure through a Vigreux column. Kugelrohr distillation of the residue at ca. 0.5 mmHg and 160 °C oven temperature affords 1.20 g of the epoxide as a colorless liquid.
¹H NMR (CDCl₃) δ 2.76, 3.19, 3.90, 7.47, 7.52, 7.58, 7.70 ppm.
OAMS supporting ions at: ESI- 144.0

**Using the general procedure of Preparation 46, the following epoxides (M-7) are obtained.**

### 3-Oxiran-2-ylfuran (Formula M-7 of Chart M, where Rₐ=furan-3-yl and R_{b}=H)

As a colorless liquid following kugelrohr distillation at ca. 0.5 mmHg and 40 °C oven temperature.
¹H NMR (CDCl₃) δ 2.88, 3.11, 3.80, 6.28, 7.39, 7.52 ppm.
OAMS supporting ions at: ESI+ 111.2.

### 2-Thien-2-yloxirane (Formula M-7 of Chart M, where Rₐ=thien-2-yl and R_{b}=H)

As a colorless liquid following kugelrohr distillation at ca. 0.5 mmHg and 75 °C oven temperature.
¹H NMR (CDCl₃) δ 3.00, 3.19, 4.10, 6.98, 7.13, 7.25 ppm.
OAMS supporting ions at: ESI+ 127.1.

### 2-(3,5-Difluorophenyl)oxirane (Formula M-7 of Chart M, where Rₐ=3,5-difluorophenyl and R_{b}=H)

As a colorless liquid following kugelrohr distillation at ca. 0.5 mmHg and 75 °C oven temperature.
¹H NMR (CDCl₃) δ 2.72, 3.15, 3.84, 6.73, 6.81 ppm.

### 5-Oxiran-2-yl-1,3-benzodioxole (Formula M-7 of Chart M, where Rₐ=1,3-benzodioxol-5-yl and R_{b}=H)

As a colorless liquid following kugelrohr distillation at ca. 0.5 mmHg and 150 °C oven temperature.
¹H NMR (CDCl₃) δ 2.75, 3.10, 3.79, 5.95, 6.7, 6.8 ppm.
OAMS supporting ions at: ESI+ 165.2.

### 6-Oxiran-2-yl-2,3-dihydro-1,4-benzodioxine (Formula M-7 of Chart M, where Rₐ=2,3-dihydro-1,4-benzodioxin-6-yl and R_{b}=H)

As a colorless oil following kugelrohr distillation at ca. 0.5 mmHg and 175 °C oven temperature.
¹H NMR (CDCl₃) δ 2.75, 3.08, 3.75, 4.23, 6.76, 6.83 ppm.
OAMS supporting ions at: ESI+ 179.2.

### 4-Oxiran-2-yl-1,3-benzodioxole (Formula M-7 of Chart M, where Rₐ=1,3-benzodioxol-4-yl and R_{b}=H)

As a colorless liquid following kugelrohr distillation at ca. 0.5 mmHg and 125 °C oven temperature.
¹H NMR (CDCl₃) δ 3.02, 3.14, 3.96, 5.97, 6.00, 6.70, 6.8 ppm.

### 2-[3,5-bis(Methoxymethoxy)phenyl]oxirane (Formula M-7 of Chart M, where Rₐ=3,5-bis(methoxymethoxy)phenyl and R_{b}=H)

As a colorless liquid following kugelrohr distillation at ca. 0.5 mmHg and 200 °C oven temperature.
¹H NMR (CDCl₃) δ 2.76, 3.11, 3.47, 3.81, 5.14, 5.16, 6.63, 6.67 ppm.
OAMS supporting ions at: ESI+ 241.2.

### 2-[2,3-bis(Methoxymethoxy)phenyl]oxirane (Formula M-7 of Chart M, where Rₐ=2,3-bis(methoxymethoxy)phenyl and R_{b}=H)

As a colorless liquid following kugelrohr distillation at ca. 0.5 mmHg and 200 °C oven temperature.
¹H NMR (CDCl₃) δ 2.74, 3.16, 3.50, 3.60, 4.28, 5.17, 5.19, 5.21, 6.81, 7.03, 7.09 ppm.
OAMS supporting ions at: ESI- 239.1.

### 2-Thien-3-yloxirane (Formula M-7 of Chart M, where Rₐ=thien-3-yl and R_{b}=H)

As a colorless liquid following kugelrohr distillation at ca. 0.5 mmHg and 75 °C oven temperature.
¹H NMR (CDCl₃) δ 2.90, 3.13, 3.93, 6.96, 7.29 ppm.
OAMS supporting ions at: ESI+ 127.1.

### 1,6-Dioxaspiro[2.5]octane (Formula M-7 of Chart M, where Rₐ, R_{b}=4-tetrahydropyran)

As a colorless liquid following kugelrohr distillation at ca. 0.5 mmHg and 100-200 °C oven temperature. The poor yield is thought to be due to troublesome distillation behavior, with sequestering of product in the viscous pot residue.
¹H NMR (CDCl₃) δ 1.54, 1.87, 2.70, 3.84 ppm.

### 6-Methyl-1-oxa-6-azaspiro[2.5]octane (Formula M-7 of Chart M, where Rₐ, R_{b}=N-methyl-4-piperidine)

As a colorless liquid following kugelrohr distillation at ca. 0.5 mmHg and 50 °C oven temperature.
¹H NMR (CDCl₃) δ 1.56, 1.87, 2.34, 2.5, 2.66 ppm.
OAMS supporting ions at: ESI+ 128.2.

### 1,7,10-Trioxadispiro[2.2.4.2]dodecane (Formula M-7 of Chart M, where Rₐ, R_{b}=cyclohexane-4-ethylene ketal)

As a white crystalline solid, melting at slightly above room temperature, following kugelrohr distillation at ca. 0.5 mmHg and 130 °C oven temperature.
¹H NMR (CDCl₃) δ 1.58, 1.77, 1.9, 2.68, 3.98 ppm.
OAMS supporting ions at: ESI+ 171.1.

### 1-Oxa-6-thiaspiro[2.5]octane (Formula M-7 of Chart M, where Rₐ, R_{b}=tetrahydrothiopyran)

As a white crystalline solid following kugelrohr distillation at ca. 0.5 mmHg and 120 °C oven temperature.
¹H NMR (CDCl₃) δ 1.74, 2.00, 2.60, 2.65, 2.91 ppm.

### Preparation 47: tert-Butyl 1-oxa-6-azaspiro[2.5]octane-6-carboxylate (Formula M-7 of Chart M, where Rₐ, R_{b}=N-Boc-4-piperidine)

**Step 1:** To a stirred mixture of 3.07 g of 4-piperidone hydrochloride hydrate and 4.80 g of di-*tert*-butyl dicarbonate in 20 mL of THE is added 7.0 mL of 3N aqueous NaOH. The biphasic mixture is stirred well for 18 h, then diluted with chloroform. The phases are separated and the aqueous phase extracted with one portion of chloroform, and the combined extract dried (Na₂SO₄) and concentrated under reduced pressure. Flash chromatography of the residue on silica using 10-20% ethyl acetate in dichloromethane provides 4.08 g of tert-butyl 4-oxopiperidine-1-carboxylate (Boc-piperidone) as a white crystalline solid.
   ¹H NMR (CDCl₃) δ 1.50, 2.44, 3.72 ppm.
   TLC R_{*f*} 0.38 (10% ethyl acetate in dichloromethane).
   OAMS supporting ions at: ESI+ 200.1
**Step 2:** Using the general procedure of Preparation **46**, the ketone product of step 1 is converted to the epoxide as a white crystalline solid.
   ¹H NMR (CDCl₃) δ 1.45, 1.48, 1.80, 2.70, 3.43, 3.7 ppm.
   OAMS supporting ions at: ESI+ 214.2.

### Preparation 48: 2,2-bis[(Methoxymethoxy)methyl]oxirane (Formula M-7 of Chart M, where Rₐ, R_{b}=methoxymethoxymethyl)

**Step 1:** To a cold (0 °C), stirred mixture of 3.60 g of dihydroxyacetone dimer and 16 mL of diisopropylethylamine in 40 mL of dichloromethane is added 6.1 mL of chloromethyl methyl ether. The mixture is stirred and allowed to warm slowly to room temperature. After 18 h, the clear solution is partitioned between ether and dilute HCl. The aqueous phase is placed in a continuous extractor and extracted overnight with ether. The combined organic phase is then dried (MgSO₄) and concentrated under reduced pressure. Kugelrohr distillation of the residue at ∼0.5 mmHg and 150 °C oven temperature affords 2.79 g of 2,4,8,10-tetraoxaundecan-6-one as a pale yellow oil.
   ¹H NMR (CDCl₃) δ 3.40, 4.34, 4.69 ppm.
**Step 2:** Using the general procedure of Preparation of **46,** the ketone product of Step 1 is converted to the epoxide as a white crystalline solid after kugelrohr distillation at ca. 0.5 mmHg and 150 °C oven temperature.
   ¹H NMR (CDCl₃) δ 2.83, 3.37, 3.72, 4.65 ppm.

### Example 36: General procedure for epoxide alkylation: N-(4-Chlorobenzyl)-2-(3-cyanophenyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=3-cyanophenyl and R_{b}=H)

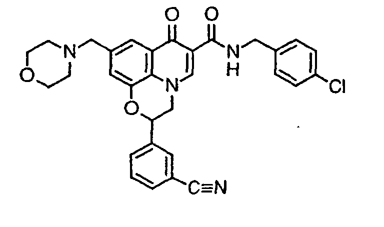

To a mixture of 172 mg of fluorodihydroquinoline **M-5** of Preparation **45** and 261 mg of cesium carbonate in 1 mL of DMF is added 116 mg of 3-oxiran-2-ylbenzonitrile (Formula **M-7** of Chart M where Rₐ is 3-cyanophenyl and R_{b} is H). The mixture is stirred and heated at 90 °C for 4-5 h, and then a second 116 mg portion of epoxide is added and heating continued overnight. The reaction is then cooled and partitioned between chloroform and water, and the aqueous phase extracted with additional chloroform as needed to remove product. The combined organic phase is dried (Na₂SO₄) and concentrated under reduced pressure, and the residue chromatographed on silica gel using 2.5-3.5% methanol in dichloromethane to afford 125 mg of the title compound. Recrystallization from acetonitrile provides orange needles.
¹H NMR (CDCl₃ + CD₃OD) δ 2.49, 3.63, 3.73, 4.26, 4.59, 4.63, 5.40, 7.30, 7.50, 7.64, 7.78, 7.90, 7.99, 8.65, 10.54 ppm.
TLC R_{*f*} 0.40 (5% methanol in dichloromethane).
OAMS supporting ions at: ESI+ 555.3.

### Example 37 N-(4-Chlorobenzyl)-2-(3-furyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=furan-3-yl and R_{b}=H)

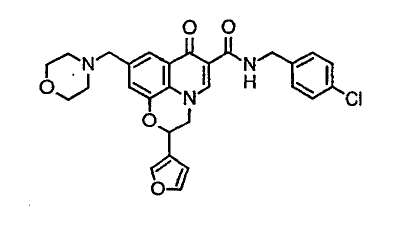

The title compound is prepared according to the general procedure of Example **36**, and is purified by flash chromatography on silica gel using 2% methanol in dichloromethane. Recrystallization from acetonitrile provides tan crystals, mp 193-196 °C.
¹H NMR (CDCl₃) δ 2.46, 3.59, 3.70, 4.34, 4.41, 4.63, 5.37, 6.49, 7.30, 7.41, 7.51, 7.60, 7.96, 8.67, 10.44 ppm.
TLC R_{*f*} 0.22 (3% methanol in dichloromethane). IR (diffuse reflectance) 1655, 1627, 1608, 1569, 1551, 1500, 1411, 1347, 1329, 1282, 1218, 1114, 885, 875, 809 cm⁻¹
OAMS supporting ions at: ESI+ 520.4
HRMS (FAB) calcd for C₂₈H₂₆CLN₃O₅ +H₁ 520.1639, found 520.1634
Anal. Calcd for C₂₈H₂₆ClN₃O₅: C, 64.68; H, 5.04; N, 8.08; Cl, 6.82; found: C, 64.50; H, 5.09; N, 8.05.

### Example 38: N-(4-Chlorobenzyl)-9-(morpholin-4-ylmethyl)-7-oxo-2-thien-2-yl-2,3-dihydro-7H-[1,4]oxazino[2,3,4-ij]quinoline-6-carboxamide (Formula M-8 of Chart M, where Rₐ=thien-2-yl and R_{b}=H)

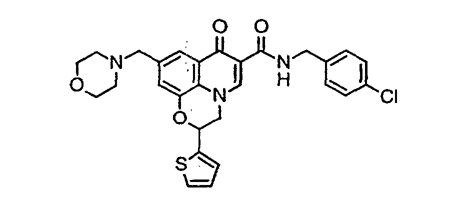

The title compound is prepared according to the general procedure of Example **36**, and is purified by flash chromatography on silica gel using 2% methanol in dichloromethane. Recrystallization from acetonitrile provides pale yellow crystals.
¹H NMR (CDCl₃) δ 2.46, 3.59, 3.70, 4.44, 4.50, 4.63, 5.64, 7.09, 7.20, 7.30, 7.44, 7.98, 8.68, 10.43 ppm.
TLC R_{*f*} 0.28 (3% methanol in dichloromethane).
IR (diffuse reflectance) 2954, 2926, 2799, 1666, 1610, 1568, 1551, 1500, 1343, 1287, 1275, 1110, 887, 860, 798 cm⁻¹
OAMS supporting ions at: ESI+ 536.3
HRMS (FAB) calcd for C₂₈H₂₆CLN₃O₄S +H₁ 536.1411, found 536.1411
Anal. Calcd for C₂₈H₂₆ClN₃O₄S: C, 62.74; H, 4.89; N, 7.84; Cl, 6.61; S, 5.98; found: C, 62.72; H, 4.92; N, 7.86.

### Example 39: N-(4-Chlorobenzyl)-2-(3,5-difluorophenyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=3,5-difluorophenyl and R_{b}=H)

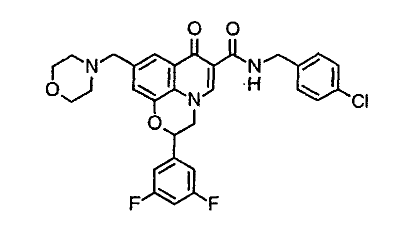

The title compound is prepared according to the general procedure of Example **36,** and is purified by flash chromatography on silica gel using 2% methanol in dichloromethane. Recrystallization from acetonitrile containing about 10% methanol affords white crystals, mp 234-236 °C.
¹H NMR (CDCl₃) δ 2.47, 3.61, 3.71, 4.23, 4.41, 4.63, 5.30, 6.91, 7.06, 7.29, 7.48, 8.00, 8.67, 10.40 ppm.
TLC R_{*f*} 0.32 (3% methanol in dichloromethane).
IR (diffuse reflectance) 1650, 1628, 1603, 1575, 1552, 1532, 1500, 1466, 1452, 1411, 1347, 1281, 1121, 864, 811 cm⁻¹
OAMS supporting ions at: ESI+ 566.4
HRMS (FAB) calcd for C₃₀H₂₆CLF₂N₃O₄ +H₁ 566.1658, found 566.1654
Anal. Calcd for C₃₀H₂₆ClF₂N₃O₄: C, 63.66; H, 4.63; N, 7.42; Cl, 6.26; F, 6.71; Found: C, 63.22; H, 4.73; N, 7.82.

### Example 40: 2-(1,3-Benzodioxol-5-yl)-N-(4-chlorobenzyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=1,3-benzodioxol-5-yl and R_{b}=H)

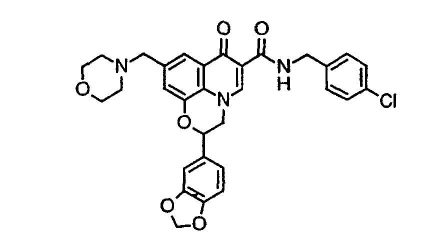

The title compound is prepared according to the general procedure of Example **36**, and is purified by flash chromatography on silica gel using 2-3% methanol in dichloromethane. Recrystallization from acetonitrile containing about 10% methanol affords pale yellow crystals, mp 227-231 °C.
¹H NMR (CDCl₃ + CD₃OD) δ 2.49, 3.62, 3.72, 4.29, 4.47, 4.63, 5.24, 6.04, 6.90, 6.98, 7.31, 7.45, 7.95, 8.64, 10.59 ppm.
TLC R_{*f*} 0.30 (3% methanol in dichloromethane).
IR (diffuse reflectance) 1650, 1627, 1608, 1552, 1500, 1449, 1411, 1353, 1330, 1282, 1246, 1225, 1116, 1038, 810 cm⁻¹
OAMS supporting ions at: ESI+ 574.4
HRMS (FAB) calcd for C₃₁H₂₈CLN₃O₆ +H₁ 574.1744, found 574.1760
Anal. Calcd for C₃₁H₂₈ClN₃O₆: C, 64.86; H, 4.92; N, 7.32; Cl, 6.18; found: C, 64.75; H, 4.96; N, 7.24.

### Example 41: N-(4-Chlorobenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=2,3-dihydro-1,4-benzodioxin-6-yl and R_{b}=H)

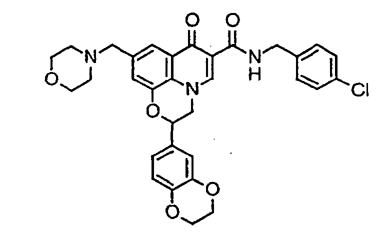

The title compound is prepared according to the general procedure of Example **36**, and is purified by flash chromatography on silica gel using 2% methanol in dichloromethane. Recrystallization from acetonitrile containing about 10% methanol affords white crystals, mp 240-243 °C.
¹H NMR (CDCl₃ + CD₃OD) δ 2.48, 3.62, 3.72, 4.29, 4.31, 4.43, 4.63, 5.21, 6.96, 7.02, 7.31, 7.44, 7.95, 8.64, 10.57 ppm.
TLC R_{*f*} 0.30 (3% methanol in dichloromethane).
IR (diffuse reflectance) 1648, 1607, 1552, 1502, 1411, 1330, 1308, 1293, 1281, 1261, 1120, 1116, 1068, 891, 811 cm⁻¹
OAMS supporting ions at: ESI+ 588.4
HRMS (FAB) calcd for C₃₂H₃₀CLN₃O₆ +H₁ 588.1901, found 588.1905
Anal. Calcd for C₃₂H₃₀ClN₃O₆: C, 65.36; H, 5.14; N, 7.14; Cl, 6.03; found: C, 65.15; H, 5.19; N, 7.04.

### Example 42: 2-(1,3-Benzodioxol-4-yl)-N-(4-chlorobenzyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=1,3-benzodioxol-4-yl and R_{b}=H)

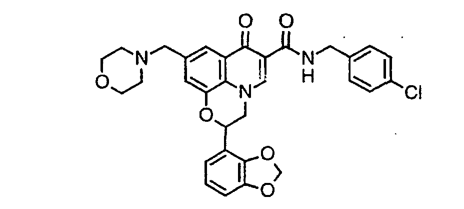

The title compound is prepared according to the general procedure of Example **36,** and is purified by flash chromatography on silica gel using 2% methanol in dichloromethane. Recrystallization from acetonitrile containing about 10% methanol affords white crystals, mp 221-225 °C (d).
¹H NMR (CDCl₃) δ 2.46, 3.60, 3.70, 4.43, 4.48, 4.63, 5.45, 6.02, 6.07, 6.91, 6.95, 7.02, 7.3, 7.46, 7.97, 8.68, 10.45 ppm.
TLC R_{*f*} 0.30 (3% methanol in dichloromethane).
IR (diffuse reflectance) 1654, 1606, 1553, 1498, 1463, 1411, 1349, 1283, 1272, 1249, 1223, 1119, 871, 808, 797 cm⁻¹
OAMS supporting ions at: ESI+ 574.4
HRMS (FAB) calcd for C₃₁H₂₈CLN₃O₆ +H₁ 574.1744, found 574.1763
Anal. Calcd for C₃₁H₂₈ClN₃O₆: C, 64.86; H, 4.92; N, 7.32; Cl, 6.18; found: C, 64.77; H, 4.98; N, 7.37.

### Example 43 2-[3,5-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=3,5-bis(methoxymethoxy)phenyl and R_{b}=H)

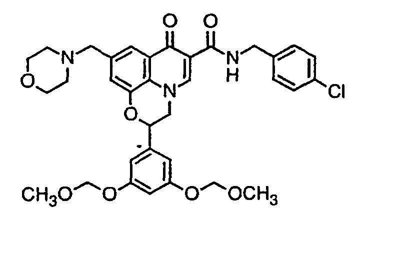

The title compound is prepared according to the general procedure of Example **36**, and is purified by flash chromatography on silica gel using 2% methanol in dichloromethane.
Recrystallization from acetonitrile affords pale pink crystals, mp 187-189 °C.
¹H NMR (CDCl₃) δ 2.47, 3.50, 3.60, 3.71, 4.29, 4.37, 4.63, 5.18, 5.21, 5.23, 6.82, 7.3, 7.46, 7.97, 8.66, 10.45 ppm.
TLC R_{*f*} 0.33 (3% methanol in dichloromethane).
IR (diffuse reflectance) 3031, 2937, 2922, 1655, 1607, 1578, 1569, 1551, 1499, 1288, 1147, 1086, 1025, 1011, 925 cm⁻¹
OAMS supporting ions at: ESI+ 650.5
HRMS (FAB) calcd for C₃₄H₃₆CLN₃O₈ +H₁ 650.2269, found 650.2283
Anal. Calcd for C₃₄H₃₆ClN₃O₈: C, 62.81; H, 5.58; N, 6.46; Cl, 5.45; found: C, 62.63; H, 5.59; N, 6.45.

### Example 44: 2-[2,3-bis(Methoxymethoxy)phenyl]-N-(4-chiorobenzyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ=2,3-bis(methoxymethoxy)phenyl and R_{b}=H)

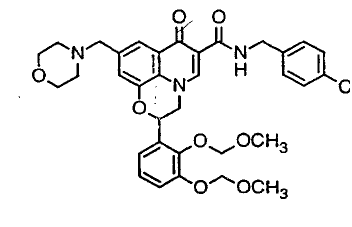

The title compound is prepared according to the general procedure of Example **36**, and is purified by flash chromatography on silica gel using 2% methanol in dichloromethane. Recrystallization from acetonitrile affords white crystals, mp 177-180 °C.
¹H NMR (CDCl₃) δ 2.47, 3.45, 3.53, 3.61, 3.71, 4.16, 4.60, 4.64, 5.18, 5.21, 5.25, 5.70, 7.2, 7.3, 7.46, 7.99, 8.67, 10.49 ppm.
TLC R_{*f*} 0.40 (3% methanol in dichloromethane).
IR (diffuse reflectance) 1648, 1605, 1571, 1551, 1526, 1500, 1482, 1280, 1260, 1159, 1115, 1077, 1004, 957, 924 cm⁻¹
OAMS supporting ions at: ESI+ 650.5
HRMS (FAB) calcd for C₃₄H₃₆CLN₃O₈ +H₁ 650.2269, found 650.2279
Anal. Calcd for C₃₄H₃₆ClN₃O₈: C, 62.81; H, 5.58; N, 6.46; Cl, 5.45; found: C, 62.63; H, 5.57; N, 6.39.

### Example 45: N-(4-Chlorobenzyl)-9-(morpholin-4-ylmethyl)-7-oxo-2-thien-3-yl-2,3-dihydro-7H-[1,4]oxazino[2,3,4-ij]quinoline-6-carboxamide (Formula M-8 of Chart M, where Rₐ=thien-3-yl and R_{b}=H)

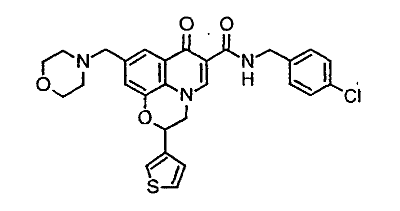

The title compound is prepared according to the general procedure of Example **36,** and is purified by flash chromatography on silica gel using 2% methanol in dichloromethane. Recrystallization from acetonitrile affords yellow crystals, mp 241-245 °C (d).
¹H NMR (CDCl₃) δ 2.46, 3.60, 3.70, 4.37, 4.44, 4.63, 5.45, 7.19, 7.3, 7.44, 7.47, 7.97, 8.68, 10.44 ppm.
TLC R_{*f*} 0.35 (3% methanol in dichloromethane).
IR (diffuse reflectance) 1656, 1627, 1608, 1569, 1551, 1500, 1411, 1348, 1330, 1319, 1282, 1227, 1113, 810, 797 cm⁻¹
OAMS supporting ions at: ESI+ 536.4
HRMS (FAB) calcd for C₂₈H₂₆CLN₃O₄S +H₁ 536.1411, found 536.1417
Anal. Calcd for C₂₈H₂₆ClN₃O₄S: C, 62.74; H, 4.89; N, 7.84; Cl, 6.61; S, 5.98; found: C, 62.68; H, 4.94; N, 7.77.

### Example 46: N-[(4-Chlorophenyl)methyl]-2,3,5,6-tetrahydro-9'-(4-morpholinylmethyl)-7'-oxospiro[4H-pyran-4,2'(3'H)-[7H]pyrido[1,2,3-de] [1,4]benzoxazine]-6'-carboxamide (Formula M-8 of Chart M, where Rₐ, R_{b}=4-tetrahydropyran)

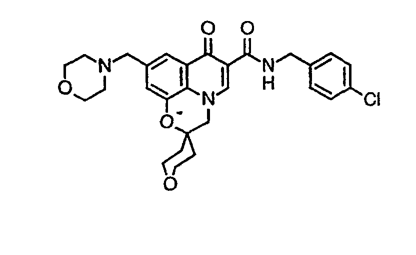

The title compound is prepared according to the general procedure of Example **36**, and is purified by flash chromatography on silica gel using 3% methanol in dichloromethane. Recrystallization from acetonitrile affords white crystals, mp 251-254 °C.
¹H NMR (CDCl₃) δ 1.80, 2.47, 3.59, 3.72, 3.86, 4.04, 4.64, 7.30, 7.40, 7.94, 8.65, 10.44 ppm.
TLC R_{*f*} 0.30 (5% methanol in dichloromethane).
IR (diffuse reflectance) 2861, 1655, 1607, 1568, 1552, 1504, 1411, 1323, 1277, 1245, 1217, 1113, 1015, 882, 811 cm⁻¹
OAMS supporting ions at: ESI+ 524.4
HRMS (FAB) calcd for C₂₈H₃₀CLN₃O₅ +H₁ 524.1952, found 524.1957
Anal. Calcd for C₂₈H₃₀ClN₃O₅: C, 64.18; H, 5.77; N, 8.02; Cl, 6.77; found: C, 64.12; H, 5.78; N, 8.03.

### Example 47: 1,1-Dimethylethyl 6-[[[(4-chlorophenyl)methyl]amino]carbonyl]-9'-(4-morpholinylmethyl)-7'-oxospiro[piperidine-4,2'(3'H)-[7H]pyrido[1,2,3-de] [1,4]benzoxazine]-1-carboxylate (Formula M-8 of Chart M, where Rₐ, R_{b}=N-Boc-4-piperidine)

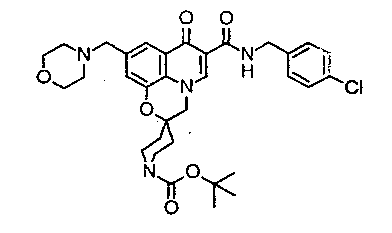

The title compound is prepared according to the general procedure of Example **36,** and is purified by flash chromatography on silica gel using 2% methanol in dichloromethane. Recrystallization from acetonitrile affords white crystals, mp 213 °C (d).
¹H NMR (CDCl₃) δ 1.47, 1.67, 1.81, 2.46, 3.23, 3.59, 3.71, 3.97, 4.03, 4.64, 7.30, 7.38, 7.94, 8.63, 10.43 ppm.
TLC R_{*f*} 0.45 (5% methanol in dichloromethane).
IR (diffuse reflectance) 1694, 1662, 1647, 1605, 1570, 1551, 1537, 1500, 1413, 1366, 1281, 1249, 1151, 1113, 811 cm⁻¹
OAMS supporting ions at: ESI+ 623.5
HRMS (FAB) calcd for C₃₃H₃₉CLN₄O₆ +H₁ 623.2636, found 623.2628
Anal. Calcd for C₃₃H₃₉ClN₄O₆: C, 63.61; H, 6.31; N, 8.99; Cl, 5.69; found: C, 63.38; H, 6.30; N, 9.02.

### Example 48: N-[(4-Chlorophenyl)methyl]-9'-(4-morpholinylmethyl)-7'-oxospiro[piperidine-4,2'(3'H)-[7H]pyrido[1,2,3-de] [1,4]benzoxazine]-6'-carboxamide (Formula M-8 of Chart M, where Rₐ, R_{b}=4-piperidine)

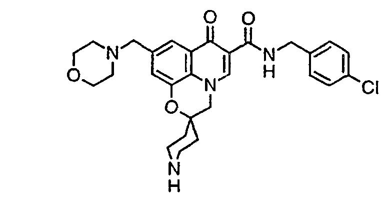

A solution of 102 mg of the product of Example **47** in 1 mL of 1:1 TFA-dichloromethane is allowed to stand for 1 h, then partitioned between excess aqueous NaHCO₃ and dichloromethane. The organic phase is dried (Na₂SO₄) and concentrated under reduced pressure. Flash chromatography of the residue on silica using 5-8% methanolic ammonia in dichloromethane provides 73 mg of the title compound as a white solid. Recrystallization from acetonitrile containing ca. 10% methanol affords white crystals, mp 246-250 °C (d).
¹H NMR (CDCl₃) δ 1.68, 1.80, 2.46, 2.92, 3.08, 3.59, 3.71, 4.03, 4.64, 7.30, 7.38, 7.92, 8.64, 10.47 ppm.
TLC R_{*f*} 0.25 (8% methanolic ammonia in dichloromethane).
IR (diffuse reflectance) 2963, 2936, 2916, 1655, 1607, 1569, 1551, 1503, 1411, 1322, 1281, 1218, 1113, 882, 811 cm⁻¹
OAMS supporting ions at: ESI+ 523.5
HRMS (FAB) calcd for C₂₈H₃₁CLN₄O₄ +H₁ 523.2112, found 523.2122
Anal. Calcd for C₂₈H₃₁ClN₄O₄: C, 64.30; H, 5.97; N, 10.71; Cl, 6.78; found: C, 64.08; H, 6.04; N, 10.76.

### Example 49: N-[(4-Chlorophenyl)methyl]-1-methyl-9'-(4-morpholinylmethyl)-7'-oxospiro[piperidine-4,2'(3'H)-[7H]pyrido[1,2,3-de][1,4]benzoxazine]-6'-carboxamide (Formula M-8 of Chart M, where Rₐ, R_{b}=N-methyl-4-piperidine)

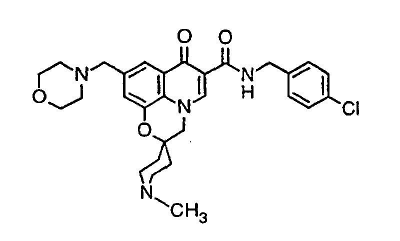

The title compound is prepared according to the general procedure of Example **36,** and is purified by flash chromatography on silica gel using 3% methanolic ammonia in dichloromethane. Recrystallization from acetonitrile affords fine white needles, mp 234-237 °C (d).
¹H NMR (CDCl₃) δ 1.83, 2.36, 2.46, 2.64, 3.59, 3.72, 4.02, 4.64, 7.30, 3.38, 7.93, 8.64, 10.45 ppm.
TLC R_{*f*} 0.41 (5% methanolic ammonia in dichloromethane).
IR (diffuse reflectance) 2951, 2938, 2797, 1653, 1607, 1552, 1501, 1413, 1306, 1289, 1276, 1216, 1144, 1116, 811 cm⁻¹
OAMS supporting ions at: ESI+ 537.2
HRMS (FAB) calcd for C₂₉H₃₃CLN₄O₄ +H₁ 537.2268, found 537.2271
Anal. Calcd for C₂₉H₃₃ClN₄O₄: C, 64.86; H, 6.19; N, 10.43; Cl, 6.60; found: C, 64.73; H, 6.16; N, 10.36.

### Example 50 N-[(4-Chlorophenyl)methyl]-9"-(4-morpholinylmethyl)dispiro[1,3-dioxolane-2,1'-cyclohexane-4',2"(3"H)-[7H]pyrido[1,2,3-de][1,4]benzoxazine]-6"-carboxamide (Formula M-8 of Chart M, where Rₐ, R_{b}=cyclohexane-4-ethylene ketal)

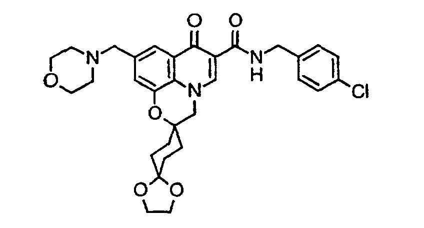

The tide compound is prepared according to the general procedure of Example **36,** and is purified by flash chromatography on silica gel using 2% methanol in dichloromethane. Recrystallization from acetonitrile affords white crystals, mp >260 °C.
¹H NMR (CDCl₃) δ 1.66, 1.84, 2.01, 2.46, 3.58, 3.71, 3.99, 4.02, 4.64, 7.30, 7.36, 7.92, 8.63, 10.47 ppm.
TLC R_{*f*} 0.43 (5% methanol in dichloromethane).
IR (diffuse reflectance) 2958, 2933, 1649, 1606, 1568, 1551, 1503, 1412, 1286, 1272, 1256, 1116, 1102, 917, 810 cm⁻¹
OAMS supporting ions at: ESI+ 580.3
HRMS (FAB) calcd for C₃₁H₃₄CLN₃O₆ +H₁ 580.2214, found 580.2227
Anal. Calcd for C₃₁H₃₄ClN₃O₆: C, 64.19; H, 5.91; N, 7.24; Cl, 6.11; found: C, 64.15; H, 5.92; N, 7.13.

### Example 51: N-(4-Chlorobenzyl)-2,2-bis[(methoxymethoxy)methyl]-9-(morpholin-4-ylmethyl)-7-oxo-2,3-dihydro-7H-[1,4]oxazino[2,3,4-ij]quinoline-6-carboxamide (Formula M-8 of Chart M, where Rₐ, R_{b}=methoxymethoxymethyl)

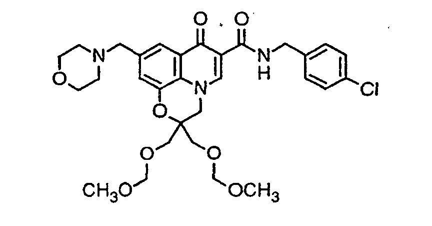

The title compound is prepared according to the general procedure of Example **36,** and is purified by flash chromatography on silica gel using 2% methanol in dichloromethane. Recrystallization from ethyl acetate affords white crystals, mp 147-149 °C.
¹H NMR (CDCl₃) δ 2.45, 3.33, 3.57, 3.66, 3.70, 3.77, 4.33, 4.63, 4.64, 7.31, 7.38, 7.94, 8.67, 10.45 ppm.
TLC R_{*f*} 0.40 (5% methanol in dichloromethane).
IR (diffuse reflectance) 2954, 2931, 2888, 1653, 1608, 1569, 1552, 1501, 1411, 1280, 1150, 1113, 1045, 919, 809 cm⁻¹
OAMS supporting ions at: ESI+ 602.2
HRMS (FAB) calcd for C₃₀H₃₆CLN₃O₈ +H₁ 602.2269, found 602.2269
Anal. Calcd for C₃₀H₃₆ClN₃O₈: C, 59.85; H, 6.03; N, 6.98; Cl, 5.89; found: C, 59.77; H, 5.96; N, 6.95.

### Example 52: N-(4-Chlorobenzyl)-2,2-bis(hydroxymethyl)-9-(morpholin-4-ylmethyl)-7-oxo-2,3-dihydro-7H-[1,4]oxazino[2,3,4-ij]quinoline-6-carboxamide (Formula M-8 of Chart M, where Rₐ, R_{b}=hydroxymethyl)

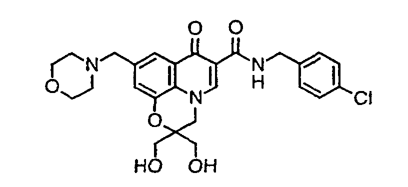

A mixture of 151 mg of the product of Example **51** in 2 mL of THF and 1 mL of conc. HCl is stirred for 18 h, then partitioned between chloroform and excess aqueous NaHCO₃. The organic phase is dried (Na₂SO₄) and concentrated under reduced pressure. Flash chromatography of the residue on silica gel using 7-10% methanol in dichloromethane affords 104 mg of the title compound as a white solid. Trituration and recrystallization from acetonitrile provides white solid with mp 230-233 °C.
¹H NMR (CDCl₃ + CD₃OD + TFA-*d*₆) δ 3.05, 3.40, 3.70, 3.78, 3.9, 4.33, 4.39, 4.64, 7.31, 7.49, 7.90, 8.73 ppm.
TLC R_{*f*} 0.24 (10% methanol in dichloromethane).
IR (diffuse reflectance) 1653, 1606, 1561, 1500, 1411, 1353, 1327, 1288, 1242, 1232, 1111, 1058, 886, 811, 795 cm⁻¹
OAMS supporting ions at: ESI+ 514.3
HRMS (FAB) calcd for C₂₆H₂₈CLN₃O₆ +H₁ 514.1744, found 514.1745
Anal. Calcd for C₂₆H₂₈ClN₃O₆: C, 60.76; H, 5.49; N, 8.18; Cl, 6.90; found: C, 60.52; H, 5.60; N, 8.22.

### Example 53: N-[(4-Chlorophenyl)methyl]-2',3',5',6'-tetrahydro-9-(4-morpholinylmethyl)-7-oxospiro[7H-pyrido[1,2,3-de]-1,4-benzoxazine-2(3H),4'-[4H]thiopyran]-6-carboxamide (Formula M-8 of Chart M, where Rₐ, R_{b}=4-tetrahydrothiopyran)

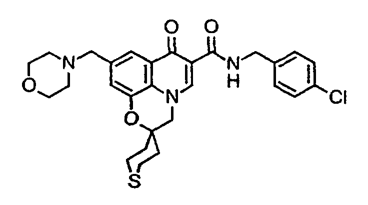

The product is prepared according to the general procedure of Example **36**, and is purified by flash chromatography on silica gel using 2% methanol in dichloromethane.
Recrystallization from ethyl acetate affords white crystals, mp 147-149 °C.
¹H NMR (CDCl₃) δ 1.88, 2.14, 2.47, 3.11, 3.59, 3.72, 3.99, 4.64, 7.30, 7.40, 7.94, 8.63, 10.43 ppm.
TLC R_{*f*} 0.35 (3% methanol in dichloromethane).
IR (diffuse reflectance) 1652, 1607, 1556, 1503, 1412, 1330, 1317, 1285, 1270, 1245, 1229, 1193, 1112, 810, 798 cm⁻¹
OAMS supporting ions at: ESI+ 540.1
HRMS (FAB) calcd for C₂₈H₃₀CLN₃O₄S +H₁ 540.1724, found 540.1722
Anal. Calcd for C₂₈H₃₀ClN₃O₄S: C, 62.27; H, 5.60; N, 7.78; Cl, 6.56; S, 5.94; found: C, 61.98; H, 5.71; N, 7.67.

### Example 54: N-[(4-Chlorophenyl)methyl]-9'-(4-morpholinylmethyl)-4,7'-dioxospiro[cyclohexane-1,2'(3'H)-[7H]pyrido[1,2,3-de] [1,4]benzoxazine]-6'-carboxamide (Formula M-8 of Chart M, where Rₐ, R_{b}=4-cyclohexanone)

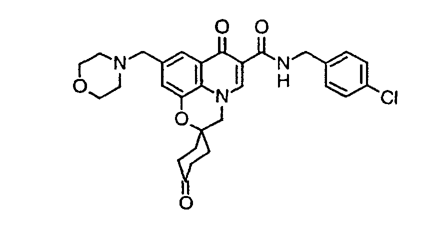

A mixture of 104 mg of compound **M-8** of Example **50** (where Rₐ, R_{b}=cyclohexane-4-ethylene ketal), 3 mL of acetone, 3 mL of THF, and 1 mL of 6N HCl is stirred for 18 h, the partitioned between chloroform and aqueous NaHCO₃. The organic phase is dried (Na₂SO₄) and concentrated under reduced pressure. Flash chromatography of the residue on silica gel using 2-3% methanol in dichloromethane affords 101 mg of the title compound as a white solid. Recrystallization from acetonitrile provides white needles, mp 249.5-252.0 °C.
¹H NMR (CDCl₃) δ 1.98, 2.24, 2.40, 2.47, 2.78, 3.61, 3.72, 4.13, 4.64, 7.31, 7.44, 7.98, 8.66, 10.42 ppm.
TLC R_{*f*} 0.33 (5% methanol in dichloromethane).
IR (diffuse reflectance) 1717, 1658, 1608, 1576, 1550, 1502, 1412, 1312, 1296, 1275, 1192, 1145, 1109, 875, 809 cm⁻¹
OAMS supporting ions at: ESI+ 536.2
HRMS (FAB) calcd for C₂₉H₃₀CLN₃O₅ +H₁ 536.1952, found 536.1951
Anal. Calcd for C₂₉H₃₀ClN₃O₅: C, 64.98; H, 5.64; N, 7.84; Cl, 6.61; found: C, 64.85; H, 5.83; N, 7.85.

### Example 55: N-[(4-Chlorophenyl)methyl]-4-hydroxy-9'-(4-morpholinylmethyl)-7'-oxospiro[cyclohexane-1,2'(3'H)-[7H]pyrido[1,2,3-de][1,4]benzoxazine]-6'-carboxamide (Formula M-8 of Chart M, where Rₐ, R_{b}=4-cyclohexanol)

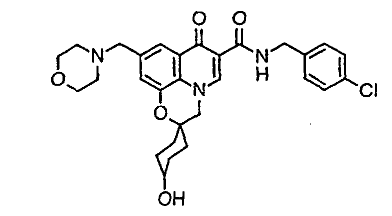

To a stirred solution of 118 mg of the produce of Example **54** in 2 mL of dichloromethane and 1 mL of methanol, cooled at 0 °C, is added 10 mg of sodium borohydride. After ten minutes, the reaction mixture is partitioned between water and dichloromethane, and the organic phase is dried (Na₂SO₄) and concentrated under reduced pressure. Flash chromatography of the residue on silica gel using 4-6% methanol in dichloromethane affords 69 mg of the title compound as a yellow solid. Recrystallization from acetonitrile provides pale yellow needles, mp 229-234 °C.
¹H NMR (CDCl₃) δ 1.54, 1.6-2.0, 2.46, 3.59, 3.72, 3.78, 4.00, 4.64, 7.3, 7.38, 7.93, 8.62, 10.47 ppm.
TLC R_{*f*} 0.29 (7% methanol in dichloromethane).
IR (diffuse reflectance) 1653, 1607, 1574, 1552, 1504, 1411, 1324, 1281, 1250, 1240, 1114, 1107, 881, 810, 801 cm⁻¹
OAMS supporting ions at: ESI+ 538.3
Anal. Calcd for C₂₉H₃₂ClN₃O₅: C, 64.74; H, 5.99; N, 7.81; Cl, 6.59; found: C, 64.63; H, 5.98; N, 7.79.

### Example 56: N-(4-Chlorobenzyl)-2-{[(4-chlorobenzyl)amino]methyl}-9-(morpholin-4-ylmethyl)-7-oxo-2,3-dihydro-7H-[1,4]oxazino[2,3,4-ij]quinoline-6-carboxamide (A-11 of Chart A, where R₆ is 4-chlorobenzylamino)

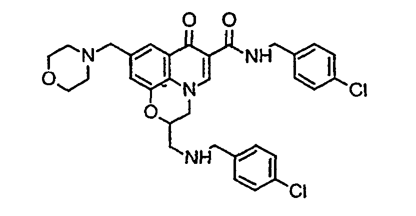

Following the procedure in Example **2**, the title compound is obtained.
¹H NMR (CDCl₃) δ 1.88, 2.45, 3.03, 3.57, 3.70, 3.85, 4.3, 4.4, 4.63, 7.30, 7.34, 7.92, 8.63, 10.45 ppm.
IR (diffuse reflectance) 2858, 1648, 1627, 1607, 1570, 1551, 1536, 1499, 1451, 1411, 1330, 1283, 1112, 811, 800 cm⁻¹.
OAMS supporting ions at: ESI+ 609.2
HRMS (FAB) calcd for C₃₂H₃₂CL₂N₄O₄ +H₁ 607.1879, found 607.1872.
Anal. Calcd for C₃₂H₃₂Cl₂N₄O₄: C, 63.26; H, 5.31; N, 9.22; Cl, 11.67; found: C, 63.12; H, 5.29; N, 9.19.

### Example 57: N-(4-Chlorobenzyl)-9-(morpholin-4-ylmethyl)-7-oxo-2-(R or S)-pyridin-2-yl-2,3-dihydro-7H-[1,4]oxazino[2,3,4-ij]quinoline-6-carboxamide (I-6 of Chart I, T=2-pyridyl)

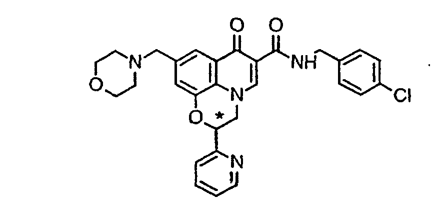

The enantiomers are prepared by resolution of racemic compound of Example **33** on chiral HPLC using a chiralcel OD column eluted with 0.025% diethylamine in ethanol, giving Isomer 1 (first eluting) and Isomer 2 (second eluting).
¹H NMR (CDCl₃) δ 2.47, 3.61, 3.72, 4.58, 4.63, 4.72, 5.51, 7.3, 7.50, 7.63, 7.79, 7.97, 8.63, 8.72, 10.44 ppm.
OAMS supporting ions at: ESI+ 531.3.

### Example 58: N-(4-Chlorobenzyl)-9-(morpholin-4-ylmethyl)-7-oxo-2-(R or S)-pyridin-3-yl-2,3-dihydro-7H-[1,4]oxazino[2,3,4-ij]quinoline-6-carboxamide (I-6 of Chart I, T=3-pyridyl)

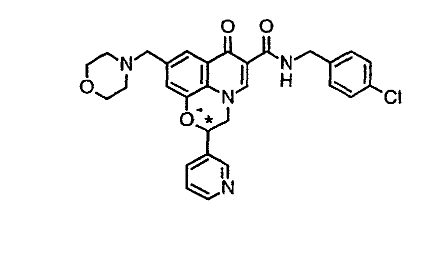

The enantiomers are prepared by resolution of racemic compound of Example **34** on chiral HPLC using a chiralcel OD column eluted with 0.1% diethylamine in ethanol, giving Isomer 1 (first eluting) and Isomer 2 (second eluting).
¹H NMR (CDCl₃) δ 2.46, 3.60, 3.71, 4.32, 4.47, 4.60, 5.37, 7.27, 7.44, 7.84, 7.99, 8.70, 8.72, 8.79, 10.41 ppm.
OAMS supporting ions at: ESI+ 531.2.

### Example 59: N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide (I-6 of Chart I, T=1-methylimidazol-2-yl)

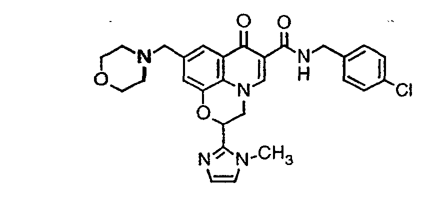

Following the procedure in Preparations **37-41** and Example **33** without making major changes except using 1-methylimidazole-2-carboxaldehyde as the starting aldehyde, the title compound is obtained as a light brown solid. Recrystallization from acetonitrile gives tan granular crystals, mp >260 °C.
¹H NMR (CDCl₃) δ 2.46, 3.56, 3.60, 3.70, 3.85, 4.63, 4.69, 4.95, 5.46, 7.00, 7.03, 7.30, 7.38, 7.97, 8.75, 10.43 ppm.
TLC Rf 0.23 (5% methanol in dichloromethane).
IR (diffuse reflectance) 3047, 2988, 2851, 1652, 1627, 1607, 1569, 1550, 1500, 1458, 1411, 1347, 1279, 1119, 810 cm⁻¹
OAMS supporting ions at: ESI+ 534.4
HRMS (FAB) calcd for C₂₈H₂₈CLN₅O₄ +H₁ 534.1908, found 534.1913
Anal. Calcd for C₂₈H₂₈ClN₅O₄: C, 62.98; H, 5.28; N, 13.11; Cl, 6.64; found: C, 63.06; H, 5.32; N, 13.11.

### Example 60: N-(4-Chlorobenzyl)-2-(2-furyl)-9-(morpholin-4-ylmethyl)-7-oxo-2,3-dihydro-7H-[1,4]oxazino[2,3,4-ij]quinoline-6-carboxamide (I-6 of Chart I, T-2-furyl)

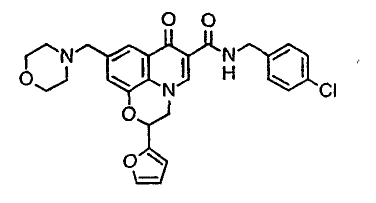

Following the procedure in Preparations **37-41** and Example **33** without making - major changes except using furfural as the starting aldehyde, the title compound is obtained as a light yellow solid. Recrystallization from acetonitrile gives tan needles, mp 220.0-222.5 °C.
¹H NMR (CDCl₃) δ 2.45, 3.58, 3.69, 4.48, 4.61, 4.63, 5.44, 6.44, 6.50, 7.30, 7.41, 7.50, 7.97, 8.70, 10.44 ppm.
TLC Rf 0.31 (3% methanol in dichloromethane).
IR (diffuse reflectance) 1655, 1627, 1608, 1551, 1500, 1411, 1347, 1330, 1282, 1225, 1114, 1011, 882, 809, 760 cm⁻¹
OAMS supporting ions at: ESI+ 520.3
HRMS (FAB) calcd for C₂₈H₂₆CLN₃O₅ +H₁ 520.1639, found 520.1638.
Anal. Calcd for C₂₈H₂₆ClN₃O₅: C, 64.68; H, 5.04; N, 8.08; Cl, 6.82; found: C, 64.47; H, 5.04; N, 8.08.

### Preparation 49: {3-[(Methoxymethoxy)methyl]phenyl}methanol (N-2 of Chart N, where substitution is meta, n=1, and P=methoxymethyl)

To a stirred, cooled (0 °C) solution of 6.91 g of 1,3-benzenedimethanol and 9.6 mL of diisopropylethylamine in 50 mL of dichloromethane is added 3.8 mL of chloromethyl methyl ether. After 90 min, 25 mL of 1N HCl is added, the phases separated, and the aqueous extracted with two additional portions of dichloromethane. The combined organic phase is dried (Na₂SO₄) and concentrated under reduced pressure. Flash chromatography on silica using 40% ethyl acetate in dichloromethane affords 4.76 g of the monoprotected compound as a pale yellow liquid.
¹H NMR (CDCl₃) δ 2.03, 3.41, 4.59, 4.68, 4.70, 7.3 ppm.
TLC R_{*f*} 0.36 (1:1 ethyl acetate in dichloromethane, vanillin staining).

### Preparation 50: 3-[(Methoxymethoxy)methyl]benzaldehyde (N-3 of Chart N, where substitution is meta, n=1, and P=methoxymethyl)

To a well-stirred mixture of 10.8 g of PCC and 820 mg of sodium acetate in 20 mL of dichloromethane is added a solution of 1.82 g of alcohol of Preparation **49** in 10 mL of dichloromethane. After 18 h, silica gel is added and the mixture filtered through a short plug of silica gel, which is rinsed with 1:1 ethyl acetate-dichloromethane. The filtrate is concentrated under reduced pressure, and the residue flash chromatographed on silica gel using 1:1 ether-hexane to afford 1.61 g of the title aldehyde as a colorless liquid.
¹H NMR (CDCl₃) δ 3.43, 4.68, 4.74, 7.53, 7.64, 7.82, 7.89, 10.03 ppm.
TLC R_{*f*} 0.32 (1:1 ether-hexane).

### Preparation 51: 2-Amino-1-{3-[(methoxymethoxy)methyl]phenyl}ethanol (N-5 of Chart N, where substitution is meta, n=1, and P=methoxymethyl)

To a mixture of 1.58 g of aldehyde of Preparation **50** and 1.3 mL of TMSCN, stirred neat under argon, is added a catalytic amount (*ca.* 10 mg) of ZnI₂. After 16 h, the resulting yellow liquid is dissolved in 15 mL of dry ether and added dropwise via cannula to a cooled (0 °C), mechanically stirred slurry of 370 mg of LAH in 20 mL of dry ether. Efficient stirring is required at this stage to assure complete reaction and reasonable product yield. Following the cyanohydrin addition, the reaction mixture is allowed to warm to ambient temperature and stirred vigorously for 2 h. The mixture is then recooled to 0 °C and quenched cautiously with 0.37 mL of water in 10 mL of THF, 0.37 mL of 3N NaOH, and 1.0 mL of water. Sodium sulfate and dichloromethane were added, and the mixture stirred well for 30-90 minutes, then filtered. The filter cake is washed well with ether-dichloromethane and then concentrated under reduced pressure to afford 2.08 g of the amino alcohol as a yellow oil.
¹H NMR (CDCl₃) δ 2.2, 2.81, 2.99, 3.42, 4.60, 4.6, 4.71, 7.3 ppm.
OAMS supporting ions at: ESI+ 212.1

### Preparation 52: Ethyl 2-(2,3-difluoro-5-iodobenzoyl)-3-[(2-hydroxy-2-{3-[(methoxymethoxy)methyl]phenyl}ethyl)amino]prop-2-enoate (N-6 of Chart N, where substitution is meta, n=1, and P=methoxymethyl)

To a stirred solution of 5.0 mmol of compound **I-1** (prepared as described in Preparation **38**) in 15 mL of ethanol is added 2.0g of crude amino alcohol of Preparation **51**. The solution is stirred for 18 h, then concentrated under reduced pressure. Flash chromatography of the residue on silica using 10-20% ethyl acetate in dichloromethane provides 2.31 g of the title compound as an yellow foam.
¹H NMR (CDCl₃) is complex due to *E*/*Z* isomers: δ 0.90, 1.02, 3.3, 3.6, 4.0, 4.58, 4.69, 4.85,7.3, 8.1 ppm.
TLC R_{*f*} 0.20 (10% ethyl acetate in dichloromethane).
OAMS supporting ions at: ESI+ 576.2.

### Preparation 53: Ethyl 9-iodo-2-{3-[(methoxymethoxy)methyl]phenyl}-7-oxo-2,3-dihydro-7H-[1,4]oxazino[2,3,4-ij]quinoline-6-carboxylate (N-7 of Chart N, where substitution is meta, n=1, and P=methoxymethyl)

A mixture of 2.3 g of compound **N-6** of Preparation **52** and 2.9 g of cesium carbonate in 8 mL of DMF is stirred at 100 °C under argon for 18 h, then cooled and partitioned between dichloromethane and dilute HCl. The organic phase is dried (Na₂SO₄) and concentrated under reduced pressure. Flash chromatography on silica gel using 2% methanol in dichloromethane provides 1.60 g of the title compound as a brown solid. Recrystallization from acetonitrile affords tan crystals, mp 153.3-156.0 °C.
¹H NMR (CDCl₃) δ 1.37, 3.44, 4.27, 4.32, 4.66, 4.75, 5.38, 7.5, 7.57, 8.22, 8.26 ppm.
TLC R_{*f*} 0.47 (5% methanol in dichloromethane).
IR (diffuse reflectance) 1680, 1632, 1610, 1586, 1550, 1496, 1316, 1257, 1224, 1182, 1148, 1113, 1048, 921, 799 cm⁻¹;
OAMS supporting ions at: ESI+ 536.1;
HRMS (FAB) calcd for C₂₃H₂₂INO₆ +H₁ 536.0572, found 536.0587;
Anal. Calcd for C₂₃H₂₂INO₆: C, 51.60; H, 4.14; N, 2.62; found: C, 51.40; H, 4.25; N, 2.50.

### Preparation 54: Ethyl 9-formyl-2-{3-[(methoxymethoxy)methyl]phenyl}-7-oxo-2,3-dihydro-7H-[1,4]oxazino[2,3,4-ij]quinoline-6-carboxylate (N-8 of Chart N, where substitution is meta, n=1, and P=methoxymethyl)

A mixture of 1.46 g of iodide N-7 of Preparation **53** and 220 mg of tetrakis (triphenylphosphine) palladium (0) in 15 mL of dry DMF is purged with CO, via a needle reaching to the bottom of the flask, and heated to 55 °C. To the stirred mixture is added, via syringe pump, 1.0 mL of tributyltin hydride in 9 mL of dry THF. Bubbling of CO through the mixture is continued throughout the addition, which took about 5 h. The mixture is then cooled and concentrated under reduced pressure. Flash chromatography of the residue on silica gel using 2% methanol in dichloromethane affords 970 mg of the title compound as a tan solid.
¹H NMR (CDCl₃) δ 1.41, 3.44, 4.33, 4.40, 4.67, 4.75, 5.36, 7.5, 7.80, 8.36, 8.54, 10.06 ppm.
TLC R_{*f*} 0.31 (3% methanol in dichloromethane).
OAMS supporting ions at: ESI+ 438.2.

### Preparation 55: Ethyl 2-{3-[(methoxymethoxy)methyl]phenyl}-9-(morpholin-4-ylmethyl)-7-oxo-2,3-dihydro-7H-[1,4]oxazino[2,3,4-ij]quinoline-6-carboxylate (N-9 of Chart N, where substitution is meta, n=1, and P=methoxymethyl)

To a mixture of 0.97 g of aldehyde of Preparation **54,** 0.58 mL of morpholine, and 0.25 mL of acetic acid in 20 mL of THF is added 940 mg of sodium triacetoxyborohydride. After 4 h, additional borohydride (300 mg) is added, and the mixture is stirred for 2 h and then partitioned between dichloromethane and aqueous NaHCO₃. The organic phase is dried (Na₂SO₄) and concentrated under reduced pressure. Flash chromatography of the residue on silica using 3-4% methanol in dichloromethane provides 940 mg of the title compound as a grey solid. Recrystallization from ethyl acetate gave white needles, mp 148-153 °C.
¹H NMR (CDCl₃) δ 1.37, 2.45, 3.44, 3.55, 3.69, 4.24, 4.3, 4.33, 4.67, 4.75, 5.36, 7.36, 7.47, 7.55, 7.93, 8.26 ppm.
TLC R_{*f*} 0.30 (5% methanol in dichloromethane).
IR (diffuse reflectance) 2953, 1686, 1638, 1608, 1555, 1506, 1322, 1291, 1271, 1221, 1183, 1148, 1115, 1041, 796 cm⁻¹;
OAMS supporting ions at: ESI+ 509.4;
HRMS (FAB) calcd for C₂₈H₃₂N₂O₇ +H₁ 509.2288, found 509.2285;
Anal. Calcd for C₂₈H₃₂N₂O₇: C, 66.13; H, 6.34; N, 5.51; found: C, 66.15; H, 6.41; N, 5.43.

### Example 61: N-(4-Chlorobenzyl)-2-{3-[(methoxymethoxy)methyl]phenyl}-9-(morpholin-4-ylmethyl)-7-oxo-2,3-dihydro-7H-[1,4]oxazino[2,3,4-ij]quinoline-6-carboxamide (N-10 of Chart N, where substitution is meta, n=1, and P=methoxymethyl)

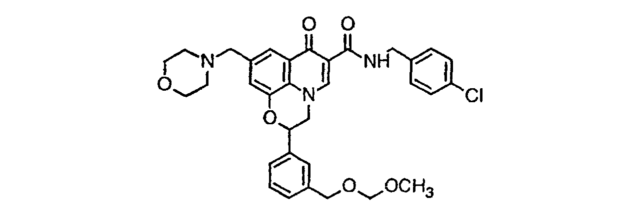

A mixture of 780 mg of ester of Preparation **55** and 1.3 g of *p*-chlorobenzylamine is stirred neat at 150 °C for 18 h, then concentrated *in vacuo*. Flash chromatography of the residue on silica using 2% methanol in dichloromethane provides 790 mg of the title compound as a white solid. Recrystallization from acetonitrile-methanol affords fine white needles, mp 186.5-189.0 °C.
¹H NMR (CDCl₃) δ 2.46, 3.43, 3.59, 3.70, 4.30, 4.41, 4.60, 4.65, 4.74, 5.30, 7.27, 7.44, 7.52, 7.97, 8.68, 10.44 ppm.
TLC R_{*f*} 0.29 (3% methanol in dichloromethane).
IR (diffuse reflectance) 1649, 1607, 1552, 1501, 1411, 1350, 1284, 1270, 1223, 1147, 1115, 1092, 1047, 810, 799 cm⁻¹;
OAMS supporting ions at: ESI+ 604.3;
HRMS (FAB) calcd for C₃₃H₃₄CLN₃O₆ +H₁ 604.2214, found 604.2225;
Anal. Calcd for C₃₃H₃₄ClN₃O₆: C, 65.61; H, 5.67; N, 6.96; Cl, 5.87; found: C, 65.57; H, 5.72; N, 6.90.

### Example 62: N-(4-Chlorobenzyl)-2-[3-(hydroxymethyl)phenyl]-9-(morpholin-4-ylmethyl)-7-oxo-2,3-dihydro-7H-[1,4]oxazino[2,3,4-ij]quinoline-6-carboxamide (N-11 of Chart N, where substitution is meta, n=1, and P=methoxymethyl)

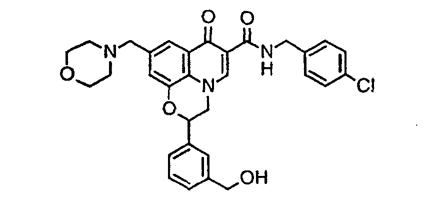

A solution of 122 mg of **N-10** of Example **61** in 1 mL of ethanol and 0.5 mL of conc. HCl is stirred for 36 h at room temperature, then added to stirred aqueous NaHCO₃. The resulting solid is filtered, washed well with water, dried under vacuum, and then absorbed onto silica gel. Flash chromatography on silica gel using 3-5% methanol in dichloromethane provides 108 mg of the title compound as a white solid. Recrystallization from acetonitrile-methanol provides material with mp 229-233 °C.
¹H NMR (CDCl₃+CD₃OD) δ 2.49, 3.62, 3.72, 4.30, 4.49, 4.62, 4.72, 5.32, 7.30, 7.43, 7.54, 7.95, 8.63, 10.58 ppm.
TLC R_{*f*} 0.25 (5% methanol in dichloromethane).
IR (diffuse reflectance) 1653, 1606, 1570, 1549, 1542, 1499, 1409, 1325, 1292, 1277, 1219, 1114, 1077, 807, 800 cm⁻¹;
OAMS supporting ions at: ESI+ 560.3;
HRMS (FAB) calcd for C₃₁H₃₀CLN₃O₅ +H₁ 560.1952, found 560.1945;
Anal. Calcd for C₃₁H₃₀ClN₃O₅: C, 66.48; H, 5.40; N, 7.50; Cl, 6.33; found (av): C, 64.94; H, 5.33; N, 7.29.

### Preparation 56: 3-{[tert-Butyl(dimethyl)silyl]oxy}benzaldehyde (N-3 of Chart N, where substitution is meta, n=0, and P=tert-butyldimethylsilyl)

A solution of 3.66 g of 3-hydroxybenzaldehyde, 3.1 g of imidazole, and 4.39 g of *tert*-butyldimethylsilyl chloride in 10 ml of DMF is stirred at room temperature for 18 h, then partitioned between ether and water. The organic phase is washed with water, dried (MgSO₄), and concentrated under reduced pressure. Flash chromatography of the residue on silica gel using 5% ether-hexane provides 5.14 g of the title compound as a pale yellow liquid.
¹H NMR (CDCl₃) 0.22, 1.00, 7.11, 7.33, 7.40, 7.48, 9.96 ppm.
TLC R_{*f*} 0.28 (5% ether-hexane).
OAMS supporting ions at: ESI+ 237.1.

### Preparation 57: 2-Amino-1-(3-{[tert-butyl(dimethyl)silyl]oxy}phenyl)ethanol (N-5 of Chart N, where substitution is meta, n=0, and P=tert-butyldimethylsilyl)

The procedure described in Preparation **51** is followed starting with 5.14 g of the product of Preparation **56** to provide 6.96 g of crude amino alcohol as an amber oil.
¹H NMR (CDCl₃) δ 0.19, 0.98, 2.1, 2.79, 2.97, 4.59, 6.74, 6.85, 6.93, 7.19 ppm.
OAMS supporting ions at: ESI+ 268.3.

### Preparation 58: Ethyl 3-{[2-(3-{[tert-butyl(dimethyl)silyl]oxy}phenyl)-2 hydroxyethyl]amino}-2-(2,3-difluoro-5-iodobenzoyl)prop-2-enoate (N-6 of Chart N, where substitution is meta, n=0, and P=tert-butyldimethylsilyl)

The procedure described in Preparation **52** is followed starting with 5.14 g of the product of Preparation **57**. Flash chromatography on silica using 30% ethyl acetate in hexane affords 4.11 g of the title compound as a yellow foam.
¹H NMR (CDCl₃) is complex due to *E*/*Z* isomers: δ 0.20, 0.99, 1.04, 2.6, 3.6, 4.0, 4.8, 6.8, 6.9, 7.2, 7.4, 8.1, 9.68, 11.03 ppm.
TLC R_{*f*} 0.28 (30% ethyl acetate-hexane).
OAMS supporting ions at: ESI+ 632.2.

### Preparation 59: Ethyl 2-(3-{[tert-butyl(dimethyl)silyl}oxy}phenyl)-9-iodo-7-oxo-2,3-dihydro-7H-[1,4]oxazino[2,3,4-ij]quinoline-6-carboxylate (N-7 of Chart N, where substitution is meta, n=0, and P=tert-butyldimethylsilyl)

A mixture of 3.44 g of the product of Preparation **58** and 3.9 g of cesium carbonate in 10 ml of DMF is stirred and heated at 100 °C under argon for 18 h, then cooled. Acetic acid (1 ml) is added, followed by 75 ml of water, and dilute HCl is added to bring the pH to about 2. The resulting solid is filtered, washed well with water, and dried *in vacuo* to provide 2.53 g of insoluble yellow-tan solid. To a mixture of this solid and 723 mg of imidazole in 6 ml of DMF is, added 1.20 g of *tert*-butyldimethylsilyl chloride. The solution is stirred overnight, then partitioned between ethyl acetate and water. The organic phase is washed with water and brine, dried (MgSO₄), and concentrated under reduced pressure. Flash chromatography on silica using 20-30% ethyl acetate in dichloromethane provides 2.15 g of the title compound as a tan solid. Recrystallization from acetonitrile provides an analytical sample; mp 227.0-228.5 °C.
¹H NMR (CDCl₃) δ 0.23, 1.00, 1.36, 4.25, 4.30, 5.34, 6.91, 6.99, 7.11, 7.34, 7.56, 8.19, 8.21 ppm.
TLC R_{*f*} 0.36 (20% ethyl acetate in dichloromethane).
IR (diffuse reflectance) 1679, 1630, 1609, 1586, 1549, 1493, 1369, 1319, 1291, 1251, 878, 865, 840, 803, 786 cm⁻¹;
OAMS supporting ions at: ESI+ 592.3;
HRMS (FAB) calcd for C₂₆H₃₀INO₅SI +H₁ 592.1018, found 592.1013;
Anal. Calcd for C₂₆H₃₀INO₅Si: C, 52.79; H, 5.11; N, 2.37; found: C, 52.57; H, 5.15; N, 2.34.

### Preparation 60: Ethyl 2-(3-{[tert-butyl(dimethyl)silyl]oxy}phenyl)-9-formyl-7-oxo-2,3-dihydro-7H-[1,4]oxazino[2,3,4-ij]quinoline-6-carboxylate (N-8 of Chart N, where substitution is meta, n=0, and P=tert-butyldimethylsilyl)

A mixture of 592 mg of the product of Preparation **59** and 81 mg of tetrakis (triphenylphosphine) palladium (0) in 5 ml of dry THF is purged with CO, via a needle reaching to the bottom of the flask, and heated to 50 °C. To the stirred mixture is added, via syringe pump, 0.35 ml of tributyltin hydride in 10 ml of dry THF. Bubbling of CO through the mixture is continued throughout the addition, which takes about 5 h. The mixture is then cooled and concentrated under reduced pressure. Flash chromatography of the residue on silica gel using 1-2% methanol in dichloromethane affords 625 mg of tan solid. This is triturated with ether-hexane, filtered, washed well with hexane, and dried *in vacuo* to provide 443 mg of the aldehyde as a tan solid. Recrystallization from acetonitrile affords white needles, mp 246-248 °C.
¹H NMR (CDCl₃) δ 0.22, 0.99, 1.41, 4.3, 4.39, 5.31, 6.9, 7.07, 7.35, 7.80, 8.36, 8.52, 10.05 ppm.
TLC R_{*f*} 0.31 (2% methanol in dichloromethane).
Summary Analytical data for: 0071676; nbk# 35116-SRT-37A:
IR (diffuse reflectance) 1727, 1679, 1646, 1603, 1556, 1501, 1373, 1288, 1258, 899, 877, 861, 840, 804, 787 cm⁻¹;
OAMS supporting ions at: ESI+ 494.3;
HRMS (FAB) calcd for C₂₇H₃₁NO₆SI +H₁ 494.1999, found 494.2005;
Anal. Calcd for C₂₇H₃₁NO₆Si: C, 65.69; H, 6.33; N, 2.84; found: C, 65.73; H, 6.41; N, 2.93

### Preparation 61: Ethyl 2-(3-{[tert-butyl(dimethyl)silyl]oxy}phenyl)-9-(4-morpholinylmethyl)-7-oxo-2,3-dihydro-7H-[1,4]oxazino[2,3,4-ij]quinoline-6-carboxylate (N-9 of Chart N, where substitution is meta, n=0, and P=tert-butyldimethylsilyl)

To a mixture of 217 mg of the product of Preparation **60**, 0.12 ml of morpholine, and 50 µL of acetic acid in 4 ml of THF is added 140 mg of sodium triacetoxyborohydride. After 18 h, additional borohydride (120 mg) is added, and the mixture is stirred for 2 h and then partitioned between dichloromethane and aqueous NaHCO₃. The organic phase is dried (Na₂SO₄) and concentrated under reduced pressure. Flash chromatography of the residue on silica using 3% methanol in dichloromethane provides 200 mg of the product as a white solid. Recrystallization from acetonitrile gives white crystals, mp 161-164 °C.
¹H NMR (CDCl₃) δ 0.23, 1.00, 1.39, 2.46, 3.57, 3.70, 4.22, 4.30, 4.35, 5.27, 6.91, 6.98, 7.10, 7.34, 7.38, 7.96, 8.29 ppm.
TLC R_{*f*} 0.33 (5% methanol in dichloromethane).
IR (diffuse reflectance) 2955, 2931, 1725, 1692, 1607, 1555, 1503, 1287, 1261, 1253, 1222, 1115, 878, 863, 840 cm⁻¹;
OAMS supporting ions at: ESI+ 565.4;
HRMS (FAB) calcd for C₃₁H₄₀N₂O₆SI +H₁ 565.2734, found 565.2740;
Anal. Calcd for C₃₁H₄₀N₂O₆Si: C, 65.93; H, 7.14; N, 4.96; found (av): C, 65.35; H, 7.16; N, 4.88.

### Preparation 62: 2-(3-{[tert-Butyl(dimethyl)silyl]oxy}phenyl)-N-(4-chlorobenzyl)-9-(4-morpholinylmethyl)-7-oxo-2,3-dihydro-7H-[1,4]oxazino[2,3,4-ij]quinoline-6-carboxamide (N-10 of Chart N, where substitution is meta, n=0, and P=tert-butyldimethylsilyl)

A mixture of 341 mg of the product of Preparation **61** and 0.70 g of *p*-chlorobenzylamine is stirred neat at 150 °C for 18 h, then concentrated *in vacuo*. Flash chromatography of the residue on silica using 2-6% methanol in dichloromethane provides 233 mg of the title compound as a tan solid, as well as 130 mg of desilylated material. Recrystallization of the main product from acetonitrile gives yellow prisms, mp 174-180 °C.
¹H NMR (CDCl₃) δ 0.22, 1.00, 2.48, 3.61, 3.71, 4.27, 4.37, 4.63, 5.25, 6.92, 6.96, 7.06, 7.30, 7.34, 7.47, 7.98, 8.66, 10.45 ppm.
TLC R_{*f*} 0.29 (3% methanol in dichloromethane).
IR (diffuse reflectance) 1655, 1607, 1569, 1551, 1499, 1411, 1281, 1262, 1114, 882, 863, 840, 809, 798, 782 cm⁻¹;
OAMS supporting ions at: ESI+ 660.5;
HRMS (FAB) calcd for C₃₆H₄₂CLN₃O₅SI +H, 660.2660, found 660.2632;
Anal. Calcd for C₃₆H₄₂ClN₃O₅Si: C, 65.49; H, 6.41; N, 6.36; Cl, 5.37; found: C, 65.42; H, 6.36; N, 6.36.

### Example 63: N-(4-Chlorobenzyl)-2-(3-hydroxyphenyl)-9-(4-morpholinylmethyl)-7-oxo-2,3-dihydro-7H-[1,4]oxazino[2,3,4-ij]quinoline-6-carboxamide (N-11 of Chart N, where substitution is meta, n=0, and P=tert-butyldimethylsilyl)

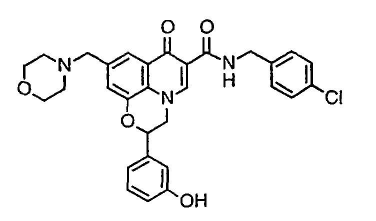

A solution of 167 mg of the product of Preparation **62** in 4 ml of ethanol and 2 ml of conc. HCl is stirred for 18 h at room temperature, then added to a stirred solution of 2 g NaHCO₃ in 50 ml of water. The resulting solid is filtered, washed well with water, dried under vacuum, and then absorbed onto silica gel. Flash chromatography on silica using 3-5% methanol in dichloromethane provides 126 mg of the title compound as a white solid. Recrystallization from acetonitrile gives glistening prisms, mp 213 °C (d).
¹H NMR (CDCl₃+CD₃OD) δ 2.49, 3.62, 3.72, 4.29, 4.48, 4.63, 5.25, 6.89, 6.97, 7.30, 7.44, 7.94, 8.62, 10.61 ppm.
TLC R_{*f*} 0.35 (5% methanol in dichloromethane).
Summary Analytical data for: 0071891; nbk# 35116-SRT-53A:
IR (diffuse reflectance) 3059, 2864, 2843, 1655, 1608, 1586, 1569, 1550, 1531, 1499, 1408, 1335, 1286, 1278, 1120 cm⁻¹;
OAMS supporting ions at: ESI+ 546.3;
HRMS (FAB) calcd for C₃₀H₂₈CLN₃O₅ +H₁ 546.1796, found 546.1781;
Anal. Calcd for C₃₀H₂₈ClN₃O₅: C, 65.99; H, 5.17; N, 7.70; Cl, 6.49; found: C, 65.57; H, 5.27; N, 8.57.

### Example 64: N-(4-Chlorobenzyl)-2-[2-(methoxymethoxy)phenyl]-9-(morpholin-4-ylmethyl)-7-oxo-2,3-dihydro-7H-[1,4]oxazino[2,3,4-ij]quinoline-6-carboxamide (I-6 of Chart I, T=2-methoxymethoxyphenyl)

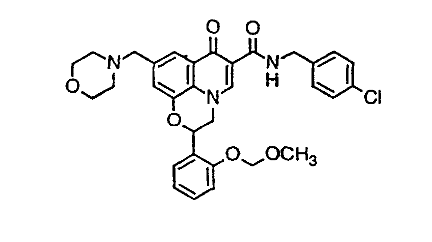

Following the procedures in Preparations **49** and **51-55** and Example **61** without making major changes except using 2-hydroxybenzaldehyde as the starting aldehyde, the title compound is obtained as a white solid. Recrystallization from acetonitrile gives 324 mg of white solid.
¹H NMR (CDCl₃) δ 2.47, 3.49, 3,61, 3.71, 4.15, 4.50, 4.63, 4.64, 5.25, 5.29, 5.65, 7.15, 7.3, 7.39, 7.47, 7.61, 7.98, 8.68, 10.49 ppm.
TLC Rf 0.34 (3% methanol in dichloromethane).
OAMS supporting ions at: ESI+ 590.3.

### Example 65: N-(4-Chlorobenzyl)-2-(4-hydroxyphenyl)-9-(morpholin-4-ylmethyl)-7-oxo-2,3-dihydro-7H-[1,4]oxazino[2,3,4-ij]quinoline-6-carboxamide (I-6 of Chart I, T=4-hydroxyphenyl)

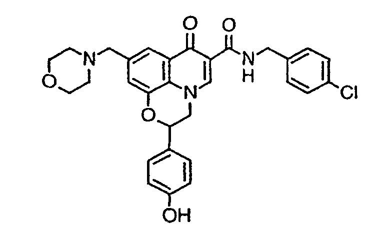

Following the procedures in Preparations **56**, **51-52**, and **59-62** and Example **63** without making major changes except using 4-hydroxybenzaldehyde as the starting aldehyde, the title compound is obtained as a beige solid. Recrystallization from acetonitrile gives pale yellow crystals, mp >260 °C:
¹H NMR (CDCl₃+ CD₃OD) δ 2.49, 3.63, 3.72, 4.33, 4.47, 4.64, 5.24, 6.93, 7.31, 7.34, 7.44, 7.95, 8.65, 10.62 ppm.
TLC Rf 0.35 (5% methanol in dichloromethane).
IR (diffuse reflectance) 3035, 1654, 1608, 1554, 1518, 1500, 1410, 1298, 1284, 1270, 1253, 1230, 1115, 809, 797 cm⁻¹
OAMS supporting ions at: ESI+ 546.3
HRMS (FAB) calcd for C₃₀H₂₈CLN₃O₅ +H₁ 546.1796, found 546.1793
Anal. Calcd for C₃₀H₂₈ClN₃O₅: C, 65.99; H, 5.17; N, 7.70; Cl, 6.49; found (av): C, 65.37; H, 5.19; N, 7.60.

### Example 66: N-(4-Chlorobenzyl)-2-{2-[(methoxymethoxy)methyl]phenyl}-9-(morpholin-4-ylmethyl)-7-oxo-2,3-dihydro-7H-[1,4]oxazino[2,3,4-ij]quinoline-6-carboxamide (I-6 of Chart I, T=2-methoxymethoxymethylphenyl)

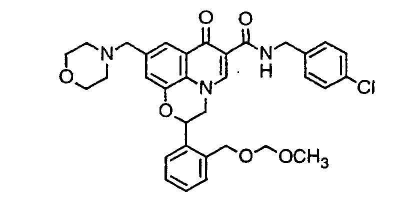

Following the procedures in Preparations **49-55** and Example **61** without making major changes except using 1,2-benzenedimethanol as the starting alcohol, the title compound is obtained as a white solid. Recrystallization from acetonitrile-methanol affords white crystals, mp 202.5-204.5 °C.
¹H NMR (CDCl₃) δ 2.48, 3.32, 3.62, 3.71, 4.23, 4.52, 4.6-4.7, 4.77, 5.57, 7.30, 7.4, 7.5, 7.66, 7.99, 8.64, 10.47 ppm.
TLC Rf 0.30 (3% methanol in dichloromethane).
IR (diffuse reflectance) 1652, 1607, 1569, 1552, 1500, 1410, 1352, 1283, 1228, 1150, 1116, 1092, 1034, 810, 748 cm⁻¹
OAMS supporting ions at: ESI+ 604.3
HRMS (FAB) calcd for C₃₃H₃₄CLN₃O₆ +H₁ 604.2214, found 604.2215
Anal. Calcd for C₃₃H₃₄ClN₃O₆: C, 65.61; H, 5.67; N, 6.96; Cl, 5.87; found: C, 65.40; H, 5.69; N, 6.95.

### Example 67: N-(4-Chlorobenzyl)-2-{4-[(methoxymethoxy)methyl]phenyl}-9-(morpholin-4-ylmethyl)-7-oxo-2,3-dihydro-7H-[1,4]oxazino[2,3,4-ij]quinoline-6-carboxamide (I-6 of Chart I, T=4-methoxymethoxymethylphenyl)

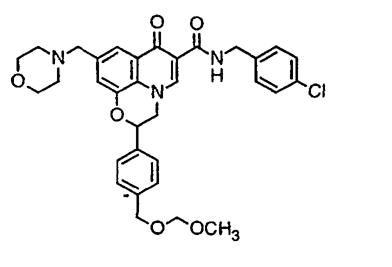

Following the procedures in Preparations **49-55** and Example **61** without making major changes except using 1,4-benzenedimethanol as the starting alcohol, the title compound is obtained as a tan solid. Recrystallization from acetonitrile-methanol affords tan crystals, mp 236-239 °C.
¹H NMR (CDCl₃) δ 2.47, 3.44, 3.60, 3.70, 4.29, 4.37, 4.63, 4.65, 4.74, 5.31, 7.3, 7.45, 7.49, 7.98, 8.67, 10.44 ppm.
TLC Rf 0.27 (3% methanol in dichloromethane).
IR (diffuse reflectance) 2931, 2852, 1653, 1607, 1569, 1551, 1536, 1500, 1411, 1328, 1280, 1149, 1113, 1044, 809 cm⁻¹
OAMS supporting ions at: ESI+ 604.3
HRMS (FAB) calcd for C₃₃H₃₄CLN₃O₆ +H₁ 604.2214, found 604.2219
Anal. Calcd for C₃₃H₃₄ClN₃O₆: C, 65.61; H, 5.67; N, 6.96; Cl, 5.87; found: C, 65.49; H, 5.73; N, 6.90.

### Example 68: N-(4-Chlorobenzyl)-2-(2-hydroxyphenyl)-9-(morpholin-4-ylmethyl)-7-oxo-2,3-dihydro-7H-[1,4]oxazino[2,3,4-ij]quinoline-6-carboxamide (I-6 of Chart I, T=2-hydroxyphenyl)

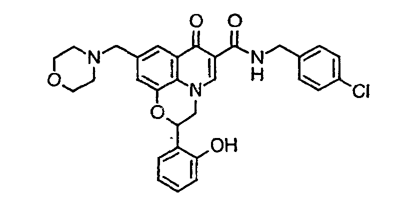

Following the procedure in Example **62** using the compound of Example **64** as the starting material, the title compound is obtained as a white solid. Trituration with hot acetonitrile affords an analytical sample; mp ∼239 °C.
¹H NMR (CDCl₃) δ 2.48, 3.61, 3.71, 4.09, 4.62, 4.68, 5.63, 6.88, 6.97, 7.17, 7.3, 7.49, 7.52, 7.97, 8.57, 9.5, 10.86 ppm.
TLC Rf 0.31 (5% methanol in dichloromethane).
IR (diffuse reflectance) 3162, 1645, 1603, 1568, 1550, 1499, 1457, 1411, 1353, 1331, 1300, 1282, 1272, 1221, 809 cm⁻¹
OAMS supporting ions at: ESI+ 545.9
HRMS (FAB) calcd for C₃₀H₂₈CLN₃O₅ +H₁ 546.1796, found 546.1796
Anal. Calcd for C₃₀H₂₈ClN₃O₅: C, 65.99; H, 5.17; N, 7.70; Cl, 6.49; found: C, 65.89; H, 5.23; N, 7.59.

### Example 69: N-(4-Chlorobenzyl)-2-[2-(hydroxymethyl)phenyl]-9-(morpholin-4-ylmethyl)-7-oxo-2,3-dihydro-7H-[1,4]oxazino[2,3,4-ij]quinoline-6-carboxamide (I-6 of Chart I, T=2-hydroxymethylphenyl)

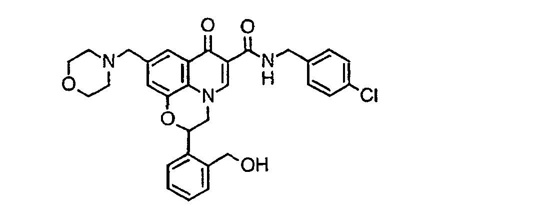

Following the procedure in Example **62** using the compound of Example **66** as the starting material, the title compound is obtained as a white solid. Recrystallization from acetonitrile gives white crystals, mp 218-223 °C (d).
¹H NMR (CDCl₃+CD₃OD) δ 2.50, 3.65, 3.73, 4.24, 4.63, 4.67, 4.72, 4.83, 5.69, 7.3, 7.4-7.5, 7.65, 7.96, 8.59, 10.62 ppm.
TLC Rf 0.32 (5% methanol in dichloromethane).
IR (diffuse reflectance) 3406, 2863, 1670, 1609, 1569, 1551, 1500, 1410, 1349, 1330, 1285, 1105, 808, 798, 752 cm⁻¹
OAMS supporting ions at: ESI+ 560.3
HRMS (FAB) calcd for C₃₁H₃₀CLN₃O₅ +H₁ 560.1952, found 560.1947
Anal. Calcd for C₃₁H₃₀ClN₃O₅: C, 66.48; H, 5.40; N, 7.50; Cl, 6.33; found: C, 66.11; H, 5.42; N, 7.47.

## Claims

**1.** A compound of formula I 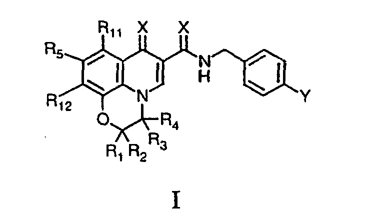 or a pharmaceutically acceptable salt, racemate, solvate, tautomer or optical isomer thereof wherein:
each X is independently O or S;
Y is Cl, F, Br, CN or NO₂;
R₁, R₂, R₃ and R₄ are independently
a) hydrogen,
b) N₃,
c) CN,
d) fluoro,
e) trifluoromethyl,
f) aryl,
g) het,
h) C₁₋₈ alkyl, optionally substituted with R₆ or OR₇, or
i) R₁ and R₂ or R₃ and R₄ together with the carbon to which they are attached form C₃₋₈cycloalkyl or het;
R₅ is C₁₋₈alkyl, which may be partially unsaturated and optionally substituted with one to three N₃, halo, CN, R₆ or R₇;
R₆ is
a) aryl,
b) het,
c) SOᵢR₈,
d) OR₈,
e) C(=O)OR₈,
f) C(=O)R₈, or
g) NR₈R₉;
R₇ is
a) P(=O)(OR₁₀)₂,
b) CO(CH₂)ⱼCON(CH₃)(CH₂)ₖSO₃⁻M⁺,
c) an amino acid,
d) C(=O)C₁₋₆alkyl, optionally substituted by NR¹⁰R¹⁰, or
e) CO(CH₂)ₙCO₂H;
R₈ and R₉ are independently
a) hydrogen,
b) C₃₋₈cycloalkyl,
c) aryl,
d) het, or
e) C₁₋₈alkyl which is further optionally substituted with one or more aryl, het, halo, CN, CO₂R₁₀, SOᵢR₁₀, OR₁₀, NR₁₀R₁₀, CF₃, or C₃₋₈cycloalkyl;
R₁₀ is
a) H or
b) C₁₋₈alkyl, optionally substituted with OH or OC₁₋₄alkyl;
R₁₁ and R₁₂ are independently
a) hydrogen,
b) halo,
c) NO₂,
d) CN,
e) R₆,
f) SOᵢNR₈R₉, or
g) C₁₋₈alkyl, which may be partially unsaturated and optionally substituted with one to three N₃, halo, CN, R₆ or OR₇;
aryl is
a phenyl radical, optionally fused with a saturated or unsaturated carbocyclic or heterocyclic ring; at each occurrence, aryl may be substituted with one or more halo, CN, CO₂R₁₀, SOᵢR₁₀, OR₁₀, NR₁₀R₁₀, CF₃, C₃₋₈cycloalkyl, or C₁₋₄alkyl wherein C₁₋₄alkyl is optionally substituted with OR₁₀;
het is
a four- (4), five- (5), six- (6), or seven- (7) membered saturated or unsaturated heterocyclic ring having 1, 2, or 3 heteroatoms selected from the group consisting of O, S, and NW, wherein W is hydrogen, C₁₋₄alkyl, C(=O)OC₁₋₄alkyl or absent, wherein het is optionally fused with a benzene ring, a carbcyclic or a heterocyclic ring; at each occurrence, het may be substituted with one or more halo, CN, CO₂R₁₀, SOᵢR₁₀, OR₁₀, NR₁₀R₁₀, C₁₋₄alkyl, CF₃, C₃₋₈cycloalkyl, oxo or oxine;
at each occurrence, a cycloalkyl may be substituted with C₁₋₄alkyl, OR¹⁰, oxo, oxine, or a spiro fused het;
i is 0, 1 or 2;
j is 1, 2, 3, 4, 5, or 6;
k is 1, 2, 3, 4, 5, or 6;
n is 1, 2, 3, 4, 5, or 6;
M is sodium, potassium, or lithium; and
with the following provisos:
a) at least one of R₁, R₂, R₃ and R₄ is other than hydrogen;
c) where R₁, R₂, R₃ and R₄ are independently C₁₋₈ alkyl, at least one of the alkyl groups is substituted with R₆ or OR₇.

**2.** A compound of formula I according to claim 1 which is formula I-A

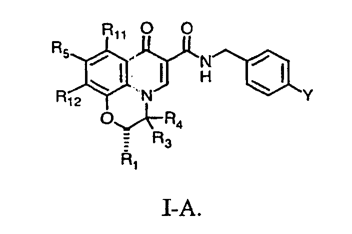

**3.** A compound of formula I according to claim 1 which is formula I-B

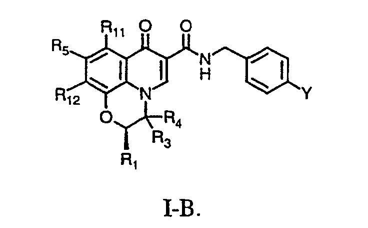

**4.** A compound of formula I according to claim 1 which is formula I-C

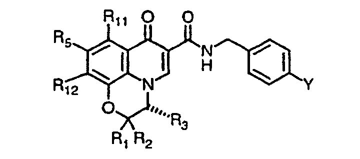

**5.** A compound of formula I according to claim 1 which is formula I-D

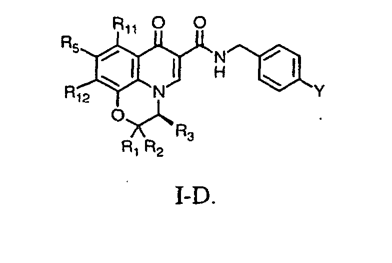

**6.** A compound of formula I according to claim 1 which is formula I-E

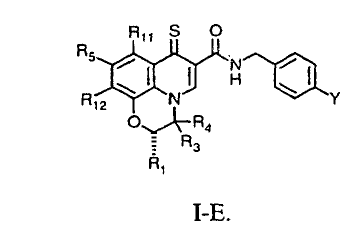

**7.** A compound of formula I according to claim 1 which is formula I-F

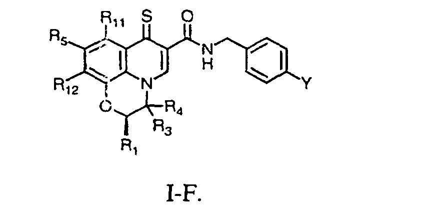

**8.** A compound of formula I according to claim 1 which is formula I-G

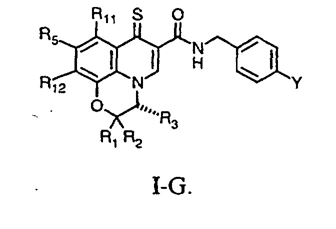

**9.** A compound of formula I according to claim 1 which is formula I-H

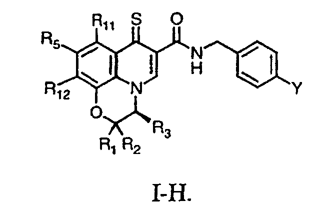

**10.** A compound of formula I according to claim 1 which is formula I-I

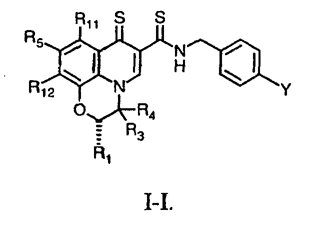

**11.** A compound of formula I according to claim 1 which is formula I-J

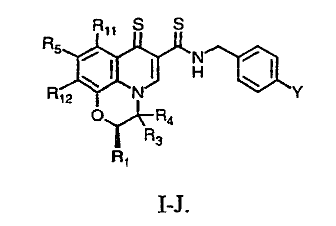

**12.** A compound of formula I according to claim 1 which is formula I-K

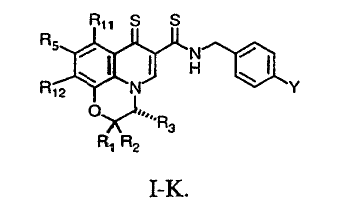

**13.** A compound of formula I according to claim 1 which is formula I-L

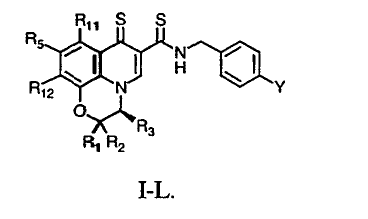

**14.** A compound of formula I according to claim 1 which is formula I-M

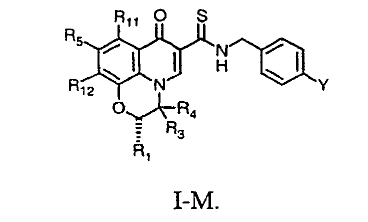

**15.** A compound of formula I according to claim 1 which is formula I-N

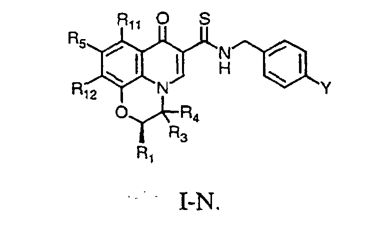

**16.** A compound of formula I according to claim 1 which is formula I-O

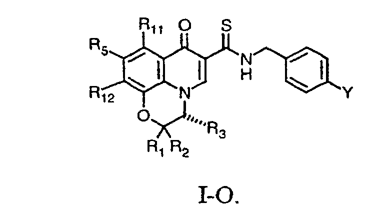

**17.** A compound of formula I according to claim 1 which is formula I-P

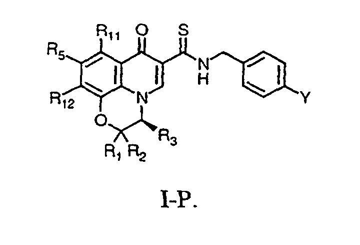

**18.** A compound of claims 1 to 17 wherein R₁₁ is H, halo, or C₁₋₄alkyl optionally substituted with one to three halo; and
R₁₂ is
a) H,
b) SO_{I}R₈,
c) OR₈,
d) C(=O)OR₈,
e) C(=O)R₈,
f) NR₈R₉,
g) SO₁R₈R₉, or
h) C₁₋₈alkyl, which may be partially unsaturated and optionally substituted with one to three N₃, halo, CN, or R₆.

**19.** A compound of claim 18 wherein R₁₁ and R₁₂ are hydrogen.

**20.** A compound of claim 19 wherein R₅ is C₁₋₈alkyl substituted with OR₈ or het.

**21.** A compound of claim 19 wherein R₅ is C₁₋₆alkyl substituted with OH.

**22.** A compound of claim 19 wherein R₅ is C₁₋₄alkyl substituted with het.

**23.** A compound of claim 22 wherein het is morpholinyl or thiomorpholinyl.

**24.** A compound claim 19 wherein R₅ is 4-morpholinylmethyl.

**25.** A compound of claim 19 wherein R₅ is C₁₋₈alkyl which is partially unsaturated and optionally substituted with OR₈.

**26.** A compound of claim 19 wherein R₅ is propynyl.

**27.** A compound of claim 26 wherein propynyl is substituted with OH.

**28.** A compound of claims 20 to 27 wherein Y is Cl.

**29.** A compound of claims 28 wherein R₃ and R₄ are independently hydrogen.

**30.** A compound of claim 29 wherein R₁ and R₂ are independently
a) hydrogen,
b) fluoro,
c) C₁₋₈ alkyl substituted with R₆ or OR₇;
d) aryl,
e) het, or
f) R₁ and R₂ together with the carbon to which they are attached form a six-(6) membered cycloalkyl or a het;
wherein R₆ is
a) het,
b) SOᵢR₈,
c) OR₈ or
d) NR₈R₉;
wherein R₇ is
a) P(=O)(OR₁₀)₂,
b) CO(CH₂)ₙCON(CH₃)(CH₂)ₙSO₃⁻M⁺, or
c) C(=O)C₁₋₆alkyl,
wherein R₈ and R₉ are independently
a) hydrogen,
b) aryl,
c) het, or
d) C₁₋₈alkyl which is further optionally substituted with one or more aryl, het, halo, CO₂R₁₀, SOᵢR₁₀, or OR₁₀;
wherein R₁₀ is
a) H or
c) C₁₋₄alkyl, optionally substituted with OH.

**31.** A compound of claim 30 wherein R₁ and R₂ are independantly H, C₁₋₄alkylsubstituted with OR₈ wherein R₈ is H, or C₁₋₄alkyl substituted with OR₁₀.

**32.** A compound of claim 30 wherein R₁ is H; R₂ is aryl wherein aryl is optionally substituted with one or two halo, CN, OR₁₀, or C₁₋₄alkylsubstituted with OR₁₀.

**33.** A compound of claim 30 wherein R₁ is H; R₂ is aryl wherein aryl is fused with a heterocyclic ring.

**34.** A compound of claim 33 wherin the R₂ is 1,3-benzodioxolyl or 1,4-benxodioxinyl.

**35.** A compound of claim 30 wherein R₁ is H; R₂ is het.

**36.** A compound of claim 35 wherein het is a five- (5) or six- (6) membered saturated or unsaturated heterocyclic ring having 1, 2, or 3 heteroatoms selected from the group consisting of O, S, and NW, wherein W is hydrogen, C₁₋₄alkyl, C(=O)OC₁₋₄alkyl or absent, wherein het may be substituted with one or more halo, C₁₋₄alkyl, CF₃, oxo or oxine.

**37.** A compound of claim 36 wherein het is pyridinyl.

**38.** A compound of claim 36 wherein het is a five- (5) membered heterocyclic ring.

**39.** A compound of claim 30 wherein R₁ and R₂ together with the carbon to which they are attached form a het.

**40.** A compound of claim 39 wherein het is a five- (5) or six- (6) membered heterocyclic ring having 1, 2, or 3 heteroatoms selected from the group consisting of O, S, and NW, wherein W is hydrogen, C₁₋₄alkyl, or C(=O)OC₁₋₄alkyl, wherein het may be substituted with one or more halo, OR₁₀, C₁₋₄alkyl, CF₃, oxo or oxine.

**41.** A compound of claim 40 wherein het is a (6) membered heterocyclic ring.

**42.** A compound of claim 41 wherein het is pyran, piperdine, or thiopyran.

**43.** A compound of claim 30 wherein R₁ and R₂ together with the carbon to which they are attached form a six- (6) membered cycloalkyl.

**44.** A compound of claim 43 wherein cycloalkyl is optionally substituted with oxo, or OR₁₀.

**45.** A compound of claim 30 wherein
R₂ is hydrogen;
R₁ is C₁₋₈ alkyl substituted with R₆ or OR₇;
where R₆ is
a) het,
b) SR₈,
c) OR₈ or
d) NR₈R₉;
wherein R₇ is
a) (P=O)(OCH₃)₂,
b) CO(CH₂)ₙCON(CH₃)(CH₂)ₙSO₃⁻M⁺, or
c) C(=O)CH₃,
wherein R₈ and R₉ are independently
a) hydrogen,
b) het, or
c) C₁₋₈alkyl, which is optionally substituted with one or two het, CO₂R₁₀, SOᵢR₁₀, or OR₁₀; and
wherein R₁₀ is
a) H or
b) C₁₋₄alkyl, optionally substituted with OH, or OC₁₋₄alkyl;

**46.** A compound of claim 45 wherein R₁ is C₁₋₈ alkyl substituted with het.

**47.** A compound of claim 46 wherein het is piperidinyl, morpholinyl, thiomorpholinyl, piperazinyl, N-C₁₋₄alky substituted piperazinyl, pyrrolidinyl, pyridyl, imidazolyl, or N-C₁₋₄alky substituted imidazol.

**47.** A compound of claim 46 wherin het is morpholinyl.

**48.** A compound of claim 47 wherein het is pyridinyl.

**49.** A compound of claim 45 wherein R₁ is C₁₋₈ alkyl substituted with OH or OC₁₋₄alkyl.

**50.** A compound of claim 49 wherein R₁ is C₁₋₄ alkyl substituted with OH.

**51.** A compound of claim 45 wherein R₁ is C₁₋₈ alkyl substituted with SR₈.

**52.** A compound of claim 51 wherein R₈ is het.

**53.** A compound of claim 51 wherein R₈ is C₁₋₄alkyl optionally substituted with one or two OR₁₀.

**54.** A compound of claim 45 wherein R₁ is C₁₋₄ alkyl substituted with NR₈R₉.

**55.** A compound of claim 54 wherein R₈ is H, and R₉ is het.

**56.** A compound of claim 55 wherein het is a six- (6) membered heterocyclic ring having 1, 2, or 3 heteroatoms selected from the group consisting of O, S, and NW, wherein W is hydrogen, C₁₋₄alkyl, or absent.

**57.** A compound of claim 56 wherein het is pyridinyl.

**58.** A compound of claim 54 wherein R₈ is H, and R₉ is C₁₋₈alkyl optionally substituted with het.

**59.** A compound of claim 58 wherein het is a six- (6) membered heterocyclic ring having 1, 2, or 3 heteroatoms selected from the group consisting of O, S, and NW, wherein
W is hydrogen, C₁₋₄alkyl, or absent.

**60.** A compound of claim 59 wherein het is pyridinyl.

**61.** A compound of claim 54 wherein R₈ is H, and R₉ is C₁₋₈alkyl optionally substituted with one or two OR₁₀.

**62.** A compound of claim 45 wherein R₁ is hydroxymethyl, morpholinylmethyl, (pyridinylmethyl)aminomethyl, (dimethylamino)methyl, (hydroxyethyl)sulfanylmethyl, (1-methyl-1H-imidazol-2-yl)sulfanylmethyl, -CH₂OCO(CH₂)₆CON(CH₃)(CH₂)₂SO₃⁻M⁺, - CH₂OC(=O)CH₃, CH₂OP(=O)(OMe)₂, (4-methyl-1-piperazinyl)methyl, 1-pyrrolidinylmethyl, (2,3-dihydroxypropyl)aminomethyl, (2-hydroxyethyl)aminomethyl, 1-piperidinylmethyl, bis(2-hydroxyethyl)aminomethyl, 1H-imidazol-1-ylmethyl, (methylsulfanyl)methyl, (tert-butylsulfanyl)methyl, methylsulfanyl acetate, (2,3-dihydroxypropyl)sulfanylmethyl, phenyl or fluoro.

**63.** A compound of claim 62 wherein R₁ is hydroxymethyl, morpholinylmethyl, (2-pyridinylmethyl)aminomethyl, (3-pyridinylmethyl)aminomethyl, (dimethylamino)methyl, (2-hydroxyethyl)sulfanylmethyl, (1-methyl-1H-imidazol-2-yl)sulfanylmethyl, OP(=O)(OCH₃)₂, -CH₂OCO(CH₂)₆CON(CH₃)(CH₂)₂SO₃⁻M⁺, or -CH₂OC(=O)CH₃.

**64.** A compound of claims 12 wherein R₁ and R₂ are independently hydrogen, R₃ and R₄ are independently
a) hydrogen,
b) fluoro, or
c) C₁₋₈ alkyl substituted with R₆ or OR₇;
where R₆ is
a) het,
b) SOᵢR₈,
c) OR₈ or
d) NR₈R₉;
wherein R₇ is
a) P=O)(OH)₂,
b) (P=O)(C₁₋₄alkoxy)₂,
c) CO(CH₂)ₙCON(CH₃)(CH₂)ₙSO₃⁻M⁺, or
d) C(=O)C₁₋₆alkyl,
wherein R₈ and R₉ are independently
a) hydrogen,
b) aryl,
c) het, or
d) C₁₋₈alkyl which is further optionally substituted with one or more aryl, het, halo, CO₂R₁₀, SOᵢR₁₀, or OR₁₀;
wherein R₁₀ is
a) H or
d) C₁₋₄alkyl, optionally substituted with OH.

**65.** A compound of claim 20 wherein R₃ and R₄ are independently fluoro or hydroxymethyl.

**66.** A compound of claim 20 wherein R₃ is hydrogen and R₄ is phenyl, morpholinylmethyl, or hydroxymethyl.

**67.** A compound of claim 22 wherein R₄ is morpholinylmethyl.

**68.** A compound of claim 1 which is
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxamide,
b) N-(4-Chlorobenzyl)-2-(*R* or *S*)- (hydroxymethyl)-9-(morpholin-4-ylmethyl)-7-oxo-2,3-dihydro-7H-[1,4]oxazino[2,3,4-ij]quinoline-6-carboxamide,
c) N-(4-Chlorobenzyl)-9-(morpholin-4-ylmethyl)-7-oxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-oxo-2-pyridin-4-yl-2,3-dihydro-7H-[ 1,4]oxazino[2,3,4-ij]quinoline-6-carboxamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4,-ij]quinoline-6-carboxamide,
g) N-(4-Chlorobenzyl)-2,9-bis(morpholin-4-ylmethyl)-7-oxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-oxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-oxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]quinoline-6-carboxamide,
k) N-(4-Chlorobenzyl)-9-(morpholin-4-ylmethyl)-7-oxo-2-{[(3-pyridinylmethyl)amino]methyl}-2,3-dihydro-7H-[1,4]oxazino[2,3,4-ij]quinoline-6-carboxamide,
l) [6-{[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-oxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-oxo-2,3-dihydro-7H-[1,4]oxazino[2,3,4-ij]quinoline-6-carboxamide,
o) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-oxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-oxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-oxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-oxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-oxo-2,3-dihydro-7H-[1,4]oxazino[2,3,4-ij]quinoline-6-carboxamide,
v) N-(4-Chlorobenzyl)-9-(morpholin-4-ylmethyl)-7-oxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-oxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-oxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]quinoline-6-carboxamide,
z) 2-( 1,3-Benzodioxol-4-yl)-N-(4-chlorobenzyl)-9-(morpholin-4-ylmethyl)-7-oxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
bb) N-(4-Chlorobenzyl)-9-(morpholin-4-ylmethyl)-7-oxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-oxo-2,3-dihydro-7H-[1,4]oxazino[2,3,4-ij]quinoline-6-carboxamide,
dd) N-[(4-Chlorophenyl)methyl]-9'-(4-morpholinylmethyl)-4,7'-dioxospiro[cyclohexane-1,2'(3'*H*)-[7H]pyrido[1,2,3-*de*][1,4]benzoxazine]-6'-carboxamide,
ee) N-[(4-Chlorophenyl)methyl]-4-hydroxy-9'-(4-morpholinylmethyl)-7'-oxospiro[cyclohexane-1,2'(3'*H*)-[7*H*]pyrido[1,2,3-*de*][1,4]benzoxazine]-6'-carboxamide,
ff) N-(4-Chlorobenzyl)-3,9-bis(morpholin-4-ylmethyl)-7-oxo-2,3-dihydro-7H-[1,4]oxazino[2,3,4-ij]quinoline-6-carboxamide,
gg) N-(4-Chlorobenzyl)-9-(morpholin-4-ylmethyl)-7-oxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-oxo-2,3-dihydro-7H-[1,4]oxazino[2,3,4-ij]quinoline-6-carboxamide,
ii) N-(4-Chlorobenzyl)-2-[(methylsulfanyl)methyl]-9-(morpholin-4-ylmethyl)-7-oxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-oxo-2,3-dihydro-7H-[1,4]oxazino[2,3,4-ij]quinoline-6-carboxamide,
kk) N-(4-Chlorobenzyl)-2-[(4-methyl-1-piperazinyl)methyl]-9-(morpholin-4-ylmethyl)-7-oxo-2,3-dihydro-7H-[1,4]oxazino[2,3,4-ij]quinoline-6-carboxamide,
ll) Methyl ({[6-{[(4-chlorobenzyl)amino]carbonyl}-9-(morpholin-4-ylmethyl)-7-oxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-oxo-2-(1-pyrrolidinylmethyl)-2,3-dihydro-7H-[1,4]oxazino[2,3,4-ij]quinoline-6-carboxamide,
nn) N-(4-Chlorobenzyl)-2-{[(2,3-dihydroxypropyl)sulfanyl]methyl}-9-(morpholin-4-ylmethyl)-7-oxo-2,3-dihydro-7H-[1,4]oxazino[2,3,4-ij]quinoline-6-carboxamide,
oo) N-(4-Chlorobenzyl)-2-{[(2,3-dihydroxypropyl)amino]methyl}-9-(morpholin-4-ylmethyl)-7-oxo-2,3-dihydro-7H-[1,4]oxazino[2,3,4-ij]quinoline-6-carboxamide,
pp) N-(4-Chlorobenzyl)-2-{[(2-hydroxyethyl)amino]methyl}-9-(morpholin-4-ylmethyl)-7-oxo-2,3-dihydro-7H-[1,4]oxazino[2,3,4-ij]quinoline-6-carboxamide,
qq) N-(4-Chlorobenzyl)-9-(morpholin-4-ylmethyl)-7-oxo-2-(1-piperidinylmethyl)-2,3-dihydro-7H-[1,4]oxazino[2,3,4-ij]quinoline-6-carboxamide,
rr) 2-{[bis(2-Hydroxyethyl)amino]methyl}-N-(4-chlorobenzyl)-9-(morpholin-4-ylmethyl)-7-oxo-2,3-dihydro-7H-[1,4]oxazino[2,3,4-ij]quinoline-6-carboxamide,
ss) N-(4-Chlorobenzyl)-9-(morpholin-4-ylmethyl)-7-oxo-2-{[(2-pyridinylmethyl)amino]methyl}-2,3-dihydro-7H-[1,4]oxazino[2,3,4-ij]quinoline-6-carboxamide,
tt) 2-[(8-{[6-{[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6-{[(4-Chlorobenzyl)ammo]carbonyl}-9-(morpholin-4-ylmethyl)-7-oxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-oxo-2-{[(4-pyridinylmethyl)amino]methyl}-2,3-dihydro-7H-[1,4]oxazino[2,3,4-ij]quinoline-6-carboxamide,
ww) N-(4-Chlorobenzyl)-2-(1H-imidazol-1-ylmethyl)-9-(morpholin-4-ylmethyl)-7-oxo-2,3-dihydro-7H-[1,4]oxazino[2,3,4-ij]quinoline-6-carboxamide,
xx) N-(4-Chlorobenzyl)-2-{[(4-chlorobenzyl)amino]methyl}-9-(morpholin-4-ylmethyl)-3-oxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-oxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-oxo-2,3-dihydro-7H-[1,4]oxazino[2,3,4-ij]quinoline-6-carboxamide,
aaa) N-(4-Chlorobenzyl)-2- {3-[(methoxymethoxy)methyl]phenyl}-9-(morpholin-4-ylmethyl}-7-oxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-oxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-oxospiro[4*H*-pyran-4,2'(3'*H*)-[7*H*]pyrido[1,2,3-*de*][1,4]benzoxazine]-6'-carboxamide,
eee) 1,1-Dimethylethyl 6-[[[(4-chlorophenyl)methyl]amino]carbonyl]-9'-(4-morpholinylmethyl)-7'-oxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-oxospiro[piperidine-4,2'(3'*H*)-[7*H*]pyrido[1,2,3-*de*],[1,4]benzoxazine]-6'-carboxamide,
ggg) N-(4-Chlorobenzyl)-2,2-bis(hydroxymethyl)-9-(morpholin-4-ylmethyl)-7-oxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-oxospiro[7*H*-pyrido[1,2,3-*de*]-1,4-benzoxazine-2(3*H*),4'-[4H]thiopyran]-6-carboxamide,
iii) N-(4-Chlorobenzyl)-9-(morpholin-4-ylmethyl)-7-oxo-3-phenyl-2,3-dihydro-7H-[1,4]oxazino[2,3,4-ij]quinoline-6-carboxamide,
jjj) N-(4-Chlorobenzyl)-3,3-bis(hydroxymethyl)-9-(3-hydroxy-1-propynyl)-7-oxo-2,3-dihydro-7H-[1,4]oxazino[2,3,4-ij]quinoline-6-carboxamide,
kkk) N-(4-Chlorobenzyl)-3,3-bis(hydroxymethyl)-9-(3-hydroxypropyl)-7-oxo-2,3-dihydro-7H-[1,4]oxazino[2,3,4-ij]quinoline-6-carboxamide,
lll) N-(4-Chlorobenzyl)-2-[2-(methoxymethoxy)phenyl]-9-(morpholin-4-ylmethyl)-7-oxo-2,3-dihydro-7H-[1,4]oxazino[2,3,4-ij]quinoline-6-carboxamide,
mmm) N-(4-Chlorobenzyl)-2-{4-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
nnn) 2-[2,3-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
ooo) N-[(4-Chlorophenyl)methyl]-1-methyl-9'-(4-morpholinylmethyl)-7'-oxospiro[piperidine-4,2'(3'*H*)-[7*H*]pyrido[1,2,3-*de*][1,4]benzoxazine]-6'-carboxamide,
ppp) N-[(4-Chlorophenyl)methyl]-9"-(4-morpholinylmethyl)dispiro[1,3-dioxolane-2,1'-cyclohexane-4',2"(3"*H*)-[7*H*]pyrido[1,2,3-*de*][1,4]benzoxazine]-6"-carboxamide, or a pharmaceutically acceptable salt.

**69.** A compound of claim 1 which is
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxamide,
b) N-(4-Chlorobenzyl)-2-(*R* or *S*)- (hydroxymethyl)-9-(morpholin-4-ylmethyl)-7-thioxo-2,3-dihydro-7H-[1,4]oxazino[2,3,4-ij]quinoline-6-carboxamide,
c) N-(4-Chlorobenzyl)-9-(morpholin-4-ylmethyl)-7-thioxo-2-pyridin-3-yl-2,3-diliydro-7H-[1,4]oxazino[2,3,4-ij]quinoline-6-carboxamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-thioxo-2-pyridin-2-yl-2,3-dihydro-H-[1,4]oxazino[2,3,4-ij]quinoline-6-carboxamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxamide,
g) N-(4-Chlorobenzyl)2,9-bis(morpholin-4-ylmethyl)-7-thioxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-thioxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-thioxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]quinoline-6-carboxamide,
k) N-(4-Chlorobenzyl)-9-(morpholin-4-ylmethyl)-7-thioxo-2-{[(3-pyridinylmethyl)amino]methyl}-2,3-dihydro-7H-[1,4]oxazino[2,3,4-ij]quinoline-6-carboxamide,
l) [6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-thioxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-thioxo-2,3-dihydro-7H-[1,4]oxazino[2,3,4-ij]quinoline-6-carboxamide,
o) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-thioxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-thioxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-thioxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-thioxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-thioxo-2,3-dihydro-7H-[1,4]oxazino[2,3,4-ij]quinoline-6-carboxamide,
v) N-(4-Chlorobenzyl)-9-(morpholin-4-ylmethyl)-7-thioxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-thioxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
bb) N-(4-Chlorobenzyl)-9-(morpholin-4-ylmethyl)-7-thioxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-thioxo-2,3-dihydro-7H-[1,4]oxazino[2,3,4-ij]quinoline-6-carboxamide,
dd) N-[(4-Chlorophenyl)methyl]-9'-(4-morpholinylmethyl)-4-oxo-7'-thioxospiro[cyclohexane-1,2'(3'*H*)-[7*H*]pyrido[1,2,3-*de*][1,4]benzoxazine]-6'-carboxamide,
ee) N-[(4-Chlorophenyl)methyl]-4-hydroxy-9'-(4-morpholinylmethyl)-7'-thioxospiro[cyclohexane-1,2'(3'*H*)-[7*H*]pyrido[1,2,3-*de*][1,4]benzoxazine]-6'-carboxamide,
ff) N-(4-Chlorobenzyl)-3,9-bis(morpholin-4-ylmethyl)-7-thioxo-2,3-dihydro-7H-[1,4]oxazino[2,3,4-ij]quinoline-6-carboxamide,
gg) N-(4-Chlorobenzyl)-9-(morpholin-4-ylmethyl)-7-thioxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-thioxo-2,3-dihydro-7H-[1,4]oxazino[2,3,4-ij]quinoline-6-carboxamide,
ii) N-(4-Chlorobenzyl)-2-[(methylsulfanyl)methyl]-9-(morpholin-4-ylmethyl)-7-thioxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-thioxo-2,3-dihydro-7H-[1,4]oxazino[2,3,4-ij]quinoline-6-carboxamide,
kk) N-(4-Chlorobenzyl)-2-[(4-methyl-1-piperazinyl)methyl]-9-(morpholin-4-ylmethyl)-7-thioxo-2,3-dihydro-7H-[1,4]oxazino[2,3,4-ij]quinoline-6-carboxamide,
ll) Methyl ({[6-{[(4-chlorobenzyl)amino]carbonyl}-9-(morpholin-4-ylmethyl)-7-thioxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-thioxo-2-(1-pyrrolidinylmethyl)-2,3-dihydro-7H-[1,4]oxazino[2,3,4-ij]quinoline-6-carboxamide,
nn) N-(4-Chlorobenzyl)-2-{[(2,3-dihydroxypropyl)sulfanyl]methyl}-9-(morpholin-4-ylmethyl)-7-thioxo-2,3-dihydro-7H-[1,4]oxazino[2,3,4-ij]quinoline-6-carboxamide,
oo) N-(4-Chlorobenzyl)-2-{[(2,3-dihydroxypropyl)amino]methyl}-9-(morpholin-4-ylmethyl)-7-thioxo-2,3-dihydro-7H-[1,4]oxazino[2,3,4-ij]quinoline-6-carboxamide,
pp) N-(4-Chlorobenzyl)-2-{[(2-hydroxyethyl)amino]methyl}-9-(morpholin-4-ylmethyl)-7-thioxo-2,3-dihydro-7H-[1,4]oxazino[2,3,4-ij]quinoline-6-carboxamide,
qq) N-(4-Chlorobenzyl)-9-(morpholin-4-ylmethyl)-7-thioxo-2-(1-piperidinylmethyl)-2,3-dihydro-7H-[1,4]oxazino[2,3,4-ij]quinoline-6-carboxamide,
rr) 2-{[bis(2-Hydroxyethyl)amino]methyl}-N-(4-chlorobenzyl)-9-(morpholin-4-ylmethyl)-7-thioxo-2,3-dihydro-7H-[1,4]oxazino[2,3,4-ij]quinoline-6-carboxamide,
ss) N-(4-Chlorobenzyl)-9-(morpholin-4-ylmethyl)-7-thioxo-2-{[(2-pyridinylmethyl)amino]methyl}-2,3-dihydro-7H-[1,4]oxazino[2,3,4-ij]quinoline-6-carboxamide,
tt) 2-[(8-{[6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-thioxo-2-([(4-pyridinylmethyl)amino]methyl}-2,3-dihydro-7H-[1,4]oxazino[2,3,4-ij]quinoline-6-carboxamide,
ww) N-(4-Chlorobenzyl)-2-(1H-imidazol-1-ylmethyl)-9-(morpholin-4-ylmethyl)-7-thioxo-2,3-dihydro-7H-[1,4]oxazino[2,3,4-ij]quinoline-6-carboxamide,
xx) N-(4-Chlorobenzyl)-2-{[(4-chlorobenzyl)amino]methyl}-9-(morpholin-4-ylmethyl)-7-thioxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-thioxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-thioxo-2,3-dihydro-7H-[1,4]oxazino[2,3,4-ij]quinoline-6-carboxamide,
aaa) N-(4-Chlorobenzyl)-2-{3-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-thioxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-thioxospiro[4*H*-pyran-4,2'(3'*H*)-[7*H*]pyrido[1,2,3-*de*] [1,4]benzoxazine]-6'-carboxamide,
eee) 1,1-Dimethylethyl 6-[[[(4-chlorophenyl)methyl]amino]carbonyl]-9'-(4-morpholinylmethyl)-7'-thioxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-thioxospiro[piperidine-4,2'(3'*H*)-[7*H*]pyrido[1,2,3-*de*] [1,4]benzoxazine]-6'-carboxamide,
ggg) N-(4-Chlorobenzyl)-2,2-bis(hydroxymethyl)-9-(morpholin-4-ylmethyl)-7-thioxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-thioxospiro[7*H*-pyrido[1,2,3-*de*]-1,4-benzoxazine-2(3*H*),4'-[4H]thiopyran]-6-carboxamide,
iii) N-(4-Chlorobenzyl)-9-(morpholin-4-ylmethyl)-7-thioxo-3-phenyl-2,3-dihydro-7H-[1,4]oxazino[2,3,4-ij]quinoline-6-carboxamide,
jjj) N-(4-Chlorobenzyl)-3,3-bis(hydroxymethyl)-9-(3-hydroxy-1-propynyl)-7-thioxo-2,3-dihydro-7H-[1,4]oxazino[2,3,4-ij]quinoline-6-carboxamide,
kkk) N-(4-Chlorobenzyl)-3,3-bis(hydroxymethyl)-9-(3-hydroxypropyl)-7-thioxo-2,3-dihydro-7H-[1,4]oxazino[2,3,4-ij]quinoline-6-carboxamide,
lll) N-(4-Chlorobenzyl)-2-[2-(methoxymethoxy)phenyl]-9-(morpholin-4-ylmethyl)-7-thioxo-2,3-dihydro-7H-[1,4]oxazino[2,3,4-ij]quinoline-6-carboxamide,
mmm) N-(4-Chlorobenzyl)-2-{4-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
nnn) 2-[2,3-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
ooo) N-[(4-Chlorophenyl)methyl]-1-methyl-9'-(4-morpholinylmethyl)-7'-thioxospiro[piperidine-4,2'(3'*H*)-[7*H*]pyrido[1,2,3-*de*][1,4]benzoxazine]-6'-carboxamide, or a pharmaceutically acceptable salt.

**70.** A compound of claim 1 which is
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxamide,
c) N-(4-Chlorobenzyl)-9-(morpholin-4-ylmethyl)-7-oxo-2-pyridin-3-yl-2,3-dihydro-7H-[ 1,4]oxazino[2,3,4-ij]quinoline-6-carboxamide,
d) N-(4-Chlorobenzyl)-9-(morpholin-4-ylmethyl)-7-oxo-2-pyridin-4-yl-2,3-dihydro-7H-[ 1,4]oxazino[2,3,4-ij]quinoline-6-carboxamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-oxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-oxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-oxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]quinoline-6-carboxamide,
k) N-(4-Chlorobenzyl)-9-(morpholin-4-ylmethyl)-7-oxo-2-{[(3-pyridinylmethyl)amino]methyl}-2,3-dihydro-7H-[1,4]oxazino[2,3,4-ij]quinoline-6-carboxamide,
l) [6- {[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl acetate,
m) - N-(4-Chlorobenzyl)-9-(morpholin-4-ylmethyl)-7-oxo-2-(*R* or *S*)-{[(3-pyridinylmethyl)amino)methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-oxo-2,3-dihydro-7H-[1,4]oxazino[2,3,4-ij]quinoline-6-carboxamide,
o) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-oxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-oxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-oxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-oxo-2,3-dihydro-7H-[1,4]oxazino[2,3,4-ij]quinoline-b-carboxamide,
u) N-(4-Chlorobenzyl)-2-(3-furyl)-9-(morpholin-4-ylmethyl)-7-oxo-2,3-dihydro-7H-[1,4]oxazino[2,3,4-ij]quinoline-6-carboxamide,
v) N-(4-Chlorobenzyl)-9-(morpholin-4-ylmethyl)-7-oxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-oxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-oxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-oxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
bb) N-(4-Chlorobenzyl)-9-(morpholin-4-ylmethyl)-7-oxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-oxo-2,3-dihydro-7H-[1,4]oxazino[2,3,4-ij]quinoline-6-carboxamide,
dd) N-[(4-Chlorophenyl)methyl]-9'-(4-morpholinylmethyl)-4,7'-dioxospiro[cyclohexane-1,2'(3'*H*)-[7*H*]pyrido[1,2,3-*de*][1,4]benzoxazine]-6'-carboxamide,
ee) N-[(4-Chlorophenyl)methyl]-4-hydroxy-9'-(4-morpholinylmethyl)-7'-oxospiro[cyclohexane-1,2'(3'*H*)-[7*H*]pyrido[1,2,3-*de*][1,4]benzoxazine]-6'-carboxamide,
ff) N-(4-Chlorobenzyl)-3,9-bis(morpholin-4-ylmethyl)-7-oxo-2,3-dihydro-7H-[1,4]oxazino[2,3,4-ij]quinoline-6-carboxamide,
gg) N-(4-Chlorobenzyl)-9-(morpholin-4-ylmethyl)-7-oxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-oxo-2,3-dihydro-7H-[1,4]oxazino[2,3,4-ij]quinoline-6-carboxamide,
ii) N-(4-Chlorobenzyl)-2-[(methylsulfanyl)methyl]-9-(morpholin-4-ylmethyl)-7-oxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-oxo-2,3-dihydro-7H-[1,4]oxazino[2,3,4-ij]quinoline-6-caxboxamide,
kk) N-(4-Chlorobenzyl)-2-[(4-methyl-1-piperazinyl)methyl]-9-(morpholin-4-ylmethyl)-7-oxo-2,3-dihydro-7H-[1,4]oxazino[2,3,4-ij]quinoline-6-carboxamide,
ll) Methyl ({[6-{[(4-chlorobenzyl)amino]carbonyl}-9-(morpholin-4-ylmethyl)-7-oxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-oxo-2-(1-pyrrolidinylmethyl)-2,3-dihydro-7H-[1,4]oxazino[2,3,4-ij]quinoline-6-carboxamide,
nn) N-(4-Chlorobenzyl)-2- {[(2,3-dihydroxypropyl)sulfanyl]methyl}-9-(morpholin-4-ylmethyl)-7-oxo-2,3-dihydro-7H-[1,4]oxazino[2,3,4-ij]quinoline-6-carboxamide,
oo) N-(4-Chlorobenzyl)-2-{[(2,3-dihydroxypropyl)amino]methyl}-9-(morpholin-4-ylmethyl)-7-oxo-2,3-dihydro-7H-[1,4]oxazino[2,3,4-ij]quinoline-6-carboxamide,
pp) N-(4-Chlorobenzyl)-2- {[(2-hydroxyethyl)amino]methyl}-9-(morpholin-4-ylmethyl)-7-oxo-2,3-dihydro-7H-[1,4]oxazino[2,3,4-ij]quinoline-6-carboxamide,
qq) N-(4-Chlorobenzyl)-9-(morpholin-4-ylmethyl)-7-oxo-2-(1-piperidinylmethyl)-2,3-dihydro-7H-[1,4]oxazino[2,3,4-ij]quinoline-6-carboxamide;
rr) 2-{[bis(2-Hydroxyethyl)amino]methyl}-N-(4-chlorobenzyl)-9-(morpholin-4-ylmethyl)-7-oxo-2,3-dihydro-7H-[1,4]oxazino[2,3,4-ij]quinoline-6-carboxamide,
ss) N-(4-Chlorobenzyl)-9-(morpholin-4-ylmethyl)-7-oxo-2-{[(2-pyridinylmethyl)amino]methyl}-2,3-dihydro-7H-[1,4]oxazino[2,3,4-ij]quinoline-6-carboxamide,
tt) 2-[(8-{[6-{[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6-{[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-oxo-2-{[(4-pyridinylmethyl)amino]methyl}-2,3-dihydro-7H-[1,4]oxazino[2,3,4-ij]quinoline-6-carboxamide,
ww) N-(4-Chlorobenzyl)-2-(1H-imidazol-1-ylmethyl)-9-(morpholin-4-ylmethyl)-7-oxo-2,3-dihydro-7H-[1,4]oxazino[2,3,4-ij]quinoline-6-carboxamide,
xx) N-(4-Chlorobenzyl)-2- {[(4-chlorobenzyl)amino]methyl}-9-(morpholin-4-ylmethyl)-7-oxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-oxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-oxo-2,3-dihydro-7H-[1,4]oxazino[2,3,4-ij]quinoline-6-carboxamide,
aaa) N-(4-Chlorobenzyl)-2-{3-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-oxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-oxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-oxospiro[4*H*-pyran-4,2'(3'*H*)-[7*H*]pyrido[1,2,3-*de*][1,4]benzoxazine]-6'-carboxamide,
eee) 1,1-Dimethylethyl 6-[[[(4-chlorophenyl)methyl]amino]carbonyl]-9'-(4-morpholinylmethyl)-7'-oxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-oxospiro[pipendine-4,2'(3'*H*)-[7*H*]pyrido[1,2,3-*de*][1,4]benzoxazine]-6'-carboxamide,
ggg) N-(4-Chlorobenzyl)-2,2-bis(hydroxymethyl)-9-(morpholin-4-ylmethyl)-7-oxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-oxospiro[7*H*-pyrido[1,2,3-*de*]-1,4-benzoxazine-2(3*H*),4'-[4H]thiopyran]-6-carboxamide, or a pharmaceutically acceptable salt.

**71.** A compound of claim 1 which is
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-thioxo-2,3-dihydro-7H-[1,4]oxazino[2,3,4-ij]quinoline-6-carboxamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-thioxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-thioxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-thioxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-thioxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]quinoline-6-carboxamide,
k) N-(4-Chlorobenzyl)-9-(morpholin-4-ylmethyl)-7-thioxo-2-{[(3-pyridinylmethyl)amino]methyl}-2,3-dihydro-7H-[1,4]oxazino[2,3,4-ij]quinoline-6-carboxamide,
l) [6-{[4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-thioxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-thioxo-2,3-dihydro-7H-[1,4]oxazino[2,3,4-ij]quinoline-6-carboxamide,
o) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-thioxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-thioxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-thioxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-thioxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-thioxo-2,3-dihydro-7H-[1,4]oxazino[2,3,4-ij]quinoline-6-carboxamide,
v) N-(4-Chlorobenzyl)-9-(morpholin-4-ylmethyl)-7-thioxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-thioxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
bb) N-(4-Chlorobenzyl)-9-(morpholin-4-ylmethyl)-7-thioxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-thioxo-2,3-dihydro-7H-[1,4]oxazino[2,3,4-ij]quinoline-6-carboxamide,
dd) N-[(4-Chlorophenyl)methyl]-9'-(4-morpholinylmethyl)-4-oxo-7'-thioxospiro[cyclohexane-1,2'(3'*H*)-[7*H*]pyrido[1,2,3-*de*] [1,4]benzoxazine]-6'-carboxamide,
ee) N-[(4-Chlorophenyl)methyl]-4-hydroxy-9'-(4-morpholinylmethyl)-7'-thioxospiro[cyclohexane-1,2'(3'*H*)-[7*H*]pyrido[1,2,3-*de*][1,4]benzoxazine]-6'-carboxamide,
ff) N-(4-Chlorobenzyl)-3,9-bis(morpholin-4-ylmethyl)-7-thioxo-2,3-dihydro-7H-[1,4]oxazino[2,3,4-ij]quinoline-6-carboxamide,
gg) N-(4-Chlorobenzyl)-9-(morpholin-4-ylmethyl)-7-thioxo-2-phenyl-2,3-dihydro-7H-[1,4]oxazino[2,3,4-ij]quinoline-6-carboxamide,
hh) N-(4-Chlorobenzyl)-2,2-difluoro-9-(morpholin-4-ylmethyl)-7-thioxo-2,3-dihydro-7H-[ 1,4]oxazino[2,3,4-ij]quinoline-6-carboxamide,
ii) N-(4-Chlorobenzyl)-2-[(methylsulfanyl)methyl]-9-(morpholin-4-ylmethyl)-7-thioxo-2,3-dihydro-7H-[1,4]oxazino[2,3,4-ij]quinoline-6-carboxamide,
jj) N-(4-Chlorobenzyl)-2-[(dimethylamino)methyl]-9-(morpholin-4-ylmethyl)-7-thioxo-2,3-dihydro-7H-[1,4]oxazino[2,3,4-ij]quinoline-6-carboxamide,
kk) N-(4-Chlorobenzyl)-2-[(4-methyl-1-piperazinyl)methyl]-9-(morpholin-4-ylmethyl)-7-thioxo-2,3-dihydro-7H-[1,4]oxazino[2,3,4-ij]quinoline-6-carboxamide,
ll) Methyl ({[6-{[(4-chlorobenzyl)amino]carbonyl}-9-(morpholin-4-ylmethyl)-7-thioxo-2,3-dihydro-7H-[1,4]oxazino[2,3,4-ij]quinolin-2-yl]methyl}thio)acetate,
mm) N-(4-Chlorobenzyl)-9-(morpholin-4-ylmethyl)-7-thioxo-2-(1-pyrrolidinylmethyl)-2,3-dihydro-7H-[1,4]oxazino[2,3,4-ij]quinoline-6-carboxamide,
nn) N-(4-Chlorobenzyl)-2- {[(2,3-dihydroxypropyl)sulfanyl]methyl}-9-(morpholin-4-ylmethyl)-7-thioxo-2,3-dihydro-7H-[1,4]oxazino[2,3,4-ij]quinoline-6-carboxamide,
oo) N-(4-Chlorobenzyl)-2-{[(2,3-dihydroxypropyl)amino]methyl}-9-(morpholin-4-ylmethyl)-7-thioxo-2,3-dihydro-7H-[1,4]oxazino[2,3,4-ij]quinoline-6-carboxamide,
pp) N-(4-Chlorobenzyl)-2-{[(2-hydroxyethyl)amino]methyl}-9-(morpholin-4-ylmethyl)-7-thioxo-2,3-dihydro-7H-[1,4]oxazino[2,3,4-ij]quinoline-6-carboxamide,
qq) N-(4-Chlorobenzyl)-9-(morpholin-4-ylmethyl)-7-thioxo-2-(1-piperidinylmethyl)-2,3-dihydro-7H-[1,4]oxazino[2,3,4-ij]quinoline-6-carboxamide,
rr) 2- {[bis(2-Hydroxyethyl)amino]methyl}-N-(4-chlorobenzyl)-9-(morpholin-4-ylmethyl)-7-thioxo-2,3-dihydro-7H-[1,4]oxazino[2,3,4-ij]quinoline-6-carboxamide,
ss) N-(4-Chlorobenzyl)-9-(morpholin-4-ylmethyl)-7-thioxo-2-{[(2-pyridinylmethyl)amino]methyl}-2,3-dihydro-7H-[1,4]oxazino[2,3,4-ij]quinoline-6-carboxamide,
tt) 2- (8-{[6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methoxy}-8-oxooctanoyl)(methyl)amino]ethanesulfonic acid sodium salt,
uu) [6- {[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl dimethyl phosphate,
vv) N-(4-Chlorobenzyl)-9-(morpholin-4-ylmethyl)-7-thioxo-2-{[(4-pyridinylmethyl)amino]methyl}-2,3-dihydro-7H-[1,4]oxazino[2,3,4-ij]quinoline-6-carboxamide,
ww) N-(4-Chlorobenzyl)-2-(1H-imidazol-1-ylmethyl)-9-(morpholin-4-ylmethyl)-7-thioxo-2,3-dihydro-7H-[1,4]oxazino[2,3,4-ij]quinoline-6-carboxamide,
xx) N-(4-Chlorobenzyl)-2-{[(4-chlorobenzyl)amino]methyl}-9-(morpholin-4-ylmethyl)-7-thioxo-2,3-dihydro-7H-[1,4]oxazino[2,3,4-ij]quinoline-6-carboxamide,
yy) N-(4-Chlorobenzyl)-3-(hydroxymethyl)-9-(morpholin-4-ylmethyl)-7-thioxo-2,3-dihydro-7H-[1,4]oxazino[2,3,4-ij]quinoline-6-carboxamide,
zz) N-(4-Chlorobenzyl)-2-(4-hydroxyphenyl)-9-(morpholin-4-ylmethyl)-7-thioxo-2,3-dihydro-7H-[1,4]oxazino[2,3,4-ij]quinoline-6-carboxamide,
aaa) N-(4-Chlorobenzyl)-2- {3-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
bbb) N-(4-Chlorobenzyl)-2-{2-[(methoxymethoxy)methyl]phenyl}-9-(morpholin-4-ylmethyl)-7-thioxo-2,3-dihydro-7H-[1,4]oxazino[2,3,4-ij]quinoline-6-carboxamide,
ccc) N-(4-Chlorobenzyl)-2-(2-hydroxyphenyl)-9-(morpholin-4-ylmethyl)-7-thioxo-2,3-dihydro-7H-[1,4]oxazino[2,3,4-ij]quinoline-6-carboxamide,
ddd) N-[(4-Chlorophenyl)methyl]-2,3,5,6-tetrahydro-9'-(4-morpholinylmethyl)-7'-thioxospiro[4*H*-pyran-4,2'(3'*H*)-[7*H*]pyrido[1,2,3-*de*][1,4]benzoxazine]-6'-carboxamide,
eee) 1,1-Dimethylethyl 6-[[[(4-chlorophenyl)methyl]amino]carbonyl]-9'-(4-morpholinylmethyl)-7'-thioxospiro[piperidine-4,2'(3'*H*)-[7*H*]pyrido[1,2,3-*de*] [1,4]benzoxazine]-1-carboxylate,
fff) N-[(4-Chlorophenyl)methyl]-9'-(4-morpholinylmethyl)-7'-thioxospiro[piperidine-4,2'(3'*H*)-[7*H*]pyrido[1,2,3-*de*][1,4]benzoxazine]-6'-carboxamide,
ggg) N-(4-Chlorobenzyl)-2,2-bis[(hydroxymethoxy)methyl]-9-(morpholin-4-ylmethyl)-7-thioxo-2,3-dihydro-7H-[1,4]oxazino[2,3,4-ij]quinoline-6-carboxamide,
hhh) N-[(4-Chlorophenyl)methyl]-2',3',5',6'-tetrahydro-9-(4-morpholinylmethyl)-7-thioxospiro[7*H*-pyrido[1,2,3-*de*]-1,4-benzoxazine-2(3*H*),4'-[4H]thiopyran]-6-carboxamide, or a pharmaceutically acceptable salt.

**72.** A compound of claim 1 which is
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxamide,
c) N-(4-Chlorobenzyl)-9-(morpholin-4-ylmethyl)-7-oxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-oxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-oxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-oxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-oxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]quinoline-6-carboxamide,
k) N-(4-Chlorobenzyl)-9-(morpholin-4-ylmethyl)-7-oxo-2-{[(3-pyridinylmethyl)amino]methyl}-2,3-dihydro-7H-[1,4]oxazino[2,3,4-ij]quinoline-6-carboxamide,
l) [6- {[(4-Chlorobenzyl)amino]carbonyl}-9-(morpholin-4-ylmethyl)-7-oxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-oxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-oxo-2,3-dihydro-7H-[1,4]oxazino[2,3,4-ij]quinoline-6-carboxamide,
o) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-oxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-oxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-oxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-oxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-oxo-2,3-dihydro-7H-[1,4]oxazino[2,3,4-ij]quinoline-6-carboxamide,
v) N-(4-Chlorobenzyl)-9-(morpholin-4-ylmethyl)-7-oxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-oxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-oxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]quinoline-6-carboxamide,
z) 2-(1,3-Benzodioxol-4-yl)-N-(4-chlorobenzyl)-9-(morpholin-4-ylmethyl)-7-oxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-oxo-2,3-dihydro-7H-[1,4]oxazino[2,3,4-ij]quinoline-6-carboxamide,
bb) N-(4-Chlorobenzyl)-9-(morpholin-4-ylmethyl)-7-oxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-oxo-2,3-dihydro-7H-[1,4]oxazino[2,3,4-ij]quinoline-6-carboxamide,
dd) N-[(4-Chlorophenyl)methyl]-9'-(4-morpholinylmethyl)-4,7'-dioxospiro[cyclohexane-1,2'(3'*H*)*-*[7*H*]pyrido[1,2,3-*de*][1,4]benzoxazine]-6'-carboxamide,
ee) N-[(4-Chlorophenyl)methyl]-4-hydroxy-9'-(4-morpholinylmethyl)-7'-oxospiro[cyclohexane-1,2'(3'*H*)-[7*H*]pyrido[1,2,3-*de*][1,4]benzoxazine]-6'-carboxamide, or a pharmaceutically acceptable salt.

**73.** A compound of claim 1 which is
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxamide,
b) N-(4-Chlorobenzyl)-2-(*R* or *S*)- (hydroxymethyl)-9-(morpholin-4-ylmethyl)-7-thioxo-2,3-dihydro-7H-[1,4]oxazino[2,3,4-ij]quinoline-6-carboxamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-thioxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxamide,
g) N-(4-Chlorobenzyl)-2,9-bis(morpholin-4-ylmethyl)-7-thioxo-2,3-dihydro-7H-[1,4]oxazino[2,3,4-ij]quinoline-6-carboxamide,
h) 2-[(*tert*-Butylsulfanyl)methyl]-N-(4-chlorobenzyl)-9-(morpholin-4-ylmethyl)-7-thioxo-2,3-dihydro-7H-[1,4]oxazino[2,3,4-ij]quinoline-6-carboxamide,
i) N-(4-Chlorobenzyl)-2-{[(2-hydroxyethyl)sulfanyl]methyl}-9-(morpholin-4-ylmethyl)-7-thioxo-2,3-dihydro-7H-[1,4]oxazino[2,3,4-ij]quinoline-6-carboxamide,
j) N-(4-Chlorobenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]quinoline-6-carboxamide,
k) N-(4-Chlorobenzyl)-9-(morpholin-4-ylmethyl)-7-thioxo-2-{[(3-pyridinylmethyl)amino]methyl}-2,3-dihydro-7H-[1,4]oxazino[2,3,4-ij]quinoline-6-carboxamide,
l) [6-{[(4-Chlorobenzyl)amino]carbonyl}-9-(morpholin-4-ylmethyl)-7-thioxo-2,3-dihydro-7H-[1,4]oxazino[2,3,4-ij]quinolin-2-yl]methyl acetate,
m) N-(4-Chlorobenzyl)-9-(morpholin-4-ylmethyl)-7-thioxo-2-(*R* or *S*)-{[(3-pyridinylmethyl)amino]methyl}-2,3-dihydro-7H-[1,4]oxazino[2,3,4-ij]quinoline-6-carboxamide,
n) N-(4-Chlorobenzyl)-2-(3-hydroxyphenyl)-9-(morpholin-4-ylmethyl)-7-thioxo-2,3-dihydro-7H-[1,4]oxazino[2,3,4-ij]quinoline-6-carboxamide,
o) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxamide,
p) N-(4-Chlorobenzyl)-2-[3-(hydroxymethyl)phenyl]-9-(morpholin-4-ylmethyl)-7-thioxo-2,3-dihydro-7H-[1,4]oxazino[2,3,4-ij]quinoline-6-carboxamide,
q) N-(4-Chlorobenzyl)-2-[2-(hydroxymethyl)phenyl]-9-(morpholin-4-ylmethyl)-7-thioxo-2,3-dihydro-7H-[1,4]oxazino[2,3,4-ij]quinoline-6-carboxamide,
r) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxamide,
s) N-(4-Chlorobenzyl)-2-(2-furyl)-9-(morpholin-4-ylmethyl)-7-thioxo-2,3-dihydro-7H-[1,4]oxazino[2,3,4-ij]quinoline-6-carboxamide,
t) N-(4-Chlorobenzyl)-2-(3-cyanophenyl)-9-(morpholin-4-ylmethyl)-7-thioxo-2,3-dihydro-7H-[1,4]oxazino[2,3,4-ij]quinoline-6-carboxamide,
u) N-(4-Chlorobenzyl)-2-(3-furyl)-9-(morpholin-4-ylmethyl)-7-thioxo-2,3-dihydro-7H-[1,4]oxazino[2,3,4-ij]quinoline-6-carboxamide,
v) N-(4-Chlorobenzyl)-9-(morpholin-4-ylmethyl)-7-thioxo-2-thien-2-yl-2,3-dihydro-7H-[1,4]oxazino[2,3,4-ij]quinoline-6-carboxamide,
w) N-(4-Chlorobenzyl)-2-(3,5-difluorophenyl)-9-(morpholin-4-ylmethyl)-7-thioxo-2,3-dihydro-7H-[1,4]oxazino[2,3,4-ij]quinoline-6-carboxamide,
x) 2-(1,3-Benzodioxol-5-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
y) N-(4-Chlorobenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]quinoline-6-carboxamide,
z) 2-(1,3-Benzodioxal-4-yl)-N-(4-chlorobenzyl)-9-(morpholin-4-ylmethyl)-7-thioxo-2,3-dihydro-7H-[1,4]oxazino[2,3,4-ij]quinoline-6-carboxamide,
aa) 2-[3,5-bis(Methoxymethoxy)phenyl]-N-(4-chlorobenzyl)-9-(morpholin-4-ylmethyl)-7-thioxo-2,3-dihydro-7H-[1,4]oxazino[2,3,4-ij]quinoline-6-carboxamide,
bb) N-(4-Chlorobenzyl)-9-(morpholin-4-ylmethyl)-7-thioxo-2-thien-3-yl-2,3-dihydro-7H-[1,4]oxazino[2,3,4-ij]quinoline-6-carboxamide,
cc) N-(4-Chlorobenzyl)-2,2-bis[(methoxymethoxy)methyl]-9-(morpholin-4-ylmethyl)-7-thioxo-2,3-dihydro-7H-[1,4]oxazino[2,3,4-ij]guinoline-6-carboxamide,
dd) N-[(4-Chlorophenyl)methyl]-9'-(4-morpholinylmethyl)-4-oxo-7'-thioxospiro[cyclohexane-1,2'(3'*H*)-[7*H*]pyrido[1,2,3-*de*][1,4]benzoxazine]-6'-carboxamide,
ee) N-[(4-Chlorophenyl)methyl]-4-hydroxy-9'-(4-morpholinylmethyl)-7'-thioxospiro[cyclohexane-1,2'(3'*H*)-[7*H*]pyrido[1,2,3-*de*][1,4]benzoxazine]-6'-carboxamide, or a pharmaceutically acceptable salt.

**74.** A compound of claim 1 which is
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxamide,
c) N-(4-Chlorobenzyl)-9-(morpholin-4-ylmethyl)-7-oxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-oxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxamide,
g) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxamide,
h) N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide, or a pharmaceutically acceptable salt.

**75.** A compound of claim 1 which is
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxamide,
b) N-(4-Chlorobenzyl)-2-(*R* or *S*)-(hydroxymethyl)-9-(morpholin-4-ylmethyl)-7-thioxo-2,3-dihydro-7H-[1,4]oxazino[2,3,4-ij]quinoline-6-carboxamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxamide,
e) N-(4-Chlorobenzyl)-9-(morpholin-4-ylmethyl)-7-thioxo-2-pyridin-2-yl-2,3-dihydro-7H-[1,4]oxazino[2,3,4-ij]quinoline-6-carboxamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxamide,
g) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxamide,
h) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxamide, or a pharmaceutically acceptable salt.

**76.** A compound of claim 1 which is
a) N-(4-Chlorobenzyl)-2-(hydroxymethyl)-9-(morpholin-4-ylmethyl)-7-thioxo-2,3-dihydro-7H-[1,4]oxazino[2,3,4-ij]quinoline-6-carboxthioamide,
b) N-(4-Chlorobenzyl)-2-(*R* or *S*)- (hydroxymethyl)-9-(morpholin-4-ylmethyl)-7-thioxo-2,3-dihydro-7H-[1,4]oxazino[2,3,4-ij]quinoline-6-carboxthioamide,
c) N-(4-Chlorobenzyl)-9-(morpholin-4-ylmethyl)-7-thioxo-2-pyridin-3-yl-2,3-dihydro-7H-[1,4]oxazino[2,3,4-ij]quinoline-6-carboxthioamide,
d) N-(4-Chlorobenzyl)-9-(morpholin-4-ylmethyl)-7-thioxo-2-pyridin-4-yl-2,3-dihydro-7H-[1,4]oxazino[2,3,4-ij]quinoline-6-carboxthioamide,
e) N-(4-Chlorobenzyl)-9-(morpholin-4-ylmethyl)-7-thioxo-2-pyridin-2-yl-2,3-dihydro-7H-[1,4]oxazino[2,3,4-ij]quinoline-6-carboxthioamide,
f) N-(4-Chlorobenzyl)-9-(morpholin-4-ylmethyl)-7-thioxo-2-(*R* or *S*)-pyridin-3-yl-2,3-dihydro-7H-[1,4]oxazino[2,3,4-ij]quinoline-6-carboxthioamide,
e) N-(4-Chlorobenzyl)-9-(morpholin-4-ylmethyl)-7-thioxo-2-(*R* or *S*)-pyridin-2-yl-2,3-dihydro-7H-[1,4]oxazino[2,3,4-ij]quinoline-6-carboxthioamide,
f) N-(4-Chlorobenzyl)-2-(1-methyl-1H-imidazol-2-yl)-9-(morpholin-4-ylmethyl)-7-thioxo-2,3-dihydro-7H-[1,4]oxazino[2,3,4-ij]quinoline-6-carboxthioamide, or a pharmaceutically acceptable salt.

**77.** A compound of claim 1 which is
a) N-(4-Chlorobenzyl)-2-(hydroxymethyl)-9-(morpholin-4-ylmethyl)-7-oxo-2,3-dihydro-7H-[1,4]oxazino[2,3,4-ij]quinoline-6-carboxthioamide,
b) N-(4-Chlorobenzyl)-2-(*R* or *S*)-(hydroxymethyl)-9-(morpholin-4-ylmethyl)-7-oxo-2,3-dihydro-7H-[1,4]oxazino[2,3,4-ij]quinoline-6-carboxthioamide,
c) N-(4-Chlorobenzyl)-9-(morpholin-4-ylmethyl)-7-oxo-2-pyridin-3-yl-2,3-dihydro-7H-[1,4]oxazino[2,3,4-ij]quinoline-6-carboxthioamide,
d) N-(4-Chlorobenzyl)-9-(morpholin-4-ylmethyl)-7-oxo-2-pyridin-4-yl-2,3-dihydro-7H-[1,4]oxazino[2,3,4-ij]quinoline-6-carboxthioamide,
e) N-(4-Chlorobenzyl)-9-(morpholin-4-ylmethyl)-7-oxo-2-pyridin-2-yl-2,3-dihydro-7H-[1,4]oxazino[2,3,4-ij]quinoline-6-carboxthioamide,
f) N-(4-Chlorobenzyl)-9-(morpholin-4-ylmethyl)-7-oxo-2-(*R* or *S*)-pyridin-3-yl-2,3-dihydro-7H-[1,4]oxazino[2,3,4-ij]quinoline-6-carboxthioamide,
g) N-(4-Chlorobenzyl)-9-(morpholin-4-ylmethyl)-7-oxo-2-(*R* or *S*)-pyridin-2-yl-2,3-dihydro-7H-[1,4]oxazino[2,3,4-ij]quinoline-6-carboxthioamide,
h) N-(4-Chlorobenzyl)-2-(1-methyl-1H-imidazol-2-yl)-9-(morpholm-4-ylmethyl)-7-oxo-2,3-dihydro-7H-[1,4]oxazino[2,3,4-ij]quinoline-6-carboxthioamide, or a pharmaceutically acceptable salt.

**78.** A compound of claim 1 which is N-(4-chlorobenzyl)-9-(morpholin-4-ylmethyl)-7-oxo-2-(*S*)-pyridin-2-yl-2,3-dihydro-7H-[1,4]oxazino[2,3,4-ij]quinoline-6-carboxamide, or a pharmaceutically acceptable salt.

**79.** A compound of claim 1 which is N-(4-chlorobenzyl)-9-(morpholin-4-ylmethyl)-7-oxo-2-(*R*)-pyridin-2-yl-2,3-dihydro-7H-[1,4]oxazino[2,3,4-ij]quinoline-6-carboxamide, or a pharmaceutically acceptable salt.

**80.** A compound of claim 1 which is N-(4-chlorobenzyl)-9-(morpholin-4-ylmethyl)-7-oxo-2-(*R*)-pyridin-3-yl-2,3-dihydro-7H-[1,4]oxazino[2,3,4-ij]quinoline-6-carboxamide, or a pharmaceutically acceptable salt.

**81.** A compound of claim 1 which is N-(4-chlorobenzyl)-9-(morpholin-4-ylmethyl)-7-oxo-2-(*S*)-pyridin-3-yl-2,3-dihydro-7H-[1,4]oxazino[2,3,4-ij]quinoline-6-carboxamide, or a pharmaceutically acceptable salt.

**82.** A compound of claim 1 which is N-(4-Chlorobenzyl)-2-(1-methyl-1H-imidazol-2-yl)-9-(morpholin-4-ylmethyl)-7-oxo-2,3-dihydro-7H-[1,4]oxazino[2,3,4-ij]quinoline-6-carboxamide, or a pharmaceutically acceptable salt.

**83.** A pharmaceutical composition comprising a compound as in one of claims 1-82 and a pharmaceutically acceptable carrier.

**84.** A use of compound as in one of claims 1-82 to prepare a medicament for treating or inhibiting a viral DNA polymerase.

**85.** A use of an effective amount of a coumpound as in one of claims 1-82 to prepare a medicament for treating infections from herpesviruses in a mammal.

**86.** A use of claim 85 wherein said herpesviruses is herpes simplex virus types 1, herpes simplex virus types 2, varicella zoster virus, cytomegalovirus, Epstein-Barr virus, human herpes viruses 6, human herpes viruses 7 or human herpes viruses 8.

**87.** A use of claim 85 wherein said herpesviruses is herpes simplex virus types 1, herpes simplex virus types 2, varicella zoster virus, cytomegalovirus, Epstein-Barr virus, human herpes viruses 7 or human herpes viruses 8.

**88.** A use of claim 85 wherein said herpesviruses is human cytomegalovirus.

**89.** A use of claim 85 wherein the effective amount of a compound of claim 1 is administered orally, parenterally, topically, rectally, nasally, dublingually, or transdermally.

**90.** A use of claim 85 wherein the effective amount of a compound of claim 1 is in an amount of from about 0.1 to about 300 mg/kg of body weight.

**91.** A use of claim 85 wherein the effective amount of a compound of claim 1 is in an amount of from about 1 to about 30 mg/kg of body weight.

**92.** A use of claim 85 wherein the mammal is human.

**93.** A use of claim 85 wherein the mammal is a food animal or a companion animal.

## Patentansprüche

**1.** Verbindung der Formel I 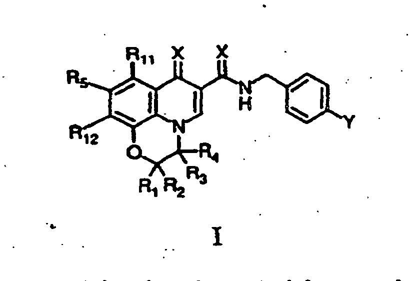 oder ein pharmazeutisch akzeptables Salz, Racemat, Solvat, Tautomer oder optisches Isomer derselben, worin:
jedes X unabhängig voneinander O oder S bedeutet;
Y Cl, F, Br, CN oder NO₂ bedeutet;
R₁, R₂, R₃ und R₄ unabhängig voneinander
a) Wasserstoff
b) N₃,
c) CN,
d) Fluor,
e) Trifluormethyl,
f) Aryl,
g) Het,
h) C₁₋₈-Alkyl, das optional mit R₆ oder OR₇ substituiert ist, bedeuten oder
i) R₁ und R₂ oder R₃ und R₄ zusammen mit dem Kohlenstoff, an den sie gebunden sind, C₃₋₈-Cycloalkyl oder Het bilden;
R₅ C₁₋₈-Alkyl, das partiell ungesättigt und optional mit einem bis drei Resten von N₃, Halogen, CN, R₆ oder R₇ substituiert sein kann, bedeutet;
R₆
a) Aryl,
b) Het,
c) SOᵢR₈,
d) OR₈,
e) C(=O)OR₈,
f) C(=O)R₈ oder
g) NR₈R₉ bedeutet;
R₇
a) P(=O)(OR₁₀)₂,
b) CO(CH₂)ⱼCON(CH₃)(CH₂)ₖSO₃⁻M⁺,
c) eine Aminosäure,
d) C(=O)-C₁₋₆-Alkyl, das optional mit NR¹⁰R¹⁰ substituiert ist, oder
e) CO(CH₂)ₙCO₂H bedeutet;
R₈ und R₉ unabhängig voneinander
a) Wasserstoff,
b) C₃₋₈-Cycloalkyl,
c) Aryl,
d) Het oder
e) C₁₋₈-Alkyl, das ferner optional mit einem oder mehreren Resten von Aryl, Het, Halogen, CN, CO₂R₁₀, SOᵢR₁₀, OR₁₀, NR₁₀R₁₀, CF₃ oder C₃₋₈-Cycloalkyl substituiert ist, bedeuten;
R₁₀
a) H oder
b) C₁₋₈-Alkyl, das optional mit OH oder O-C₁₋₄-Alkyl substituiert ist, bedeutet;
R₁₂ und R₁₂ unabhängig voneinander
a) Wasserstoff,
b) Halogen,
c) NO₂,
d) CN,
e) R₆,
f) SOᵢNR₈R₉ oder
g) C₁₋₈-Alkyl, das partiell ungesättigt und optional mit einem bis drei Resten von N₃, Halogen, CN, R₆ oder OR₇ substituiert sein kann, bedeuten;
Aryl einen Phenylrest, der optional mit einem gesättigten oder ungesättigten carbocyclischen oder heterocyclischen Ring kondensiert ist, bedeutet; wobei Aryl bei jedem Vorkommen mit einem oder mehreren Resten von Halogen, CN, CO₂R₁₀, SOᵢR₁₀, OR₁₀, NR₁₀R₁₀, CF₃, C₃₋₈-Cycloalkyl oder C₁₋₄-Alkyl, wobei C₁₋₄-Alkyl optional mit OR₁₀ substituiert ist, substituiert sein kann;
Het einen vier(4)-, fünf(5)-, sechs(6)- oder sieben(7)-gliedrigen gesättigten oder ungesättigten heterocyclischen Ring mit 1, 2 oder 3 Heteroatomen, die aus der aus O, S und NW, wobei W Wasserstoff, C₁₋₄-Alkyl, C=(O)O-C₁₋₄-Alkyl bedeutet oder nicht vorhanden ist, bestehenden Gruppe ausgewählt sind, bedeutet, wobei Het optional mit einem Benzolring, einem carbocyclischen oder heterocyclischen Ring kondensiert ist; wobei Het bei jedem Vorkommen mit einem oder mehreren Resten von Halogen, CN, CO₂R₁₀, SOᵢR₁₀, OR₁₀, NR₁₀R₁₀, C₁₋₄-Alkyl, CF₃, C₃-C₈-Cycloalkyl, Oxo oder Oxin substituiert sein kann;
ein Cycloalkyl bei jedem Vorkommen mit C₁₋₄-Alkyl, OR¹⁰, Oxo, Oxin oder einen spirokondensierten Het substituiert sein kann;
i 0, 1 oder 2 bedeutet;
j 1, 2, 3, 4, 5 oder 6 bedeutet;
k 1, 2, 3, 4, 5 oder 6 bedeutet;
n 1, 2, 3, 4, 5 oder 6 bedeutet;
M Natrium, Kalium oder Lithium bedeutet; und
mit den folgenden Vorbehalten:
a) mindestens einer der Reste von R₁, R₂, R₃ und R₄ ist von Wasserstoff verschieden;
c) wenn R₁, R₂, R₃ und R₄ unabhängig voneinander C₁₋₈-Alkyl sind, ist mindestens eine der Alkylgruppen mit R₆ oder OR₇ substituiert.

**2.** Verbindung der Formel I gemäß Anspruch 1, die die Formel I-A aufweist.

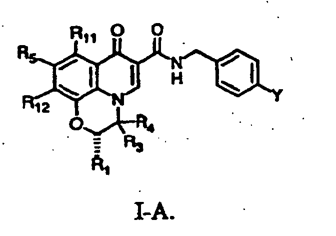

**3.** Verbindung der Formel I gemäß Anspruch 1, die die Formel I-B aufweist.

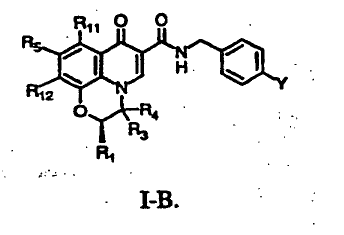

**4.** Verbindung der Formel I gemäß Anspruch 1, die die Formel I-C aufweist.

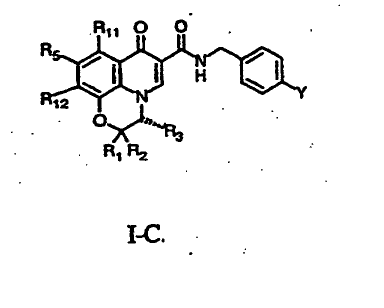

**5.** Verbindung der Formel I gemäß Anspruch 1, die die Formel I-D aufweist.

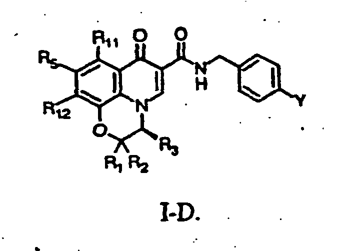

**6.** Verbindung der Formel I gemäß Anspruch 1, die die Formel I-E aufweist.

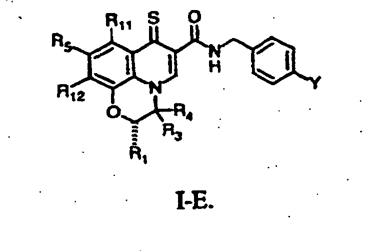

**7.** Verbindung der Formel I gemäß Anspruch 1, die die Formel I-F aufweist.

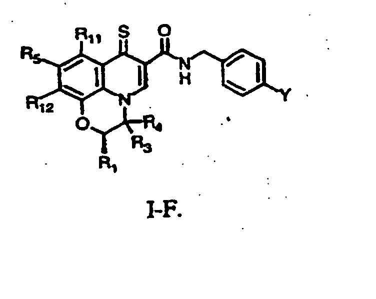

**8.** Verbindung der Formel I gemäß Anspruch 1, die die Formel I-G aufweist.

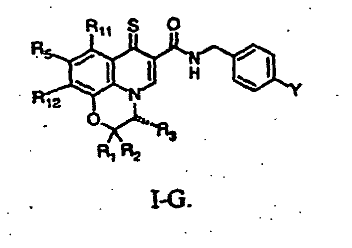

**9.** Verbindung der Formel I gemäß Anspruch 1, die die Formal I-H aufweist.

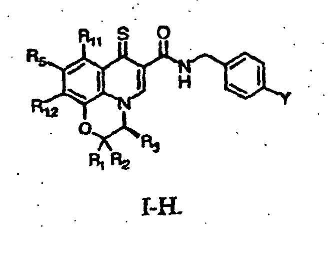

**10.** Verbindung der Formel I gemäß Anspruch 1, die die Formel I-I aufweist.

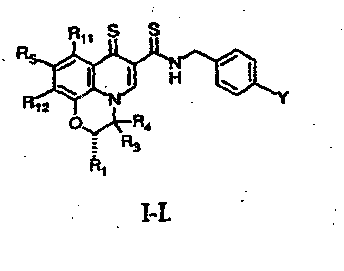

**11.** Verbindung der Formel I gemäß Anspruch 1, die die Formel I-J aufweist.

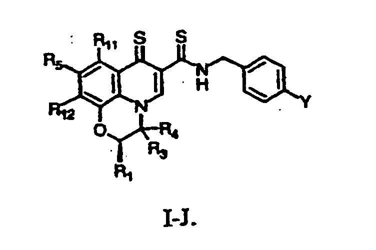

**12.** Verbindung der Formel I gemäß Anspruch 1, die die Formel I-K aufweist.

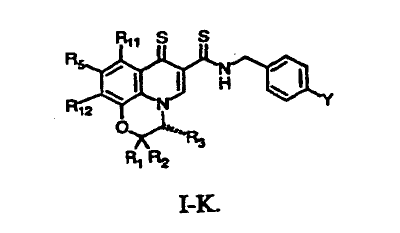

**13.** Verbindung der Formel I gemäß Anspruch 1, die die Formel I-L aufweist.

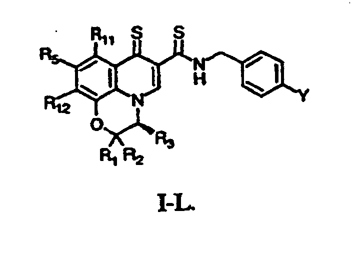

**14.** Verbindung der Formel I gemäß Anspruch 1, die die Formel I-M aufweist.

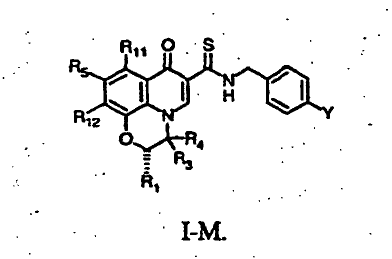

**15.** Verbindung der Formel I gemäß Anspruch 1, die die Formel I-N aufweist.

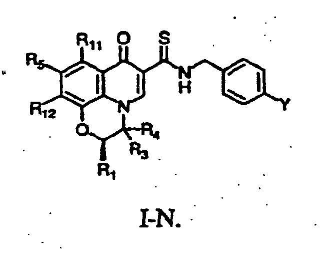

**16.** Verbindung der Formel I gemäß Anspruch 1, die die Formel I-O aufweist.

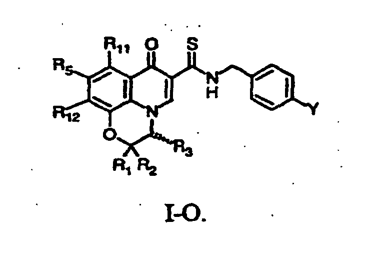

**17.** Verbindung der Formel I gemäß Anspruch 1, die die Formel I-P aufweist.

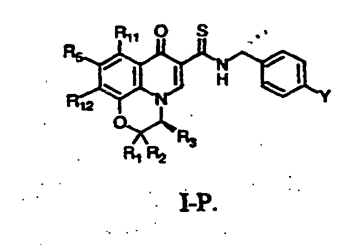

**18.** Verbindung nach den Ansprüchen 1 bis 17, wobei R₁₁ H, Halogen oder C₁₋₄-Alkyl, das optional mit einem bis drei Halogenen substituiert ist, bedeutet; und
R₁₂
a) H,
b) SOᵢR₈,
c) OR₈,
d) C(=O)OR₈,
e) C(=O)R₈,
f) NR₈R₉,
g) SOᵢR₈R₉ oder
h) C₁₋₈-Alkyl, das partiell ungesättigt und optional mit einem bis drei Resten von N₃, Halogen, CN oder R₆ substituiert sein kann, bedeutet.

**19.** Verbindung nach Anspruch 18, worin R₁₁ und R₁₂ Wasserstoff sind.

**20.** Verbindung nach Anspruch 19, worin R₅ C₁₋₈-Alkyl, das mit OR₈ oder Het substituiert ist, bedeutet.

**21.** Verbindung nach Anspruch 19, worin R₅ C₁₋₆-Alkyl, das mit OH substituiert ist, bedeutet.

**22.** Verbindung nach Anspruch 19, worin R₅ C₁₋₄-Alkyl, das mit Het substituiert ist, bedeutet.

**23.** Verbindung nach Anspruch 22, worin Het Morpholinyl oder Thiomorpholinyl ist.

**24.** Verbindung nach Anspruch 19, worin R₅ 4-Morpholinylmethyl ist.

**25.** Verbindung nach Anspruch 19, worin R₅ C₁₋₈-Alkyl, das partiell ungesättigt und optional mit OR₈ substituiert ist, bedeutet.

**26.** Verbindung nach Anspruch 19, worin R₅ Propinyl ist.

**27.** Verbindung nach Anspruch 26, worin das Propinyl mit OH substituiert ist.

**28.** Verbindung nach den Ansprüchen 20 bis 27, worin Y Cl ist.

**29.** Verbindung nach Anspruch 28, worin R₃ und R₄ unabhängig voneinander Wasserstoff sind.

**30.** Verbindung nach Anspruch 29, worin R₁ und R₂ unabhängig voneinander
a) Wasserstoff,
b) Fluor,
c) C₁₋₈-Alkyl, das mit R₆ oder OR₇ substituiert ist;
d) Aryl,
e) Het bedeuten, oder
f) R₁ und R₂ zusammen mit dem Kohlenstoff, an den sie gebunden sind, ein sechs(6)gliedriges Cycloalkyl oder ein Het bilden;
worin R₆
a) Het,
b) SOᵢR₈,
c) OR₈ oder
d) NR₈R₉ bedeutet;
worin R₇
a) P(=O)(OR₁₀)₂,
b) CO (CH₂)ₙCON(CH₃)(CH₂)ₙSO₃⁻M⁺ oder
c) C(=O)-C₁₋₆-Alkyl bedeutet;
worin R₈ und R₉ unabhängig voneinander
a) Wasserstoff,
b) Aryl,
c) Het oder
d) C₁₋₈-Alkyl, das ferner optional mit einem oder mehreren Resten von Aryl, Het, Halogen, CO₂R₁₀, SOᵢR₁₀ oder OR₁₀ substituiert ist, bedeuten;
worin R₁₀
a) H oder
c) C₁₋₄-Alkyl, das optional mit OH substituiert ist, bedeutet.

**31.** Verbindung nach Anspruch 30, worin R₁ und R₂ unabhängig voneinander H, C₁₋₄-Alkyl, das mit OR₈, worin R₈ H ist, substituiert ist, oder C₁₋₄-Alkyl, das mit OR₁₀ substituiert ist, bedeuten.

**32.** Verbindung nach Anspruch 30, worin R₁ H bedeutet; R₂ Aryl bedeutet, wobei Aryl optional mit einem oder zwei Resten von Halogen, CN, OR₁₀ oder mit OR₁₀ substituiertem C₁₋₄-Alkyl substituiert ist.

**33.** Verbindung nach Anspruch 30, worin R₁ H bedeutet; R₂ Aryl bedeutet, wobei Aryl mit einem heterocyclischen Ring kondensiert ist.

**34.** Verbindung nach Anspruch 33, worin R₂ 1,3-Benzodioxolyl oder 1,4-Benzodioxinyl ist.

**35.** Verbindung nach Anspruch 30, worin R₁ H ist; R₂ Het ist.

**36.** Verbindung nach Anspruch 35, worin Het einen fünf(5)- oder sechs (6) gliedrigen gesättigten oder ungesättigten heterocyclischen Ring mit 1, 2 oder 3 Heteroatomen, die aus der aus O, S und NW, wobei W Wasserstoff, C₁₋₄-Alkyl, C(=O)O-C₁₋₄-Alkyl bedeutet oder nicht vorhanden ist, bestehenden Gruppe ausgewählt sind, bedeutet, wobei Het mit einem oder mehreren Resten von Halogen, C₁₋₄-Alkyl, CF₃, Oxo oder Oxin substituiert sein kann.

**37.** Verbindung nach Anspruch 36, worin Het Pyridinyl ist.

**38.** Verbindung nach Anspruch 36, worin Het ein fünf(5)-gliedriger heterocyclischer Ring ist.

**39.** Verbindung nach Anspruch 30, worin R₁ und R₂ zusammen mit dem Kohlenstoff, an den sie gebunden sind, ein Het bilden.

**40.** Verbindung nach Ansprucn 39, worin Het einen fünf(5)- oder sechs(6)gliedrigen heterocyclischen Ring mit 1, 2 oder 3 Heteroatomen, die aus der aus O, S und NW, wobei W Wasserstoff, C₁₋₄-Alkyl, C(=O)O-C₁₋₄-Alkyl bedeutet, bestehenden Gruppe ausgewählt sind, bedeutet, wobei Het mit einem oder mehreren Resten von Halogen, OR₁₀, C₁₋₄-Alkyl, CF₃, Oxo oder Oxin substituiert sein kann.

**41.** Verbindung nach Anspruch 40, worin Het ein (6)-gliedriger heterocyclischer Ring ist.

**42.** Verbindung nach Anspruch 41, worin Het Pyran, Piperidin oder Thiopyran ist.

**43.** Verbindung nach Anspruch 30, worin R₁ und R₂ zusammen mit dem Kohlenstoff, an den sie gebunden sind, ein sechs(6)gliedriges Cycloalkyl bilden.

**44.** Verbindung nach Anspruch 43, worin Cycloalkyl optional mit Oxo oder OR₁₀ substituiert ist.

**45.** Verbindung nach Anspruch 30, worin
R₂ Wasserstoff bedeutet;
R₁ C₁₋₈-Alkyl, das mit R₆ oder OR₇ substituiert ist, bedeutet;
worin R₆
a) Het,
b) SR₈,
c) OR₈ oder
d) NR₈R₉ bedeutet;
worin R₇
a) P(=O)(OCH₃)₂,
b) CO(CH₂)ₙCON(CH₃)(CH₂)ₙSO₃⁻M⁺ oder
c) C(=O)CH₃ bedeutet;
worin R₈ und R₉ unabhängig voneinander
a) Wasserstoff,
b) Het oder
c) C₁₋₈-Alkyl, das optional mit einem oder zwei Resten von Het, CO₂R₁₀, SOᵢR₁₀ oder OR₁₀ substituiert ist, bedeuten; und
worin R₁₀
a) H oder
b) C₁₋₄-Alkyl, das optional mit OH oder O-C₁₋₄-Alkyl substituiert ist, bedeutet.

**46.** Verbindung nach Anspruch 45, worin R₁ mit Het substituiertes C₁₋₈-Alkyl bedeutet.

**47.** Verbindung nach Anspruch 46, worin Het Piperidinyl, Morpholinyl, Thiomorpholinyl, Piperazinyl, N-C₁₋₄-Alkylsubstituiertes Piperazinyl, Pyrrolidinyl, Pyridyl, Imidazolyl oder N-C₁₋₄-Alkyl-substituiertes Imidazol bedeutet.

**47.** Verbindung nach Anspruch 46, worin Het Morpholinyl ist.

**48.** Verbindung nach Anspruch 47, worin Het Pyridinyl ist.

**49.** Verbindung nach Anspruch 45, worin R₁ C₁₋₈-Alkyl, das mit OH oder O-C₁₋₄-Alkyl substituiert ist, bedeutet.

**50.** Verbindung nach Anspruch 49, worin R₁ C₁₋₄-Alkyl, das mit OH substituiert ist, bedeutet.

**51.** Verbindung nach Anspruch 45, worin R₁ C₁₋₈-Alkyl, das mit SR₈ substituiert ist, bedeutet.

**52.** Verbindung nach Anspruch 51, worin R₈ Het ist.

**53.** Verbindung nach Anspruch 51, worin R₈ C₁₋₄-Alkyl, das optional mit einem oder zwei Resten OR₁₀ substituiert ist, bedeutet.

**54.** Verbindung nach Anspruch 45, worin R₁ C₁₋₄-Alkyl, das mit NR₈R₉ substituiert ist, bedeutet.

**55.** Verbindung nach Anspruch 54, worin R₈ H ist und R₉ Het ist.

**56.** Verbindung nach Anspruch 55, worin Het einen sechs(6)-gliedrigen heterocyclischen Ring mit 1, 2 oder 3 Heteroatomen, die aus der aus O, S und NW, wobei W Wasserstoff, C₁₋₄-Alkyl bedeutet oder nicht vorhanden ist, bestehenden Gruppe ausgewählt sind, bedeutet.

**57.** Verbindung nach Anspruch 56, worin Het Pyridinyl ist.

**58.** Verbindung nach Anspruch 54, worin R₈ H ist und R₉ C₁₋₈-Alkyl, das optional mit Het substituiert ist, bedeutet.

**59.** Verbindung nach Anspruch 58, worin Het einen sechs(6)-gliedrigen heterocyclischen Ring mit 1, 2 oder 3 Heteroatomen, die aus der aus O, S und NW, wobei W Wasserstoff, C₁₋₄-Alkyl bedeutet oder nicht vorhanden ist, bestehenden Gruppe ausgewählt sind, bedeutet.

**60.** Verbindung nach Anspruch 59, worin Het Pyridinyl ist.

**61.** Verbindung nach Anspruch 54, worin R₈ H ist und R₉ C₁₋₈-Alkyl, das optional mit einem oder zwei Resten OR₁₀ substituiert ist, bedeutet.

**62.** Verbindung nach Anspruch 45, worin R₁ Hydroxymethyl, Morpholinylmethyl, (Pyridinylmethyl)aminomethyl, (Dimethylamino)methyl, (Hydroxyethyl)sulfanylmethyl, (1-Methyl-1H-imidazol-2-yl)sulfanylmethyl, -CH₂OCO(CH₂)₆CON(CH₃)(CH₂)₂SO₃⁻M⁺, -CH₂OC(=O)CH₃, CH₂OP(=O)(OMe)₂, (4-Methyl-1-piperazinyl)methyl, 1-Pyrrolidinylmethyl, (2, 3-Dihydroxpropyl)aminomethyl, (2-Hydroxyethyl)aminomethyl, 1-Piperidinylmethyl, Bis(2-hydroxyethyl)aminomethyl, 1H-Imidazol-1-ylmethyl, (Methylsulfanyl)methyl, (tert-Butylsulfanyl)methyl, Methylsulfanylacetat, (2,3-Dihydroxypropyl)sulfanylmethyl, Phenyl oder Fluor bedeutet.

**63.** Verbindung nach Anspruch 62, worin R₁ Hydroxymethyl, Morpholinylmethyl, (2-Pyridinylmethyl)aminomethyl, (3-Pyridinylmethyl)aminomethyl, (Dimethylamino)methyl, (2-Hydroxyethyl) sulfanylmethyl, (1-Methyl-1H-imidazol-2-yl) sulfanylmethyl, OP(=O)(OCH₃)₂, -CH₂OCO(CH₂)₆CON(CH₃)(CH₂)₂SO₃⁻M⁺ oder -CH₂OC(=O)CH₃ ist.

**64.** Verbindung nach Anspruch 12, worin R₁ und R₂ unabhängig voneinander Wasserstoff bedeuten,
R₃ und R₄ unabhängig voneinander
a) Wasserstoff,
b) Fluor oder
c) C₁₋₈-Alkyl, das mit R₆ oder OR₇ substituiert ist, bedeuten;
worin R₆
a) Het,
b) SOᵢR₈,
c) OR₈ oder
d) NR₈R₉ bedeutet;
worin R₇
a) P(=O)(OH)₂,
b) (P=O) (C₁₋₄-Alkoxy)₂,
c) CO(CH₂)ₙCON(CH₃)(CH₂)ₙSO₃⁻M⁺ oder
d) C(=O)-C₁₋₆-Alkyl bedeutet;
worin R₈ und R₉ unabhängig voneinander
a) Wasserstoff,
b) Aryl,
c) Het oder
d) C₁₋₈-Alkyl, das ferner optional mit einem oder mehreren Resten von Aryl, Het, Halogen, CO₂R₁₀, SOᵢR₁₀ oder OR₁₀ substituiert ist, bedeuten;
worin R₁₀
a) H oder
d) C₁₋₄-Alkyl, das optional mit OH substituiert ist, bedeutet.

**65.** Verbindung nach Anspruch 20, worin R₃ und R₄ unabhängig voneinander Fluor oder Hydroxymethyl bedeuten.

**66.** Verbindung nach Anspruch 20, worin R₃ Wasserstoff ist und R₄ Phenyl, Morpholinylmethyl oder Hydroxymethyl bedeutet.

**67.** Verbindung nach Anspruch 22, worin R₄ Morpholinylmethyl ist.

**68.** Verbindung nach Anspruch 1, nämlich
a) N-(4-Chlorbenzyl)-2-(hydroxymethyl)-9-(morpholin-4-ylmethyl)-7-oxo-2,3-dihydro-7H-[1,4]oxazino[2,3,4-ij]chinolin-6-carboxamid,
b) N-(4-Chlorbenzyl)-2-(R oder S)-(hydroxymethyl)-9-(morpholin-4-ylmethyl)-7-oxo-2,3-dihydro-7H-[1,4]oxazino[2,3,4-ij]chinolin-6-carboxamid,
c) N-(4-Chlorbenzyl)-9-(morpholin-4-ylmethyl)-7-oxo-2-pyridin-3-yl-2,3-dihydro-7H-[1,4]oxazino[2,3,4-ij]-chinolin-6-carboxamid,
d) N-(4-Chlorbenzyl)-9-(morpholin-4-ylmethyl)-7-oxo-2-pyridin-4-yl-2,3-dihydro-7H-[1,4]oxazino[2,3,4-ij]-chinolin-6-carboxamid,
e) N-(4-Chlorbenzyl)-9-(morpholin-4-ylmethyl)-7-oxo-2-pyridin-2-yl-2,3-dihydro-7H-[1,4]oxazino[2,3,4-ij]-chinolin-6-carboxamid,
f) N-(4-Chlorbenzyl)-9-(morpholin-4-ylmethyl)-7-oxo-2-(R oder S)-pyridin-3-yl-2,3-dihydro-7H-[1,4]oxazino[2,3,4-ij]chinolin-6-carboxamid,
g) N-(4-Chlorbenzyl)-2,9-bis(morpholin-4-ylmethyl)-7-oxo-2,3-dihydro-7H-[1,4]oxazino[2,3,4-ij]chinolin-6-carboxamid,
h) 2- [(tert-Butylsulfanyl)methyl]-N-(4-chlorbenzyl)-9-(morpholin-4-ylmethyl)-7-oxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-oxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]chinolin-6-carboxamid,
k) N-(4-Chlorbenzyl)-9-(morpholin-4-ylmethyl)-7-oxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]methylacetat,
m) N-(4-Chlorbenzyl)-9-(morpholin-4-ylmethyl)-7-oxo-2-(R oder S)-{[(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl)-7-oxo-2,3-dihydro-7H-[1,4]oxazino[2,3,4-ij]chinolin-6-carboxamid,
o) N-(4-Chlorbenzyl)-9-(morpholin-4-ylmethyl)-7-oxo-2-(R oder S)-pyridin-2-yl-2,3-dihydro-7H-[1,4]oxazino[2,3,4-ij]chinolin-6-carboxamid,
p) N-(4-Chlorbenzyl)-2-[3-(hydroxymethyl)phenyl]-9-(morpholin-4-ylmethyl)-7-oxo-2,3-dihydro-7H-[1,4]oxazino[2,3,4-ij]chinolin-6-carboxamid,
q) N-(4-Chlorbenzyl)-2-[2-(hydroxymethyl)phenyl]-9-(morpholin-4-ylmethyl)-7-oxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-oxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-oxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-oxo-2,3-dihydro-7H-[1,4]oxazino[2,3,4-ij]chinolin-6-carboxamid,
u) N-(4-Chlorbenzyl)-2-(3-furyl)-9-(morpholin-4-ylmethyl)-7-oxo-2,3-dihydro-7H-[1,4]oxazino[2,3,4-ij]chinolin-6-carboxamid,
v) N-(4-Chlorbenzyl)-9-(morpholin-4-ylmethyl)-7-oxo-2-thien-2-yl-2,3-dihydro-7H-[1,4]oxazino[2,3,4-ij]chinolin-6-carboxamid,
w) N-(4-Chlorbenzyl)-2-(3,5-difluorphenyl)-9-(morpholin-4-ylmethyl)-7-oxo-2,3-dihydro-7H-[1,4]oxazino[2,3,4-ij]chinolin-6-carboxamid,
x) 2-(1,3-Benzodioxol-5-yl)-N-(4-chlorbenzyl)-9-(morpholin-4-ylmethyl)-7-oxo-2,3-dihydro-7H-[1,4]oxazino[2,3,4-ij]chinolin-6-carboxamid,
y) N-(4-Chlorbenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]chinolin-6-carboxamid,
z) 2-(1,3-Benzodioxol-4-yl)-N-(4-chlorbenzyl)-9-(morpholin-4-ylmethyl)-7-oxo-2,3-dihydro-7H-[1,4]oxazino[2,3,4-ij]chinolin-6-carboxamid,
aa) 2-[3,5-Bis(methoxymethoxy)phenyl]-N-(4-chlorbenzyl)-9-(morpholin-4-ylmethyl)-7-oxo-2,3-dihydro-7H-[1,4]oxazino[2,3,4-ij]chinolin-6-carboxamid,
bb) N-(4-Chlorbenzyl)-9-(morpholin-4-ylmethyl)-7-oxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-oxo-2,3-dihydro-7H-[1,4]oxazino[2,3,4-ij]chinolin-6-carboxamid,
dd) N-[(4-Chlorphenyl)methyl]-9'-(4-morpholinylmethyl)-4,7'-dioxospiro[cyclohexan-1,2'(3'H)-[7H]pyrido[1,2,3-de][1,4]benzoxazin]-6'-carboxamid,
ee) N-[(4-Chlorphenyl)methyl]-4-hydroxy-9'-(4-morpholinylmethyl)-7'-oxospiro[cyclohexan-1,2'(3'H)-[7H]pyrido[1,2,3-de][1,4]benzoxazin]-6'carboxamid,
ff) N-(4-Chlorbenzyl)-3,9-bis(morpholin-4-ylmethyl)-7-oxo-2,3-dihydro-7H-[1,4]oxazino[2,3,4-ij]chinolin-6-carboxamid,
gg) N-(4-Chlorbenzyl)-9-(morpholin-4-ylmethyl)-7-oxo-2-phenyl-2,3-dihydro-7H-[1,4]oxazino[2,3,4-ij]-chinolin-6-carboxamid,
hh) N-(4-Chlorbenzyl)-2,2-difluor-9-(morpholin-4-ylmethyl)-7-oxo-2,3-dihydro-7H-[1,4]oxazino[2,3,4-ij]chinolin-6-carboxamid,
ii) N-(4-Chlorbenzyl)-2-[(methylsulfanyl)methyl]-9-(morpholin-4-ylmethyl)-7-oxo-2,3-dihydro-7H-[1,4]oxazino[2,3,4-ij]chinolin-6-carboxamid,
jj) N-(4-Chlorbenzyl)-2-[(dimethylamino)methyl]-9-(morpholin-4-ylmethyl)-7-oxo-2,3-dihydro-7H-[1,4]oxazino[2,3,4-ij]chinolin-6-carboxamid,
kk) N-(4-Chlorbenzyl)-2-[(4-methyl-1-piperazinyl)-methyl]-9-(morpholin-4-ylmethyl)-7-oxo-2,3-dihydro-7H-[1,4]oxazino[2,3,4-ij]chinolin-6-carboxamid,
ll) Methyl-({[6-{[(4-chlorbenzyl)amino]carbonyl}-9-(morpholin-4-ylmethyl)-7-oxo-2,3-dihydro-7H-[1,4]oxazino[2,3,4-ij]chinolin-2-yl]methyl}thio)-acetat,
mm) N- (4-Chlorbenzyl) -9-(morpholin-4-ylmethyl)-7-oxo-2-(1-pyrrolidinylmethyl)-2,3-dihydro-7H-[1,4]oxazino[2, 3, 4-ij]chinolin-6-carboxamid,
nn) N-(4-Chlorbenzyl)-2-{[(2,3-dihydroxypropyl)-sulfanyl]methyl}-9-(morpholin-4-ylmethyl)-7-oxo-2,3-dihydro-7H-[1,4]oxazino[2,3,4-ij]chinolin-6-carboxamid,
oo) N-(4-Chlorbenzyl)-2-{[(2,3-dihydroxypropyl)amino]-methyl}-9-(morpholin-4-ylmethyl)-7-oxo-2,3-dihydro-7H- [1,4]oxazino[2,3,4-ij]chinolin-6-carboxamid,
pp) N-(4-Chlorbenzyl)-2-{[(2-hydroxyethyl)amino]methyl}-9-(morpholin-4-ylmethyl)-7-oxo-2,3-dihydro-7H-[1,4]oxazino[2,3,4-ij]chinolin-6-carboxamid,
qq) N-(4-Chlorbenzyl)-9-(morpholin-4-ylmethyl)-7-oxo-2-(1-piperidinylmethyl)-2,3-dihydro-7H-[1,4]oxazino[2, 3, 4-ij]chinolin-6-carboxamid,
rr) 2-{[Bis (2-hydroxyethyl)amino]methyl}-N-(4-chlorbenzyl)-9-(morpholin-4-ylmethyl)-7-oxo-2,3-dihydro-7H-[1,4]oxazino[2,3,4-ij]chinolin-6-carboxamid,
ss) N-(4-Chlorbenzyl)-9-(morpholin-4-ylmethyl)-7-oxo-2-{[(2-pyridinylmethyl)amino]methyl}-2,3-dihydro-7H-[1,4]oxazino[2,3,4-ij]chinolin-6-carboxamid,
tt) 2-[(8-{[6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]methoxy}-8-oxooctanoyl)(methyl)amino]ethansulfonsäurenatriumsalz,
uu) [6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]methyldimethylphosphat,
vv) N-(4-Chlorbenzyl)-9-(morpholin-4-ylmethyl)-7-oxo-2-{[(4-pyridinylmethyl)amino]methyl}-2,3-dihydro-7H-[1,4]oxazino[2,3,4-ij]chinolin-6-carboxamid,
ww) N-(4-Chlorbenzyl)-2-(1H-imidazol-1-ylmethyl)-9-(morpholin-4-ylmethyl)-7-oxo-2,3-dihydro-7H-[1,4]oxazino[2,3,4-ij]chinolin-6-carboxamid,
xx) N-(4-Chlorbenzyl)-2-{[(4-Chlorbenzyl)amino]methyl}-9-(morpholin-4-ylmethyl)-7-oxo-2,3-dihydro-7H-[1,4]oxazino[2,3,4-ij]chinolin-6-carboxamid,
yy) N-(4-Chlorbenzyl)-3-(hydroxymethyl)-9-(morpholin-4-ylmethyl)-7-oxo-2,3-dihydro-7H-[1,4]oxazino[2,3,4-ij]chinolin-6-carboxamid,
zz) N- (4-Chlorbenzyl)-2-(4-hydroxyphenyl) -9- (morpholin-4-ylmethyl)-7-oxo-2,3-dihydro-7H-[1,4]oxazino[2,3,4-ij]chinolin-6-carboxamid,
aaa) N-(4-Chlorbenzyl)-2-{3-[(methoxymethoxy)methyl]-phenyl}-9-(morpholin-4-ylmethyl)-7-oxo-2,3-dihydro-7H-[1,4]oxazino[2, 3, 4-ij] chinolin-6-carboxamid,
bbb) N-(4-Chlorbenzyl)-2-{2-[(methoxymethoxy)methyl]-phenyl}-9-(morpholin-4-ylmethyl)-7-oxo-2,3-dihydro-7H-[1,4]oxazino[2,3,4-ij]chinolin-6-carboxamid,
ccc) N-(4-Chlorbenzyl)-2-(2-hydroxyphenyl)-9-(morpholin-4-ylmethyl)-7-oxo-2,3-dihydro-7H-[1,4]oxazino[2,3,4-ij]chinolin-6-carboxamid,
ddd) N-[(4-Chlorphenyl)methyl]-2,3,5,6-tetrahydro-9'-(4-morpholinylmethyl)-7'-oxospiro[4H-pyran-4,2'(3'H)-[7H]pyrido[1,2,3-de][1,4]benzoxazin]-6'-carboxamid,
eee) 1,1-Dimethylethyl-6-[[[(4-chlorphenyl)methyl]amino]-carbonyl]-9'-(4-morpholinylmethyl)-7'-oxospiro[piperidin-4,2' (3'H)-[7H]pyrido[1,2,3-de][1,4]-benzoxazin]-1-carboxylat,
fff) N-[(4-Chlorphenyl)methyl]-9'-(4-morpholinylmethyl)-7'-oxospiro[piperidin-4,2'(3'H)-[7H]pyrido[1,2,3-de][1,4]benzoxazin]-6'-carboxamid,
ggg) N-(4-Chlorbenzyl)-2,2-bis(hydroxymethyl)-9-(morpholin-4-ylmethyl)-7-oxo-2,3-dihydro-7H-[1,4]oxazino[2,3,4-ij]chinolin-6-carboxamid,
hhh) N- [(4-Chlorphenyl)methyl]-2',3',5',6'-tetrahydro-9-(4-morpholinylmethyl)-7-oxospiro[7H-pyrido[1,2,3-de]-1,4-benzoxazin-2(3H),4'-[4H]thiopyran]-6-carboxamid,
iii) N-(4-Chlorbenzyl)-9-(morpholin-4-ylmethyl)-7-oxo-3-phenyl-2,3-dihydro-7H-[1,4]oxazino[2,3,4-ij]-chinolin-6-carboxamid,
jjj) N-(4-Chlorbenzyl)-3, 3-bis(hydroxymethyl)-9- (3-hydroxy-1-propinyl)7-oxo-2,3-dihydro-7H-[1,4]oxazino-[2,3,4-ij]chinolin-6-carboxamid,
kkk) N-(4-Chlorbenzyl)-3,3-bis(hydroxymethyl)-9-(3-hydroxypropyl)-7-oxo-2,3-dihydro-7H-[1,4]oxazino[2, 3, 4-ij]chinolin-6-carboxamid,
lll) N-(4-Chlorbenzyl)-2-[2-(methoxymethoxy)phenyl]-9-(morpholin-4-ylmethyl)-7-oxo-2,3-dihydro-7H-[1,4]oxazino[2,3,4-ij]chinolin-6-carboxamid,
mmm) N-(4-Chlorbenzyl)-2-{4-[(methoxymethoxy)methyl]-phenyl}-9-(morpholin-4-ylmethyl)-7-oxo-2,3-dihydro-7H-[1,4]oxazino[2,3,4-ij]chinolin-6-carboxamid,
nnn) 2-[2,3-Bis(methoxymethoxy)phenyl]-N-(4-chlorbenzyl)-9-(morpholin-4-ylmethyl)-7-oxo-2,3-dihydro-7H-[1,4]oxazino[2,3,4-ij]chinolin-6-carboxamid,
ooo) N-[(4-Chlorphenyl)methyl]-1-methyl-9'-(4-morpholinylmethyl)-7'-oxospiro[piperidin-4,2'(3'H)-[7H]pyrido[1,2,3-de][1,4]benzoxazin]-6'-carboxamid,
ppp) N-[(4-Chlorphenyl)methyl]-9"-(4-morpholinylmethyl)-dispiro[1,3-dioxolan-2,1'-cyclohexan-4',2"(3"H)-[7H]-pyrido[1,2,3-de][1,4]benzoxazin]-6"-carboxamid, oder ein pharmazeutisch akzeptables Salz.

**69.** Verbindung nach Anspruch 1, nämlich
a) N- (4-Chlorbenzyl) -2-(hydroxymethyl) -9- (morphol in-4-ylmethyl)-7-thioxo-2,3-dihydro-7H-[1,4]oxazino-2,3,4-ij]chinolin-6-carboxamid,
b) N-(4-Chlorbenzyl)-2-(R oder S)-(hydroxymethyl)-9-(morpholin-4-ylmethyl)-7-thioxo-2,3-dihydro-7H-[1,4]-oxazino[2,3,4-ij]chinolin-6-carboxamid,
c) N-(4-Chlorbenzyl)-9-(morpholin-4-ylmethyl)-7-thioxo-2-pyridin-3-yl-2,3-dihydro-7H-[1,4]oxazino[2,3,4-ij]chinolin-6-carboxamid,
d) N- (4-Chlorbenzyl)-9-(morpholin-4-ylmethyl)-7-thioxo-2-pyridin-4-yl-2,3-dihydro-7H-[1,4]oxazino[2,3,4-ij]chinalin-6-carboxamid,
e) N-(4-Chlorbenzyl)-9-(morpholin-4-ylmethyl)-7-thioxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-thioxo-2-(R oder S)-pyridin-3-yl-2,3-dihydro-7H-[1,4]oxazino[2,3,4-ij]chinolin-6-carboxamid,
g) N-(4-Chlorbenzyl)-2,9-bis (morpholin-4-ylmethyl)-7-thioxo-2,3-dihydro-7H-[1,4]oxazino[2,3,4-ij]-chinolin-6-carboxamid,
h) 2-[(tert-Butylsulfanyl)methyl]-N-(4-chlorbenzyl)-9-(morpholin-4-ylmethyl)-7-thioxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-thioxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]-chinolin-6-carboxamid,
k) N-(4-Chlorbenzyl)-9-(morpholin-4-ylmethyl)-7-thioxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methylacetat,
m) N-(4-Chlorbenzyl)-9-(morpholin-4-ylmethyl)-7-thioxo-2-(R oder S)-{([(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl)-7-thioxo-2,3-dihydro-7H-[1,4]oxazino[2,3,4-ij]chinolin-6-carboxamid,
o) N-(4-Chlorbenzyl)-9-(morpholin-4-ylmethyl)-7-thioxo-2-(R oder S)-pyridin-2-yl-2,3-dihydro-7H-[1,4]-oxazino[2,3,4-ij]chinolin-6-carboxamid,
p) N-(4-Chlorbenzyl)-2-[3-(hydroxymethyl)phenyl]-9-(morpholin-4-ylmethyl)-7-thioxo-2,3-dihydro-7H-[1,4]oxazino[2,3,4-ij]chinolin-6-carboxamid,
q) N-(4-Chlorbenzyl)-2-[2-(hydroxymethyl)phenyl]-9-(morpholin-4-ylmethyl)-7-thioxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-thioxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-thioxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-thioxo-2,3-dihydro-7H-[1,4]oxazino[2,3,4-ij]chinolin-6-carboxamid,
u) N-(4-Chlorbenzyl)-2-(3-furyl)-9-(morpholin-4-ylmethyl)-7-thioxo-2,3-dihydro-7H-[1,4]oxazino[2,3,4-ij]chinolin-6-carboxamid,
v) N-(4-Chlorbenzyl)-9-(morpholin-4-ylmethyl)-7-thioxo-2-thien-2-yl-2,3-dihydro-7H-[1,4]oxazino[2,3,4-ij]chinolin-6-carboxamid,
w) N-(4-Chlorbenzyl)-2-(3,5-difluorphenyl)-9-(morpholin-4-ylmethyl)-7-thioxo-2,3-dihydro-7H-[1,4]oxazino[2,3,4-ij]chinolin-6-carboxamid,
x) 2-(1,3-Benzodioxol-5-yl)-N-(4-chlorbenzyl)-9-(morpholin-4-ylmethyl)-7-thioxo-2,3-dihydro-7H-[1,4]oxazino[2,3,4-ij]chinolin-6-carboxamid,
y) N-(4-Chlorbenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]chinolin-6-carboxamid,
z) 2-(1,3-Benzodioxol-4-yl)-N-(4-chlorbenzyl)-9-(morpholin-4-ylmethyl)-7-thioxo-2,3-dihydro-7H-[1,4]oxazino[2,3,4-ij]chinolin-6-carboxamid,
aa) 2- [3, 5-Bis (methoxymethoxy)phenyl]-N-(4-chlorbenzyl)-9-(morpholin-4-ylmethyl)-7-thioxo-2,3-dihydro-7H-[1,4]oxazino[2,3,4-ij]chinolin-6-carboxamid,
bb) N-(4-Chlorbenzyl)-9-(morpholin-4-ylmethyl)-7-thioxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-thioxo-2,3-dihydro-7H-[1,4]oxazino[2,3,4-ij]chinolin-6-carboxamid,
dd) N-[(4-Chlorphenyl)methyl]-9'-(4-morpholinylmethyl)-4-oxo-7'-thioxospiro[cyclohexan-1,2'(3'H)-[7H]pyrido[1,2,3-de][1,4]benzoxazin]-6'-carboxamid,
ee) N-[(4-Chlorphenyl)methyl]-4-hydroxy-9'-(4-morpholinylmethyl)-7'-thioxospiro[cyclohexan-1,2'(3'H)-[7H]pyrido[1,2,3-de]-[1,4]benzoxazin]-6'carboxamid,
ff) N-(4-Chlorbenzyl)-3,9-bis(morpholin-4-ylmethyl)-7-thioxo-2,3-dihydro-7H-[1,4]oxazino[2,3,4-ij]-chinolin-6-carboxamid,
gg) N-(4-Chlorbenzyl)-9-(morpholin-4-ylmethyl)-7-thioxo-2-phenyl-2,3-dihydro-7H-[1,4]oxazino[2,3,4-ij]chinolin-6-carboxamid,
hh) N-(4-Chlorbenzyl)-2,2-difluor-9-(morpholin-4-ylmethyl)-7-thioxo-2,3-dihydro-7H-[1,4]oxazino[2,3,4-ij]chinolin-6-carboxamid,
ii) N-(4-Chlorbenzyl)-2-[(methylsulfanyl)methyl]-9-(morpholin-4-ylmethyl)-7-thioxo-2,3-dihydro-7H-[1,4]oxazino[2,3,4-ij]chinolin-6-carboxamid,
jj) N-(4-Chlorbenzyl)-2-[(dimethylamino)methyl]-9-(morpholin-4-ylmethyl)-7-thioxo-2,3-dihydro-7H-[1,4]oxazino[2,3,4-ij]chinolin-6-carboxamid,
kk) N-(4-Chlorbenzyl)-2-[(4-methyl-1-piperazinyl)-methyl]-9-(morpholin-4ylmethyl)-7-thioxo-2,3-dihydro-7H-[1,4]oxazino[2,3,4-ij]chinolin-6-carboxamid,
ll) Methyl-({[6-{[(4-chlorbenzyl)amino]carbonyl}-9-(morpholin-4-ylmethyl)-7-thioxo-2,3-dihydro-7H-[1,4]oxazino[2,3,4-ij]chinolin-2-yl]methyl)-thio)acetat,
mm) N-(4-Chlorbenzyl)-9-(morpholin-4-ylmethyl)-7-thioxo 2-(1-pyrrolidinylmethyl)-2,3-dihydro-7H-[1,4]oxazino[2,3,4-ij]chinolin-6-carboxamid,
nn) N-(4-Chlorbenzyl)-2-{[(2,3-dihydroxypropyl)-sulfanyl]methyl}-9-(morpholin-4-ylmethyl)-7-thioxo-2,3-dihydro-7H-[1,4]oxazino[2,3,4-ij]chinolin-6-carboxamid,
oo) N-(4-Chlorbenzyl)-2-{[(2,3-dihydroxypropyl)-amino]methyl}-9-(morpholin-4-ylmethyl)-7-thioxo-2,3-dihydro-7H-[1,4]oxazino[2,3,4-ij]chinolin-6-carboxamid,
pp) N-(4-Chlorbenzyl)-2-{[(2-hydroxyethyl)amino]methyl}-9-(morpholin-4-ylmethyl)-7-thioxo-2,3-dihydro-7H-[1,4]oxazino[2,3,4-ij]chinolin-6-carboxamid,
qq) N-(4-Chlorbenzyl)-9-(morpholin-4-ylmethyl)-7-thioxo-2-(1-piperidinylmethyl)-2,3-dihydro-7H-[1,4]oxazino[2,3,4ij]chinolin-6-carboxamid,
rr) 2-{[Bis(2-hydroxyethyl)amino]methyl}-N-(4-chlorbenzyl)-9-(morpholin-4-ylmethyl)-7-thioxo-2,3-dihydro-7H-[1,4]oxazino[2,3,4-ij]chinolin-6-carboxamid,
ss) N-(4-Chlorbenzyl)-9-(morpholin-4-ylmethyl)-7-thioxo-2- {[(2-pyridinylmethyl)amino]methyl}-2,3-dihydro-7H-[1,4]oxazino [2, 3, 4-ij]chinolin-6-carboxamid,
tt) 2-[(8-{[6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methoxy}-8-oxooctanoyl) (methyl)amino]ethansulfonsäurenatriumsalz,
uu) [6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methyldimethylphosphat,
vv) N-(4-Chlorbenzyl)-9-(morpholin-4-ylmethyl)-7-thioxo-2-{[(4-pyridinylmethyl)amino]methyl}-2,3-dihydro-7H-[1,4]oxazino[2,3,4-ij]chinolin-6-carboxamid,
ww) N- (4-Chlorbenzyl) -2- (1H-imidazol-1-ylmethyl)-9-(morpholin-4-ylmethyl)-7-thioxo-2,3-dihydro-7H-1,4]oxazino[2,3,4-ij]chinolin-6-carboxamid,
xx) N-(4-Chlorbenzyl)-2-{[(4-chlorbenzyl)amino]methyl}-9-(morpholin-4-ylmethyl)-7-thioxo-2,3-dihydro-7H-[1,4]oxazino[2,3,4-ij]chinolin-6-carboxamid,
yy) N-(4-Chlorbenzyl)-3-(hydroxymethyl)-9-(morpholin-4-ylmethyl)-7-thioxo-2,3-dihydro-7H-[1,4]oxazino[2,3,4-ij]chinolin-6-carboxamid,
zz) N-(4-Chlorbenzyl)-2-(4-hydroxyphenyl)-9-(morpholin-4-ylmethyl)-7-thioxo-2,3-dihydro-7H-[1,4]oxazino[2,3,4-ij]chinolin-6-carboxamid,
aaa) N-(4-Chlorbenzyl)-2-{3-[(methoxymethoxy)methyl]-phenyl}-9-(morpholin-4-ylmethyl)-7-thioxo-2,3-dihydro-7H-[1,4]oxazino[2,3,4-ij]chinolin-6-carboxamid,
bbb) N-(4-Chlorbenzyl)-2-{2-[(methoxymethoxy)methyl]-phenyl}-9-(morpholin-4-ylmethyl)-7-thioxo-2,3-dihydro-7H-[1,4]oxazino[2,3,4-ij]chinolin-6-carboxamid,
ccc) N-(4-Chlorbenzyl)-2-(2-hydroxyphenyl)-9-(morpholin-4-ylmethyl)-7-thioxo-2,3-dihydro-7H-[1,4]oxazino[2,3, 4-ij]chinolin-6-carboxamid,
ddd) N-((4-Chlorphenyl)methyl]-2,3,5,6-tetrahydro-9'-(4-morpholinylmethyl)-7'-thioxospiro[4H-pyran-4,2'-(3'H)-[7H]pyrido[1,2,3-de][1,4]benzoxazin]-6'carboxamid,
eee) 1,1-Dimethylethyl-6-[[[(4-chlorphenyl)methyl]amino]-carbonyl]-9'-(4-morpholinylmethyl)-7'-thioxospiro[piperidin-4,2'(3'H)-[7H]pyrido[1,2,3-de][1,4]-benzoxazin]-1-carboxylat,
fff) N-[(4-Chlorphenyl)methyl]-9'-(4-morpholinylmethyl)-7'-thioxospiro(piperidin-4,2'(3'H)-[7H]pyrido[1,2,3-de][1,4]benzoxazin]-6'-carboxamid,
ggg) N-(4-Chlorbenzyl)-2,2-bis(hydroxymethyl)-9-(morpholin-4-ylmethyl)-7-thioxo-2,3-dihydro-7H-[1,4]oxazino[2,3,4-ij]chinolin-6-carboxamid,
hhh) N-[(4-Chlorphenyl)methyl]-2',3',5',6'-tetrahydro-9-(4-morpholinylmethyl)-7-thioxospiro[7H-pyrido[1,2,3-de]-1,4-benzoxazin-2(3H),4'-[4H]thiopyran]-6-carboxamid,
iii) N-(4-Chlorbenzyl)-9-(morpholin-4-ylmethyl)-7-thioxo-3-phenyl-2,3-dihydro-7H-[1,4]oxazino[2,3,4-ij]chinolin-6-carboxamid,
jjj) N-(4-Chlorbenzyl)-3,3-bis (hydroxymethyl)-9-(3-hydroxy-1-propinyl)-7-thioxo-2,3-dihydro-7H-[1,4]oxazino[2,3,4-ij]chinolin-6-carboxamid,
kkk) N-(4-Chlorbenzyl)-3,3-bis(hydroxymethyl)-9-(3-hydroxypropyl)-7-thioxo-2,3-dihydro-7H-[1,4]oxazino[2,3,4-ij]chinolin-6-carboxamid,
lll) N-(4-Chlorbenzyl)-2-[2-(methoxymethoxy)phenyl]-9-(morpholin-4-ylmethyl)-7-thioxo-2,3-dihydro-7H-[1,4]oxazino[2,3,4-ij]chinolin-6-carboxamid,
mmm) N-(4-Chlorbenzyl)-2-{4-[(methoxymethoxy)methyl]-phenyl}-9-(morpholin-4-ylmethyl)-7-thioxo-2,3-dihydro-7H-[1,4]oxazino[2,3,4-ij]chinolin-6-carboxamid,
nnn) 2-[2,3-Bis(methoxymethoxy)phenyl]-N-(4-chlorbenzyl)-9-(morpholin-4-ylmethyl)-7-thioxo-2,3-dihydro-7H-[1,4]oxazino[2,3,4-ij]chinolin-6-carboxamid,
ooo) N-[(4-Chlorphenyl)methyl]-1-methyl-9'-(4-morpholinylmethyl)-7'-thioxospiro[piperidin-4,2' (3'H)-[7H]pyrido[1,2,3-de) [1,4]benzoxazin]-6'carboxamid, oder ein pharmazeutisch akzeptables Salz.

**70.** Verbindung nach Anspruch 1, nämlich
a) N-(4-Chlorbenzyl)-2-(hydroxymethyl)-9-(morpholin-4-ylmethyl)-7-oxo-2,3-dihydro-7H-[1,4]oxazino[2,3,4-ij]chinolin-6-carboxamid,
b) N-(4-Chlorbenzyl)-2-(R oder S)-(hydroxymethyl)-9-(morpholin-4-ylmethyl)-7-oxo-2,3-dihydro-7H-[1,4]oxazino[2,3,4-ij]chinolin-6-carboxamid,
c) N-(4-Chlorbenzyl)-9-(morpholin-4-ylmethyl)-7-oxo-2-pyridin-3-yl-2,3-dihydro-7H-[1,4]oxazino[2,3,4-ij]chinolin-6-carboxamid,
d) N-(4-Chlorbenzyl)-9-(morpholin-4-ylmethyl)-7-oxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-oxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-oxo-2-(R oder S)-pyridin-3-yl-2,3-dihydro-7H-[1,4]oxazino[2,3,4-ij]chinolin-6-carboxamid,
g) N-(4-Chlorbenzyl)-2,9-bis(morpholin-4-ylmethyl)-7-oxo-2,3-dihydro-7H-[1,4]oxazino[2,3,4-ij]chinolin-6-carboxamid,
h) 2-[(tert-Butylsulfanyl)methyl]-N-(4-chlorbenzyl)-9-(morpholin-4-ylmethyl)-7-oxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-oxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4ij]chinolin-6-carboxamid,
k) N-(4-Chlorbenzyl)-9-(morpholin-4-ylmethyl)-7-oxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]methylacetat,
m) N-(4-Chlorbenzyl)-9-(morpholin-4-ylmethyl)-7-oxo-2-(R oder S)-{[(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl) -7-oxo-2,3-dihydro-7H-[1,4]oxazino[2,3,4-ij]chinolin-6-carboxamid;
o) N- (4-Chlorbenzyl)-9- (morpholin-4-ylmethyl)-7-oxo-2-(R oder S)-pyridin-2-yl-2,3-dihydro-7H-[1,4]oxazino[2,3,4-ij]chinolin-6-carboxamid,
p) N-(4-Chlorbenzyl)-2-[3-(hydroxymethyl)phenyl]-9-(morpholin-4-ylmethyl)-7-oxo-2,3-dihydro-7H-[1,4]oxazino[2,3,4-ij]chinolin-6-carboxamid,
q) N-(4-Chlorbenzyl)-2-[2-(hydroxymethyl)phenyl]-9-(morpholin-4-ylmethyl)-7-oxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-oxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-oxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-oxo-2,3-dihydro-7H-[1,4]oxazino[2,3,4-ij]chinolin-6-carboxamid,
u) N-(4-Chlorbenzyl)-2-(3-furyl)-9-(morpholin-4-ylmethyl)-7-oxo-2,3-dihydro-7H-[1,4]oxazino[2,3,4-ij]chinolin-6-carboxamid,
v) N- (4-Chlorbenzyl) -9- (morpholin-4-ylmethyl)-7-oxo-2-thien-2-yl-2,3-dihydro-7H-[1,4]oxazino[2,3,4-ij]chinolin-6-carboxamid,
w) N- (4-Chlorbenzyl) -2- (3, 5-difluorphenyl) -9- (morpholin-4-ylmethyl)-7-oxo-2,3-dihydro-7H-[1,4]oxazino[2,3,4-ij]chinolin-6-carboxamid,
x) 2-(1,3-Benzodioxol-5-yl)-N-(4-chlorbenzyl)-9-(morpholin-4-ylmethyl)-7-oxo-2,3-dihydro-7H-[1,4]oxazino[2,3,4-ij]chinolin-6-carboxamid,
y) N-(4-Chlorbenzyl)-2-(2,3-dihydro-1,4-benzodioxin-6-yl)-9-(morpholin-4-ylmethyl)-7-oxo-2,3-dihydro-7H-[1,4]oxazino[2,3,4-ij]chinolin-6-carboxamid,
z) 2- (1,3-Benzodioxol-4-yl)-N-(4-chlorbenzyl)-9-(morpholin-4-ylmethyl)-7-oxo-2,3-dihydro-7H-[1,4]oxazino[2,3,4-ij]chinolin-6-carboxamid,
aa) 2-[3,5-Bis(methoxymethoxy)phenyl]-N-(4-chlorbenzyl)-9-(morpholin-4-ylmethyl)-7-oxo-2,3-dihydro-7H-[1,4]oxazino[2,3,4-ij]chinolin-6-carboxamid,
bb) N-(4-Chlorbenzyl)-9-(morpholin-4-ylmethyl)-7-oxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-oxo-2,3-dihydro-7H-[1,4]oxazino[2,3,4-ij]chinolin-6-carboxamid,
dd) N-[(4-Chlorphenyl)methyl]-9'-(4-morpholinylmethyl)-4,7'-dioxospiro[cyclohexan-1,2'(3'H)-[7H]pyrido[1,2,3-de][1,4]benzoxazin]-6'-carboxamid,
ee) N-[(4-Chlorphenyl)methyl]-4-hydroxy-9'-(4-morpholinylmethyl)-7'-oxospiro[cyclohexan-1,2'(3'H)-[7H]pyrido[1,2,3-de][1,4]benzoxazin]-6'-carboxamid,
ff) N-(4-Chlorbenzyl)-3,9-bis(morpholin-4-ylmethyl)-7-oxo-2,3-dihydro-7H-[1,4]oxazino(2,3,4-ij]chinolin-6-carboxamid,
gg) N-(4-Chlorbenzyl)-9-(morpholin-4-ylmethyl)-7-oxo-2-phenyl-2,3-dihydro-7H-[1,4)oxazino[2,3,4-ij]chinolin-6-carboxamid,
hh) N-(4-Chlorbenzyl)-2,2-difluor-9-(morpholin-4-ylmethyl)-7-oxo-2,3-dihydro-7H-[1,4]oxazino[2,3,4-ij)chinolin-6-carboxamid,
ii) N-(4-Chlorbenzyl)-2-[(methylsulfanyl)methyl]-9-(morpholin-4-ylmethyl)-7-oxo-2,3-dihydro-7H-[1,4]oxazino[2,3,4-ij]chinolin-6-carboxamid,
jj) N-(4-Chlorbenzyl)-2-[(dimethylamino)methyl]-9-(morpholin-4-ylmethyl)-7-oxo-2,3-dihydro-7H-[1,4]oxazino[2,3,4-ij]chinolin-6-carboxamid,
kk) N-(4-Chlorbenzyl)-2-[(4-methyl-1-piperazinyl)methyl]-9-(morpholin-4-ylmethyl)-7-oxo-2,3-dihydro-7H-[1,4]oxazino[2,3,4-ij]chinolin-6-carboxamid,
ll) Methyl-({[6-{[(4-chlorbenzyl)amino)carbonyl}-9-(morpholin-4-ylmethyl)-7-oxo-2,3-dihydro-7H-[1,4]oxazino[2,3,4-ij]chinolin-2-yl]methyl}thio)-acetat,
mm) N- (4-Chlorbenzyl)-9- (morpholin-4-ylmethyl) -7-oxo-2-(1-pyrrolidinylmethyl)-2,3-dihydro-7H-[1,4]oxazino[2,3,4-ij]chinolin-6-carboxamid,
nn) N-(4-Chlorbenzyl)-2-{[(2,3-dihydroxypropyl)sulfanyl]-methyl}-9-(morpholin-4-ylmethyl)-7-oxo-2,3-dihydro-7H- [1,4]oxazino [2, 3, 4-ij]chinolin-6-carboxamid,
oo) N-(4-Chlorbenzyl)-2-{[(2,3-dihydroxypropyl)amino]-methyl}-9-(morpholin-4ylmethyl)-7-oxo-2,3-dihydro-7H-[1,4]oxazino[2, 3, 4-ij]chinolin-6-carboxamid,
pp) N-(4-Chlorbenzyl)-2-{[(2-hydroxyethyl)amino]methyl}-9-(morpholin-4-ylmethyl)-7-oxo-2,3-dihydro-7H-[1,4]oxazino [2, 3, 4-ij]chinolin-6-carboxamid,
qq) N-(4-Chlorbenzyl)-9-(morpholin-4-ylmethyl)-7-oxo-2-(1-piperidinylmethyl)-2,3-dihydro-7H-[1,4]oxazino[2,3,4-ij]chinolin-6-carboxamid,
rr) 2-{[Bis(2-hydroxyethyl)amino]methyl}-N-(4-chlorbenzyl)-9-(morpholin-4-ylmethyl)-7-oxo-2,3-dihydro-7H-[1,4]oxazino[2,3,4-ij]chinolin-6-carboxamid,
ss) N-(4-Chlorbenzyl)-9-(morpholin-4-ylmethyl)-7-oxo-2-{[(2-pyridinylmethyl)amino]methyl}-2,3-dihydro-7H-[1,4]oxazino[2,3,4-ij]chinolin-6-carboxamid,
tt) 2-[(8-{[6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]methoxy}-8-oxooctanoyl)(methyl)amino]ethansulfonsäurenatriumsalz,
uu) [6-{[(4-Chlorbenzyl)amino]carbonyl}-9-(morpholin-4-ylmethyl)-7-oxo-2,3-dihydro-7H-[1,4]oxazino[2,3,4-ij]chinolin-2-yl]dimethylphosphat,
vv) N-(4-Chlorbenzyl)-9-(morpholin-4-ylmethyl)-7-oxo-2-{[(4-pyridinylmethyl)amino]methyl}-2,3-dihydro-7H-[1,4]oxazino[2,3,4-ij]chinolin-6-carboxamid,
ww) N-(4-Chlorbenzyl)-2-(1H-imidazol-1-ylmethyl)-9-(morpholin-4-ylmethyl)-7-oxo-2,3-dihydro-7H-[1,4]oxazino(2,3,4-ij]chinolin-6-carboxamid,
xx) N-(4-Chlorbenzyl)-2-{[(4-chlorbenzyl)amino]methyl}-9-(morpholin-4-ylmethyl)-7-oxo-2,3-dihydro-7H-[1,4]oxazino[2,3,4-ij]chinolin-6-carboxamid,
yy) N-(4-Chlorbenzyl)-3-(hydroxymethyl)-9-(morpholin-4-ylmethyl)-7-oxo-2,3-dihydro-7H-[1,4]oxazino[2,3,4-ij]chinolin-6-carboxamid,
zz) N-(4-Chlorbenzyl)-2-(4-hydroxyphenyl)-9-(morpholin-4-ylmethyl)-7-oxo-2,3-dihydro-7H-[1,4]oxazino[2,3,4-ij]chinolin-6-carboxamid,
aaa) N-(4-Chlorbenzyl)-2-{3-[(methoxymethoxy)methyl]-phenyl}-9-(morpholin-4-ylmethyl)-7-oxo-2,3-dihydro-7H-[1,4 ] oxazino [2, 3, 4-ij]chinolin-6-carboxamid,
bbb) N-(4-Chlorbenzyl)-2-{2-[(methoxymethoxy)methyl]-phenyl}-9-(morpholin-4-ylmethyl)-7-oxo-2,3-dihydro-7H-[1,4]oxazino[2, 3, 4-ij]chinolin-6-carboxamid,
ccc) N-(4-Chlorbenzyl)-2-(2-hydroxyphenyl)-9-(morpholin-4-ylmethyl)-7-oxo-2,3-dihydro-7H-[1,4]oxazino[2,3,4-ij]chinolin-6-carboxamid,
ddd) N-[(4-Chlorphenyl)methyl]-2,3,5,6-tetrahydro-9'-(4-morpholinylmethyl)-7'-oxospiro[4H-pyran-4,2'(3'H)-[7H]pyrido[1,2,3-de][1,4]benzoxazin]-6'-carboxamid,
eee) 1,1-Dimethylethyl-6-[[[(-4-chlorphenyl)methyl]-amino]-carbonyl]-9'-(4-morpholinylmethyl)-7'oxospiro-(piperidin-4,2'(3'H)-[7H]pyrido[1,2,3-de][1,4]benzoxazin]-1-carboxylat,
fff) N-[(4-Chlorphenyl)methyl]-9'-(4-morpholinylmethyl)-7'-oxospiro[piperidin-4,2'(3'H)-[7H]pyrido[1,2,3-de]-[1,4]benzoxazin]-6'-carboxamid,
ggg) N-(4-Chlorbenzyl)-2,2-bis(hydroxymethyl)-9-(morpholin-4-ylmethyl)-7-oxo-2,3-dihydro-7H-[1,4]oxazino[2,3,4-ij]chinolin-6-carboxamid,
hhh) N-[(4-Chlorphenyl)methyl]-2',3',5',6'-tetrahydro-9-(4-morpholinylmethyl)-7-oxospiro[7H-pyrido[1,2,3-de]-1,4-benzoxazin-2(3H),4'-[4H]thiopyran]-6-carboxamid oder ein pharmazeutisch akzeptables Salz.

**71.** Verbindung nach Anspruch 1, nämlich
a) N-(4-Chlorbenzyl)-2-(hydroxymethyl)-9-(morpholin-4-ylmethyl)-7-thioxo-2,3-dihydro-7H-[1,4]oxazino[2,3,4-ij]chinolin-6-carboxamid,
b) N-(4-Chlorbenzyl)-2-(R oder S)-(hydroxymethyl)-9-(morpholin-4-ylmethyl)-7-thioxo-2,3-dihydro-7H-[1,4]oxazino[2,3,4ij]chinolin-6-carboxamid,
c) N-(4-Chlorbenzyl)-9-(morpholin-4-ylmethyl)-7-thioxo-2-pyridin-3-yl-2,3-dihydro-7H-[1,4]oxazino[2,3,4-ij]chinolin-6-carboxamid,
d) N-(4-Chlorbenzyl)-9-(morpholin-4-ylmethyl)-7-thioxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-thioxo-2-pyridin-2-yl-2,3-dihydro-7H-[1,4]oxazino[2,3,4-ij]-chinolin-6-carboxamid,
f) N-(4-Chlorbenzyl)-9-(morpholin-4-ylmethyl)-7-thioxo-2-(R oder S)-pyridin-3-yl-2,3-dihydro-7H-[1,4]oxazino[2,3,4-ij]chinolin-carboxamid,
g) N-(4-Chlorbenzyl)-2,9-bis(morpholin-4-ylmethyl)-7-thioxo-2,3-dihydro-7H-[1,4]oxazino[2,3,4-ij]chinolin-6-carboxamid,
h) 2-[(tert-Butylsulfanyl)methyl]-N-(4-chlorbenzyl)-9-(morpholin-4-ylmethyl)-7-thioxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-thioxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij)chinolin-6-carboxamid,
k) N-(4-Chlorbenzyl)-9-(morpholin-4-ylmethyl)-7-thioxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methylacetat,
m) N-(4-Chlorbenzyl)-9-(morpholin-4-ylmethyl)-7-thioxo-2-(R oder S)-{[(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl)-7-thioxo-2,3-dihydro-7H-[1,4]oxazino[2,3,4-ij]chinolin-6-carboxamid,
o) N-(4-Chlorbenzyl)-9-(morpholin-4-ylmethyl)-7-thioxo-2-(R oder S)-pyridin-2-yl-2,3-dihydro-7H-[1,4]oxazino-2,3,4-ij]chinolin-6-carboxamid,
p) N-(4-Chlorbenzyl)-2-[3-(hydroxymethyl)phenyl]-9-(morpholin-4-ylmethyl)-7-thioxo-2,3-dihydro-7H-[1,4]oxazino[2,3,4-ij]chinolin-6-carboxamid,
q) N-(4-Chlorbenzyl)-2-[2-(hydroxymethyl)phenyl]-9-(morpholin-4-ylmethyl)-7-thioxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-thioxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-thioxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-thioxo-2,3-dihydro-7H-[1,4]oxazino[2,3,4-ij]chinolin-6-carboxamid,
u) N-(4-Chlorbenzyl)-2-(3-furyl)-9-(morpholin-4-ylmethyl)-7-thioxo-2,3-dihydro-7H-[1,4]oxazino[2,3,4-ij]chinolin-6-carboxamid,
v) N-(4-Chlorbenzyl)-9-(morpholin-4-ylmethyl)-7-thioxo-2-thien-2-yl-2,3-dihydro-7H-[1,4]oxazino[2,3,4-ij]-chinolin-6-carboxamid,
w) N-(4-Chlorbenzyl)-2-(3,5-difluorphenyl)-9-(morpholin-4-ylmethyl)-7-thioxo-2,3-dihydro-7H-[1,4]oxazino[2,3,4-ij]chinolin-6-carboxamid,
x) 2-(1,3-Benzodioxol-5-yl)-N-(4-chlorbenzyl)-9-(morpholin-4-ylmethyl)-7-thioxo-2,3-dihydro-7H-[1,4]oxazino[2,3,4-ij]chinolin-6-carboxamid,
y) N-(4-Chlorbenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]chinolin-6-carboxamid,
z) 2-(1,3-Benzodioxol-4-yl)-N-(4-chlorbenzyl)-9-(morpholin-4-ylmethyl)-7-thioxo-2,3-dihydro-7H-[1,4]oxazino[2,3,4-ij]chinolin-6-carboxamid,
aa) 2-[3,5-Bis(methoxymethoxy)phenyl]-N-(4-chlorbenzyl)-9-(morpholin-4-ylmethyl)-7-thioxo-2,3-dihydro-7H-[1,4]oxazino[2,3,4-ij]chinolin-6-carboxamid,
bb) N-(4-Chlorbenzyl)-9-(morpholin-4-ylmethyl)-7-thioxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-thioxo-2,3-dihydro-7H-[1,4]oxazino[2,3,9-ij]chinolin-6-carboxamid,
dd) N-[(4-Chlorphenyl)methyl]-9'-(4-morpholinylmethyl)-4-oxo-7'-thioxospiro[cyclohexan-1,2'(3'H)-[7H]-pyrido[1,2,3-de][1,4]benzoxazin]-6'-carboxamid,
ee) N-[(4-Chlorphenyl)methyl]-4-hydroxy-9'-(4-morpholinylmethyl)-7'-thioxospiro[cyclohexan-1,2'(3'H)-[7H]pyrido[1,2,3-de][1,4]benzoxazin]-6'-carboxamid,
ff) N-(4-Chlorbenzyl)-3,9-bis(morpholin-4-ylmethyl)-7-thioxo-2,3-dihydro-7H-[1,4]oxazino[2,3,4-ij]-chinolin-6-carboxamid,
gg) N-(4-Chlorbenzyl)-9-(morpholin-4-ylmethyl)-7-thioxo-2-phenyl-2,3-dihydro-7H-[1,4]oxazino[2,3,4-ij]-chinolin-6-carboxamid,
hh) N-(4-Chlorbenzyl)-2,2-difluor-9-(morpholin-4-ylmethyl)-7-thioxo-2,3-dihydro-7H-[1,4]oxazino[2,3,4-ij]chinolin-6-carboxamid,
ii) N-(4-Chlorbenzyl)-2-[(methylsulfanyl)methyl]-9-(morpholin-4-ylmethyl)-7-thioxo-2,3-dihydro-7H-[1,4]oxazino[2,3,4-ij]chinolin-6-carboxamid,
jj) N-(4-Chlorbenzyl)-2-[(dimethylamino)methyl]-9-(morpholin-4-ylmethyl)-7-thioxo-2,3-dihydro-7H-[1,4]oxazino[2,3,4-ij]chinolin-6-carboxamid,
kk) N-(4-Chlorbenzyl)-2-[(4-methyl-1-piperazinyl)-methyl]-9-(morpholin-4-ylmethyl)-7-thioxo-2,3-dihydro-7H-[1,4]oxazino[2,3,4-ij]chinolin-6-carboxamid,
ll) Methyl-({[6-{[(4-Chlorbenzyl)amino]carbonyl}-9-(morpholin-4-ylmethyl)-7-thioxo-2,3-dihydro-7H-[1,4]oxazino(2,3,4-ij]chinolin-2-yl]methyl}thio)-acetat,
mm) N-(4-Chlorbenzyl)-9-(morpholin-4-ylmethyl)-7-thioxo-2-(1-pyrrolidinylmethyl)-2,3-dihydro-7H-[1,4]oxazino[2,3,4-ij]chinolin-6-carboxamid,
nn) N-(4-Chlorbenzyl)-2-{[(2,3-dihydroxypropyl)-sulfanyl]methyl}-9-(morpholin-4-ylmethyl)-7-thioxo-2, 3-dihydro-7H-[1,4]oxazino[2,3, 4-ij]chinolin-6-carboxamid,
oo) N-(4-Chlorbenzyl)-2-{[(2,3-dihydroxypropyl)-amino]methyl}-9-(morpholin-4-ylmethyl)-7-thioxo-2,3-dihydro-7H-[1,4]oxazino[2,3,4-ij]chinolin-6-carboxamid,
pp) N-(4-Chlorbenzyl)-2-{[(2-hydroxyethyl)amino]methyl}-9-(morpholin-4-ylmethyl)-7-thioxo-2,3-dihydro-7H-[1,4]oxazino[2,3,4-ij]chinolin-6-carboxamid,
qq) N-(4-Chlorbenzyl)-9-(morpholin-4-ylmethyl)-7-thioxo-2-(1-piperidinylmethyl)-2,3-dihydro-7H-[1,4]oxazino[2,3,4-ij]chinolin-6-carboxamid,
rr) 2-{[Bis(2-hydroxyethyl)amino]methyl}-N-(4-chlorbenzyl)-9-(morpholin-4-ylmethyl)-7-thioxo-2,3-dihydro-7H-[1,4]oxazino[2,3,4-ij]chinolin-6-carboxamid,
ss) N-(4-Chlorbenzyl)-9-(morpholin-4-ylmethyl)-7-thioxo-2-{[(2-pyridinylmethyl)amino]methyl}-2,3-dihydro-7H-[1,4]oxazino[2,3,4-ij]chinolin-6-carboxamid,
tt) 2- [(8-{[6-{[(4-Chlorbenzyl)amino]carbonyl}-9-(morpholin-4-ylmethyl)-7-thioxo-2,3-dihydro-7H-[1,4]oxazino [2,3,4-ij]chinolin-2-yl]methoxy}-8-oxooctanoyl)(methyl)amino]ethersulfonsäurenatriumsalz,
uu) [6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methyldimethylphosphat,
vv) N-(4-Chlorbenzyl)-9-(morpholin-4-ylmethyl)-7-thioxo-2-{[(4-pyridinylmethyl)amino]methyl}-2,3-dihydro-7H-[1,4]oxazino[2,3,4-ij]chinolin-6-carboxamid,
ww) N-(4-Chlorbenzyl)-2-(1H-imidazol-1-ylmethyl)-9-(morpholin-4-ylmethyl)-7-thioxo-2,3-dihydro-7H-[1,4]oxazino[2,3,4-ij]chinolin-6-carboxamid,
xx) N-(4-Chlorbenzyl)-2-{[(4-chlorbenzyl)amino]methyl}-9-(morpholin-4-ylmethyl)-7-thioxo-2,3-dihydro-7H-[1,4]oxazino [2, 3, 4-ij]chinolin-6-carboxamid,
yy) N-(4-Chlorbenzyl)-3-(hydroxymethyl)-9-(morpholin-4-ylmethyl)-7-thioxo-2,3-dihydro-7H-[1,4]oxazino[2,3,4-ij]chinolin-6-carboxamid,
z z ) N-(4-Chlorbenzyl)-2-(4-hydroxyphenyl)-9-(morpholin-4-ylmethyl)-7-thioxo-2,3-dihydro-7H-[1,4]oxazino[2,3,4-ij]chinolin-6-carboxamid,
aaa) N-(4-Chlorbenzyl)-2-{3-[(methoxymethoxy)methyl]-phenyl}-9-(morpholin-4-ylmethyl)-7-thioxo-2,3-dihydro-7H-[1,4]oxazino[2,3,4-ij]chinolin-6-carboxamid,
bbb) N-(4-Chlorbenzyl)-2-{2-[(methoxymethoxy)methyl]-phenyl}-9-(morpholin-4-ylmethyl)-7-thioxo-2,3-dihydro-7H-[1,4]oxazino [2, 3,4-ij]chinolin-6-carboxamid,
ccc) N- (4-Chlorbenzyl)-2-(2-hydroxyphenyl)-9- (morpholin-4-ylmethyl)-7-thioxo-2,3-dihydro-7H-[1,4]oxazino[2, 3, 4-ij]chinolin-6-carboxamid,
ddd) N-[(4-Chlorphenyl)methyl]-2,3,5,6-tetrahydro-9'-(4-morpholinylmethyl)-7'-thioxospiro[4H-pyran-4,2'(3'H)-[7H]pyrido[1,2,3-de][1,4]benzoxazin]-6'-carboxamid,
eee) 1,1-Dimethylethyl-6-[[[(4-chlorphenyl)methyl]-amino]carbonyl]-9'-(4-morpholinylmethyl)-7'thioxospiro[piperidin-4,2'(3'H)-[7H]pyrido[1,2,3-de] [1,4]benzoxazin]-1-carboxylat,
fff) N-[(4-Chlorphenyl)methyl]-9'-(4-morpholinylmethyl)-7'-thioxospiro[piperidin-4,2'(3'H)-[7H]pyrido[1,2,3-de][1,4]benzoxazin]-6'-carboxamid,
ggg) N-(4-Chlorbenzyl)-2,2-bis[(hydroxymethoxy)methyl]-9-(morpholin-4-ylmethyl)-7-thioxo-2,3-dihydro-7H-[1,4]oxazino[2,3,4-ij]chinolin-6-carboxamid,
hhh) N- [(4-Chlorphenyl)methyl]-2',3',5',6'-tetrahydro-9-(4-morpholinylmethyl)-7-thioxospiro[7H-pyrido[1,2,3-de]-1,4-benzoxazin-2(3H),4'-[4H]thiopyran]-6-carboxamid oder ein pharmazeutisch akzeptables Salz.

**72.** Verbindung nach Anspruch 1, nämlich
a) N-(4-Chlorbenzyl)-2-(hydroxymethyl)-9-(morpholin-4-ylmethyl)-7-oxo-2,3-dihydro-7H-[1,4]oxazino[2,3,4-ij]chinolin-6-carboxamid,
b) N-(4-Chlorbenzyl)-2-(R oder S)-(hydroxymethyl)-9-(morpholin-4-ylmethyl)-7-oxo-2,3-dihydro-7H-[1,4]oxazino[2,3,4-ij]chinolin-6-carboxamid,
c) N-(4-Chlorbenzyl)-9-(morpholin-4-ylmethyl)-7-oxo-2-pyridin-3-yl-2,3-dihydro-7H-(1,4]oxazino[2,3,4-ij]chinolin-6-carboxamid,
d) N-(4-Chlorbenzyl)-9-(morpholin-4-ylmethyl)-7-oxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-oxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-oxo-2-(R oder S)-pyridin-3-yl-2,3-dihydro-7H-[1,4]oxazino[2,3,4-ij]chinolin-6-carboxamid,
g) N-(4-Chlorbenzyl)-2,9-bis(morpholin-4-ylmethyl)-7-oxo-2,3-dihydro-7H-[1,4]oxazino[2,3,4-ij]chinolin-6-carboxamid,
h) 2-[(tert-Butylsulfanyl)methyl]-N-(4-chlorbenzyl)-9-(morpholin-4-ylmethyl)-7-oxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-oxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-oxo-2,3-dihydro-7H-[1,4]oxazino[2,3,4-ij]chinolin-6-carboxamid,
k) N-(4-Chlorbenzyl)-9-(morpholin-4-ylmethyl)-7-oxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl) -7-oxo-2,3-dihydro-7H-[1,4]oxazino[2,3, 4-ij]quinolin-2-yl]methylacetat,
m) N-(4-Chlorbenzyl)-9-(morpholin-4-ylmethyl)-7-oxo-2-(R oder S)-{[(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl)-7-oxo-2,3-dihydro-7H-[1,4]oxazino[2,3,4-ij]chinolin-6-carboxamid,
o) N-(4-Chlorbenzyl)-9-(morpholin-4-ylmethyl)-7-oxo-2-(R oder S)-pyridin-2-yl-2,3-dihydro-7H-[1,4]oxazino[2,3,4-ij]chinolin-6-carboxamid,
p) N-(4-Chlorbenzyl)-2-[3-(hydroxymethyl)phenyl]-9-(morpholin-4-ylmethyl)-7-oxo-2,3-dihydro-7H-[1,4]oxazino[2,3,4-ij]chinolin-6-carboxamid,
q) N-(4-Chlorbenzyl)-2-[2-(hydroxymethyl)phenyl]-9-(morpholin-4-ylmethyl)-7-oxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-oxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-oxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-oxo-2,3-dihydro-7H-[1,4]oxazino(2,3,4-ij]chinolin-6-carboxamid,
u) N-(4-Chlorbenzyl)-2-(3-furyl)-9-(morpholin-4-ylmethyl)-7-oxo-2,3-dihydro-7H-[1,4]oxazino[2,3,4-ij]chinolin-6-carboxamid,
v) N-(4-Chlorbenzyl)-9-(morpholin-4-ylmethyl)-7-oxo-2-thien-2-yl-2,3-dihydro-7H-[1,4]oxazino[2,3,4-ij]chinolin-6-carboxamid,
w) N-(4-Chlorbenzyl)-2-(3,5-difluorphenyl)-9-(morpholin-4-ylmethyl)-7-oxo-2,3-dihydro-7H-[1,4]oxazino[2,3,4-ij)chinolin-6-carboxamid,
x) 2-(1,3-Benzodioxol-5-yl)-N-(4-chlorbenzyl)-9-(morpholin-4-ylmethyl)-7-oxo-2,3-dihydro-7H-[1,4]oxazino[2,3,4-ij]chinolin-6-carboxamid,
y) N- (4-Chlorbenzyl) -2- (2, 3-dihydro-1,4-benzodioxin-6-yl)-9-(morpholin-4-ylmethyl)-7-oxo-2,3-dihydro-7H-[1,4]oxazino[2,3,4-ij]chinolin-6-carboxamid,
z) 2-(1,3-Benzodioxol-4-yl)-N-(4-chlorbenzyl)-9-(morpholin-4-ylmethyl)-7-oxo-2,3-dihydro-7H-[1,4]oxazino[2,3,4-ij]chinolin-6-carboxamid,
aa) 2-[3,5-Bis(methoxymethoxy)phenyl]-N-(4-chlorbenzyl)-9-(morpholin-4-ylmethyl)-7-oxo-2,3-dihydro-7H-[1,4]oxazino[2,3,4-ij)chinolin-6-carboxamid,
bb) N-(4-Chlorbenzyl)-9-(morpholin-4-ylmethyl)-7-oxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-oxo-2,3-dihydro-7H-[1,4]-oxazino[2,3,4-ij]chinolin-6-carboxamid,
dd) N- [(4-Chlorphenyl)methyl]-9'-(4-morpholinylmethyl)-4,7'-dioxospiro[cyclohexan-1,2'(3'H)-[7H]pyrido[1,2,3-de)[1,4]benzoxazin]-6'-carboxamid,
ee) N-[(4-Chlorphenyl)methyl]-4-hydroxy-9'-(4-morpholinylmethyl)-7'-oxospiro[cyclohexan-1,2'(3'H)-[7H]pyrido[1,2,3-de][1,4]benzoxazin]-6'-carboxamid oder ein pharmazeutisch akzeptables Salz.

**73.** Verbindung nach Anspruch 1, nämlich
a) N- (4-Chlorbenzyl) -2- (hydroxymethyl)-9-(morpholin-4-ylmethyl)-7-thioxo-2,3-dihydro-7H-[1,4]oxazino[2, 3, 4-ij]chinolin-6-carboxamid,
b) N-(4-Chlorbenzyl)-2-(R oder S)-(hydroxymethyl)-9-(morpholin-4-ylmethyl)-7-thioxo-2,3-dihydro-7H-[1,4]oxazino[2,3,4-ij]chinolin-6-carboxamid,
c) N-(4-Chlorbenzyl)-9-(morpholin-4-ylmethyl)-7-thioxo-2-pyridin-3-yl-2,3-dihydro-7H-[1,4]oxazino(2,3,4-ij]chinolin-6-carboxamid,
d) N-(4-Chlorbenzyl)-9-(morpholin-4-ylmethyl)-7-thioxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-thioxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-thioxo-2-(R oder S)-pyridin-3-yl-2,3-dihydro-7H-[1,4]oxazino[2,3,4-ij]chinolin-6-carboxamid,
g) N-(4-Chlorbenzyl)-2,9-bis(morpholin-4-ylmethyl)-7-thioxo-2,3-dihydro-7H-[1,4]oxazino[2,3,4-ij]chinolin-6-carboxamid,
h) 2-[(tert-Butylsulfanyl)methyl]-N-(4-chlorbenzyl)-9-(morpholin-4-ylmethyl)-7-thioxo-2,3-dihydro-7H-[1,4]oxazino[2,3,4-ij]chinolin-6-carboxamid,
i) N-(4-Chlorbenzyl)-2-{[(2-hydroxyethyl)sulfanyl]-methyl}-9-(morpholin-4-ylmethyl)-7-thioxo-2,3-dihydro-7H-[1,4]oxazino[2,3,4-ij]chinolin-6-carboxamid,
j) N-(4-Chlorbenzyl)-2-{[(1-methyl-1H-imidazol-2-yl)sulfanyl]methyl}-9-(morpholin-4-ylmethyl)-7-thioxo-2,3-dihydro-7H-[1,4]oxazino[2,3,4-ij]chinolin-6-carboxamid,
k) N-(4-Chlorbenzyl)-9-(morpholin-4-ylmethyl)-7-thioxo-2-{[(3-pyridinylmethyl)amino]methyl}-2,3-dihydro-7H-[1,4]oxazino[2,3,4-ij]chinolin-6-carboxamid,
l) [6-{[(4-Chlorbenzyl)amino]carbonyl}-9-(morpholin-4-ylmethyl)-7-thioxo-2,3-dihydro-7H-[1,4]oxazino[2,3,4-ij]chinolin-2-yl]methylacetat,
m) N-(4-Chlorbenzyl)-9-(morpholin-4-ylmethyl)-7-thioxo-2-(R oder S)-{[(3-pyridinylmethyl)amino]methyl}-2,3-dihydro-7H-[1,4]oxazino[2,3,4-ij]chinolin-6-carboxamid,
n) N-(4-Chlorbenzyl)-2-(3-hydroxyphenyl)-9-(morpholin-4-ylmethyl)-7-thioxo-2,3-dihydro-7H-[1,4]oxazino[2,3,4-ij]chinolin-6-carboxamid,
o) N-(4-Chlorbenzyl)-9-(morpholin-4-ylmethyl)-7-thioxo-2-(R oder S)-pyridin-2-yl-2,3-dihydro-7H-[1,4]oxazino[2,3,4-ij]chinolin-6-carboxamid,
p) N-(4-Chlorbenzyl)-2-[3-(hydroxymethyl)phenyl]-9-(morpholin-4-ylmethyl)-7-thioxo-2,3-dihydro-7H-[1,4]oxazino[2,3,4-ij]chinolin-6-carboxamid,
q) N-(4-Chlorbenzyl)-2-[2-(hydroxymethyl)phenyl)-9-(morpholin-4-ylmethyl)-7-thioxo-2,3-dihydro-7H-[1,4]oxazino[2,3,4-ij]chinolin-6-carboxamid,
r) N-(4-Chlorbenzyl)-2-(1-methyl-1H-imidazol-2-yl)-9-(morpholin-4-ylmethyl)-7-thioxo-2,3-dihydro-7H-[1,4]oxazino[2,3,4-ij]chinolin-6-carboxamid,
s) N-(4-Chlorbenzyl)-2-(2-furyl)-9-(morpholin-4-ylmethyl)-7-thioxo-2,3-dihydro-7H-[1,4]oxazino[2,3,4-ij]chinolin-6-carboxamid,
t) N-(4-Chlorbenzyl)-2-(3-cyanophenyl)-9-(morpholin-4-ylmethyl)-7-thioxo-2,3-dihydro-7H-[1,4]oxazino[2,3,4-ij ]chinolin-6-carboxamid,
u) N-(4-Chlorbenzyl)-2-(3-furyl)-9-(morpholin-4-ylmethyl)-7-thioxo-2,3-dihydro-7H-[1,4]oxazino[2,3,4-ij]chinolin-6-carboxamid,
v) N-(4-Chlorbenzyl)-9-(morpholin-4-ylmethyl)-7-thioxo-2-thien-2-yl-2, 3-dihydro-7H-[1,4]oxazino[2,3,4-ij]chinolin-6-carboxamid,
w) N-(4-Chlorbenzyl)-2-(3,5-difluorphenyl)-9-(morpholin-4-ylmethyl)-7-thioxo-2,3-dihydro-7H-[1,4]oxazino[2,3,4-ij]chinolin-6-carboxamid,
x) 2-(1,3-Benzodioxol-5-yl)-N-(4-chlorbenzyl)-9-(morpholin-4-ylmethyl)-7-thioxo-2,3-dihydro-7H-[1,4]oxazino[2,3,4-ij]chinolin-6-carboxamid,
y) N-(4-Chlorbenzyl)-2-(2,3-dihydro-1,4-benzodioxin-6-yl)-9-(morpholin-4-ylmethyl)-7-thioxo-2,3-dihydro-7H-[1,4]oxazino[2,3,4-ij]chinolin-6-carboxamid,
z) 2-(1,3-Benzodioxol-4-yl)-N-(4-chlorbenzyl)-9-(morpholin-4-ylmethyl)-7-thioxo-2,3-dihydro-7H-[1,4] oxazino[2,3,4-ij]chinolin-6-carboxamid,
aa) 2- [ 3, 5-Bis(methoxymethoxy)phenyl]-N-(4-chlorbenzyl)-9-(morpholin-4-ylmethyl)-7-thioxo-2,3-dihydro-7H-[1,4]oxazino[2,3,4-ij]chinolin-6-carboxamid,
bb) N-(4-Chlorbenzyl)-9-(morpholin-4-ylmethyl)-7-thioxo-2-thien-3-yl-2,3-dihydro-7H-[1,4]oxazino[2,3,4-ij]chinolin-6-carboxamid,
cc) N-(4-Chlorbenzyl)-2,2-bis[(methoxymethoxy)methyl]-9-(morpholin-4-ylmethyl)-7-thioxo-2,3-dihydro-7H-[1,4]oxazino[2,3,4-ij]chinolin-6-carboxamid,
dd) N-[(4-Chlorphenyl)methyl]-9'-(4-morpholinylmethyl)-4-oxo-7'-thioxospiro[cyclohexan-1,2'(3'H)-[7H]pyrido[1,2,3-de][1,4]benzoxazin]-6'-carboxamid,
ee) N-[(4-Chlorphenyl)methyl)-4-hydroxy-9'-(4-morpholinylmethyl)-7'-thioxospiro[cyclohexan-1,2'(3'H)-[7H]pyrido[1,2,3-de][1,4]benzoxazin]-6'-carboxamid,
oder ein pharmazeutisch akzeptables Salz.

**74.** Verbindung nach Anspruch 1, nämlich
a) N-(4-Chlorbenzyl)-2-(hydroxymethyl)-9-(morpholin-4-ylmethyl)-7-oxo-2,3-dihydro-7H-[1,4]oxazino[2,3,4-ij]chinolin-6-carboxamid,
b) N-(4-Chlorbenzyl)-2-(R oder S)-(hydroxymethyl)-9-(morpholin-4-ylmethyl)-7-oxo-2,3-dihydro-7H-[1,4]oxazino[2,3,4-ij]chinolin-6-carboxamid,
c) N-(4-Chlorbenzyl)-9-(morpholin-4-ylmethyl)-7-oxo-2-pyridin-3-yl-2,3-dihydro-7H-[1,4]oxazino[2,3,4-ij]chinolin-6-carboxamid,
d) N-(4-Chlorbenzyl)-9-(morpholin-4-ylmethyl)-7-oxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-oxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-oxo-2-(R oder S)-pyridin-3-yl-2,3-dihydro-7H-[1,4]oxazino[2,3,4-ij]chinolin-6-carboxamid,
g) N-(4-Chlorbenzyl)-9-(morpholin-4-ylmethyl)-7-oxo-2-(R oder S)-pyridin-2-yl-2,3-dihydro-7H-[1,4]-oxazino[2,3,4-ij]chinolin-6-carboxamid,
h) N-(4-Chlorbenzyl)-2-(1-methyl-1H-imidazol-2-yl)-9-(morpholin-4-ylmethyl)-7-oxo-2,3-dihydro-7H-[1,4]oxazino[2,3,4-ij]chinolin-6-carboxamid oder ein pharmazeutisch akzeptables Salz.

**75.** Verbindung nach Anspruch 1, nämlich
a) N-(4-Chlorbenzyl)-2-(hydroxymethyl)-9-(morpholin-4-ylmethyl)-7-thioxo-2,3-dihydro-7H-[1,4]oxazino[2,3,4-ij)chinolin-6-carboxamid,
b) N-(4-Chlorbenzyl)-2- (R oder S)-(hydroxymethyl)-9-(morpholin-4-ylmethyl)-7-thioxo-2,3-dihydro-7H-[1,4]oxazino[2,3,4-ij]chinolin-6-carboxamid,
c) N-(4-Chlorbenzyl)-9-(morpholin-4-ylmethyl)-7-thioxo-2-pyridin-3-yl-2,3-dihydro-7H-[1,4]oxazino[2,3,4-ij]chinolin-6-carboxamid,
d) N-(4-Chlorbenzyl)-9-(morpholin-4-ylmethyl)-7-thioxo-2-pyridin-4-yl-2,3-dihydro-7H-[1,4]oxazino[2,3,4-ij]chinolin-6-carboxamid,
e) N-(4-Chlorbenzyl)-9-(morpholin-4-ylmethyl)-7-thioxo-2-pyridin-2-yl-2,3-dihydro-7H-[1,4]oxazino[2,3,4-ij]chinolin-6-carboxamid,
f) N-(4-Chlorbenzyl)-9-(morpholin-4-ylmethyl)-7-thioxo-2-(R oder S-pyridin-3-yl-2,3-dihydro-7H-[1,4]oxazino[2, 3, 4-ij]chinolin-6-carboxamid,
g) N-(4-Chlorbenzyl)-9-(morpholin-4-ylmethyl)-7-thioxo-2-(R oder S)-pyridin-2-yl-2,3-dihydro-7H-[1,4]oxazino(2,3,4-ij]chinolin-6-carboxamid,
h) N-(4-Chlorbenzyl)-2-(1-methyl-1H-imidazol-2-yl)-9-(morpholin-4-ylmethyl)-7-thioxo-2,3-dihydro-7H-[1,4]oxazino[2,3,4-ij]chinolin-6-carboxamid oder ein pharmazeutisch akzeptables Salz.

**76.** Verbindung nach Anspruch 1, nämlich
a) N- (4-Chlorbenzyl) -2-(hydroxymethyl) -9- (morpholin-4-ylmethyl)-7-thioxo-2,3-dihydro-7H-[1,4]oxazino[2, 3, 4-ij ]chinolin-6-carboxthioamid,
b) N-(4-Chlorbenzyl)-2-(R oder S)-(hydroxymethyl)-9-(morpholin-4-ylmethyl)-7-thioxo-2,3-dihydro-7H-[1,4]oxazino[2, 3, 4-ij]chinolin-6-carboxthioamid,
c) N-(4-Chlorbenzyl)-9-(morpholin-4-ylmethyl)-7-thioxo-2-pyridin-3-yl-2,3-dihydro-7H-[1,4]oxazino[2,3,4-ij]chinolin-6-carboxthioamid,
d) N-(4-Chlorbenzyl)-9-(morpholin-4-ylmethyl)-7-thioxo-2-pyridin-4-yl-2,3-dihydro-7H-[1,4]oxazino(2,3,4-ij]chinolin-6-carboxthioamid,
e) N-(4-Chlorbenzyl)-9-(morpholin-4-ylmethyl)-7-thioxo-2-pyridin-2-yl-2,3-dihydro-7H-[1,4]oxazino[2,3,4-ij]chinolin-6-carboxthioamid,
f) N-(4-Chlorbenzyl)-9-(morpholin-4-ylmethyl)-7-thioxo-2-(R oder S)-pyridin-3-yl-2,3-dihydro-7H-[1,4]-oxazino[2,3,4-ij]chinolin-6-carboxthioamid,
e) N-(4-Chlorbenzyl)-9-(morpholin-4-ylmethyl)-7-thioxo-2-(R oder S)-pyridin-2-yl-2,3-dihydro-7H-[1,4]oxazino[2,3,4-ij]chinolin-6-carboxthioamid,
f) N-(4-Chlorbenzyl)-2-(1-methyl-1H-imidazol-2-yl)-9-(morpholin-4-ylmethyl)-7-thioxo-2,3-dihydro-7H-[1,4]oxazino[2,3,4-ij]chinolin-6-carboxthioamid oder ein pharmazeutisch akzeptables Salz.

**77.** Verbindung nach Anspruch 1, nämlich
a) N-(4-Chlorbenzyl)-2-(hydroxymethyl)-9-(morpholin-4-ylmethyl)-7-oxo-2,3-dihydro-7H-[1,4]oxazino[2,3,4-ij]chinolin-6-carboxthioamid,
b) N-(4-Chlorbenzyl)-2-(R oder S)-(hydroxymethyl)-9-(morpholin-4-ylmethyl)-7-oxo-2,3-dihydro-7H-[1,4]oxazino[2,3,4-ij]chinolin-6-carboxthioamid,
c) N-(4-Chlorbenzyl)-9-(morpholin-4-ylmethyl)-7-oxo-2-pyridin-3-yl-2,3-dihydro-7H-[1,4]oxazino[2,3,4-ij] chinolin-6-carboxthioamid,
d) N-(4-Chlorbenzyl)-9-(morpholin-4-ylmethyl)-7-oxo-2-pyridin-4-yl-2,3-dihydro-7H-[1,4]oxazino[2,3,4-ij]-chinolin-6-carboxthioamid,
e) N-(4-Chlorbenzyl)-9-(morpholin-4-ylmethyl)-7-oxo-2-pyridin-2-yl-2, 3-dihydro-7H-[1,4]oxazino[2,3,4-ij]chinolin-6-carboxthioamid,
f) N-(4-Chlorbenzyl)-9-(morpholin-4-ylmethyl)-7-oxo-2-(R oder S)-pyridin-3-yl-2,3-dihydro-7H-[1,4]oxazino[2, 3, 4-ij]chinolin-6-carboxthioamid,
g) N- (4-Chlorbenzyl) -9-(morpholin-4-ylmethyl)-7-oxo-2-(R oder S)-pyridin-2-yl-2,3-dihydro-7H-[1,4]oxazino[2,3,4-ij]chinolin-6-carboxthioamid,
h) N-(4-Chlorbenzyl)-2-(1-methyl-1H-imidazol-2-yl)-9-(morpholin-4-ylmethyl)-7-oxo-2,3-dihydro-7H-[1,4]oxazino[2,3,4-ij]chinolin-6-carboxthioamid oder ein pharmazeutisch akzeptables Salz.

**78.** Verbindung nach Anspruch 1, nämlich N-(4-Chlorbenzyl)-9-(morpholin-4-ylmethyl)-7-oxo-2-(S)-pyridin-2-yl-2,3-dihydro-7H-[1,4]oxazino[2,3,4-ij]chinolin-6-carboxamid oder ein pharmazeutisch akzeptables Salz.

**79.** Verbindung nach Anspruch 1, nämlich N-(4-Chlorbenzyl)-9-(morpholin-4-ylmethyl)-7-oxo-2-(R)-pyridin-2-yl-2,3-dihydro-7H-[1,4]oxazino[2,3,4-ij]chinolin-6-carboxamid oder ein pharmazeutisch akzeptables Salz.

**80.** Verbindung nach Anspruch 1, nämlich N- (4-Chlorbenzyl) -9-(morpholin-4-ylmethyl)-7-oxo-2-(R)-pyridin-3-yl-2,3-dihydro-7H-[1,4] oxazino [2, 3,4-ij]chinolin-6-carboxamid oder ein pharmazeutisch akzeptables Salz.

**81.** Verbindung nach Anspruch 1, nämlich N-(4-Chlorbenzyl)-9-(morpholin-4-ylmethyl) -7-oxo-2-(S)-pyridin-3-yl-2,3-dihydro-7H-[1,4]oxazino[2,3,4-ij]chinolin-6-carboxamid oder ein pharmazeutisch akzeptables Salz.

**82.** Verbindung nach Anspruch 1, nämlich N- (4-Chlorbenzyl) -2-(1-methyl-1H-imidazol-2-yl)-9-(morpholin-4-ylmethyl)-7-oxo-2,3-dihydro-7H-[1,4]oxazino[2,3,4-ij]chinolin-6-carboxamid oder ein pharmazeutisch akzeptables Salz.

**83.** Pharmazeutische Zusammensetzung, die eine Verbindung gemäß einem der Ansprüche 1 - 82 und einen pharmazeutisch akzeptablen Träger umfasst.

**84.** Verwendung einer Verbindung gemäß einem der Ansprüche 1 - 82 zur Herstellung eines Medikaments zur Behandlung oder Hemmung einer viralen DNA-Polymerase.

**85.** Verwendung einer wirksamen Menge einer Verbindung gemäß einem der Ansprüche 1 - 82 zur Herstellung eines Medikaments zur Behandlung von Infektionen von Herpesviren bei einem Säuger.

**86.** Verwendung nach Anspruch 85, wobei die Herpesviren das Herpes-simplex-Virus Typ 1, Herpes-simplex-Virus Typ 2, Varicella-zoster-Virus, Cytomegalovirus, Epstein-Barr-Virus, die Human-Herpes-Viruses 6, Human-Herpes-Viruses 7 oder Human-Herpes-Viruses 8 sind.

**87.** Verwendung nach Anspruch 85, wobei die Herpesviren das Herpes-simplex-Virus Typ 1, Herpes-simplex-Virus Typ 2, Varicella-zoster-Virus, Cytomegalovirus, Epstein-Barr-Virus, die Human-Herpes-Viruses 7 oder Human-Herpes-Viruses 8 sind.

**88.** Verwendung nach Anspruch 85, wobei die Herpesviren das humane Cytomegalovirus sind.

**89.** Verwendung nach Anspruch 85, wobei die wirksame Menge einer Verbindung nach Anspruch 1 oral, parenteral, topisch, rektal, nasal, sublingual oder transdermal verabreicht wird.

**90.** Verwendung nach Anspruch 85, wobei die wirksame Menge einer Verbindung nach Anspruch 1 eine Menge von etwa 0,1 bis etwa 300 mg/kg Körpergewicht ist.

**91.** Verwendung nach Anspruch 85, wobei die wirksame Menge einer Verbindung nach Anspruch 1 eine Menge von etwa 1 bis etwa 30 mg/kg Körpergewicht ist.

**92.** Verwendung nach Anspruch 85, wobei der Säuger ein Mensch ist.

**93.** Verwendung nach Anspruch 85, wobei der Säuger ein Lebensmitteltier oder ein Begleittier ist.

## Revendications

**1.** Composé de formule I 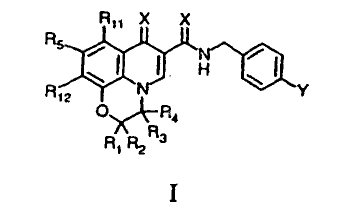 ou un de ses sels, racémates, produits de solvatation ou une de ses formes tautomères ou un de ses isomères optiques , pharmaceutiquement acceptables, formule dans laquelle :
chaque groupe X représente indépendamment un atome de O ou S ;
Y représente un groupe Cl, F, Br, CN ou NO₂ ;
R₁, R₂, R₃ et R₄ représentent indépendamment
a) un atome d'hydrogène,
b) un groupe N₃,
c) un groupe CN,
d) un groupe fluoro,
e) un groupe trifluorométhyl,
f) un groupe aryle,
g) un groupe het,
h) un groupe alkyle en C₁ à C₈, facultativement substitué avec un substituant R₆ ou OR₇, ou
i) R₁ et R₂ ou R₃ et R₄, conjointement avec l'atome de carbone auquel ils sont fixés, forment un groupe cycloalkyle en C₃ à C₈ ou het ;
R₅ représente un groupe alkyle en C₁ à C₈, qui peut être partiellement insaturé et facultativement substitué avec un à trois substituants N₃, halogéno, CN, R₆ ou R₇ ;
R₆ représente un groupe
a) aryle,
b) het,
c) SOᵢR₈,
d) OR₈,
e) C(=O)OR₈,
f) C(=O)R₈, ou
g) NR₈R₉ ;
R₇ représente
a) P(=O) (OR₁₀)₂,
b) CO (CH₂)ⱼCON(CH₃)(CH₂)ₖSO₃⁻M⁺,
c) un aminoacide,
d) un groupe C(=O)(alkyle en C₁ à C₆), facultativement substitué avec un substituant NR¹⁰R¹⁰, ou
e) un groupe CO(CH₂)ₙCO₂H ;
R₈ et R₉ représentent indépendamment
a) un atome d'hydrogène,
b) un groupe cycloalkyle en C₃ à C₈,
c) un groupe aryle,
d) un groupe het, ou
e) un groupe alkyle en C₁ à C₈ qui est en outre facultativement substitué avec un ou plusieurs substituants aryle, het, halogéno, CN, CO₂R₁₀, SOᵢR₁₀, OR₁₀, NR₁₀R_{10'} CF₃ ou cycloalkyle en C₃ à C₈ ;
R₁₀ représente
a) un atome de H ou
b) un groupe alkyle en C₁ à C₈, facultativement substitué avec un substituant OH ou O(alkyle en C₁ à C₄) ;
R₁₁ et R₁₂ représentent indépendamment
a) un atome d'hydrogène,
b) un groupe halogéno,
c) un groupe NO₂,
d) un groupe CN,
e) un groupe R₆,
f) un groupe SOᵢNR₈R₉, ou
g) un groupe alkyle en C₁ à C₈, qui peut être partiellement insaturé et facultativement substitué avec un à trois substituants N₃, halogéno, CN, R₆ ou OR₇ ;
le groupe aryle représente
un radical phényle, facultativement condensé avec un noyau carbocyclique ou hétérocyclique saturé ou insaturé ; à chaque occurrence, le groupe aryle peut être substitué avec un ou plusieurs substituants halogéno, CN, CO₂R₁₀, SOᵢR₁₀, OR₁₀, NR₁₀R₁₀, CF₃, cycloalkyle en C₃ à C₈ ou alkyle en C₁ à C₄, le groupe alkyle en C₁ à C₄ étant facultativement substitué avec un substituant OR₁₀ ;
le groupe het représente
un noyau hétérocyclique tétra-(4), penta-(5), hexa-(6) ou hepta-(7)gonal saturé ou insaturé ayant 1, 2 ou 3 hétéroatomes choisis dans le groupe consistant en O, S et NW, dans lequel W représente un atome d'hydrogène, un groupe alkyle en C₁ à C₄ ou C(=O)O(alkyle en C₁ à C₄) ou bien est absent, le groupe het étant facultativement condensé avec un noyau benzénique, un noyau carbocyclique ou un noyau hétérocyclique ; à chaque occurrence, het peut être substitué avec un ou plusieurs substituants halogéno, CN, CO₂R₁₀, SOᵢR₁₀, OR₁₀, NR₁₀R₁₀, alkyle en C₁ à C₄, CF₃, cycloalkyle en C₃ à C₈, oxo ou oxine ;
à chaque occurrence, un groupe cycloalkyle peut être substitué avec un substituant alkyle en C₁ à C₄, OR¹⁰, oxo, oxine ou un groupe het spiro condensé ;
i est égal à 0, 1 ou 2 ;
j est égal à 1, 2, 3, 4, 5 ou 6 ;
k est égal à 1, 2, 3, 4, 5 ou 6 ;
n est égal à 1, 2, 3, 4, 5 ou 6 ;
M représente un atome de sodium, de potassium ou de lithium ; et
sous les réserves suivantes :
a) au moins un des groupes R₁, R₂, R₃ et R₄ est autre qu'un atome d'hydrogène ;
c) lorsque R₁, R₂, R₃ et R₄ représentent indépendamment des groupes alkyle en C₁ à C₈, au moins un des groupes alkyle est substitué avec un substituant R₆ ou R₇ ;

**2.** Composé de formule I suivant la revendication 1, qui répond à la formule I-A

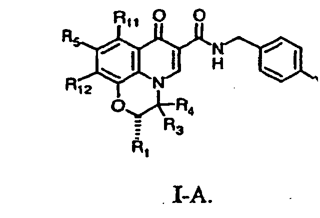

**3.** Composé de formule I suivant la revendication 1, qui répond à la formule I-B

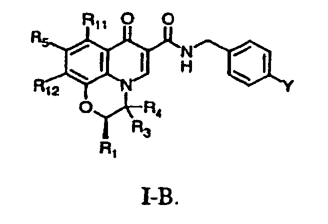

**4.** Composé de formule I suivant la revendication 1, qui répond à la formule I-C

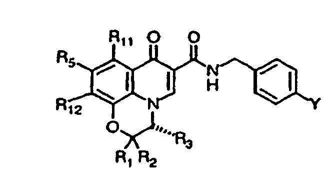

**5.** Composé de formule I suivant la revendication 1, qui répond à la formule I-D

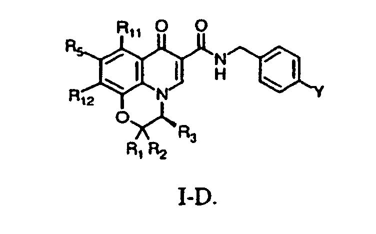

**6.** Composé de formule I suivant la revendication 1, qui répond à la formule I-E

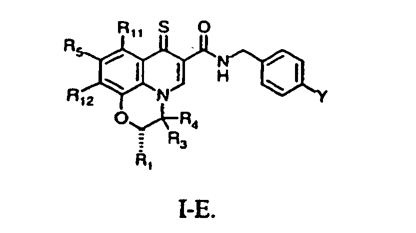

**7.** Composé de formule I suivant la revendication 1, qui répond à la formule I-F

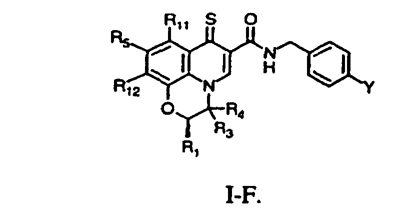

**8.** Composé de formule I suivant la revendication 1, qui répond à la formule I-G

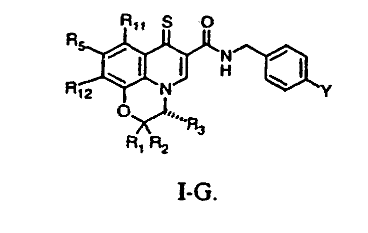

**9.** Composé de formule I suivant la revendication 1, qui répond à la formule I-H

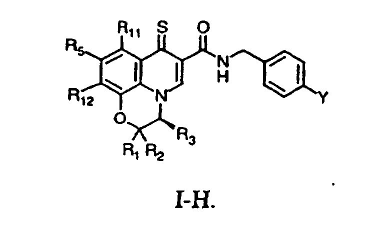

**10.** Composé de formule I suivant la revendication 1, qui répond à la formule I-1

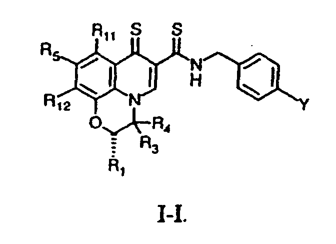

**11.** Composé de formule I suivant la revendication 1, qui répond à la formule I-J

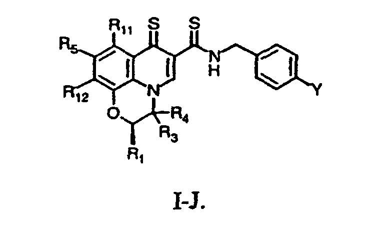

**12.** Composé de formule I suivant la revendication 1, qui répond à la formule I-K

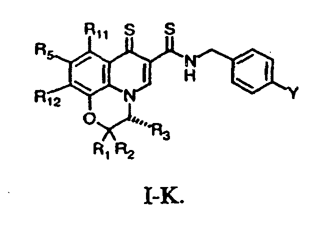

**13.** Composé de formule I suivant la revendication 1, qui répond à la formule I-L

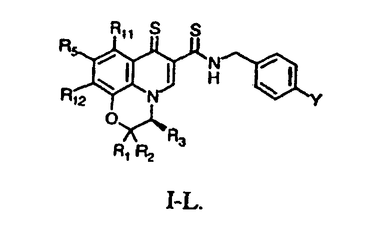

**14.** Composé de formule I suivant la revendication 1, qui répond à la formule I-M

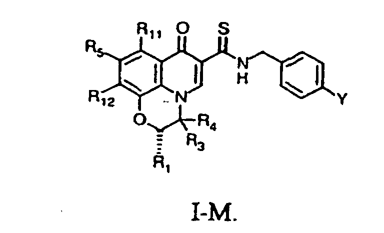

**15.** Composé de formule I suivant la revendication 1, qui répond à la formule I-N

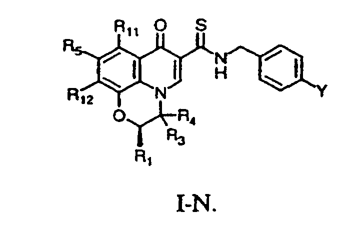

**16.** Composé de formule I suivant la revendication 1, qui répond à la formule I-O

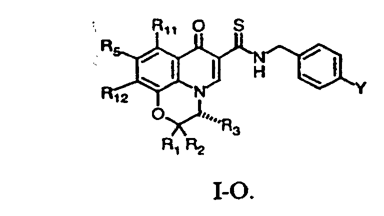

**17.** Composé de formule I suivant la revendication 1, qui répond à la formule I-P

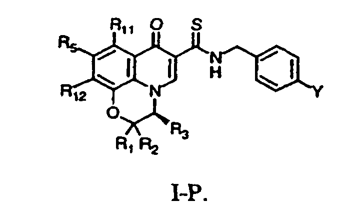

**18.** Composé de formule I suivant les revendications 1 à 17, dans lequel R₁₁ représente H, un groupe halogéno, ou un groupe alkyle en C₁ à C₄ facultativement substitué avec un à trois substituants halogéno ; et
R₁₂ représente
a) H,
b) SO_{I}R₈,
c) OR₈,
d) C(=O)OR₈,
e) C(=O)R₈,
f) NR₈R₉,
g) SO_{I}R₈R₉, ou
h) un groupe alkyle en C₁ à C₈, qui peut être partiellement insaturé et facultativement substitué avec un à trois substituants N₃, halogéno, CN ou R₆.

**19.** Composé suivant la revendication 18, dans lequel R₁₁ et R₁₂ représentent des atomes d'hydrogène.

**20.** Composé suivant la revendication 19, dans lequel R₅ représente un groupe alkyle en C₁ à C₈ substitué avec un substituant OR₈ ou het.

**21.** Composé suivant la revendication 19, dans lequel R₅ représente un groupe alkyle en C₁ à C₆ substitué avec un substituant OH.

**22.** Composé suivant la revendication 19, dans lequel R₅ représente un groupe alkyle en C₁ à C₄ substitué avec un substituant het.

**23.** Composé suivant la revendication 22, dans lequel het représente un groupe morpholinyle ou thiomorpholinyle.

**24.** Composé suivant la revendication 19, dans lequel R₅ représente un groupe 4-morpholinylméthyle.

**25.** Composé suivant la revendication 19, dans lequel R₅ représente un groupe alkyle en C₁ à C₈ qui est partiellement insaturé et facultativement substitué avec un substituant OR₈.

**26.** Composé suivant la revendication 19, dans lequel R₅ représente un groupe propynyle.

**27.** Composé suivant la revendication 26, dans lequel le groupe propynyle est substitué avec un substituant OH.

**28.** Composé suivant les revendications 20 à 27, dans lequel Y représente un groupe Cl.

**29.** Composé suivant la revendication 28, dans lequel R₃ et R₄ représentent indépendamment de atomes d'hydrogène.

**30.** Composé suivant la revendication 29, dans lequel R₁ et R₂ représentent indépendamment
a) un atome de d'hydrogène,
b) un groupe fluoro,
c) un groupe alkyle en C₁ à C₈ substitué avec un substituant R₆ ou OR₇,
d) un groupe aryle,
e) un groupe het, ou
f) R₁ et R₂, conjointement avec l'atome de carbone auquel ils sont fixés, forment un groupe cycloalkyle hexa-(6)gonal ou un groupe het ;
où R₆ représente un groupe
a) het,
b) SOᵢR₈,
c) OR₈, ou
d) NR₈R₉ ;
où R₇ représente un groupe
a) P (=O) (PR₁₀)₂,
b) CO (CH₂)ₙCON(CH₃)(CH₂)ₙSO₃⁻M⁺, ou
c) C(=O)alkyle en C₁ à C₆,
où R₈ et R₉ représentent indépendamment
a) un atome d'hydrogène,
b) un groupe aryle,
c) un groupe het, ou
d) un groupe alkyle en C₁ à C₈ qui en outre est facultativement substitué avec un ou plusieurs substituants aryle, het, halogéno, CO₂R₁₀, SOᵢR₁₀ ou OR₁₀,
où R₁₀ représente
a) un atome de H, ou
c) un groupe alkyle en C₁ à C₄, facultativement substitué avec un substituant OH.

**31.** Composé suivant la revendication 30, dans lequel R₁ et R₂ représentent indépendamment H, un groupe alkyle en C₁ à C₄ substitué avec un groupe OR₈ dans lequel R₈ représente un atome de H, ou un groupe alkyle en C₁ à C₄ substitué avec un groupe OR₁₀.

**32.** Composé suivant la revendication 30, dans lequel R₁ représente un atome de H ; R₂ représente un groupe aryle, le groupe aryle étant facultativement substitué avec un ou deux substituants halogéno, CN, OR₁₀ ou alkyle en C₁ à C₄ substitué avec un groupe OR₁₀.

**33.** Composé suivant la revendication 30, dans lequel R₁ représente un atome de H ; R₂ représente un groupe aryle, le groupe aryle étant condensé avec un noyau hétérocyclique.

**34.** Composé suivant la revendication 33, dans lequel le groupe R₂ représente un groupe 1,3-benzodioxolyle ou 1,4-benzodoxinyle.

**35.** Composé suivant la revendication 30, dans lequel R₁ représente un atome de H ; R₂ représente un groupe het.

**36.** Composé suivant la revendication 35, dans lequel het représente un noyau hétérocyclique penta-(5) ou hexa-(6)gonal saturé ou insaturé ayant 1, 2 ou 3 hétéroatomes choisis dans le groupe consistant en O, S et NW, dans lequel W représente un atome d'hydrogène, un groupe alkyle en C₁ à C₄ ou C(=O) (alkyle en C₁ à C₄) ou bien est absent, le groupe het pouvant être substitué avec un ou plusieurs substituants halogéno, alkyle en C₁ à C₄, CF3 ou oxo ou oxine.

**37.** Composé suivant la revendication 36, dans lequel het représente un groupe pyridinyle.

**38.** Composé suivant la revendication 36, dans lequel het représente un noyau hétérocyclique penta-(5)gonal.

**39.** Composé suivant la revendication 30, dans lequel R₁ et R₂, conjointement avec l'atome de carbone auquel ils sont fixés, forment un groupe het.

**40.** Composé suivant la revendication 39, dans lequel le groupe het est un noyau hétérocyclique penta-(5) ou hexa-(6)gonal ayant 1, 2 ou 3 hétéroatomes choisis dans le groupe consistant en O, S et NW, dans lequel W représente un atome d'hydrogène, un groupe alkyle en C₁ à C₄ ou C(=O)(alkyle en C₁ à C₄), le groupe het pouvant être substitué avec un ou plusieurs substituants halogéno OR₁₀, alkyle en C₁ à C₄, CF₃, oxo ou oxine.

**41.** Composé suivant la revendication 40, dans lequel het représente un noyau hétérocyclique hexa-(6)gonal.

**42.** Composé suivant la revendication 41, dans lequel le groupe het est un groupe pyranne, pipéridine ou thiopyranne.

**43.** Composé suivant la revendication 30, dans lequel R₁ et R₂, conjointement avec l'atome de carbone auquel ils sont fixés, forment un groupe cycloalkyle hexa-(6)gonal.

**44.** Composé suivant la revendication 43, dans lequel le groupe cycloalkyle est facultativement substitué avec un substituant oxo ou OR₁₀.

**45.** Composé suivant la revendication 30, dans lequel
R₂ représente un atome d'hydrogène ;
R₁ représente un groupe alkyle en C₁ à C₈ substitué avec un substituant R₆ ou OR₇ ;
où R₆ représente un groupe
a) het,
b) SR₈,
c) OR₈, ou
d) NR₈R₉ ;
où R₇ représente un groupe
a) (P=O) (OCH₃)₂,
b) CO (CH₂)ₙCON (CH₃) (CH₂)ₙSO₃⁻M⁺, ou
c) C(=O)CH₃,
où R₈ et R₉ représentent indépendamment
a) un atome d'hydrogène,
b) un groupe het, ou
c) un groupe alkyle en C₁ à C₈, qui est facultativement substitué avec un ou deux substituants het, CO₂R₁₀, SOᵢR₁₀ ou OR₁₀ ; et
où R₁₀ représente
a) un atome de H, ou
b) un groupe alkyle en C₁ à C₄, facultativement substitué avec un substituant OH ou O(alkyle en C₁ à C₄).

**46.** Composé suivant la revendication 45, dans lequel R₁ représente un groupe alkyle en C₁ à C₈ substitué avec un substituant het.

**47.** Composé suivant la revendication 46, dans lequel le groupe het est un groupe pipéridinyle, morpholinyle, thiomorpholinyle, pipérazinyle, pipérazinyle à substituant N(alkyle C₁ à C₄), pyrrolidinyle, pyridyle, imidazolyle ou imidazole à substituant N(alkyle en C₁ à C₄).

**47.** Composé suivant la revendication 46, dans lequel le groupe het est un groupe morpholinyle.

**48.** Composé suivant la revendication 47, dans lequel le groupe het est un groupe pyridinyle.

**49.** Composé suivant la revendication 45, dans lequel R₁ représente un groupe alkyle en C₁ à C₈ substitué avec un substituant OH ou O(alkyle en C₁ à C₄).

**50.** Composé suivant la revendication 49, dans lequel R₁ représente un groupe alkyle en C₁ à C₄ substitué avec un substituant OH.

**51.** Composé suivant la revendication 45, dans lequel R₁ représente un groupe alkyle en C₁ à C₈ substitué avec un substituant SR₈.

**52.** Composé suivant la revendication 51, dans lequel R₈ représente un groupe het.

**53.** Composé suivant la revendication 51, dans lequel R₈ représente un groupe alkyle en C₁ à C₄ facultativement substitué avec un ou deux substituants OR₁₀.

**54.** Composé suivant la revendication 45, dans lequel R₁ représente un groupe alkyle en C₁ à C₄ substitué avec un substituant NR₈R₉.

**55.** Composé suivant la revendication 54, dans lequel R₈ représente H et R₉ représente un groupe het.

**56.** Composé suivant la revendication 55, dans lequel het représente un noyau hétérocyclique hexa-(6)gonal ayant 1, 2 ou 3 hétéroatomes choisis dans le groupe consistant en 0, S et NW, dans lequel W représente un atome d'hydrogène ou un groupe alkyle en C₁ à C₄ ou bien est absent.

**57.** Composé suivant la revendication 56, dans lequel het représente un groupe pyridinyle.

**58.** Composé suivant la revendication 54, dans lequel R₈ représente un atome de H et R₉ représente un groupe alkyle en C₁ à C₈ facultativement substitué avec un substituant het.

**59.** Composé suivant la revendication 58, dans lequel het représente un noyau hétérocyclique hexa-(6)gonal ayant 1, 2 ou 3 hétéroatomes choisis dans le groupe consistant en O, S et NW, dans lequel W représente un atome d'hydrogène, un groupe alkyle en C₁ à C₄ ou bien est absent.

**60.** Composé suivant la revendication 59, dans lequel het représente un groupe pyridinyle.

**61.** Composé suivant la revendication 54, dans lequel R₈ représente H et R₉ représente un groupe alkyle en C₁ à C₈ facultativement substitué avec un ou deux substituants OR₁₀.

**62.** Composé suivant la revendication 45, dans lequel R₁ représente un groupe hydroxyméthyle, morpholinylméthyle, (pyridinylméthyl)aminométhyle, (diméthylamino)méthyle, (hydroxyéthyl)sulfanylméthyle, (1-méthyl-1H-imidazole-2-yl)-sulfanylméthyle, -CH₂OCO(CH₂)₆CON(CH₃)(CH₂)₂SO₃⁻M⁺, -CH₂OC(=O)CH₃, CH₂OP(=O)(OMe)₂, (4-méthyl-1-pipérazinyl)méthyle, 1-pyrrolidinylméthyle, (2,3-dihydroxypropyl)aminométhyle, (2-hydroxéthyl)-aminométhyle, 1-pipéridinylméthyle, bis(2-hydroxyéthyl)-aminométhyle, 1H-imidazole-1-ylméthyle, (méthylsulfanyl)-méthyle, (tertiobutylsulfanyl)méthyle, méthylsulfanylacétate, (2,3-dihydroxypropyl)sulfanylméthyle, phényle ou fluoro.

**63.** Composé suivant la revendication 62, dans lequel R₁ représente un groupe hydroxyméthyle, morpholinylméthyle, (2-pyridinylméthyl)aminométhyle, (3-pyridinylméthyl)aminométhyle, (diméthylamino)méthyle, (2-hydroxyéthyl)sulfanylméthyle, (1-méthyl-1H-imidazole-2-yl)sulfanylméthyle, OP(=O)(OCH₃)₂, -CH₂OCO(CH₂)₆CON(CH₃)(CH₂)₂SO₃⁻M⁺ ou -CH₂OC(=O)CH₃.

**64.** Composé suivant la revendication 12, dans lequel
R₁ et R₂ représentent indépendamment un atome d'hydrogène,
R₃ et R₄ représentent indépendamment
a) un atome d'hydrogène,
b) un groupe fluoro, ou
c) un groupe alkyle en C₁ à C₈ substitué avec un substituant R₆ ou OR₇ ;
où
R₆ représente
a) het,
b) SORᵢR₈,
c) OR₈, ou
d) NR₈R₉ ;
où R₇ représente un groupe
a) P(=O)(OH)₂,
b) P(=O)(alkoxy en C₁ à C₄)₂,
c) CO(CH₂)ₙCON(CH₃)(CH₂)ₙSO₃⁻M⁺, ou
d) C(=O)alkyle en C₁ à C₆ ;
où R₈ et R₉ représentent indépendamment
a) un atome d'hydrogène,
b) un groupe aryle,
c) un groupe het, ou
d) un groupe alkyle en C₁ à C₈ qui est en outre est facultativement substitué avec un ou plusieurs substituants aryle, het, halogéno, CO₂R₁₀, SOᵢR₁₀ ou OR₁₀ ;
où R₁₀ représente
a) un atome de H, ou
d un groupe alkyle en C₁ à C₄, facultativement substitué avec un substituant OH.

**65.** Composé suivant la revendication 20, dans lequel R₃ et R₄ représentent indépendamment un groupe fluoro ou hydroxyméthyle.

**66.** Composé suivant la revendication 20, dans lequel R₃ représente un atome d'hydrogène et R₄ représente un groupe phényle, morpholinylméthyle ou hydroxyméthyle.

**67.** Composé suivant la revendication 22, dans lequel R₄ représente un groupe morpholinylméthyle.

**68.** Composé suivant la revendication 1, qui est le
a) N-(4-chlorobenzyl)-2-(hydroxyméthyl)-9- (morpholine-4-ylméthyl)-7-oxo-2,3-dihydro-7H-[1,4]oxazino[2,3,4-ij]quinoléine-6-carboxamide,
b) N-(4-chlorobenzyl)-2-(*R* ou *S*)-(hydroxyméthyl)-9-(morpholine-4-ylméthyl)-7-oxo-2,3-dihydro-7H-[1,4]oxazino[2,3,9-ij]quinoléine-6-carboxamide,
c) N-(4-chlorobenzyl)-9- (morpholine-4-ylméthyl)-7-oxo-2-pyridine-3-yl-2,3-dihydro-7H-[1,4]oxazino[2,3,4-ij]quinoléine-6-carboxamide,
d) N-(4-chlorobenzyl)-9-(morpholine-4-ylméthyl)-7-oxo-2-pyridine-4-yl-2,3-dihydro-7H-[1,4]oxazino[2,3,4-ij]-quinoléine-6-carboxamide,
e) N-(4-chlorobenzyl)-9- (morpholine-4-ylméthyl ) -7-oxo-2-pyridine-2-yl-2,3-dihydro-7H-[1,4]oxazino[2, 3, 4-ij]-quinoléine-6-carboxamide,
f) N-(4-chlorobenzyl)-9-(morpholine-4-ylméthyl)-7-oxo-2-(*R* ou *S*)-pyridine-3-yl-2,3-dihydro-7H-[1,4]oxazino[2,3,4-ij]-quinoléine-6-carboxamide,
g) N-(4-chlorobenzyl)-2,9-bis(morpholine-4-ylméthyl)-7-oxo-2,3-dihydro-7H-[1,4]oxazino[2,3,4-ij]quinoléine-6-carboxamide,
h) 2- [(*tertio*butylsulfanyl)méthyl]-N-(4-chlorobenzyl)-9-(morpholine-4-ylméthyl)-7-oxo-2, 3-dihydro-7H-[1,4]oxazino[2,3,4-ij]quinoléine-6-carboxamide,
i) N-(4-chlorobenzyl)-2-{[(2-hydroxyéthyl)sulfanyl]méthyl}-9-(morpholine-4-ylméthyl)-7-oxo-2,3-dihydro-7H-[1,4]oxazino[2,3,4-ij]quinoléine-6-carboxamide,
j) N-(4-chlorobenzyl)-2-{[(1-méthyl-1H-imidazole-2-yl)sulfanyl]-méthyl}-9-(morpholine-4-ylméthyl)-7-oxo-2,3-dihydro-7H-[1,4]oxazino[2,3,4-ij]quinoléine-6-carboxamide,
k) N-(4-chlorobenzyl)-9- (morpholine-4-ylméthyl)-7-oxo-2-{[(3-pyridinylméthyl)amino]méthyl}-2,3-dihydro-7H-[1,4]-oxazino[2,3,4-ij]quinoléine-6-carboxamide,
l) acétate de [6-{[(4-chlorobenzyl)amino]carbonyl}-9-(morpholine-4-ylméthyl)-7-oxo-2,3-dihydro-7H-[1,4 ]oxazino[2,3,4-ij]quinoléine-2-yl]méthyle,
m) N-(4-chlorobenzyl)-9-(morpholine-4-ylméthyl)-7-oxo-2-(*R* ou *S*)-{[ (3-pyridinylméthyl)amino]méthyl}-2,3-dihydro-7H-[1,4]-oxazino [2, 3, 4-ij]quinoléine-6-carboxamide,
n) N-(4-chlorobenzyl)-2-(3-hydroxyphényl)-9-(morpholine-4-ylméthyl)-7-oxo-2,3-dihydro-7H-[1,4]oxazino[2,3,4-ij]-quinoléine-6-carboxamide,
o) N-(4-chlorobenzyl)-9-(morpholine-4-ylméthyl)-7-oxo-2-(*R* ou *S*)-pyridine-2yl-2,3-dihydro-7H-[1,4]oxazino[2,3,4-ij]-quinoléine-6-carboxamide,
p) N-(4-chlorobenzyl)-2-[3-(hydroxyméthyl)phényl]-9-(morpholine-4-ylméthyl)-7-oxo-2,3-dihydro-7H-[1,4]-oxazino[2,3,4-ij]quinoléine-6-carboxamide,
q) N-(4-chlorobenzyl)-2-[2-(hydroxyméthyl)phényl]-9- (morpholine-4-ylméthyl)-7-oxo-2,3-dihydro-7H-[1,4]-oxazino [2,3,4-ij]quinoléine-6-carboxamide,
r) N-(4-chlorobenzyl)-2-(1-méthyl-1H-imidazol-2-yl)-9-(morpholine-4-ylméthyl)-7-oxo-2,3-dihydro-7H-[1,4]-oxazino[2,3,4-ij]quinoléine-6-carboxamide,
s) N-(4-chlorobenzyl)-2-(2-furyl)-9-(morpholine-4-ylméthyl)-7-oxo-2,3-dihydro-7H-[1,4]oxazino[2,3,4-ij]quinoléine-6-carboxamide,
t) N-(4-chlorobenzyl)-2-(3-cyanophényl)-9-(morpholine-4-ylméthyl)-7-oxo-2,3-dihydro-7H-[1,4]oxazino[2,3,4-ij]quinoléine-6-carboxamide,
u) N-(4-chlorobenzyl)-2-(3-furyl)-9-(morpholine-4-ylméthyl)-7-oxo-2,3-dihydro-7H-[1,4]oxazino[2,3,9-ij]quinoléine-6-carboxamide,
v) N-(9-chlorobenzyl)-9-(morpholine-4-ylméthyl)-7-oxo-2-thiène-2-yl-2,3-dihydro-7H-[1,4]oxazino[2,3,4-ij]-quinoléine-6-carboxamide,
w) N-(4-chlorobenzyl)-2-(3,5-difluorophényl)-9-(morpholine-4-ylméthyl)-7-oxo-2,3-dihydro-7H-[1,4]oxazino[ 2 , 3 , 4 - ij]quinoléine-6-carboxamide,
x) 2- (1, 3-benzodioxol-5-yl)-N-(4-chlorobenzyl)-9-(morpholine-4-ylméthyl)-7-oxo-2,3-dihydro-7H-[1,4]-oxazino[2,3,4-ij]quinoléine-6-carboxamide,
y) N-(4-chlorobenzyl)-2-(2,3-dihydro-1,4-benzodioxine-6-yl)-9-(morpholine-4-ylméthyl)-7-oxo-2,3-dihydro-7H-[1,4]oxazino[2,3,4-ij]quinoléine-6-carboxamide,
z) 2-(1,3-benzodioxol-4-yl)-N-(4-chlorobenzyl)-9-(morpholine-4-ylméthyl)-7-oxo-2,3-dihydro-7H-[1,4]oxazino[2,3,4-ij]-quinoléine-6-carboxamide,
aa) 2-[3,5-bis(méthoxyméthoxy)phényl]-N-(4-chlorobenzyl)-9- (morpholine-4-ylméthyl) -7-oxo-2,3-dihydro-7H-[1,4]-oxazino[2,3,4-ij]quinoléine-6-carboxamide,
bb) N-(4-chlorobenzyl)-9-(morpholine-4-ylméthyl)-7-oxo-2-thiène-3-yl-2, 3-dihydro-7H-[1,4]oxazino[2,3, 4-ij]-quinoléine-6-carboxamide,
cc) N-(4-chlorobenzyl)-2, 2-bis [ (méthoxyméthoxy)méthyl]-9-(morpholine-4-ylméthyl)-7-oxo-2,3-dihydro-7H-[1,4]-oxazino [2, 3,4-ij]quinoléine-6-carboxamide,
dd) N-[(4-chlorophényl)méthyl]-9'-(4-morpholinylméthyl)-4,7'-dioxospiro[cyclohexane-1,2'(3'*H*)-[7*H*]pyrido[1,2,3-*de*]-[1,4]benzoxazine]-6'-carboxamide,
ee) N- [ (4-chlorophényl)méthyl]-4-hydroxy-9'-(4-morpholinylméthyl)-7'-oxospiro[cyclohexane-1,2'(3'*H*)-[7*H*]pyrido[1,2,3-*de*]-[1,4]benzoxazine]-6'-carboxamide,
ff) N-[(4-chlorobenzyl)-3,9-bis(morpholine-4-ylméthyl)-7-oxo-2, 3-dihydro-7H-[1,4]oxazino[2,3, 4-ij]quinoléine-6-carboxamide,
gg) N-(4-chlorobenzyl) -9- (morpholine-4-ylméthyl) -7-oxo-2-phényl-2,3-dihydro-7H-[1,4]oxazino[2,3,4-ij]-quinoléine-6-carboxamide,
hh) N-(4-chlorobenzyl)-2,2-difluoro-9-(morpholine-4-ylméthyl)-7-oxo-2,3-dihydro-7H-[1,4]oxazino[2,3,4-ij]-quinoléine-6-carboxamide,
ii) N-(4-chlorobenzyl)-2-[(méthylsulfanyl)méthyl]-9-(morpholine-4-ylméthyl)-7-oxo-2,3-dihydro-7H-[1,4]-oxazino[2,3,4-ij]quinoléine-6-carboxamide,
jj) N-(4-chlorobenzyl)-2-[(diméthylamino)méthyl]-9-(morpholine-4-ylméthyl)-7-oxo-2,3-dihydro-7H-[1,4]-oxazino[2,3,4-ij]quinoléine-6-carboxamide,
kk) N-(4-chlorobenzyl)-2-[(4-méthyl-1-pipérazinyl)méthyl]-9-(morpholine-4-ylméthyl)-7-oxo-2,3-dihydro-7H-[1,4]-oxazino[2,3,4-ij]quinoléine-6-carboxamide,
ll) ({[6-{[(4-chlorobenzyl)amino]carbonyl}-9-(morpholine-4-ylméthyl)-7-oxo-2,3-dihydro-7H-[1,4]oxazino[2,3,4-ij]quinoléine-2-yl]méthyl}thio)acétate de méthyle,
mm) N- (4-chlorobenzyl) -9- (morpholine-4-ylméthyl)-7-oxo-2-(1-pyrrolidinylméthyl)-2,3-dihydro-7H-[1,4]oxazino[2,3,4-ij]-quinoléine-6-carboxamide,
nn) N- (4-chlorobenzyl)-2-{[(2,3-dihydroxypropyl)sulfanyl]méthyl}-9-(morpholine-4-ylméthyl)-7-oxo-2,3-dihydro-7H-[1,4]oxazino[2,3,4-ij]quinoléine-6-carboxamide,
oo) N-(4-chlorobenzyl)-2-{[ (2,3-dihydroxypropyl)amino]méthyl)-9-(morpholine-4-ylméthyl)-7-oxo-2,3-dihydro-7H-[1,4]oxazino[2, 3, 4-ij ] quinoléine-6-carboxamide,
pp) N- (4-chlorobenzyl)-2- {[(2-dihydroxyéthyl)amino]méthyl}-9-(morpholine-4-ylméthyl)-7-oxo-2,3-dihydro-7H-[1,4]oxazino[2,3, 4-ij]quinoléine-6-carboxamide,
qq) N- (4-chlorobenzyl)-9-(morpholine-4-ylméthyl)-7-oxo-2-(1-pipéridinylméthyl)-2,3-dihydro-7H-[1,4]oxazino[2,3,4-ij]-quinoléine-6-carboxamide,
rr) 2-{[bis(2-hydroxyéthyl)amino]méthyl}-N-(4-chlorobenzyl)-9-(morpholine-4-ylméthyl)-7-oxo-2, 3-dihydro-7H-[1,4]oxazino[2,3,4-ij]quinoléine-6-carboxamide,
ss) N-(4-chlorobenzyl)-9-(morpholine-4-ylméthyl)-7-oxo-{[(2-pyridinylméthyl)amino]méthyl)-2,3-dihydro-7H-[1,4]oxazino[2,3,4-ij]quinoléine-6-carboxamide,
tt) sel de sodium d'acide 2-[(8-{[6-{[(4-chlorobenzyl)-amino]carbonyl}-9-(morpholine-4-ylméthyl)-7-oxo-2,3-dihydro-7H-[1, 4]oxazino[2,3,4-ij]quinoléine-2-yl]méthoxy}-8-oxooctanoyl) (méthyl)amino]éthanesulfonique,
uu) [6-{[(4-chlorobenzyl)amino]carbonyl}-9-(morpholine-4-ylméthyl)-7-oxo-2,3-dihydro-7H-[1,4]oxazino[2,3,4-ij]-quinoléine-2-yl]méthyl-diméthyl-phosphate,
vv) N-(4-chlorobenzyl)-9-(morpholine-4-ylméthyl)-7-oxo-2-{[(4-pyridinylméthyl) amino]méthyl}-2,3-dihydro-7H-[1,4]oxazino[2,3,4-ij]quinoléine-6-carboxamide,
ww) N-(4-chlorobenzyl)-2-(1H-imidazol-1-ylméthyl)-9-(morpholine-4-ylméthyl)-7-oxo-2,3-dihydro-7H-[1,4]oxazino[2,3,4-ij]-quinoléine-6-carboxamide,
xx) N-(4-chlorobenzyl)-2-{[(4-chlorobenzyl)amino]méthyl}-9-(morpholine-4-ylméthyl)-7-oxo-2,3-dihydro-7H-[1,4]-oxazino [2,3,4-ij]quinoléine-6-carboxamide,
yy) N- (4-chlorobenzyl)-3-(hydroxyméthyl)-9-(morpholine-4-ylméthyl)-7-oxo-2,3-dihydro-7H-[1,4]oxazino[2,3,4-ij]quinoléine-6-carboxamide,
zz) N-(4-chlorobenzyl)-2-(4-hydroxyphényl)-9-(morpholine-4-ylméthyl)-7-oxo-2,3-dihydro-7H-[1,4]oxazino[2,3,4-ij]quinoléine-6-carboxamide,
aaa) N-(4-chlorobenzyl)-2-{3-[(méthoxyméthoxy)méthyl]-phényl)-9-(morpholine-4-ylméthyl)-7-oxo-2,3-dihydro-7H-[1,4]oxazino[2,3,4-ij]quinoléine-6-carboxamide,
bbb) N-(4-chlorobenzyl)-2-{2-[(méthoxyméthoxy)méthyl]-phényl)-9-(morpholine-4-ylméthyl)-7-oxo-2,3-dihydro-7H-[1,4]oxazino[2,3,4-ij]quinoléine-6-carboxamide,
ccc) N-(4-chlorobenzyl)-2-(2-hydroxyphényl)-9-(morpholine-4-ylméthyl) -7-oxo-2,3-dihydro-7H-[1,4]oxazino[2,3,4-ij]quinoléine-6-carboxamide,
ddd) N- [(4-chlorophényl)méthyl]-2,3,5,6-tétrahydro-9'-(4-morpholinylméthyl)-7'-oxospiro[4H-pyranne-4,2'(3'*H*)-[7*H*]pyrido[1,2,3-de][1,4]benzoxazine]-6'-carboxamide,
eee) 6- [ [ [4-chlorophényl)méthyl]amino]carbonyl]-9'-(4-morpholinylméthyl)-7'-oxospiro[pipéridine-4, 2' (3*H*)-[7*H*]pyrido [1,2,3-*de*]-[1,4]benzoxazine)-1-carboxylate de 1,1-diméthyléthyle,
fff) N-[(4-chlorophényl)méthyl]-9'-(4-morpholinylméthyl)-7'-oxospiro[pipéridine-4,2'(3'*H*)-[7*H*]pyrido[1,2,3-*de*]-[1,4]benzoxazine]-6'-carboxamide,
ggg) N- (4-chlorobenzyl) -2,2-bis(hydroxyméthyl) -9- (morpholine-4-yl-méthyl)-7-oxo-2,3-dihydro-7*H*-[1,4]oxazino [2,3, 4-ij]quinoléine-6-carboxamide,
hhh) N- [ (4-chlorophényl)méthyl]-2',3',5' , 6'-tétrahydro-9-(4-morpholinylméthyl)-7-oxospiro[7*H*-pyrido[1,2,3-*de*]-1,4-benzoxazine-2(*3H*),4'-[4*H*]thiopyranne]-6-carboxamide,
iii) N- (4-chlorobenzyl) -9- (morpholine-4-ylméthyl) -7-oxo-3-phényl-2,3-dihydro-7H-[1,4]oxazino[2,3,4-ij]quinoléine-6-carboxamide,
jjj) N-(4-chlorobenzyl)-3,3-bis(hydroxyméthyl)-9-(3-hydroxy-1-propynyl)-7-oxo-2,3-dihydro-7H-[1,4]oxazino[2,3, 4-ij]quinoléine-6-carboxamide,
kkk) N-(4-chlorobenzyl)-3,3-bis(hydroxyméthyl)-9-(3-hydroxypropyl)-7-oxo-2,3-dihydro-7H-[1,4]oxazino[ 2 , 3 , 4 - ij] quinoléine-6-carboxamide,
lll) N- (4-chlorobenzyl)-2-[2-(méthoxyméthoxy)phényl]-9-(morpholine-4-ylméthyl)-7-oxo-2,3-dihydro-7H-[1,4]oxazino[2,3,4-ij]quinoléine-6-carboxamide,
mmm) N-(4-chlorobenzyl)-2-{4-[(méthoxyméthoxy)méthyl]-phényl}-9-(morpholine-4-ylméthyl)-7-oxo-2,3-dihydro-7H-[1,4]oxazino[2,3,4-ij]quinoléine-6-carboxamide,
nnn) 2-[2,3-bis(méthoxyméthoxy)phényl]-N-(4-chlorobenzyl)-9-(morpholine-4-ylméthyl)-7-oxo-2,3-dihydro-7H-[1,4]-oxazino[2,3,4-ij]quinoléine-6-carboxamide,
ooo) N- [(4-chlorophényl)méthyl)-1-méthyl-9'-(4-morpholinylméthyl ) -7' -oxospiro [pipéridine-4, 2' (3*H*)-[7*H*]pyrido[1,2,3-*de*][1,4]benzoxazine]-6'-carboxamide,
ppp) N-[(4-chlorophényl)méthyl]-9"-(4-morpholinylméthyl)-dispiro[1,3-dioxolane-2,1'-cyclohexane-4',2"(3"*H*)-[7*H*] -pyrido[1,2,3-*de*][1,4]benzoxazine]-6"-carboxamide,
ou un de ses sels pharmaceutiquement acceptables.

**69.** Composé suivant la revendication 1, qui est le
a) N-(4-chlorobenzyl)-2-(hydroxyméthyl)-9-(morpholine-4-ylméthyl)-7-thioxo-2,3-dihydro-7H-[1,4]oxazino[2,3,4-ij]-quinoléine-6-carboxamide,
b) N-(4-chlorobenzyl)-2-(R ou *S*)-(hydroxyméthyl)-9-(morpholine-4-ylméthyl)-7-thioxo-2,3-dihydro-7H-[1,4]oxazino[2,3,4-ij]quinoléine-6-carboxamide,
c) N-(4-chlorobenzyl)-9-(morpholine-4-ylméthyl)-7-thioxo-2-pyridine-3-yl-2,3-dihydro-7H-[1,4]oxazino[2,3,4-ij]-quinoléine-6-carboxamide,
d) N-(4-chlorobenzyl)-9- (morpholine-4-ylméthyl)-7-thioxo-2-pyridine-4-yl-2, 3-dihydro-7H-[1,4]oxazino[2,3, 4-ij]quinoléine-6-carboxamide,
e) N-(4-chlorobenzyl)-9-(morpholine-4-ylméthyl)-7-thioxo-2-pyridine-2-yl-2,3-dihydro-7H-[1,4]oxazino[2,3,4-ij]-quinoléine-6-carboxamide,
f) N-(4-chlorobenzyl)-9-(morpholine-4-ylméthyl)-7-thioxo-2-(*R* ou *S*)-pyridine-3-yl-2,3-dihydro-7H-[1,4]oxazino[2,3,4-ij]quinoléine-6-carboxamide,
g) N-(4-chlorobenzyl)-2,9-bis(morpholine-4-ylméthyl)-7-thioxo-2,3-dihydro-7H-[1,4]oxazino[2,3,4-ij]quinoléine-6-carboxamide,
h) 2-[ ( *tertio*butylsulfanyl)méthyl]-N-(4-chlorobenzyl)-9- (morpholine-4-ylméthyl)-7-thioxo-2,3-dihydro-7H-[1,4]-oxazino[2,3,4-ij]quinoléine-6-carboxamide,
i) N-(4-chlorobenzyl)-2-{[(2-hydroxyéthyl)sulfanyl]méthyl}-9-(morpholine-4-ylméthyl)-7-thioxo-2,3-dihydro-7H-[1, 4 ] oxazino [2, 3,4-ij]quinoléine-6-carboxamide,
j) N-(4-chlorobenzyl)-2-{[(1-méthyl-1H-imidazol-2-yl)sulfanyl]-méthyl }-9- (morpholine-4-ylméthyl)-7-thioxo-2, 3-dihydro-7H- [1,4]oxazino[2,3,4-ij]quinoléine-6-carboxamide,
k) N- (4-chlorobenzyl)-9- (morpholine-4-ylméthyl)-7-thioxo-2-{[(3-pyridinylméthyl)amino]méthyl}-2,3-dihydro-7H-[1,4]oxazino[2,3,4-ij]quinoléine-6-carboxamide,
l) acétate de [6-{[(4-chlorobenzyl)amino]carbonyl}-9-(morpholine-4-ylméthyl)-7-thioxo-2,3-dihydro-7H-[1,4]-oxazino[2,3,4-ij]quinoléine-2-yl]méthyle,
m) N-(4-chlorobenzyl)-9-(morpholine-4-ylméthyl)-7-thioxo-2-(*R* ou *S*)-{[(3-pyridinylméthyl)amino]méthyl}-2,3-dihydro-7H-[1,4]-oxazino[2,3,4-ij]quinoléine-6-carboxamide,
n) N-(4-chlorobenzyl)-2-(3-hydroxyphényl)-9-(morpholine-4-ylméthyl)-7-thioxo-2,3-dihydro-7H-[1,4]oxazino[2,3,4-ij]quinoléine-6-carboxamide,
o) N-(4-chlorobenzyl)-9-(morpholine-4-ylméthyl)-7-thioxo-2-(*R* ou *S*)-pyridine-2yl-2,3-dihydro-7H-[1,4]oxazino[2,3,4-ij]-quinoléine-6-carboxamide,
p) N-(4-chlorobenzyl)-2-[3-(hydroxyméthyl)phényl]-9- (morpholine-4-ylméthyl)-7-thioxo-2,3-dihydro-7H-[1,4]-oxazino[2,3,4-ij]quinoléine-6-carboxamide,
q) N-(4-chlorobenzyl)-2-[2-(hydroxyméthyl)phényl]-9- (morpholine-4-ylméthyl)-7-thioxo-2,3-dihydro-7H-[1, 4]oxazino[2,3,4-ij]quinoléine-6-carboxamide,
r) N-(4-chlorobenzyl)-2-(1-méthyl-1H-imidazol-2-yl)-9-(morpholine-4-ylméthyl)-7-thioxo-2,3-dihydro-7H-[1,4]-oxazino[2,3,4-ij]quinoléine-6-carboxamide,
s) N-(4-chlorobenzyl)-2-(2-furyl)-9-(morpholine-4-ylméthyl)-7-thioxo-2,3-dihydro-7H-[1,4]oxazino[2,3,4-ij]-quinoléine-6-carboxamide,
t) N- (4-chlorobenzyl)-2- (3-cyanophényl)-9-(morpholine-4-ylméthyl)-7-thioxo-2,3-dihydro-7H-[1,4]oxazino[2,3,4-ij]quinoléine-6-carboxamide,
u) N-(4-chlorobenzyl)-2-(3-furyl)-9-(morpholine-4-ylméthyl)-7-thioxo-2,3-dihydro-7H-[1,4]oxazino[2,3,4-ij]-quinoléine-6-carboxamide,
v) N-(4-chlorobenzyl)-9-(morpholine-4-ylméthyl)-7-thioxo-2-thiène-2-yl-2,3-dihydro-7H-[1,4]oxazino[2,3,4-ij]-quinoléine-6-carboxamide,
w) N-(4-chlorobenzyl)-2-(3,5-difluorophényl)-9-(morpholine-4-ylméthyl)-7-thioxo-2,3-dihydro-7H-[1,4]oxazino[2,3,4-ij]-quinoléine-6-carboxamide,
x) 2-(1,3-benzodioxol-5-yl)-N-(4-chlorobenzyl)-9-(morpholine-4-ylméthyl) -7-thioxo-2,3-dihydro-7H-[1,4]oxazino[2,3,4-ij]quinoléine-6-carboxamide,
y) N-(4-chlorobenzyl)-2-(2,3-dihydro-1,4-benzodioxine-6-yl)-9- (morpholine-4-ylméthyl)-7-thioxo-2,3-dihydro-7H-[1,4]oxazino[2,3,4-ij]quinoléine-6-carboxamide,
z) 2-(1,3-benzodioxol-4-yl)-N-(4-chlorobenzyl)-9-(morpholine-4-ylméthyl) -7-thioxo-2,3-dihydro-7H-[1,4]oxazino[2,3, 4-ij]quinoléine-6-carboxamide,
aa) 2-[3,5-bis(méthoxyméthoxy)phényl]-N-(4-chlorobenzyl)-9- (morpholine-4-ylméthyl)-7-thioxo-2,3-dihydro-7H-[1,4]oxazino[2,3,4-ij]quinoléine-6-carboxamide,
bb) N- (4-chlorobenzyl)-9- (morpholine-4-ylméthyl) -7-thioxo-2-thiène-3-yl-2,3-dihydro-7H-[1,4]oxazino[2,3,4-ij]quinoléine-6-carboxamide,
cc) N-(4-chlorobenzyl)-2,2-bis [ (méthoxyméthoxy)méthyl]-9- (morpholine-4-ylméthyl)-7-thioxo-2,3-dihydro-7H-[ 1 , 4 ]oxazino[2,3,4-ij]quinoléine-6-carboxamide,
dd) N-[(4-chlorophényl)méthyl]-9'-(4-morpholinylméthyl)-4-oxo-7'-thioxospiro[cyclohexane-1, 2' (3'*H*)-[7*H*]pyrido[1,2,3-*de*][1,4]benzoxazine]-6'-carboxamide,
e e ) N- [ (4-chlorophényl)méthyl ]-4-hydroxy-9'-(4-morpholinylméthyl)-7' -thioxospiro [cyclohexane-1, 2' (3' H) - [7*H*]pyrido[1,2,3-de] [1,4]benzoxazine]-6'-carboxamide,
ff) N-[(4-chlorobenzyl)-3,9-bis(morpholine-4-ylméthyl) -7-thioxo-2,3-dihydro-7H-[1,4]oxazino[2,3,4-ij]-quinoléine-6-carboxamide,
gg) N-(4-chlorobenzyl)-9-(morpholine-4-ylméthyl)-7-thioxo-2-phényl-2,3-dihydro-7H-[1,4]oxazino[2,3,4-ij]-quinoléine-6-carboxamide,
hh) N-(4-chlorobenzyl)-2,2-difluoro-9-(morpholine-4-ylméthyl)-7-thioxo-2,3-dihydro-7H-[1,4]oxazino[2,3,4-ij]-quinoléine-6-carboxamide,
ii) N-(4-chlorobenzyl)-2-[(méthylsulfanyl)méthyl]-9-(morpholine-4-ylméthyl)-7-thioxo-2,3-dihydro-7H-[1,4]oxazino[2,3,4-ij]quinoléine-6-carboxamide,
jj) N-(4-chlorobenzyl) -2- [ (diméthylamino)méthyl]-9- (morpholine-4-ylméthyl)-7-thioxo-2,3-dihydro-7H-[1,4]oxazino[2,3,4-ij]quinoléine-6-carboxamide,
kk) N-(4-chlorobenzyl)-2-[(4-méthyl-1-pipérazinyl)méthyl]-9- (morpholine-4-ylméthyl)-7-thioxo-2,3-dihydro-7H-[1,4]oxazino[2,3,4-ij]quinoléine-6-carboxamide,
ll) ({[6-{[ (4-chlorobenzyl)amino]carbonyl}-9-(morpholine-4-ylméthyl)-7-thioxo-2,3-dihydro-7H-[1,4]oxazino[2,3,4-ij]-quinoléine-2-yl]méthyl}thio)acétate de méthyle,
mm) N-(4-chlorobenzyl)-9-(morpholine-4-ylméthyl)-7-thioxo-2-(1-pyrrolidinylméthyl)-2,3-dihydro-7H-[1,4]oxazino[2,3,4-ij]quinoléine-6-carboxamide,
nn) N- (4-chlorobenzyl) -2-{[ (2,3-dihydroxypropyl)sulfanyl]méthyl}-9-(morpholine-4-ylméthyl)-7-thioxo-2,3-dihydro-7H-[1,4]-oxazino [2, 3, 4-ij]quinoléine-6-carboxamide,
oo) N-(4-chlorobenzyl)-2-{[(2,3-dihydroxypropyl)amino]méthyl}-9-(morpholine-4-ylméthyl)-7-thioxo-2,3-dihydro-7H-[1,4]-oxazino[2,3, 4-ij ]quinoléine-6-carboxamide,
pp) N- (4-chlorobenzyl)-2-{[(2-dihydroxypropyl)amino]méthyl}-9-(morpholine-4-ylméthyl)-7-thioxo-2,3-dihydro-7H-[1,4]-oxazino[2, 3, 4-ij]quinoléine-6-carboxamide,
qq) N- (4-chlorobenzyl) -9- (morpholine-4-ylméthyl)-7-thioxo-2-(1-pipéridinylméthyl)-2,3-dihydro-7H-[1,4]oxazino[2,3,4-ij]quinoléine-6-carboxamide,
rr) 2-{[bis(2-hydroxyéthyl)amino]méthyl}-N-(4-chlorobenzyl)-9-(morpholine-4-ylméthyl)-7-thioxo-2,3-dihydro-7H-[1,4]-oxazino [2, 3, 4-ij]quinoléine-6-carboxamide,
ss) N-(4-chlorobenzyl)-9-(morpholine-4-ylméthyl)-7-thioxo-2-{[(2-pyridinylméthyl)amino]méthyl}-2,3-dihydro-7H-[1,4]oxazino[2,3,4-ij]quinoléine-6-carboxamide,
tt) sel de sodium d'acide 2-[(8-{[6-{[(4-chlorobenzyl)-amino]carbonyl}-9-(morpholine-4-ylméthyl)-7-thioxo-2,3-dihydro-7H-[1,4]oxazino[2,3,4-ij]quinoléine-2-yl]méthoxy}-8-oxo-octanoyl) (méthyl)amino]éthanesulfonique,
uu) [6-{[(4-chlorobenzyl)amino]carbonyl}-9-(morpholine-4-ylméthyl)-7-thioxo-2,3-dihydro-7H-[1,4]oxazino[2,3,4-ij]-quinoléine-2-yl]méthyl-diméthyl-phosphate,
vv) N-(4-chlorobenzyl)-9-(morpholine-4-ylméthyl)-7-thioxo-2-{[(4-pyridinylméthyl)amino]méthyl}-2,3-dihydro-7H-[1,4]-oxa zi no [2, 3, 4-ij]quinoléine-6-car-boxamide,
ww) N-(4-chlorobenzyl)-2-(1H-imidazol-1-ylméthyl)-9-(morpholine-4-ylméthyl)-7-thioxo-2,3-dihydro-7H-[1,4]oxazino[2,3,4-ij]quinoléine-6-carboxamide,
xx) N-(4-chlorobenzyl)-2-{[(4-chlorobenzyl)amino]méthyl}-9-(morpholine-4-ylméthyl)-7-thioxo-2,3-dihydro-7H-[1,4]oxazino[2,3,4-ij]quinoléine-6-carboxamide,
yy) N- (4-chlorobenzyl)-3-(hydroxyméthyl)-9-(morpholine-4-ylméthyl)-7-thioxo-2,3-dihydro-7H-[1,4]oxazino[2,3,4-ij]quinoléine-6-carboxamide,
zz) N-(4-chlorobenzyl)-2-(4-hydroxyphényl)-9-(morpholine-4-ylméthyl) -7-thioxo-2,3-dihydro-7H-[1,4]oxazino[2,3,4-ij]-quinoléine-6-carboxamide,
aaa) N-(4-chlorobenzyl)-2-{3-[(méthoxyméthoxy)méthyl]-phényl}-9-(morpholine-4-ylméthyl)-7-thioxo-2,3-dihydro-7H-[1,4]oxazino[2,3,9-ij]quinoléine-6-carboxamide,
bbb) N-(4-chlorobenzyl)-2-{2-[(méthoxyméthoxy)méthyl]phényl}-9-(morpholine-4-ylméthyl)-7-thioxo-2,3-dihydro-7H-[1,4]oxazino[2, 3,4-ij]quinoléine-6-carboxamide,
ccc) N- (4-chlorobenzyl) -2-(2-hydroxyphényl)-9- (morpholine-4-ylméthyl) -7-thioxo-2,3-dihydro-7H-[1,4]oxazino[2,3,4-ij]-quinoléine-6-carboxamide,
ddd) N- [(4-chlorophényl)méthyl]-2,3,5,6-tétrahydro-9'-(4-morpholinylméthyl)-7'-thioxospiro[4H-pyranne-4,2'(3'*H*)-[7*H*]-pyrido[1,2,3-*de*][1,4]benzoxazine]-6'-carboxamide,
eee) 6- [ [ [ (4-chlorophényl)méthyl]amino]carbonyl]-9'-(4-morpholinylméthyl) -7'-thioxospiro[pipéridine-4,2' (3'*H*) *-* [7*H*]pyrido[1,2,3-*de*]-[1,4]benzoxazine]-1-carboxylate de 1,1-diméthyléthyle,
fff) N-[(4-chlorophényl)méthyl]-9'-(4-morpholinylméthyl)-7'-thioxospiro[pipéridine-4,2'(3'*H*)-[7*H*]pyrido[1,2,3-*de*][1,4]-benzoxazine]-6'-carboxamide,
ggg) N-(4-chlorobenzyl)-2,2-bis(hydroxyméthyl)-9-(morpholine-4-ylméthyl) -7-thioxo-2,3-dihydro-7*H*-[1,4]oxazino[2,3,4-ij]-quinoléine-6-carboxamide,
hhh) N-[(4-chlorophényl)méthyl]-2',3',5',6'-tétrahydro-9-(4-morpholinylméthyl)-7-thioxospiro[7*H*-pyrido[1,2,3-*de*]-1,4-benzoxazine-2(3*H*),4'-[4H]thiopyranne]-6-carboxamide,
iii) N-(4-chlorobenzyl)-9-(morpholine-4-ylméthyl)-7-thioxo-3-phényl-2,3-dihydro-7H-[1,4]oxazino[2,3,4-ij]-quinoléine-6-carboxamide,
jjj) N-(4-chlorobenzyl)-3,3-bis(hydroxyméthyl)-9-(3-hydroxy-1-propanyl)-7-thioxo-2,3-dihydro-7H-[1,4]-oxazino[2,3,4-ij]quinoléine-6-carboxamide,
kkk) N-(4-chlorobenzyl)-3,3-bis(hydroxyméthyl)-9-(3-hydroxypropyl)-7-thioxo-2,3-dihydro-7H-[1,4]oxazino[2,3,4-ij]-quinoléine-6-carboxamide,
lll) N- (4-chlorobenzyl)-2- [2- (méthoxyméthoxy) phényl]-9-(morpholine-4-ylméthyl) -7-thioxo-2,3-dihydro-7H-[1,4]oxazino[2,3,4-ij]quinoléine-6-carboxamide,
mmm) N-(4-chlorobenzyl)-2-{4-[(méthoxyméthoxy)méthyl]-phényl}-9-(morpholine-4-ylméthyl)-7-thioxo-2,3-dihydro-7H-[1,4] oxazino[2,3,4-ij]quinoléine-6-carboxamide,
nnn) 2-[2,3-bis(méthoxyméthoxy)phényl]-N-(4-chlorobenzyl)-9- (morpholine-4-ylméthyl)-7-thioxo-2,3-dihydro-7H-[1,4]-oxazino[2,3,4-ij]quinoléine-6-carboxamide,
ooo) N-[(4-chlorophényl)méthyl]-1-méthyl-9'-(4-morpholinylméthyl)-7'-thioxospiro[pipéridine-4,2' (3*H*)-[7*H*]-pyrido[1,2,3-*de*][1,4]benzoxazine]-6'-carboxamide ou un de ses sels pharmaceutiquement acceptables.

**70.** Composé suivant la revendication 1, qui est le
a) N-(4-chlorobenzyl)-2-(hydroxyméthyl)-9-(morpholine-4-yl-méthyl)-7-oxo-2,3-dihydro-7H-[1,4]oxazino[2,3,4-ij]-quinoléine-6-carboxamide,
b) N-(4-chlorobenzyl)-2-(*R* ou *S*)-(hydroxyméthyl)-9-(morpholine-4-ylméthyl)-7-oxo-2,3-dihydro-7H-[1,4]oxazino[2,3,4-ij]quinoléine-6-carboxamide,
c) N-(4-chlorobenzyl)-9- (morpholine-4-ylméthyl) -7-oxo-2-pyridine-3-yl-2,3-dihydro-7H-[1,4]oxazino[2,3,4-ij]-quinoléine-6-carboxamide,
d) N-(4-chlorobenzyl)-9- (morpholine-4-ylméthyl)-7-oxo-2-pyridine-4-yl-2,3-dihydro-7H-[1,4]oxazino[2,3,4-ij]-quinoléine-6-carboxamide,
e) N-(4-chlorobenzyl)-9-(morpholine-4-ylméthyl)-7-oxo-2-pyridine-2-yl-2,3-dihydro-7H-[1,4]oxazino[2,3,4-ij]-quinoléine-6-carboxamide,
f) N-(4-chlorobenzyl)-9-(morpholine-4-ylméthyl)-7-oxo-2-(*R* ou *S*)-pyridine-3-yl-2,3-dihydro-7H-[1,4]oxazino[2,3,4-ij]-quinoléine-6-carboxamide,
g) N-(4-chlorobenzyl)-2,9-bis(morpholine-4-ylméthyl)-7-oxo-2,3-dihydro-7H-[1,4]oxazino[2,3,4-ij]quinoléine-6-carboxamide,
h) 2-[(*tertio*butylsulfanyl)méthyl]-N-(4-chlorobenzyl)-9-(morpholine-4-ylméthyl)-7-oxo-2,3-dihydro-7H-[1,4]oxazino[2,3,4-ij]quinoléine-6-carboxamide,
i) N-(4-chlorobenzyl)-2-{[(2-hydroxyéthyl)sulfanyl]méthyl}-9-(morpholine-4-ylméthyl)-7-oxo-2,3-dihydro-7H-[1,4]oxazino[2,3,4-ij]quinoléine-6-carboxamide,
j ) N-(4-chlorobenzyl)-2-{[(1-méthyl-1H-imidazol-2-yl)sulfanyl]-méthyl}-9-(morpholine-4-ylméthyl)-7-oxo-2, 3-dihydro-7H-[1,4]oxazino[2,3,4-ij]quinoléine-6-carboxamide,
k) N-(4-chlorobenzyl)-9-(morpholine-4-ylméthyl)-7-oxo-2-{[(3-pyridinylméthyl)amino]méthyl)-2,3-dihydro-7H-[1,4]-oxazino[2,3,4-ij]quinoléine-6-carboxamide,
l) acétate de [6-{[(4-chlorobenzyl)amino]carbonyl}-9-(morpholine-4-ylméthyl)-7-oxo-2,3-dihydro-7H-[1,4]-oxazino[2,3,4-ij]quinoléine-2-yl]méthyle,
m) N-(4-chlorobenzyl)-9-(morpholine-4-ylméthyl)-7-oxo-2-(*R* ou *S*)-{[(3-pyridinylméthyl)amino]méthyl)-2,3-dihydro-7H-[1,4]-oxazino [2,3,4-ij]quinoléine-6-carboxamide,
n) N-(4-chlorobenzyl)-2-(3-hydroxyphényl)-9-(morpholine-4-ylméthyl)-7-oxo-2,3-dihydro-7H-[1,4]oxazino[2,3,4-ij]-quinoléine-6-carboxamide,
o) N-(4-chlorobenzyl)-9-(morpholine-4-ylméthyl)-7-oxo-2-(*R* ou *S*)-pyridine-2yl-2,3-dihydro-7H-[1,4]oxazino[2,3,4-ij]-quinoléine-6-carboxamide,
p) N-(4-chlorobenzyl)-2-[3-(hydroxyméthyl)phényl]-9-(morpholine-4-ylméthyl)-7-oxo-2,3-dihydro-7H-[1,4]oxazino[2,3, 4-ij]quinoléine-6-carboxamide,
q) N-(4-chlorobenzyl)-2-[2-(hydroxyméthyl)phényl]-9-(morpholine-4-ylméthyl)-7-oxo-2,3-dihydro-7H-[1,4]-oxazino[2,3,4-ij]quinoléine-6-carboxamide,
r) N-(4-chlorobenzyl)-2-(1-méthyl-1H-imidazol-2-yl)-9-(morpholine-4-ylméthyl)-7-oxo-2,3-dihydro-7H-[1,4]oxazino[2,3,4-ij]quinoléine-6-carboxamide,
s) N-(4-chlorobenzyl)-2-(2-furyl)-9-(morpholine-4-ylméthyl)-7-oxo-2,3-dihydro-7H-[1, 4]oxazino[2, 3, 4-ij ]quinoléine-6-carboxamide,
t) N-(4-chlorobenzyl)-2-(3-cyanophényl)-9-(morpholine-4-ylméthyl)-7-oxo-2,3-dihydro-7H-[1,4]oxazino[2,3,4-ij]quinoléine-6-carboxamide,
u) N-(4-chlorobenzyl)-2-(3-furyl)-9-(morpholine-4-ylméthyl)-7-oxo-2,3-dihydro-7H-[1,4]oxazino[2,3,4-ij]quinoléine-6-carboxamide,
v) N-(4-chlorobenzyl)-9-(morpholine-4-ylméthyl)-7-oxo-2-thiène-2-yl-2,3-dihydro-7H-[1,4]oxazino[2,3,4-ij]-quinoléine-6-carboxamide,
w) N-(4-chlorobenzyl)-2-(3,5-difluorophényl)-9-(morpholine-4-ylméthyl)-7-oxo-2,3-dihydro-7H-[1,4]oxazino[2,3,4-ij]quinoléine-6-carboxamide,
x) 2-(1,3-benzodioxol-5-yl)-N-(4-chlorobenzyl)-9-(morpholine-4-ylméthyl)-7-oxo-2,3-dihydro-7H-[1,4]oxazino[2,3,4-ij]quinoléine-6-carboxamide,
y) N-(4-chlorobenzyl)-2-(2,3-dihydro-1,4-benzodioxine-6-yl)-9-(morpholine-4-ylméthyl)-7-oxo-2,3-dihydro-7H-[1,4]-oxazino[2, 3, 4-ij]quinoléine-6-carboxamide,
z) 2-(1,3-benzodioxol-4-yl)-N-(4-chlorobenzyl)-9-(morpholine-4-ylméthyl)-7-oxo-2,3-dihydro-7H-[1,4]-oxazino[2,3,4-ij]quinoléine-6-carboxamide,
aa) 2-[3,5-bis(méthoxyméthoxy)phényl]-N-(4-chlorobenzyl)-9- (morpholine-4-ylméthyl)-7-oxo-2,3-dihydro-7H-[1,4]-oxazino [2, 3, 4-ij ]quinoléine-6-carboxamide,
bb) N-(4-chlorobenzyl)-9-(morpholine-4-ylméthyl)-7-oxo-2-thiène-3-yl-2,3-dihydro-7H-[1,4]oxazino[2,3,4-ij]-quinoléine-6-carboxamide,
cc) N-(4-chlorobenzyl)-2, 2-bis [ (méthoxyméthoxy)méthyl]-9- (morpholine-4-ylméthyl)-7-oxo-2,3-dihydro-7H-[1,4]-oxazino[2,3,4-ij]quinoléine-6-carboxamide,
dd) N-[(4-chlorophényl)méthyl]-9'-(4-morpholinylméthyl)-4,7'-dioxospiro [cyclohexane-1, 2'(3' H) - [7*H*]pyrido[1,2,3-de]-[1,4]benzoxazine]-6'-carboxamide,
ee ) N- [ (4-chlorophényl)méthyl]-4-hydroxy-9'-(4-morpholinylméthyl)-7'-oxospiro[cyclohexane-1,2'(3'*H*)-[7*H*]pyrido[1,2,3-*de*]-[1,4]benzoxazine]-6'-carboxamide,
ff) N-(4-chlorobenzyl)-3,9-bis(morpholine-4-ylméthyl)-7-oxo-2,3-dihydro-7H-[1,4]oxazino[2,3,4-ij]quinoléine-6-carboxamide,
gg) N-(4-chlorobenzyl)-9-(morpholine-9-ylméthyl)-7-oxo-2-phényl-2 , 3-dihydro-7H-[1,4]oxazino[2,3,4-ij]quinoléine-6-carboxamide,
hh) N-(4-chlorobenzyl)-2,2-difluoro-9-(morpholine-4-ylméthyl)-7-oxo-2,3-dihydro-7H-[1,4]oxazino[2,3, 4-ij ]quinoléine-6-carboxamide,
ii) N- (4-chlorobenzyl) -2- [(méthylsulfanyl)méthyl]-9-(morpholine-4-ylméthyl)-7-oxo-2,3-dihydro-7H-[1,4]oxazino[2,3,4-ij]-quinoléine-6-carboxamide,
jj) N- (4-chlorobenzyl) -2- [(diméthylamino)méthyl]-9-(morpholine-4-ylméthyl)-7-oxo-2,3-dihydro-7H-[1,4]oxazino[2,3,4-ij]-quinoléine-6-carboxamide,
kk) N-(4-chlorobenzyl)-2-[(4-méthyl-1-pipérazinyl)méthyl]-9- (morpholine-4-ylméthyl)-7-oxo-2,3-dihydro-7H-[1,4]oxazino[ 2, 3, 4-ij]quinoléine-6-carboxamide,
ll) ({[6-{[(4-chlorobenzyl)amino]carbonyl}-9-(morpholine-4-ylméthyl)-7-oxo-2,3-dihydro-7H-[1,4]oxazino[2,3,4-ij]-quinoléine-2-yl]méthyl}thio)acétate de méthyle,
mm) N-(9-chlorobenzyl)-9-(morpholine-4-ylméthyl)-7-oxo-2-(1-pyrrolidinylméthyl)-2,3-dihydro-7H-[1,4]oxazino[2,3,4-ij]quinoléine-6-carboxamide,
nn) N- (4-chlorobenzyl) -2-{[(2,3-dihydroxypropyl)sulfanyl]méthyl}-9-(morpholine-4-ylméthyl)-7-oxo-2,3-dihydro-7H-[1,4]-oxazino [2,3,4-ij]quinoléine-6-carboxamide,
oo) N-(4-chlorobenzyl)-2-{[(2,3-dihydroxypropyl)amino]méthyl}-9-(morpholine-4-ylméthyl)-7-oxo-2,3-dihydro-7H-[1,4]oxazino[2, 3, 4-ij]quinoléine-6-carboxamide,
pp) N-(4-chlorobenzyl)-2-{[(2-hydroxyéthyl)amino]méthyl}-9-(morpholine-4-ylméthyl)-7-oxo-2,3-dihydro-7H-[1,4]oxazino[2, 3, 4-ij]quinoléine-6-carboxamide,
qq) N-(4-chlorobenzyl)-9-(morpholine-4-ylméthyl)-7-oxo-2-(1-pipéridinylméthyl)-2,3-dihydro-7H-[1,4]oxazino[2,3,4-ij] quinoléine-6-carboxamide,
rr) 2-{[bis(2-hydroxyéthyl)amino]méthyl}-N-(4-chlorobenzyl)-9-(morpholine-4-ylméthyl)-7-oxo-2,3-dihydro-7H-[1,4]-oxazino[2,3,4-ij]quinoléine-6-carboxamide,
ss) N-(4-chlorobenzyl)-9-(morpholine-4-ylméthyl)-7-oxo-2-{[2-pipéridinylméthyl)amino]méthyl}-2,3-dihydro-7H-[1,4]oxazino[2,3,4-ij]quinoléine-6-carboxamide,
tt) sel de sodium d'acide 2-[(8-{[6-{[(4-chlorobenzyl)-amino]carbonyl}-9-(morpholine-4-ylméthyl)-7-oxo-2,3-dihydro-7H- [1,4]oxazino[2,3,4-ij]quinoléine-2-yl]méthoxy}-8-oxo-octanoyl)(méthyl)amino]éthanesulfonique,
uu) [6-{[(4-chlorobenzyl)amino]carbonyl}-9-(morpholine-4-ylméthyl)-7-oxo-2,3-dihydro-7H-[1,4]oxazino[2,3,4-ij]-quinoléine-2-yl]méthyl-diméthyl-phosphate,
vv) N-(4-chlorobenzyl)-9-(morpholine-4-ylméthyl)-7-oxo-2-{[(4-pyridinylméthyl)amino]méthyl}-2,3-dihydro-7H-[1,4]-oxazino[2,3,4-ij]quinoléine-6-carboxamide,
ww) N- (4-chlorobenzyl) -2- (1H-imidazol-1-ylméthyl) -9- (morpholine-4-ylméthyl)-7-oxo-2,3-dihydro-7H-[1,4]oxazino[2,3,4-ij]-quinoléine-6-carboxamide,
xx) N- (4-chlorobenzyl) -2-{[(4-chlorobenzyl)amino]méthyl}-9-(morpholine-4-ylméthyl) -7-oxo-2,3-dihydro-7H-[1,4]-oxazino [2, 3,4-ij]quinoléine-6-carboxamide,
yy) N- (4-chlorobenzyl)-3-(hydroxyméthyl)-9-(morpholine-4-ylméthyl)-7-oxo-2,3-dihydro-7H-[1,4]oxazino[2,3,4-ij]quinoléine-6-carboxamide,
zz) N-(4-chlorobenzyl)-2-(4-hydroxyphényl)-9-(morpholine-4-ylméthyl)-7-oxo-2,3-dihydro-7H-[1,4]oxazino[2,3,4-ij]quinoléine-6-carboxamide,
aaa) N-(4-chlorobenzyl)-2-{3-[(méthoxyméthoxy)méthyl]-phényl}-9-(morpholine-4-ylméthyl)-7-oxo-2,3-dihydro-7H-[1,4]oxazino[2,3,4-ij]quinoléine-6-carboxamide,
bbb) N-(4-chlorobenzyl)-2-{2-[(méthoxyméthoxy)méthyl]-phényl}-9-(morpholine-4-ylméthyl)-7-oxo-2,3-dihydro-7H-[1,4]oxazino[2,3,4-ij]quinoléine-6-carboxamide,
ccc) N-(4-chlorobenzyl)-2-(2-hydroxyphényl)-9-(morpholine-4-ylméthyl)-7-oxo-2,3-dihydro-7H-[1,4]oxazino[2,3,4-ij]quinoléine-6-carboxamide,
ddd) N-[(4-chlorophényl)méthyl]-2,3,5,6-tétrahydro-9'-(4-morpholinylméthyl)-7'-oxospiro[4*H*-pyranne-4,2'(3'*H*)-[7*H*]-pyrido[1,2,3-de][1,4]benzoxazine]-6'-carboxamide,
eee) 6-[[[4-chlorophényl)méthyl]amino]carbonyl]-9'-(4-morpholinylméthyl)-7'-oxospiro[pipéridine-4 , 2' (3*H*)-[7*H*]pyrido[1,2,3-*de*]-[1,4]benzoxazine]-1-carboxylate de 1,1-diméthyléthyle,
fff) N-[(4-chlorophényl)méthyl)-9'-(4-morpholinylméthyl)-7'-oxo-spiro[pipéridine-4,2'(3'*H*)-[7*H*]pyrido[1,2,3-*de*][1,4]-benzoxazine]-6'-carboxamide,
ggg) N-(4-chlorobenzyl)-2,2-bis(hydroxyméthyl)-9-(morpholine-4-ylméthyl)-7-oxo-2,3-dihydro-7H-[1, 4]-oxazino[2,3,4-ij]quinoléine-6-carboxamide,
hhh) N-[(4-chlorophényl)méthyl]-2',3',5',6'-tétrahydro-9-(4-morpholinylméthyl)-7-oxospiro[7*H*-pyrido[1,2,3-*de*]-1,4-benzoxazine-2(3*H*),4'-[4H]thiopyranne]-6-carboxamide ou un de ses sels pharmaceutiquement acceptables.

**71.** Composé suivant la revendication 1, qui est le
a) N-(4-chlorobenzyl)-2-(hydroxyméthyl)-9-(morpholine-9-ylméthyl)-7-thioxo-2,3-dihydro-7H-[1,4]oxazino[2,3,4-ij]quinoléine-6-carboxamide,
b) N-(4-chlorobenzyl)-2-(*R* ou *S*)-(hydroxyméthyl)-9-(morpholine-4-ylméthyl)-7-thioxo-2,3-dihydro-7H-[1,4]oxazino[ 2, 3, 4-ij]quinoléine-6-carboxamide,
c) N-(4-chlorobenzyl)-9-(morpholine-4-ylméthyl)-7-thioxo-2-pyridine-3-yl-2,3-dihydro-7H-[1,4]oxazino[2,3,4-ij] quinoléine-6-carboxamide,
d) N-(4-chlorobenzyl)-9-(morpholine-4-ylméthyl)-7-thioxo-2-pyridine-4-yl-2,3-dihydro-7H-[1,4]oxazino[2,3,4-ij]quinoléine-6-carboxamide,
e) N- (4-chlorobenzyl) -9- (morpholine-4-ylméthyl)-7-thioxo-2-pyridine-2-yl-2,3-dihydro-7H-[1,4]oxazino[2,3,4-ij] quinoléine-6-carboxamide,
f ) N- (4-chlorobenzyl)-9-(morpholine-4-ylméthyl)-7-thioxo-2-(*R* ou *S*)-pyridine-3-yl-2,3-dihydro-7H-[1,4]oxazino[2,3,4-ij]quinoléine-6-carboxamide,
g) N-(4-chlorobenzyl)-2,9-bis(morpholine-4-ylméthyl)-7-thioxo-2,3-dihydro-7H-[1,4]oxazino[2,3,4-ij]-quinoléine-6-carboxamide,
h) 2- [(*tertio*butylsulfanyl)méthyl]-N-(4-chlorobenzyl)-9- (morpholine-4-ylméthyl)-7-thioxo-2,3-dihydro-7H-[1,4]-oxazino[2,3,4-ij]quinoléine-6-carboxamide,
i) N-(4-chlorobenzyl )-2-{[(2-hydroxyéthyl)sulfanyl]méthyl}-9-(morpholine-4-ylméthyl)-7-thioxo-2,3-dihydro-7H-[1,4]oxazino[2,3,4-ij]quinoléine-6-carboxamide,
j ) N-(4-chlorobenzyl)-2-{[(1-méthyl-1H-imidazol-2-yl)sulfanyl]-méthyl }-9-(morpholine-4-ylméthyl)-7-thioxo-2, 3-dihydro-7H- [ 1,4]oxazino[2, 3, 4-ij]quinoléine-6-carboxamide,
k) N- (4-chlorobenzyl)-9- (morpholine-4-ylméthyl)-7-thioxo-2-{[(3-pyridinylméthyl)amino]méthyl}-2,3-dihydro-7H-[1,4]oxazino[2,3,4-ij]quinoléine-6-carboxamide,
l) acétate de [6-{[(4-chlorobenzyl)amino]carbonyl}-9-(morpholine-4-ylméthyl)-7-thioxo-2,3-dihydro-7H-[1,4]oxazino[2, 3,4-ij]quinoléine-2-yl]méthyle,
m) N- (4-chlorobenzyl)-9-(morpholine-4-ylméthyl)-7-thioxo-2-(*R* ou *S*)-([(3-pyridinylméthyl)amino]méthyl)-2,3-dihydro-7H-[1,4] oxazino[2, 3, 4-ij]quinoléine-6-carboxamide,
n) N-(4-chlorobenzyl)-2-(3-hydroxyphényl)-9-(morpholine-4-ylméthyl)-7-thioxo-2,3-dihydro-7H-[1,4]oxazino[2,3,4-ij]quinoléine-6-carboxamide,
o) N-(4-chlorobenzyl)-9-(morpholine-4-ylméthyl)-7-thioxo-2-(*R* ou *S*) -pyridine-2yl-2,3-dihydro-7H-[1,4]oxazino[2, 3, 4-ij]quino-léine-6-carboxamide,
p) N-(4-chlorobenzyl)-2-[3-(hydroxyméthyl)phényl]-9- (morpholine-4-ylméthyl)-7-thioxo-2,3-dihydro-7H-[1,4]-oxazino [ 2, 3, 4-ij]quinoléine-6-carboxamide,
q) N-(4-chlorobenzyl)-2-[2-(hydroxyméthyl)phényl]-9-(morpholine-4-ylméthyl)-7-thioxo-2,3-dihydro-7H-[1,4]oxazino[2, 3, 4-ij]quinoléine-6-carboxamide,
r) N-(4-chlorobenzyl)-2-(1-méthyl-1H-imidazol-2-yl)-9-(morpholine-4-ylméthyl ) -7-thioxo-2,3-dihydro-7H-[1,4]-oxazino[2,3,4-ij]quinoléine-6-carboxamide,
s) N- (4-chlorobenzyl)-2-(2-furyl)-9-(morpholine-4-ylméthyl)-7-thioxo-2,3-dihydro-7H-[1,4]oxazino[2,3,4-ij]-quinoléine-6-carboxamide,
t) N- (4-chlorobenzyl)-2-(3-cyanophényl)-9-(morpholine-4-ylméthyl ) -7-thioxo-2,3-dihydro-7H-[1,4]oxazino[2,3,4-ij] quinoléine-6-carboxamide,
u) N-(4-chlorobenzyl)-2-(3-furyl)-9-(morpholine-4-ylméthyl)-7-thioxo-2,3-dihydro-7H-[1,4]oxazino[2,3,4-ij]-quinoléine-6-carboxamide,
v) N- (4-chlorobenzyl)-9-(morpholine-4-ylméthyl)-7-thioxo-2-thiène-2-yl-2,3-dihydro-7H-[1,4]oxazino[2,3,4-ij]quinoléine-6-carboxamide,
w) N- (4-chlorobenzyl)-2-(3, 5-difluorophényl)-9-(morpholine-4-ylméthyl)-7-thioxo-2,3-dihydro-7H-[1,4]oxazino[2,3,4-ij]quinoléine-6-carboxamide,
x) 2-(1,3-benzodioxol-5-yl)-N-(4-chlorobenzyl)-9-(morpholine-4-ylméthyl)-7-thioxo-2,3-dihydro-7H-[1,4]oxazino[2,3,4-ij]quinoléine-6-carboxamide,
y) N-(4-chlorobenzyl)-2-(2,3-dihydro-1,4-benzodioxine-6-yl)-9-(morpholine-4-ylméthyl)-7-thioxo-2,3-dihydro-7H-[1,4]oxazino[2,3,4-ij]quinoléine-6-carboxamide,
z) 2-(1,3-benzodioxol-4-yl)-N-(4-chlorobenzyl)-9-(morpholine-4-ylméthyl) -7-thioxo-2,3-dihydro-7H-[1,4]oxazino[2,3,4-ij]quinoléine-6-carboxamide,
aa) 2-[3,5-bis(méthoxyméthoxy)phényl]-N-(4-chlorobenzyl)-9- (morpholine-4-ylméthyl) -7-thioxo-2,3-dihydro-7H-[1,4]oxazino[2,3,4-ij]quinoléine-6-carboxamide,
bb) N- (4-chlorobenzyl) -9- (morpholine-4-ylméthyl)-7-thioxo-2-thiène-3-yl-2,3-dihydro-7H-[1,4]oxazino[2,3,4-ij]quinoléine-6-carboxamide,
cc) N-(4-chlorobenzyl)-2, 2-bis [ (méthoxyméthoxy)méthyl]-9- (morpholine-4-ylméthyl)-7-thioxo-2,3-dihydro-7H-[1,4]oxazino[2,3,4-ij]quinoléine-6-carboxamide,
dd) N-[(4-chlorophényl)méthyl]-9'-(4-morpholinylméthyl)-4-oxo-7'-thioxospiro[cyclohexane-1,2'(3' *H*) - [7*H*]pyrido[1,2,3-*de*][1, 4]benzoxazine]-6'-carboxamide,
ee) N- [(4-chlorophényl)méthyl]-4-hydroxy-9'-(4-morpholinylméthyl)-7'-thioxospiro[cyclohexane-1,2'(3'*H*)-[7*H*]-pyrido[1,2,3-*de*][1,4]benzoxazine]-6'-carboxamide,
ff) N-[(4-chlorobenzyl)-3,9-bis(morpholine-4-ylméthyl)-7-thioxo-2,3-dihydro-7H-[1,4]oxazino[2,3,4-ij]-quinoléine-6-carboxamide,
gg) N-(4-chlorobenzyl)-9-(morpholine-4-ylméthyl)-7-thioxo-2-phényl-2,3-dihydro-7H-[1,4]oxazino[2,3,4-ij] quinoléine-6-carboxamide,
hh) N-(4-chlorobenzyl)-2,2-difluoro-9-(morpholine-4-ylméthyl)-7-thioxo-2,3-dihydro-7H-[1,4]oxazino[2,3,4-ij]-quinoléine-6-carboxamide,
ii) N-(4-chlorobenzyl)-2-[(méthylsulfanyl)méthyl]-9-(morpholine-4-ylméthyl)-7-thioxo-2,3-dihydro-7H-[1,4]oxazino[2,3,4-ij]quinoléine-6-carboxamide,
jj) N-(4-chlorobenzyl)-2-[(diméthylamino)méthyl]-9-(morpholine-4-ylméthyl)-7-thioxo-2,3-dihydro-7H-[1,4]oxazino[2,3,4-ij]quinoléine-6-carboxamide,
kk) N-(4-chlorobenzyl)-2-[(4-méthyl-1-pipérazinyl)méthyl]-9-(morpholine-4-ylméthyl)-7-thioxo-2,3-dihydro-7H-[1,4]oxazino[2,3,4-ij]quinoléine-6-carboxamide,
ll) ({[6-{[(4-chlorobenzyl)amino]carbonyl}-9-(morpholine-4-ylméthyl)-7-thioxo-2,3-dihydro-7H-[1,4]oxazino[2,3,4-ij]quinoléine-2-yl]méthyl}thio)acétate de méthyle,
mm) N-(4-chlorobenzyl)-9-(morpholine-4-ylméthyl)-7-thioxo-2-(1-pyrrolidinylméthyl)-2,3-dihydro-7H-[1,4]oxazino[2,3,4-ij]quinoléine-6-carboxamide,
nn) N-(4-chlorobenzyl)-2-{[(2,3-dihydroxypropyl)sulfanyl]méthyl}-9- (morpholine-4-ylméthyl)-7-thioxo-2,3-dihydro-7H-[1,4]-oxazino[2,3,4-ij]quinoléine-6-carboxamide,
oo) N-(4-chlorobenzyl)-2-{[(2,3-dihydroxypropyl)amino]méthyl}-9-(morpholine-4-ylméthyl)-7-thioxo-2,3-dihydro-7H-[1,4]oxazino[2,3,4-ij]quinoléine-6-carboxamide,
pp) N-(4-chlorobenzyl)-2-{[(2-dihydroxyéthyl)amino]méthyl}-9-(morpholine-4-ylméthyl)-7-thioxo-2,3-dihydro-7H-[1,4]-oxazino[2,3,4-ij]quinoléine-6-carboxamide,
qq) N-(4-chlorobenzyl)-9-(morpholine-4-ylméthyl)-7-thioxo-2-(1-pipéridinylméthyl)-2,3-dihydro-7H-[1,4]oxazino[2,3,4-ij]quinoléine-6-carboxamide,
rr) 2-{[bis(2-hydroxyéthyl)amino]méthyl}-N-(4-chlorobenzyl)-9- (morpholine-4-ylméthyl) -7-thioxo-2,3-dihydro-7H-[1,4]oxazino[2,3,4-ij] quinoléine-6-carboxamide,
ss) N-(4-chlorobenzyl)-9-(morpholine-4-ylméthyl)-7-thioxo-2-{[(2-pyridinylméthyl)amino]méthyl}-2,3-dihydro-7H-[1,4]oxazino[2,3,4-ij]quinoléine-6-carboxamide,
tt) sel de sodium d'acide 2-[(8-{[6-{[(4-chlorobenzyl)-amino]carbonyl}-9-(morpholine-4-ylméthyl)-7-thioxo-2,3-dihydro-7H-[1,4]oxazino[2,3,4-ij]quinoléine-2-yl]méthoxy}-8-oxo-octanoyl) (méthyl)amino]éthanesulfonique,
uu) [6-{[(4-chlorobenzyl)amino]carbonyl}-9-(morpholine-4-ylméthyl) -7-thioxo-2,3-dihydro-7H-[1,4]oxazino[2,3,4-ij]-quinoléine-2-yl]méthyl-diméthyl-phosphate,
vv) N-(4-chlorobenzyl)-9-(morpholine-4-ylméthyl)-7-thioxo-2-{[(4-pyridinylméthyl)amino]méthyl}-2,3-dihydro-7H-[1,4]oxazino[2, 3,4-ij]quinoléine-6-carboxamide,
ww) N-(4-chlorobenzyl)-2-(1H-imidazol-1-ylméthyl)-9-(morpholine-4-ylméthyl)-7-thioxo-2,3-dihydro-7H-[1,4]oxazino[2,3,4-ij]quinoléine-6-carboxamide,
xx) N- (4-chlorobenzyl)-2- {[(4-chlorobenzyl)amino]méthyl}-9-(morpholine-4-ylméthyl)-7-thioxo-2,3-dihydro-7H-[1,4]-oxazino[2,3,4-ij]quinoléine-6-carboxamide,
yy) N- (4-chlorobenzyl) -3-(hydroxyméthyl)-9-(morpholine-4-ylméthyl)-7-thioxo-2,3-dihydro-7H-[1,4]oxazino[2,3,4-ij]quinoléine-6-carboxamide,
zz) N-(4-chlorobenzyl)-2-(4-hydroxyphényl)-9-(morpholine-4-ylméthyl)-7-thioxo-2,3-dihydro-7H-[1,4]oxazino[2,3,4-ij]quinoléine-6-carboxamide,
aaa) N- (4-chlorobenzyl)-2-{3-[(méthoxyméthoxy)méthyl]phényl}-9-(morpholine-4-ylméthyl)-7-thioxo-2,3-dihydro-7H-[1,4]oxazino[2,3, 4-ij]quinoléine-6-carboxamide,
bbb) N-(4-chlorobenzyl)-2-{2-[(méthoxyméthoxy)méthyl]phényl}-9-(morpholine-4-ylméthyl)-7-thioxo-2,3-dihydro-7H-[1,4]oxazino[2,3,4-ij]quinoléine-6-carboxamide,
ccc) N- (4-chlorobenzyl)-2- (2-hydroxyphényl)-9- (morpholine-4-ylméthyl)-7-thioxo-2,3-dihydro-7H-[1,4]oxazino[2,3,4-ij]-quinoléine-6-carboxamide,
ddd) N- [ (4-chlorophényl)méthyl]-2,3,5,6-tétrahydro-9'-(4-morpholinylméthyl)-7'-thioxospiro[4H-pyranne-4,2'(3'H)-[7H]-pyrido[1,2,3-de][1,4]benzoxazine]-6'-carboxamide,
eee) 6-[[[(4-chlorophényl)méthyl]amino]carbonyl]-9'-(4-morpholinylméthyl)-7'-thioxospiro[pipéridine-4,2'(3'H)-[7H]pyrido[1,2,3-de][1,4]benzoxazine]-1-carboxylate de 1,1-diméthyléthyle,
fff) N-[(4-chlorophényl)méthyl]-9'-(4-morpholinylméthyl)-7'-thioxospiro[pipéridine-4,2'(3'*H*)-[7*H*]pyrido[1,2,3-*de*][1,4]-benzoxazine]-6'-carboxamide,
ggg) N-(4-chlorobenzyl)-2,2-bis[(hydroxyméthoxy)méthyl]-9-(morpholine-4-ylméthyl)-7-thioxo-2,3-dihydro-7H-[1,4]-oxazino[2,3,4-ij]quinoléine-6-carboxamide,
hhh) N-[(4-chlorophényl)méthyl]-2',3',5',6'-tétrahydro-9-(4-morpholinylméthyl)-7-thioxospiro[7H-pyrido [1,2,3-de] -1, 4-benzoxazine-2(3H),4'-[4H]thiopyranne]-6-carboxamide ou un de ses sels pharmaceutiquement acceptables.

**72.** Composé suivant la revendication 1, qui est le
a) N-(4-chlorobenzyl)-2-(hydroxyméthyl)-9-(morpholine-4-ylméthyl)-7-oxo-2,3-dihydro-7H-[1,4]oxazino[2,3,4-ij]-quinoléine-6-carboxamide,
b) N-(4-chlorobenzyl)-2-(*R* ou *S*)-(hydroxyméthyl)-9-(morpholine-4-ylméthyl)-7-oxo-2,3-dihydro-7H-[1,4]oxazino[2,3,4-ij]quinoléine-6-carboxamide,
c) N-(4-chlorobenzyl)-9-(morpholine-4-ylméthyl)-7-oxo-2-pyridine-3-yl-2,3-dihydro-7H-[1,4]oxazino[2,3,4-ij]-quinoléine-6-carboxamide,
d) N-(4-chlorobenzyl)-9-(morpholine-4-ylméthyl) -7-oxo-2-pyridine-4-yl-2,3-dihydro-7H-[1,4]oxazino[2,3,4-ij]-quinoléine-6-carboxamide,
e) N-(4-chlorobenzyl)-9-(morpholine-4-ylméthyl)-7-oxo-2-pyridine-2-yl-2,3-dihydro-7H-[1,4]oxazino[2,3,4-ij]-quinoléine-6-carboxamide,
f) N-(4-chlorobenzyl)-9-(morpholine-4-ylméthyl)-7-oxo-2-(*R* ou *S*)-pyridine-3-yl-2,3-dihydro-7H-[1,4]oxazino[2,3,4-ij]-quinoléine-6-carboxamide,
g) N-(4-chlorobenzyl)-2,9-bis(morpholine-4-ylméthyl)-7-oxo-2,3-dihydro-7H-[1,4]oxazino[2,3,4-ij]quinoléine-6-carboxamide,
h) 2-[(*tertio*butylsulfanyl)méthyl]-N-(4-chlorobenzyl)-9-(morpholine-4-ylméthyl)-7-oxo-2,3-dihydro-7H-[1,4]oxazino[2,3,4-ij]quinoléine-6-carboxamide,
i) N-(4-chlorobenzyl)-2-{[(2-hydroxyéthyl)sulfanyl]méthyl}-9-(morpholine-4-ylméthyl)-7-oxo-2,3-dihydro-7H-[1,4]-oxazino[2,3,4-ij]quinoléine-6-carboxamide,
j) N- (4-chlorobenzyl)-2-{[(1-méthyl-1H-imidazol-2-yl)sulfanyl]-méthyl}-9-(morpholine-4-ylméthyl)-7-oxo-2, 3-dihydro-7H-[1,4]oxazino[2,3,4-ij]quinoléine-6-carboxamide,
k) N-(4-chlorobenzyl)-9-(morpholine-4-ylméthyl)-7-oxo-2-{[(3-pyridinylméthyl)amino]méthyl}-2,3-dihydro-7H-[1,4]-oxazino[2,3,4-ij]quinoléine-6-carboxamide,
l) acétate de [6-{[(4-chlorobenzyl)amino]carbonyl}-9-(morpholine-4-ylméthyl)-7-oxo-2,3-dihydro-7H-[1,4]-oxazino[2,3,4-ij]quinoléine-2-yl]méthyle,
m) N-(4-chlorobenzyl)-9-(morpholine-4-ylméthyl)-7-oxo-2-(*R* ou *S*)-{[(3-pyridinylméthyl)amino]méthyl}-2,3-dihydro-7H-[1,4]-oxa z ino [2, 3, 4-ij]quinoléine-6-carboxamide,
n) N- (4-chlorobenzyl)-2-(3-hydroxyphényl)-9-(morpholine-4-ylméthyl)-7-oxo-2,3-dihydro-7H-[1,4]oxazino[2,3,4-ij]-quinoléine-6-carboxamide,
o) N-(4-chlorobenzyl)-9-(morpholine-4-ylméthyl)-7-oxo-2-(*R* ou *S*)-pyridine-2yl-2,3-dihydro-7H-[1,4]oxazino[2,3,4-ij]-quinoléine-6-carboxamide,
p) N-(4-chlorobenzyl)-2-[3-(hydroxyméthyl)phényl]-9-(morpholine-4-ylméthyl)-7-oxo-2,3-dihydro-7H-[1,4]-oxazino [2,3,4-ij]quinoléine-6-carboxamide,
q) N- (4-chlorobenzyl)-2-[2-(hydroxyméthyl)phényl]-9-(morpholine-4-ylméthyl)-7-oxo-2,3-dihydro-7H-[1,4]-oxazino[2,3,4-ij]quinoléine-6-carboxamide,
r) N-(4-chlorobenzyl)-2-(1-méthyl-1H-imidazol-2-yl)-9-(morpholine-4-ylméthyl)-7-oxo-2,3-dihydro-7H-[1,4]-oxazino[2,3,4-ij]quinoléine-6-carboxamide,
s) N-(4-chlorobenzyl)-2-(2-furyl)-9-(morpholine-4-ylméthyl)-7-oxo-2,3-dihydro-7H-[1,4]oxazino[2,3,4-ij]quinoléine-6 - carboxamide,
t) N-(4-chlorobenzyl)-2-(3-cyanophényl)-9-(morpholine-4-ylméthyl)-7-oxo-2,3-dihydro-7H-[1,4]oxazino[2,3,4-ij]quinoléine-6-carboxamide,
u) N-(4-chlorobenzyl)-2-(3-furyl)-9-(morpholine-4-ylméthyl)-7-oxo-2,3-dihydro-7H-[1,4]oxazino[2,3,4-ij]quinoléine-6-carboxamide,
v) N-(4-chlorobenzyl)-9-(morpholine-4-ylméthyl)-7-oxo-2-thiène-2-yl-2, 3-dihydro-7H-[1,4]oxazino[2,3,4-ij]-quinoléine-6-carboxamide,
w) N-(4-chlorobenzyl)-2-(3,5-difluorophényl)-9-(morpholine-4-ylméthyl) -7-oxo-2,3-dihydro-7H-[1,4]oxazino[2,3,4-ij]quinoléine-6-carboxamide,
x) 2- (1,3-benzodioxol-5-yl)-N- (4-chlorobenzyl) -9-(morpholine-4-ylméthyl)-7-oxo-2,3-dihydro-7H-[1,4]-oxazino [2,3,4-ij]quinoléine-6-carboxamide,
y ) N-(4-chlorobenzyl)-2-(2,3-dihydro-1,4-benzodioxine-6-yl)-9-(morpholine-4-ylméthyl)-7-oxo-2,3-dihydro-7H-[1,4]-oxazino [2,3,4-ij]quinoléine-6-carboxamide,
z) 2-(1,3-benzodioxol-4-yl)-N-(4-chlorobenzyl)-9-(morpholine-4-ylméthyl)-7-oxo-2,3-dihydro-7H-[1,4]-oxazino [2,3,4-ij]quinoléine-6-carboxamide,
aa) 2-[3,5-bis(méthoxyméthoxy)phényl]-N-(4-chlorobenzyl)-9-(morpholine-4-ylméthyl)-7-oxo-2,3-dihydro-7H-[1,4]-oxazino[2,3,4-ij]quinoléine-6-carboxamide,
bb) N-(4-chlorobenzyl)-9-(morpholine-4-ylméthyl)-7-oxo-2-thiène-3-yl-2, 3-dihydro-7H-[1,4]oxazino[2,3,4-ij]-quinoléine-6-carboxamide,
cc) N-(4-chlorobenzyl)-2,2-bis [(méthoxyméthoxy)méthyl]-9- (morpholine-4-ylméthyl)-7-oxo-2, 3-dihydro-7H-[1,4]-oxazino [2,3,4-ij]quinoléine-6-carboxamide,
dd) N-[(4-chlorophényl)méthyl]-9'-(4-morpholinylméthyl)-4,7'-dioxospiro[cyclohexane-1,2'(3'*H*)-[7*H*]pyrido[1,2,3-*de*]-[1,4]benzoxazine]-6'-carboxamide,
ee) N-[(4-chlorophényl)méthyl]-4-hydroxy-9'-(4-morpholinylméthyl)-7'-oxospiro[cyclohexane-1,2'(3'*H*)-[7*H*]pyrido[1,2,3-*de*]-[1,4]benzoxazine]-6'-carboxamide ou un de ses sels pharmaceutiquement acceptables.

**73.** Composé suivant la revendication 1, qui est le
a) N-(4-chlorobenzyl)-2-(hydroxyméthyl)-9-(morpholine-4-ylméthyl)-7-thioxo-2,3-dihydro-7H-[1,4]oxazino[2,3,4-ij]-quinoléine-6-carboxamide,
b) N- (4-chlorobenzyl) -2- (*R* ou *S*) - (hydroxyméthyl) -9- (morpholine-4-ylméthyl)-7-thioxo-2,3-dihydro-7H-[1,4]oxazino[2,3,4-ij]quinoléine-6-carboxamide,
c) N-(4-chlorobenzyl)-9-(morpholine-4-ylméthyl)-7-thioxo-2-pyridine-3-yl-2,3-dihydro-7H-[1,4]oxazino[2,3,4-ij]quinoléine-6-carboxamide,
d) N-(4-chlorobenzyl)-9- (morpholine-4-ylméthyl)-7-thioxo-2-pyridine-4-yl-2,3-dihydro-7H-[1,4]oxazino[2,3,4-ij]quinoléine-6-carboxamide,
e) N-(4-chlorobenzyl)-9-(morpholine-4-ylméthyl)-7-thioxo-2-pyridine-2-yl-2,3-dihydro-7H-[1,4]oxazino[2,3,4-ij] quinoléine-6-carboxamide,
f) N-(4-chlorobenzyl)-9-(morpholine-4-ylméthyl)-7-thioxo-2-(*R* ou *S*)-pyridine-3-yl-2,3-dihydro-7H-[1,4]oxazino[2,3,4-ij]quinoléine-6-carboxamide,
g) N-(4-chlorobenzyl)-2,9-bis(morpholine-4-ylméthyl)-7-thioxo-2,3-dihydro-7H-[1,4]oxazino[2,3,4-ij]-quinoléine-6-carboxamide,
h) 2-[(*tertio*butylsulfanyl)méthyl]-N-(4-chlorobenzyl)-9-(morpholine-4-ylméthyl)-7-thioxo-2,3-dihydro-7H-[1,4]oxazino[2,3,4-ij]quinoléine-6-carboxamide,
i) N-(4-chlorobenzyl)-2-{[(2-hydroxyéthyl)sulfanyl]méthyl}-9-(morpholine-4-ylméthyl)-7-thioxo-2,3-dihydro-7H-[1,4]oxazino[2,3,4-ij]quinoléine-6-carboxamide,
j ) N-(4-chlorobenzyl)-2-{[(1-méthyl-1H-imidazol-2-yl)sulfanyl]-méthyl }-9- (morpholine-4-ylméthyl)-7-thioxo-2,3-dihydro-7H-[1,4]oxazino[2,3,4-ij]quinoléine-6-carboxamide,
k) N-(4-chlorobenzyl)-9-(morpholine-4-ylméthyl)-7-thioxo-2-{[(3-pyridinylméthyl)amino]méthyl}-2,3-dihydro-7H-[1,4]oxazino[2,3,4-ij]quinoléine-6-carboxamide,
l) acétate de [6-{[(4-chlorobenzyl)amino]carbonyl}-9-(morpholine-4-ylméthyl)-7-thioxo-2,3-dihydro-7H-[1,4]oxazino[2,3,4-ij]quinoléine-2-yl]méthyle,
m) N-(4-chlorobenzyl)-9-(morpholine-4-ylméthyl)-7-thioxo-2-(*R* ou *S*)-{[(3-pyridinylméthyl)amino]méthyl}-2,3-dihydro-7H-[1 , 4]oxazino[2, 3, 4-ij]quinoléine-6-carboxamide,
n) N-(4-chlorobenzyl)-2-(3-hydroxyphényl)-9-(morpholine-4-ylméthyl)-7-thioxo-2,3-dihydro-7H-[1,4]oxazino[2,3,4-ij]quinoléine-6-carboxamide,
o) N-(4-chlorobenzyl)-9-(morpholine-4-ylméthyl)-7-thioxo-2-(*R* ou *S*)-pyridine-2yl-2,3-dihydro-7H-[1,4]oxazino[2,3,4-ij]-quinoléine-6-carboxamide,
p) N-(4-chlorobenzyl)-2-[3-(hydroxyméthyl)phényl]-9- (morpholine-4-ylméthyl) -7-thioxo-2,3-dihydro-7H-[1,4]-oxazino [2,3,4-ij ]quinoléine-6-carboxamide,
q) N-(4-chlorobenzyl)-2-[2-(hydroxyméthyl)phényl]-9-(morpholine-4-ylméthyl)-7-thioxo-2,3-dihydro-7H-[1,4]oxazino[2,3, 4-ij]quinoléine-6-carboxamide,
r) N- (4-chlorobenzyl)-2-(1-méthyl-1H-imidazol-2-yl)-9-(morpholine-4-ylméthyl)-7-thioxo-2,3-dihydro-7H-[1,4]-oxazino[2,3,4-ij]quinoléine-6-carboxamide,
s) N-(4-chlorobenzyl)-2-(2-furyl)-9-(morpholine-4-ylméthyl)-7-thioxo-2,3-dihydro-7H-[1,4]oxazino[2,3,4-ij]-quinoléine-6-carboxamide,
t) N- (4-chlorobenzyl)-2- (3-cyanophényl)-9-(morpholine-4-ylméthyl)-7-thioxo-2,3-dihydro-7H-[1,4]oxazino[2,3,4-ij]quinoléine-6-carboxamide,
u) N- (4-chlorobenzyl) -2- (3-furyl)-9-(morpholine-4-ylméthyl)-7-thioxo-2,3-dihydro-7H-[1,4]oxazino[2,3,4-ij]-quinoléine-6-carboxamide,
v) N-(4-chlorobenzyl)-9-(morpholine-4-ylméthyl)-7-thioxo-2-thiène-2-yl-2, 3-dihydro-7H-[1,4]oxazino [2,3,4-ij]quinoléine-6-carboxamide,
w) N- (4-chlorobenzyl) -2- (3,5-difluorophényl)-9-(morpholine-4-ylméthyl)-7-thioxo-2,3-dihydro-7H-[1,4]oxazino[2,3,4-ij]quinoléine-6-carboxamide,
x) 2-(1,3-benzodioxol-5-yl)-N-(4-chlorobenzyl)-9-(morpholine-4-ylméthyl)-7-thioxo-2,3-dihydro-7H-[1,4]oxazino[2,3,4-ij]quinoléine-6-carboxamide,
y) N-(4-chlorobenzyl)-2-(2,3-dihydro-1,9-benzodioxine-6-yl)-9-(morpholine-4-ylméthyl)-7-thioxo-2,3-dihydro-7H-[1,4]oxazino[2,3,4-ij]quinoléine-6-carboxamide,
z) 2-(1,3-benzodioxol-4-yl)-N-(4-chlorobenzyl)-9-(morpholine-4-ylméthyl)-7-thioxo-2,3-dihydro-7H-[1,4]oxazino[2,3,4-ij]quinoléine-6-carboxamide,
aa) 2-[3,5-bis(méthoxyméthoxy)phényl]-N-(4-chlorobenzyl)-9-(morpholine-4-ylméthyl)-7-thioxo-2,3-dihydro-7H-[1,4]oxazino[2,3,4-ij]quinoléine-6-carboxamide,
bb) N-(4-chlorobenzyl)-9-(morpholine-4-ylméthyl)-7-thioxo-2-thiène-3-yl-2,3-dihydro-7H-[1,4]oxazino[2,3,4-ij]quinoléine-6-carboxamide,
cc) N-(4-chlorobenzyl)-2,2-bis[(méthoxyméthoxy)méthyl]-9-(morpholine-4-ylméthyl)-7-thioxo-2,3-dihydro-7H-[1,4]oxazino[2,3,4-ij]quinoléine-6-carboxamide,
dd) N-[(4-chlorophényl)méthyl]-9'-(4-morpholinylméthyl)-4-oxo-7'-thioxospiro[cyclohexane-1,2'(3'*H*)-[7*H*]pyrido[1,2,3-*de*][1,4]benzoxazine]-6'-carboxamide,
ee) N-[(4-chlorophényl)méthyl]-4-hydroxy-9'-(4-morpholinyiméthyl)-7'-thioxospiro[cyclohexane-1,2'(3'*H*)-[7*H*]pyrido[1,2,3-*de*][1,4]benzoxazine]-6'-carboxamide ou un de ses sels pharmaceutiquement acceptables.

**74.** Composé suivant la revendication 1, qui est le
a) N-(4-chlorobenzyl)-2-(hydroxyméthyl)-9-(morpholine-4-ylméthyl)-7-oxo-2,3-dihydro-7H-[1,4]oxazino[2,3,4-ij]-quinoléine-6-carboxamide,
b) N- (4-chlorobenzyl)-2-(*R* ou *S*)-(hydroxyméthyl)-9-(morpholine-4-ylméthyl)-7-oxo-2,3-dihydro-7H-[1,4]oxazino[2,3,4-ij]quinoléine-6-carboxamide,
c) N- (4-chlorobenzyl) -9- (morpholine-4-ylméthyl)-7-oxo-2-pyridine-3-yl-2, 3-dihydro-7H-[1,4]oxazino[2,3,4-ij]quinoléine-6-carboxamide,
d) N-(4-chlorobenzyl)-9- (morpholine-4-ylméthyl)-7-oxo-2-pyridine-4-yl-2,3-dihydro-7H-[1,4 ]oxazino[2,3,4-ij]quinoléine-6-carboxamide,
e) N-(4-chlorobenzyl)-9-(morpholine-4-ylméthyl)-7-oxo-2-pyridine-2-yl-2,3-dihydro-7H-[2,4]oxazino[2,3,4-ij]quinoléine-6-carboxamide,
f ) N-(4-chlorobenzyl)-9-(morpholine-4-ylméthyl)-7-oxo-2-(*R* ou *S*)-pyridine-3-yl-2,3-dihydro-7H-[1,4]oxazino[2,3,4-ij]quinoléine-6-carboxamide,
g) N-(4-chlorobenzyl)-9-(morpholine-4-ylméthyl)-7-oxo-2-(*R* ou *S*)-pyridine-2-yl-2,3-dihydro-7H-[1,4]oxazino[2,3,4-ij]quinoléine-6-carboxamide,
h) N-(4-chlorobenzyl)-2-(1-méthyl-1H-imidazol-2-yl)-9-(morpholine-4-ylméthyl)-7-oxo-2,3-dihydro-7H-[1,4]-oxazino[2,3,4-ij]quinoléine-6-carboxamide ou un de ses sels pharmaceutiquement acceptables.

**75.** Composé suivant la revendication 1, qui est le
a) N-(4-chlorobenzyl)-2-(hydroxyméthyl)-9-(morpholine-4-ylméthyl)-7-thioxo-2,3-dihydro-7H-[1,4]oxazino[2,3,4-ij]quinoléine-6-carboxamide,
b ) N- (4-chlorobenzyl) -2- (R ou S)-(hydroxyméthyl)-9-(morpholine-4-ylméthyl)-7-thioxo-2,3-dihydro-7H-[1,4]oxazino[2,3,4-ij]quinoléine-6-carboxamide,
c) N-(4-chlorobenzyl)-9-(morpholine-4-ylméthyl)-7-thioxo-2-pyridine-3-yl-2,3-dihydro-7B-[1,4]oxazino[2,3, 4 -ij] quinoléine-6-carboxamide,
d) N-(4-chlorobenzyl)-9-(morpholine-4-ylméthyl)-7-thioxo-2-pyridine-4-yl-2,3-dihydro-7H-[1,4]oxazino[2,3,4-ij]quinoléine-6-carboxamide,
e) N-(4-chlorobenzyl)-9- (morpholine-4-ylméthyl)-7-thioxo-2-pyridine-2-yl-2,3-dihydro-7H-[1,4]oxazino[2,3,4-ij]quinoléine-6-carboxamide,
f) N-(4-chlorobenzyl)-9-(morpholine-4-ylméthyl)-7-thioxo-2-(*R* ou *S*)-pyridine-3-yl-2,3-dihydro-7H-[1,4]oxazino[2,3,4-ij]quinoléine-6-carboxamide,
g) N-(4-chlorobenzyl)-9-(morpholine-4-ylméthyl)-7-thioxo-2-(R ou *S*)-pyridine-2-yl-2,3-dihydro-7H-[1,4]oxazino[2,3,4-ij]quinoléine-6-carboxamide,
h) N-(4-chlorobenzyl)-2-(1-méthyl-1H-imidazol-2-yl)-9-(morpholine-4-ylméthyl)-7-thioxo-2,3-dihydro-7H-[1,4]-oxazino[2,3,4-ij]quinoléine-6-carboxamide ou un de ses sels pharmaceutiquement acceptables.

**76.** Composé suivant la revendication 1, qui est le
a) N-(4-chlorobenzyl)-2- (hydroxyméthyl) -9-(morpholine-4-ylméthyl)-7-thioxo-2,3-dihydro-7H-[1,9]oxazino[2,3,4-ij]quinoléine-6-carboxthioamide,
b) N-(4-chlorobenzyl)-2-(*R* ou *S*)-(hydroxyméthyl)-9-(morpholine-4-ylméthyl)-7-thioxo-2,3-dihydro-7H-[1,4]oxazino[2,3,4-ij]quinoléine-6-carboxthioamide,
c) N-(4-chlorobenzyl)-9-(morpholine-4-ylméthyl)-7-thioxo-2-pyridine-3-yl-2,3-dihydro-7H-[1,4]oxazino[2,3,4-ij]quinoléine-6-carboxthioamide,
d) N-(4-chlorobenzyl)-9-(morpholine-4-ylméthyl)-7-thioxo-2-pyridine-4-yl-2,3-dihydro-7H-[1,4]oxazino[2,3,4-ij]quinoléine-6-carboxthioamide,
e) N-(4-chlorobenzyl)-9- (morpholine-4-ylméthyl)-7-thioxo-2-pyridine-2-yl-2,3-dihydro-7H-[1,4]oxazino[2,3,4-ij]quinoléine-6-carboxthioamide,
f) N-(4-chlorobenzyl)-9-(morpholine-4-ylméthyl)-7-thioxo-2-(*R* ou *S*)-pyridine-3-yl-2,3-dihydro-7H-[1,4]oxazino[2,3,4-ij]quinoléine-6-carboxthioamide,
e) N-(4-chlorobenzyl)-9-(morpholine-4-ylméthyl)-7-thioxo-2-(*R* ou *S*)-pyridine-2-yl-2,3-dihydro-7H-[1,4]oxazino[2, 3, 4-ij]quinoléine-6-carboxthioamide,
f) N-(4-chlorobenzyl)-2-(1-méthyl-1H-imidazol-2-yl)-9-(morpholine-4-ylméthyl)-7-thioxo-2,3-dihydro-7H-[1,4]-oxazino[2,3,4-ij]quinoléine-6-carboxthioamide ou un de ses sels pharmaceutiquement acceptables.

**77.** Composé suivant la revendication 1, qui est le
a) N-(4-chlorobenzyl)-2-(hydroxyméthyl)-9-(morpholine-4-ylméthyl)-7-oxo-2,3-dihydro-7H-[1,4]oxazino[2,3,4-ij]-quinoléine-6-carboxthioamide,
b) N- (4-chlorobenzyl) -2- (*R* ou *S*)-(hydroxyméthyl)-9-(morpholine-4-ylméthyl)-7-oxo-2,3-dihydro-7H-[1,4]oxazino[2,3,4-ij]quinoléine-6-carboxthioamide,
c) N-(4-chlorobenzyl)-9-(morpholine-4-ylméthyl ) -7-oxo-2-pyridine-3-yl-2, 3-dihydro-7H-[1,4]oxazino[2,3,4-ij]quinoléine-6-carboxthioamide,
d) N-(4-chlorobenzyl)-9- (morpholine-4-ylméthyl)-7-oxo-2-pyridine-4-yl-2,3-dihydro-7H-[1,4]oxazino[2,3,4-ij]quinoléine-6-carboxthioamide,
e) N-(4-chlorobenzyl)-9- (morpholine-4-ylméthyl) -7-oxo-2-pyridine-2-yl-2,3-dihydro-7H-[1,4]oxazino[2,3,4-ij]quinoléine-6-carboxthioamide,
f) N-(4-chlorobenzyl)-9-(morpholine-4-ylméthyl)-7-oxo-2-(*R* ou *S*)-pyridine-3-yl-2, 3-dihydro-7H-[1,4]oxazino[2,3,4-ij]quinoléine-6-carboxthioamide,
g) N-(4-chlorobenzyl)-9-(morpholine-4-ylméthyl)-7-oxo-2-(*R* ou *S*)-pyridine-2-yl-2,3-dihydro-7H-[1,4]oxazino[2,3,4-ij]quinoléine-6-carboxthioamide,
h) N-(4-chlorobenzyl)-2-(1-méthyl-1H-imidazol-2-yl)-9-(morpholine-4-ylméthyl)-7-oxo-2,3-dihydro-7H-[1,4]-oxazino[2,3,4-ij]quinoléine-6-carboxthioamide ou un de ses sels pharmaceutiquement acceptables.

**78.** Composé suivant la revendication 1, qui est le N-(4-chlorobenzyl) -9- (morpholine-4-ylméthyl)-7-oxo-2-(*S*) -pyridine-2-yl-2,3-dihydro-7H-[1,4]oxazino[2,3,4-ij]quinoléine-6-carboxamide ou un de ses sels pharmaceutiquement acceptables.

**79.** Composé suivant la revendication 1, qui est le N-(4-chlorobenzyl)-9-(morpholine-4-ylméthyl)-7-oxo-2-(*R*)-pyridine-2-yl-2,3-dihydro-7H-[1,4]oxazino[2,3,4-ij]quinoléine-6-carboxamide ou un de ses sels pharmaceutiquement acceptables.

**80.** Composé suivant la revendication 1, qui est le N-(4-chlorobenzyl)-9-(morpholine-4-ylméthyl)-7-oxo-2-(*R*)-pyridine-3-yl-2,3-dihydro-7H-[1,4]oxazino[2,3,4-ij]quinoléine-6-carboxamide ou un de ses sels pharmaceutiquement acceptables.

**81.** Composé suivant la revendication 1, qui est le N-(4-chlorobenzyl)-9-(morpholine-4-ylméthyl)-7-oxo-2-(*S*)-pyridine-3-yl-2,3-dihydro-7H-[1,4]oxazino[2,3,4-ij]quinoléine-6-carboxamide ou un de ses sels pharmaceutiquement acceptables.

**82.** Composé suivant la revendication 1, qui est le N-(4-chlorobenzyl)-2-(1-méthyl-1H-imidazol-2-yl)-9-(morpholine-4-ylméthyl)-7-oxo-2,3-dihydro-7H-[1,4]oxazino[2,3,4-ij]-quinoléine-6-carboxamide ou un de ses sels pharmaceutiquement acceptables.

**83.** Composition pharmaceutique comprenant un composé suivant l'une quelconque des revendications 1 à 82 et un support pharmaceutiquement acceptable.

**84.** Utilisation d'un composé suivant l'une quelconque des revendications 1 à 82 pour la préparation d'un médicament destiné au traitement ou à l'inhibition d'une ADN-polymérase virale.

**85.** Utilisation d'une quantité efficace d'un composé suivant l'une quelconque des revendications 1 à 82 pour la préparation d'un médicament destiné au traitement d'infections par des virus de l'herpès chez un mammifère.

**86.** Utilisation suivant la revendication 85, dans laquelle ledit virus de l'herpès est le virus d'Herpes simplex de type 1, le virus d'Herpes simplex de type 2, le virus Varicella zoster, le cytomégalovirus, le virus d'Epstein-Barr, le virus herpétique humain 6, le virus herpétique humain 7 ou le virus herpétique humain 8.

**87.** Utilisation suivant la revendication 85, dans laquelle ledit virus de l'herpès est le virus d'Herpes simplex de type 1, le virus d'Herpes simplex de type 2, le virus Varicella zoster, le cytomégalovirus, le virus d'Epstein-Barr, le virus herpétique humain 7 ou le virus herpétique humain 8.

**88.** Utilisation suivant la revendication 85, dans laquelle ledit virus de l'herpès est le cytomégalovirus humain.

**89.** Utilisation suivant la revendication 85, dans laquelle la quantité efficace d'un composé suivant la revendication 1 est administrée par voie orale, parentérale, topique, rectale, nasale, sublinguale ou transdermique.

**90.** Utilisation suivant la revendication 85, dans laquelle la quantité efficace d'un composé suivant la revendication 1 est une quantité comprise dans l'intervalle d'environ 0,1 à environ 300 mg/kg de poids corporel.

**91.** Utilisation suivant la revendication 85, dans laquelle la quantité efficace d'un composé suivant la revendication 1 est une quantité comprise dans l'intervalle d'environ 1 à environ 30 mg/kg de poids corporel.

**92.** Utilisation suivant la revendication 85, dans laquelle le mammifère est l'homme.

**93.** Utilisation suivant la revendication 85, dans laquelle le mammifère est un animal destiné à la consommation ou un animal de compagnie.
